(19) Europäisches Patentamt
European Patent Office
Office européen des brevets

(11) **EP 3 877 054 B1**

(12) **EUROPEAN PATENT SPECIFICATION**

(45) Date of publication and mention
of the grant of the patent:
**01.11.2023   Bulletin 2023/44**

(21) Application number: **19835949.9**

(22) Date of filing: **05.11.2019**

(51) International Patent Classification (IPC):
**A61K 35/17** (2015.01)      **A61P 35/00** (2006.01)
**C07K 14/36** (2006.01)     **C07K 14/725** (2006.01)
**C07K 16/28** (2006.01)     **C12N 5/00** (2006.01)
**C12N 5/0783** (2010.01)

(52) Cooperative Patent Classification (CPC):
**C07K 14/36; A61K 35/17; A61P 35/00;
C07K 14/7051; C07K 16/2803; C07K 16/2896;
C12N 5/0087; C12N 5/0636;** C07K 2319/00;
C07K 2319/03; C07K 2319/33; C12N 2501/50;
C12N 2501/505; C12N 2501/515; C12N 2501/599;

(Cont.)

(86) International application number:
**PCT/US2019/059946**

(87) International publication number:
**WO 2020/097132 (14.05.2020 Gazette 2020/20)**

(54) **PROCESS FOR PRODUCING GENETICALLY ENGINEERED T CELLS**

VERFAHREN ZUR HERSTELLUNG VON GENETISCH VERÄNDERTEN T-ZELLEN

PROCÉDÉ DE PRODUCTION DE CELLULES T GÉNÉTIQUEMENT MODIFIÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **06.11.2018   US 201862756571 P
29.01.2019   US 201962798457 P**

(43) Date of publication of application:
**15.09.2021   Bulletin 2021/37**

(73) Proprietor: **Juno Therapeutics, Inc.
Seattle, WA 98109 (US)**

(72) Inventors:
• **COOPER, Sara**
  **Seattle, Washington 98109 (US)**
• **COSSETTE, Daniel**
  **Seattle, Washington 98109 (US)**
• **LARSON, Ryan**
  **Seattle, Washington 98109 (US)**
• **TEOH, Jeffrey**
  **Seattle, Washington 98109 (US)**

(74) Representative: **Carpmaels & Ransford LLP
One Southampton Row
London WC1B 5HA (GB)**

(56) References cited:
**WO-A1-2016/187216**

• **MATTHIAS W?LFL ET AL: "Antigen-specific
activation and cytokine-facilitated expansion of
naive, human CD8+ T cells", NATURE
PROTOCOLS, vol. 9, no. 4, 27 March 2014
(2014-03-27), pages 950-966, XP055306243, GB
ISSN: 1754-2189, DOI: 10.1038/nprot.2014.064**

- **ANNA CASATI ET AL: "Clinical-scale selection and viral transduction of human naïve and central memory CD8+ T cells for adoptive cell therapy of cancer patients", CANCER IMMUNOLOGY, IMMUNOTHERAPY, NIH AUTHOR MANUSCRIPT, vol. 62, no. 10, 1 August 2013 (2013-08-01), pages 1563-1573, XP055547685, Berlin/Heidelberg ISSN: 0340-7004, DOI: 10.1007/s00262-013-1459-x**
- **C. S. HINRICHS ET AL: "Human effector CD8+ T cells derived from naive rather than memory subsets possess superior traits for adoptive immunotherapy", BLOOD : JOURNAL OF THE AMERICAN SOCIETY OF HEMATOLOGY, vol. 117, no. 3, 20 January 2011 (2011-01-20), pages 808-814, XP055235116, US ISSN: 0006-4971, DOI: 10.1182/blood-2010-05-286286**
- **Takashi Ohkawa ET AL: "Systematic characterization of human CD8+ T cells with natural killer cell markers in comparison with natural killer cells and normal CD8+ T cells", Immunology, 1 July 2001 (2001-07-01), pages 281-290, XP055669646, Oxford, UK DOI: 10.1046/j.1365-2567.2001.01248.x Retrieved from the Internet: URL:https://www.ncbi.nlm.nih.gov/pmc/artic les/PMC1783250/pdf/imm0103-0281.pdf**

Remarks:

The complete document including Reference Table(s) and the Sequence Listing(s) can be downloaded from the EPO website

(52) Cooperative Patent Classification (CPC): (Cont.)
C12N 2510/00; C12N 2740/16043

**Description**

Field

The present disclosure relates in some aspects to cell populations enriched for CD57 negative T cells, or depleted for CD57 positive cells, and methods for stimulating, cultivating, expanding, and/or genetically engineering cell populations enriched for CD57 negative T cells or depleted for CD57 positive T cells. Also included are methods for generating, isolating, enriching, or selecting CD57 negative T cells, or depleting CD57 positive cells, such as by negative selection.

Background

Various cell therapy methods are available for treating diseases and conditions. Among cell therapy methods are methods involving immune cells, such as T cells, genetically engineered with a recombinant receptor, such as a chimeric antigen receptors. However, in some cases, some of the existing processes may result in a population with low consistency, potency or persistence *in vivo.* Improved methods for manufacturing and/or engineering such cell therapies are needed, including to provide for an improved cell therapy product with high consistency, potency and persistence *in vivo*.

WO2016/187216 describes compositions for adoptive cell therapies for targeting cancers that express receptor tyrosine kinase-like orphan receptor 1 (ROR1). Wölf et al, Nature Protocols, 2014, vol. 9, no. 4, pages 950-966 describes antigen-specific activation of cytokine-facilitated expansion of naive, human CD8+ T cells. Casati et al, Cancer Immunology, 2016, vol. 62, no. 10, pages 1563-1573 describes clinical-scale selection and viral transcution of human naive and central memory CD8+ T-cells for adoptice cell therapy of cancer patients. Hinrichs et al, Journal of the American Society of Hematology, 2011, vol. 117, no. 3, pages 808-814 describes that human effector CD8+ T cells derived from naïve rather than memory subsets possess superior traits for adoptive immunotherapy. Ohkawa et al, Immunology Oxford, 2001, pages 281-290 describes systematic characterization of human CD8+ T cells with natural killer cell markers in comparison with natural killer cells and normal CD8+ T cells.

Summary

The invention is defined in the appended claims.

The invention provides a method for enriching T cells and generating an engineered population of T cells, the method comprising:

(a) performing a first selection, the first selection comprising enriching for either of CD57- or CD3+ T cells from a biological sample comprising primary human T cells, thereby generating an enriched T cell population; and
(b) performing a second selection on the cells from the enriched T cell population, wherein:

the first selection comprises enriching for CD57- T cells and the second selection comprises enriching for CD3+ T cells from the enriched population; or
the first selection comprises enriching for CD3+ T cells and the second selection comprises removing CD57+ T cells from the enriched T cell population,
wherein the method generates a depleted population that comprises fewer CD57+ T cells than the biological sample and is enriched for CD3+ T cells, and

(c) introducing a heterologous polynucleotide encoding a recombinant receptor into a population of T cells obtained from the depleted population, thereby generating an engineered population of T cells.

In some of any such embodiments, the depleted population contains at least one of the following: (i) less than at or about 5% CD57+ T cells; (ii) a frequency of CD57+ T cells that is less than at or about 35% of the frequency of CD57+ T cells present the biological sample; (iii) CD4+ T cells, wherein at least at or about 95% of the CD4+ T cells are CD57-; and (iv) CD8+ T cells, wherein at least at or about 95% of the CD8+ T cells are CD57-. In some of any such embodiments, at least at or about 95% of the CD3+ T cells of the depleted population contains CD57-CD3+ T cells. T

In some of any such embodiments, the biological sample comprises an apheresis product or a leukapheresis product.

In some of any such embodiments, the depleted population contains CD57-CD4+ T cells. In some of any such embodiments, the depleted population contains CD57- CD8+ T cells. In some of any such embodiments, the depleted population contains CD57-CD4+ T cells and CD57-CD8+ T cells.

In some of any such embodiments, the frequency of the CD57+ cells in the depleted population is less than at or about 35%, 30%, 20%, 10%, 5%, 1%, or 0.1% of the frequency of CD57+ T cells in the biological sample. In some of any such embodiments, the frequency of the CD57+ T cells in the depleted population is less than at or about 35%, 30%, 20%, 10%, 5%, 1%, or 0.1% of the frequency of CD57+ T cells in the biological sample. In some of any such embodiments, the depleted population comprises less than at or about 3%, less than at or about 2%, less than at or about 1%, less than at or about 0.1%, or less than at or about 0.01% CD57+ T cells. In some of any such embodiments, the depleted population is free or is essentially free of CD57+ T cells. In some embodiments, a depleted population that is essentially free of CD57+ T cells comprises less than about or about 3%, less than about or about 2%, less than about or about 1%, less than about or about 0.1% or less than about or about 0.01% CD57+ T cells.

In some of any of such embodiments, at least at or about 95% of the CD4+ T cells of the depleted population comprises CD57-CD4+ T cells. In some of any of such embodiments, at least at or about 95% of the CD8+ T cells of the depleted population comprises CD57-CD8+ T cells. In some of any of such embodiments, at least at or about 95% of the CD4+ T cells and 95% of the CD8+ T cells of the depleted population comprises CD57-CD4+ T cells and CD57-CD8+ T cells, respectively.

In some of any such embodiments, the frequency of the naïve-like cells in the depleted population is at least at or about 10%, 20%, 30%, 40%, or 50% greater than at or about the frequency of naïve-like cells in the biological sample. In some of any such embodiments, the frequency of one or more of CD25+ T cells, CD27+ T cells, CD28+ T cells, CCR7+ T cells, or CD45RA+ T cells in the depleted population is at least at or about 10%, 20%, 30%, 40%, or 50% greater than at or about the frequency of the respective cells in the biological sample.

In some of any such embodiments, the depleted population comprises at least at or about 15%, 20%, 25%, 30%, 35%, or 40% CD27+ T cells. In some of any such embodiments, the depleted population comprises at least at or about 10%, 15%, 20%, 25%, 25%, 30%, 35%, or 40% CD28+ T cells. In some of any such embodiments, the depleted population comprises at least at or about 10%, 15%, 20%, 25%, 25%, 30%, 35%, or 40% CD27+CD28+ T cells. In some of any such embodiments, the depleted population comprises at least at or about 10%, 15%, 20%, 25%, 30%, 35%, 40%, 50%, 60%, 70% or 80% CD27+CD28+ T cells. In some of any such embodiments, the depleted population comprises at least at or about 70% or 80% CD27+CD28+ T cells. In some of any such embodiments, the depleted population comprises at least at or about 10%, 15%, 20%, or 25% CCR7+ T cells.

In some of any of the provided embodiments, the depleted population is used as an input composition for subsequent steps in a process for genetically engineering T cells. In some embodiments, the input composition includes CD57 depleted populations enriched for CD4+ cells (CD57-CD4+) and enriched for CD8+ cells (CD57-CD8+). In some embodiments, the input composition includes CD57 depleted populations enriched for CD4+ T cells (CD57-CD4+) and enriched for CD8+ T cells (CD57-CD8+).

In some of any of the provided methods, the method produces a T cell composition containing at least at or about 90%, at least at or about 95%, at least at or about 97%, at least at or about 99% or at least at or about 99.9% CD57-CD4+ T cells. In some of any of the provided methods, the method produces a T cell composition containing at least at or about 90%, at least at or about 95%, at least at or about 97%, at least at or about 99% or at least at or about 99.9% CD57-CD8+ T cells. In some of any of the provided methods, the method produces a T cell composition containing at least at or about 90%, at least at or about 95%, at least at or about 97%, at least at or about 99% or at least at or about 99.9% CD57-CD3+ T cells.

In some of any of the provided embodiments, the ratio of CD4+ to CD8+ T cells in the composition is between 3:1 and 1:3, each inclusive. In some of any of the provided embodiments, the ratio of CD4+ to CD8+ T cells in the composition is between 2:1 and 1:2, each inclusive. In some of any of the provided embodiments, the ratio of CD4+ to CD8+ T cells in the composition is between 1.5:1 and 1:1.5, each inclusive. In some of any of the provided embodiments, the ratio of CD4+ to CD8+ T cells in the composition is between 1.2:1 and 1:1.2, each inclusive.. In some of any of the provided embodiments, the ratio of CD4+ and CD8+ T cells in the composition is at or about 1:1 CD4+. In some of any of the provided embodiments, the primary T cells are from a subject with a disease or condition. In some of any of the provided embodiments, the disease or condition is a cancer. In some of any of the provided embodiments, the primary T cells are from a healthy subject.

In some of any of the provided embodiments, the removing of the CD57+ T cells includes immunoaffinity-based selection. In some of any of the provided embodiments, the immununoaffinity-based selection includes contacting cells with an antibody capable of specifically binding to CD57 and recovering cells not bound to the antibody, thereby effecting negative selection, wherein the recovered cells are depleted for the CD57+ cells. In some of any of the provided embodiments, the enriching cells comprises immunoaffinity-based selection. In some of any of the provided embodiments, the selection enriches for CD3 T cells and the immunoaffinity-based selection is effected by contacting cells with an antibody capable of specifically binding to CD3, and recovering cells bound to the antibody, thereby effecting positive selection, wherein the recovered cells are enriched for the CD3+ cells. In some of any of the provided embodiments, the antibody is immobilized on a solid surface. In some of any of the provided embodiments, the solid surface is a magnetic particle. In some of any of the provided embodiments, the antibody is immobilized on or attached to an affinity chromatography matrix.

In some of any of the provided embodiments, the antibody further includes one or more binding partners capable of forming a reversible bond with a binding reagent immobilized on the matrix, whereby the antibody is reversibly bound to said chromatography matrix during said contacting. In some of any of the provided embodiments, the binding reagent is a streptavidin mutein that reversibly binds to the binding partner. In some of any of the provided embodiments, the streptavidin mutein contains the amino acid sequence Ile44-Gly45-Ala46-Arg47 at sequence positions corresponding to positions 44 to 47 with reference to positions in streptavidin in the sequence of amino acids set forth in SEQ ID NO:66; or the streptavidin mutein contains the amino acid sequence Va144-Thr45-Ala46-Arg47 at sequence positions corresponding to positions 44 to 47 with reference to positions in streptavidin in the sequence of amino acids set forth in SEQ ID NO: 66. In some of any of the provided embodiments, the binding partner is a streptavidin binding peptide. In some of any of the provided embodiments, the streptavidin-binding peptide is selected from the group consisting of Trp-Ser-His-Pro-Gln-Phe-Glu-Lys (SEQ ID NO: 69), Ser-Ala-Trp-Ser-His-Pro-Gln-Phe-Glu-Lys-(GlyGlyGlySer)3-Trp-Ser-His-Pro-Gln-Phe-Glu-Lys (SEQ ID NO:78), SAWSHPQFEKGGGSGGGSGGSAWSHPQFEK (SEQ ID NO:79), Trp-Ser-His-Pro-Gln-Phe-Glu-Lys-(GlyGlyGlySer)3-Trp-Ser-His-Pro-Gln-Phe-Glu-Lys (SEQ ID NO: 70), Trp-Ser-His-Pro-Gln-Phe-Glu-Lys-(GlyGlyGlySer)2-Trp-Ser-His-Pro-Gln-Phe-Glu-Lys (SEQ ID NO: 71) and Trp-Ser-His-Pro-Gln-Phe-Glu-Lys-(GlyGlyGlySer)2Gly-Gly-Ser-Ala-Trp-Ser-His-Pro-Gln-Phe-Glu-Lys (SEQ ID NO: 72). In some of any of the provided embodiments, the streptavidin-binding peptide has the sequence SAWSHPQFEKGGGSGGGSGGSAWSHPQFEK (SEQ ID NO:79). In some of any of the provided embodiments, the method further includes, after contacting cells in the sample to the affinity chromatography matrix, applying a competition reagent to disrupt the bond between the binding partner and binding reagent, thereby recovering the cells bound to the antibody. In some of any of the provided embodiments, the competition reagent is biotin or a biotin analog. In some of any of the provided embodiments, the chromatography matrix is packed in a separation vessel, which is a column. In some of any of the provided embodiments, the biological sample comprising the primary T cells is a sample formulated with a cyroprectant. In some of any of the provided embodiments, the apheresis or leukapheresis sample is a sample formulated with a cryoprotectant.

In some of any such embodiments, the stimulating conditions include the presence of a stimulatory reagent, said stimulatory reagent capable of activating one or more intracellular signaling domains of one or more components of a TCR complex and one or more intracellular signaling domains of one or more costimulatory molecules. In some of any such embodiments, the stimulatory reagent contains (i) a primary agent that specifically binds to a member of a TCR complex, optionally that specifically binds to CD3 and (ii) a secondary agent that specifically binds to a T cell costimulatory molecule, optionally wherein the costimulatory molecule is selected from CD28, CD137 (4-1-BB), OX40, or ICOS.

In some of any such embodiments, the one or both of the primary and secondary agents include an antibody or an antigen-binding fragment thereof. In some of any such embodiments, the primary and secondary agents include an antibody, optionally wherein the stimulatory reagent includes incubation with an anti-CD3 antibody and an anti-CD28 antibody, or an antigen-binding fragment thereof. In some of any such embodiments, the primary agent is an anti-CD3 antibody or an antigen-binding fragment thereof and the secondary agent is an anti-CD28 antibody or an antigen-binding fragment thereof. In some of any such embodiments, the antigen binding fragment is a monovalent antibody fragment selected from the group consisting of a Fab fragment, an Fv fragment, and a single-chain Fv fragment (scFv). In some of any such embodiments, the primary agent is an anti-CD3 Fab and the secondary agent comprises an anti-CD28 Fab. In some of any such embodiments, the primary agent and secondary agent are present or attached on the surface of a solid support. In some of any such embodiments, the solid support is or comprises a bead, optionally a paramagnetic bead. In some of any such embodiments, the solid support is a paramagnetic bead with surface attached anti-CD3 and anti-CD28 antibodies, and the stimulatory reagent is present at a ratio of less than about or about 3:1 beads to cells.

In some of any such embodiments, the stimulating conditions include the presence of stimulatory reagent containing a paramagnetic bead with surface attached anti-CD3 and anti-CD28 antibodies, said stimulatory reagent present at a ratio of less than at or about 3:1 beads to cells. In some of any such embodiments, said stimulatory reagent present at a ratio of or of about 1:1 beads to cells.

In some of any such embodiments, the method further includes separating the stimulatory reagent from the cells, said separating including exposing the cells to a magnetic field.

In some of any such embodiments, the primary agent and secondary agent are reversibly bound on the surface of an oligomeric particle reagent comprising a plurality of streptavidin or streptavidin mutein molecules. In some of any such embodiments, the streptavidin or streptavidin mutein molecules bind to or are capable of binding to biotin, avidin, a biotin analog or mutein, an avidin analog or mutein, and/or a biologically active fragment thereof. In some of any such embodiments, the streptavidin mutein molecules include the amino acid sequence $Val^{44}$-$Thr^{45}$-$Ala^{46}$-$Arg^{47}$ or $Ile^{44}$-$Gly^{45}$-$Ala^{46}$-$Arg^{47}$ at sequence positions corresponding to positions 44 to 47 with reference to positions in streptavidin in the sequence of amino acids set forth in SEQ ID NO: 66. In some of any such embodiments, the streptavidin mutein molecules contain: a) the sequence of amino acids set forth in any of SEQ ID NOS: 66-68, 73, 80-82, or 85-88; b) a sequence of amino acids that exhibit at least at or about 85%, 86%, 87%, 88%, 89%, 89%, 90%, 91%, 92%, 93%, 94%, 95%, 96%, 97%, 98%, 99% or more sequence identity to any of SEQ ID NOs: 70-73, 78, and 85-89 and contain the amino acid sequence corresponding to Val$^{44}$-Thr$^{45}$-Ala$^{46}$-Arg$^{47}$ or Ile$^{44}$-Gly$^{45}$-Ala$^{46}$-Arg$^{47}$ and/or reversibly bind to biotin, a biotin analog or a streptavidin-binding peptide; or c) a functional fragment of a) or b) that reversibly binds to biotin, a biotin analog, or a streptavidin-binding peptide.

In some of any such embodiments, the streptavidin mutein molecules contain the sequence of amino acids set forth in SEQ ID NO: 73 or 78. In some of any such embodiments, the streptavidin mutein molecules contain the sequence of amino acids set forth in SEQ ID NO: 73. In some of any such embodiments, the streptavidin mutein molecules contain the sequence of amino acids set forth in SEQ ID NO: 78. In some of any such embodiments, the primary agent and the secondary agent each contain a streptavidin-binding peptide. In some of any such embodiments, the streptavidin-binding peptide selected from the group consisting of Trp-Ser-His-Pro-Gln-Phe-Glu-Lys (SEQ ID NO: 69), Trp-Ser-His-Pro-Gln-Phe-Glu-Lys-(GlyGlyGlySer)$_3$-Trp-Ser-His-Pro-Gln-Phe-Glu-Lys (SEQ ID NO: 76), Trp-Ser-His-Pro-Gln-Phe-Glu-Lys-(GlyGlyGlySer)$_2$-Trp-Ser-His-Pro-Gln-Phe-Glu-Lys (SEQ ID NO: 77) and Trp-Ser-His-Pro-Gln-Phe-Glu-Lys-(GlyGlyGlySer)$_2$Gly-Gly-Ser-Ala-Trp-Ser-His-Pro-Gln-Phe-Glu-Lys (SEQ ID NO: 89). In some of any such embodiments, the streptavidin-binding peptide selected from the group consisting of Trp-Ser-His-Pro-Gln-Phe-Glu-Lys (SEQ ID NO: 69), Trp-Ser-His-Pro-Gln-Phe-Glu-Lys-(GlyGlyGlySer)$_3$-Trp-Ser-His-Pro-Gln-Phe-Glu-Lys (SEQ ID NO: 70), Trp-Ser-His-Pro-Gln-Phe-Glu-Lys-(GlyGlyGlySer)$_2$-Trp-Ser-His-Pro-Gln-Phe-Glu-Lys (SEQ ID NO: 71) and Trp-Ser-His-Pro-Gln-Phe-Glu-Lys-(GlyGlyGlySer)$_2$Gly-Gly-Ser-Ala-Trp-Ser-His-Pro-Gln-Phe-Glu-Lys (SEQ ID NO: 89). In some of any such embodiments, the primary agent includes an anti-CD3 Fab and wherein the secondary agent contains an anti-CD28 Fab.

In some of any such embodiments, the oligomeric particle reagent contains a radius of greater than at or about 60 nm, greater than at or about 70 nm, greater than at or about 80 nm, or greater than at or about 90 nm. In some of any such embodiments, the oligomeric particle reagent contains a molecular weight of: at least at or about $5 \times 10^7$ g/mol, or at least at or about $1 \times 10^8$ g/mol; and/or between $5 \times 10^7$ g/mol and $5 \times 10^8$ g/mol, between $1 \times 10^8$ g/mol and $5 \times 10^8$ g/mol, or between $1 \times 10^8$ g/mol and $2 \times 10^8$ g/mol. In some of any such embodiments, the oligomeric particle reagent contains at least at or about 500 streptavidin or streptavidin mutein tetramers, at least at or about 1,000 streptavidin or streptavidin mutein tetramers, at least at or about 1,500 streptavidin or streptavidin mutein tetramers, or at least at or about 2,000 streptavidin or streptavidin mutein tetramers; and/or; between 1,000 and 20,000 streptavidin or streptavidin mutein tetramers, between 1,000 and 10,000 streptavidin or streptavidin mutein tetramers, or between 2,000 and 5,000 streptavidin or streptavidin mutein tetramers.

In some of any such embodiments, the method further includes separating the stimulatory reagent from the cells, said separating including contacting a substance to the cells, said substance being capable of reversing bonds between the primary and secondary agent and the oligomeric particle reagent.

In some of any such embodiments, the substance is a free binding partner and/or is a competition agent. In some of any such embodiments, the presence of the substance terminates or lessens the signal induced or modulated by the primary and secondary agent in the T cells. In some of any such embodiments, the substance is or includes a streptavidin-binding peptide, biotin or a biologically active fragment, or a biotin analog or biologically active fragment. In some of any such embodiments, the substance is or includes a biotin analog. In some of any such embodiments, the substance is or includes a streptavidin-binding peptide and the streptavidin-binding peptide is selected from the group consisting of Trp-Ser-His-Pro-Gln-Phe-Glu-Lys (SEQ ID NO: 69), Trp-Ser-His-Pro-Gln-Phe-Glu-Lys-(GlyGlyGlySer)$_3$-Trp-Ser-His-Pro-Gln-Phe-Glu-Lys (SEQ ID NO: 70), Trp-Ser-His-Pro-Gln-Phe-Glu-Lys-(GlyGlyGlySer)$_2$-Trp-Ser-His-Pro-Gln-Phe-Glu-Lys (SEQ ID NO: 77, e.g. SEQ ID NO:71) and Trp-Ser-His-Pro-Gln-Phe-Glu-Lys-(GlyGlyGlySer)zGly-Gly-Ser-Ala-Trp-Ser-His-Pro-Gln-Phe-Glu-Lys (SEQ ID NO: 89, also set forth SEQ ID NO:72). In some of any such embodiments, the substance is or includes biotin or a biologically active fragment, or a biotin analog or biologically active fragment.

In some of any such embodiments, the stimulating conditions include the presence of one or more recombinant cytokines. In some of any such embodiments, the stimulating conditions include the presence of one or more of recombinant IL-2, IL-7, and IL-15.

In some of any of such embodiments, prior to the introducing, the depleted population is incubated under stimulatory conditions.

In some of any such embodiments, the introducing includes transduction with a viral vector comprising the heterologous polynucleotide. In some of any such embodiments, the viral vector is a gammaretroviral vector or a lentiviral vector. In some of any such embodiments, the viral vector is a lentiviral vector.

In some of any such embodiments, the method further includes incubating the composition containing transduced cells for up to 96 hours subsequent to the introducing. In some of any such embodiments, the incubating is at a temperature of at or about $37° \pm 2°$ C. In some of any such embodiments, the incubating is carried out for up to 72 hours subsequent to the introducing. In some of any such embodiments, the incubating is carried out for up to 48 hours subsequent to the introducing. In some of any such embodiments, the incubating is carried out for up to 24 hours subsequent to the introducing. In some of any such embodiments, the incubating results in integration of the viral vector into the genome of the T cells.

In some of any such embodiments, the method optionally further includes cultivating cells of the transformed population under conditions to promote proliferation or expansion of the engineered cells, thereby generating an expanded population of cells. In some of any such embodiments, the cultivating is carried out in the presence of one or more recombinant cytokines. In some embodiments, the one or more recombinant cytokines is one or more of IL-2, IL-7, and IL-15. In some of any such embodiments, the proliferation or expansion results in, in about, or in at least at or about a 2-fold, 3-fold, 4-fold, 5-fold, or greater than at or about a 5-fold increase in the number of viable T cells comprising the heterologous polynucleotide.

In some of any such embodiments, the method further includes:(i) cultivating the populations of engineered T cells under conditions to promote proliferation or expansion of the engineered population, thereby generating an expanded population of T cells; and (ii) harvesting or collecting the expanded population.

In some of any such embodiments, the stimulatory reagent includes a paramagnetic bead with surface attached anti-CD3 and anti-CD28 antibodies. In some of any such embodiments, the stimulatory reagent includes an oligomeric particle reagent containing a plurality of streptavidin or streptavidin mutein molecules with reversibly bound anti-CD3 and anti-CD28 Fabs.

In some of any such embodiments, the harvesting is performed at or after the time in which the engineered population or the expanded population of T cells include a threshold number of T cells, viable T cells, engineered T cells or viable engineered T cells, or a threshold concentration of T cells, viable T cells, engineered T cells or viable engineered T cells. In some of any such embodiments, the threshold number or concentration of T cells, viable T cells, engineered T cells or viable engineered T cells is reached within at or about 4, 5, 6 or 7 days after the initiation of stimulation.

In some of any such embodiments, among a plurality of populations of engineered T cells or populations of expanded T cells, the threshold number or concentration of T cells, viable T cells, engineered T cells or viable engineered T cells is reached within at or about 5 or 6 days after the initiation of stimulation in at least at or about or at least at or about 70%, 80%, 90% or 95% of the plurality. In some of any such embodiments, the threshold number or concentration of T cells, viable T cells, engineered T cells or viable engineered T cells is reached within at or about 2, 3, 4 or 5 population doublings after the initiation of stimulation.

In some of any such embodiments, the engineered population or the expanded population contains less than at or about 3%, less than at or about 2%, less than at or about 1%, less than at or about 0.1%, or less than at or about 0.01% CD57+ T cells. In some of any such embodiments, the engineered population or the expanded population is free or is essentially free of CD57+ T cells. In some embodiments, a depleted population that is essentially free of CD57+ T cells comprises less than about or about 3%, less than about or about 2%, less than about or about 1%, less than about or about 0.1% or less than about or about 0.01% CD57+ T cells.

In some of any such embodiments, the frequency of the naïve-like cells in the engineered population or the expanded population is at least at or about 10%, 20%, 30%, 40%, or 50% of the cells in the population. In some of any such embodiments, the frequency of one or more of CD25+ T cells, CD27+ T cells, CD28+ T cells, CCR7+ T cells, or CD45RA+ T cells in the engineered population or the expanded population is at least at or about 10%, 20%, 30%, 40%, or 50% greater than at or about the frequency of the respective cells in the population. In some of any such embodiments, the engineered population or the expanded population contains at least at or about 15%, 20%, 25%, 30%, 35%, or 40% CD27+ T cells. In some of any such embodiments, the engineered population or the expanded population contains at least at or about 10%, 15%, 20%, 25%, 25%, 30%, 35%, or 40% CD28+ T cells. In some of any such embodiments, the engineered population or the expanded population contains at least at or about 10%, 15%, 20%, 25%, 30%, 35%, 40%, 50%, 60%, 70% or 80% CD27+CD28+ T cells.

In some of any such embodiments, the engineered population or the expanded population contains at least at or about 70% or 80% CD27+CD28+ T cells. In some of any such embodiments, the engineered population or the expanded population contains at least at or about 10%, 15%, 20%, or 25% CCR7+ T cells.

In some of any such embodiments, harvesting the cells includes removing cellular debris by rinsing or washing the cells. In some of any such embodiments, the harvesting or collecting further includes formulating the cells for cryopreservation or administration to a subject. In some of any such embodiments, the harvested or collected cells are formulated in the presence of a pharmaceutically acceptable excipient. In some of any such embodiments, the harvested or collected cells are formulated for cryopreservation in the presence of a cryoprotectant. In some of any such embodiments, the cryoprotectant comprises DMSO. In some of any such embodiments, the harvested or collected cells are formulated in a container, optionally a vial or a bag.

In some of any such embodiments, the separation occurs prior to the harvesting. In some of any such embodiments, the separation occurs prior to or during the cultivation. In some of any such embodiments, the separation occurs subsequent to the introducing.

In some of any such embodiments, the recombinant receptor is capable of binding to a target antigen that is associated with, specific to, and/or expressed on a cell or tissue of a disease, disorder or condition. In some of any such embodiments, the disease, disorder or condition is an infectious disease or disorder, an autoimmune disease, an inflammatory disease, or a tumor or a cancer. In some of any such embodiments, the target antigen is a tumor antigen.

In some of any such embodiments, the target antigen is selected from among $\alpha\nu\beta6$ integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Ra), IL-13 receptor alpha 2 (IL-13R$\alpha$2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens.

In some of any such embodiments, the recombinant protein is or includes a functional non-TCR antigen receptor or a TCR or antigen-binding fragment thereof. In some of any such embodiments, the recombinant protein is a chimeric antigen receptor (CAR). In some of any such embodiments, the recombinant receptor is an anti-BCMA CAR. In some of any such embodiments, the recombinant protein is an anti-CD19 CAR. In some of any such embodiments, the chimeric antigen receptor includes an extracellular domain containing an antigen-binding domain, a spacer and/or a hinge region, a transmembrane domain, and an intracellular signaling domain containing a costimulatory signaling region. In some of any such embodiments, the extracellular domain including an antigen-binding domain includes an scFv.

In some of any such embodiments, the intracellular signaling domain is or includes a primary signaling domain, a signaling domain that is capable of inducing a primary activation signal in a T cell, a signaling domain of a T cell receptor (TCR) component, and/or a signaling domain including an immunoreceptor tyrosine-based activation motif (ITAM). In some of any such embodiments, the intracellular signaling domain is or includes an intracellular signaling domain of a CD3 chain, optionally a CD3-zeta (CD3$\zeta$) chain, or a signaling portion thereof. In some of any such embodiments, the costimulatory signaling region includes an intracellular signaling domain of a CD28, a 4-1BB or an ICOS or a signaling portion thereof.

Also provided herein are compositions of cells produced by the methods of the invention.

The invention further provides a dose of T cells from an engineered population of T cells produced by a method of the invention, for use in a method of treating a subject having or suspected of having a disease, disorder or condition, wherein the method comprises administering the dose of T cells to the subject. The invention further provides a dose of T cells from an engineered population of T cells produced by a method of the invention, for use in a method of treating a subject having or suspected of having a disease or condition, wherein the disease or condition is a cancer, and wherein the method comprises administering the dose of T cells to the subject.

Brief Description of the Drawings

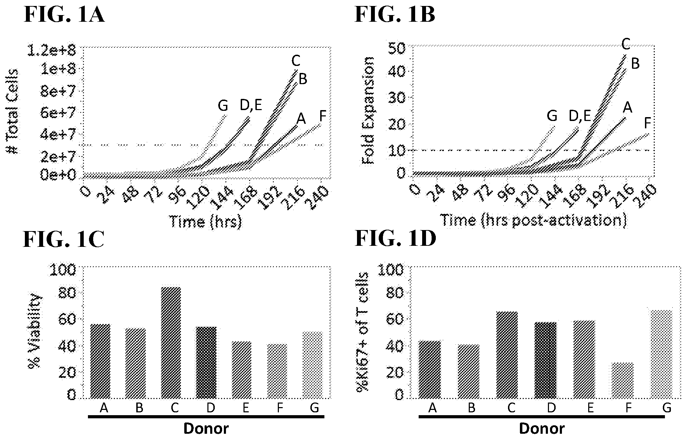

FIGS. 1A-1D show the total cell number (FIG. 1A), fold expansion (FIG. 1B), percent viability (FIG. 1C) and percent Ki-67+ cells (marker associated with cell cycle entry; FIG. 1D) among CD8+ T cells obtained from seven different donors (Donors A-G), over approximately 240 hours after the start of stimulation in an exemplary manufacturing process for primary human T cells, or a similar process without transduction of the cells.

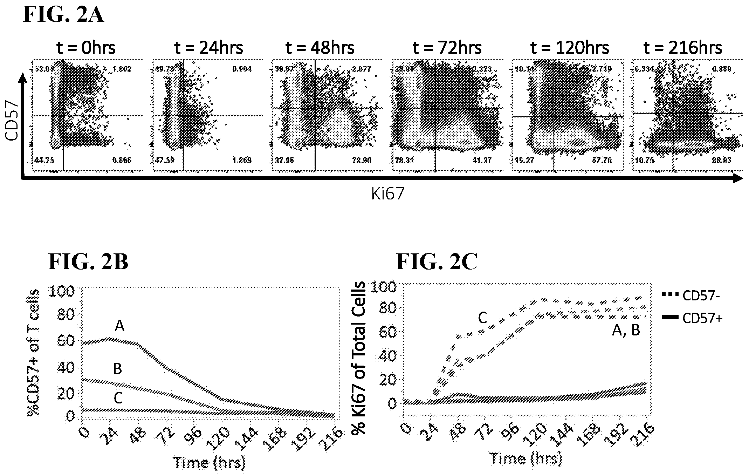

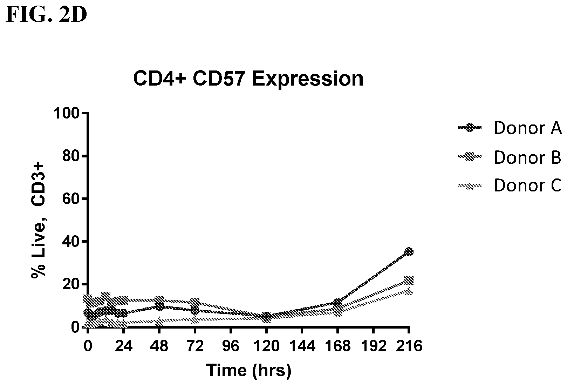

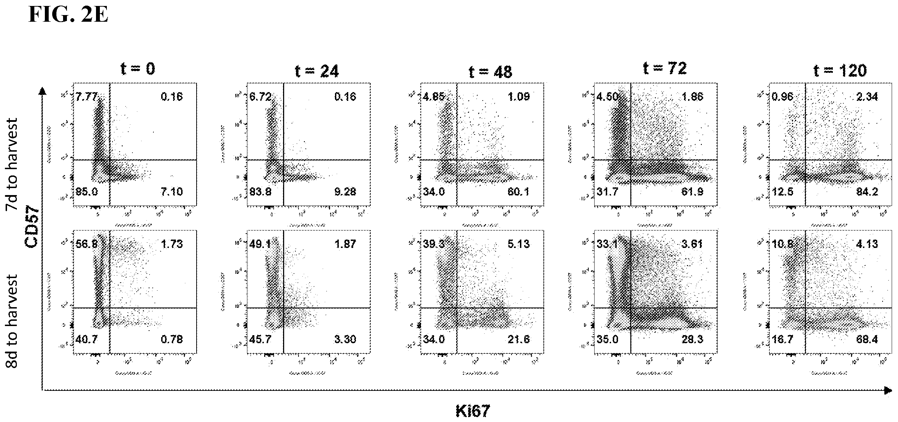

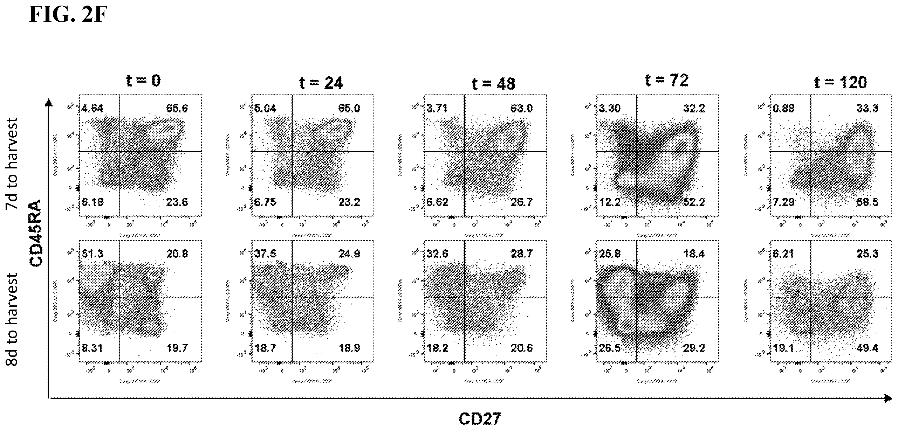

FIG. 2A shows a flow cytometry analysis for CD57 and Ki-67 expression at various time points over approximately 216 hours after the start of stimulation in an exemplary process for primary human T cells. FIG. 2B shows the percentage of CD57+ cells in the cell population during stimulation and cultivation, in three different donors (Donors A-C). FIG. 2C shows the percentage of Ki-67+ cells among CD57+ cells or CD57- cells in the cell population during stimulation and cultivation. **FIG. 2D** shows the percentage of CD57+ cells in the CD4+ cell population during stimulation and cultivation, in three different donors (Donors A-C). **FIG. 2E** shows CD57 and Ki67 expression on cells from donor cell compositions, over time, following stimulation. The cells of one donor cell composition required 8 days to reach harvest criterion ("8d to harvest"), while the cells of the other donor cell composition required only 7 days to reach harvest criterion ("7d to harvest"). **FIG. 2F** shows CD45RA and CD27 expression on cells from the donor cell compositions described in FIG. 2E.

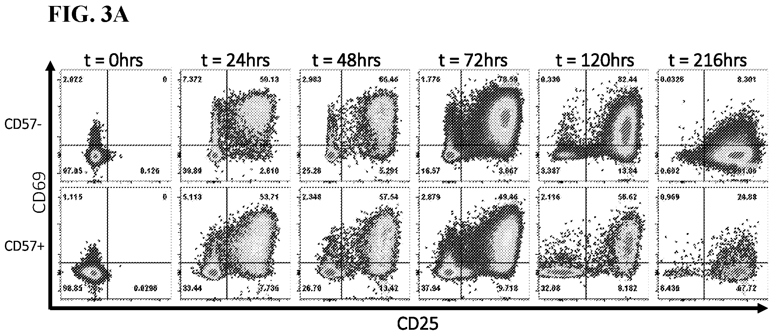

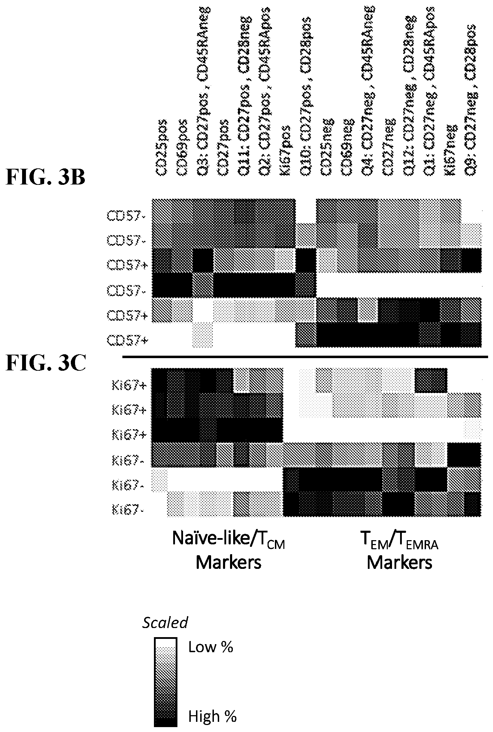

**FIG. 3A** shows a flow cytometry analysis for CD69 and CD25 expression (markers associated with activation) among CD57+ cells and CD57- cells in the cell population during stimulation and cultivation in an exemplary process for primary human T cells. **FIGS. 3B** and **3C** show hierarchical clustering analysis for expression of various surface markers associated with T cell differentiation phenotypes (e.g., naïve-like T cells, effector T ($T_{EFF}$) cells, memory T cells, central memory T cells ($T_{CM}$), effector memory T ($T_{EM}$) cells, effector memory RA T ($T_{EMRA}$) cells), and the expression of CD57 (**FIG. 3B**) or Ki-67 (**FIG. 3C**). Each row represents a cell population from an individual donor, columns represent markers or combinations of markers that are associated with T cell differentiation phenotypes.

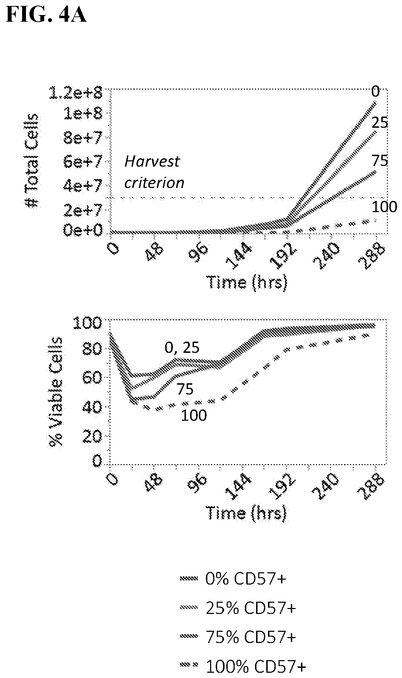

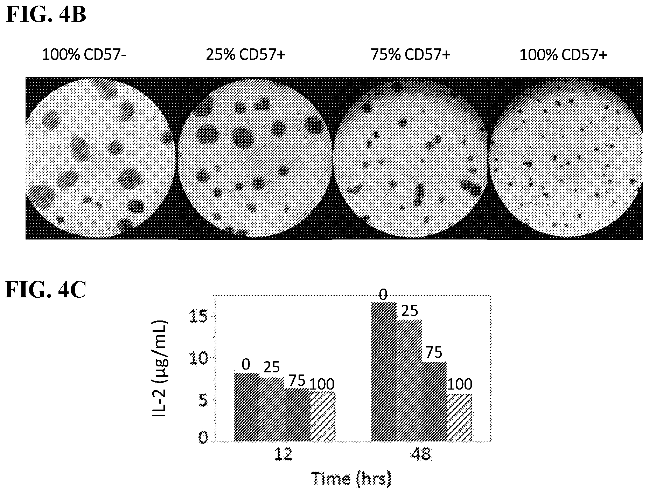

**FIG. 4A** shows the number of total cells and the percentage of viable cells over approximately 240 hours after the start of stimulation in an exemplary manufacturing process, for titrated cell compositions containing a mixture of CD57+ and CD57- cells at the following frequency: (1) 100% CD57+ cells; (2) 75% CD57+ cells; (3) 25% CD57+ cells; and (4) 0% CD57+ cells. An exemplary harvest criterion is indicated. **FIG. 4B** shows images of cell culture wells at 48 hours after the start of stimulation, showing cell clustering in the presence of a stimulating reagent, in the stimulation and cultivation of the titrated cell compositions. **FIG. 4C** shows the amount of IL-2 ($\mu$g/mL) present in the culture media at 12 and 48 hours after the start of stimulation, in the stimulation and cultivation of the titrated cell compositions.

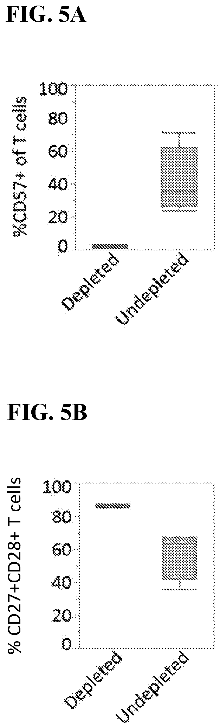

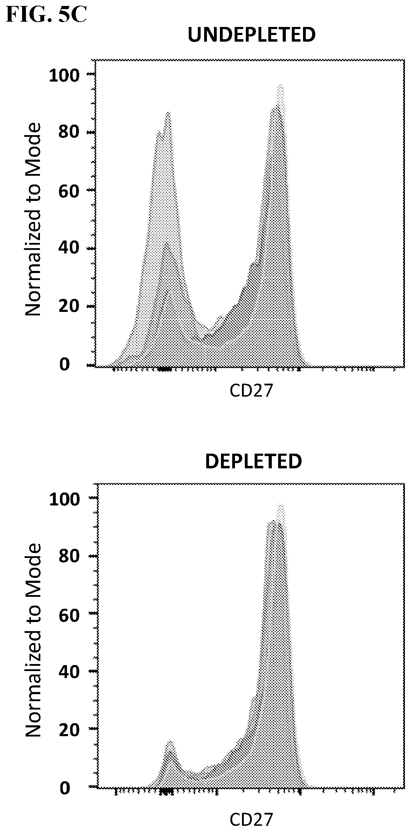

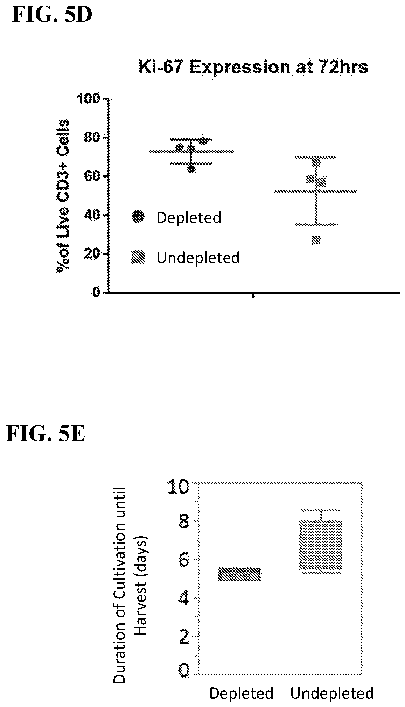

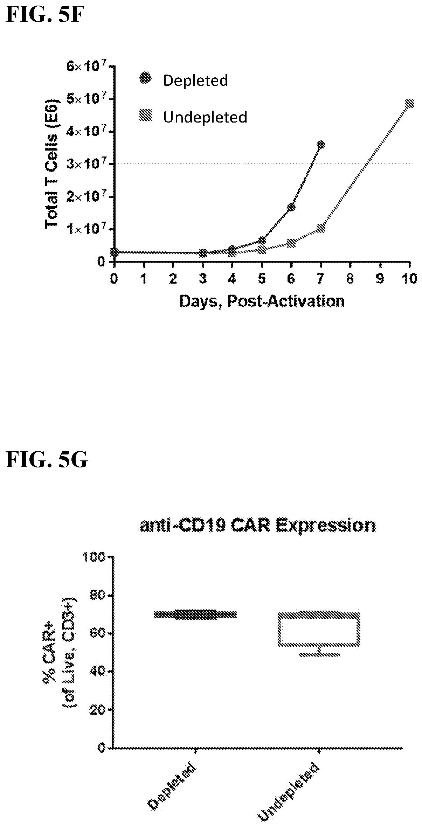

**FIGS. 5A and 5B** show the percentage of CD57+ T cells (**FIG. 5A**), CD27+CD28+ T cells (**FIG. 5B**). **FIG. 5C** shows the expression of CD27 for four donor cell compositions that were not depleted of CD57+ cells (non-depleted) or that were depleted of CD57+ cells (depleted). **FIG. 5D** shows Ki67 expression in depleted and undepleted CD3+ cell compositions. **FIG. 5E** shows the duration of cultivation to harvest (from the start of stimulation to the time at which harvest criterion was reached) for primary CD8+ cells obtained from subjects that were depleted of CD57+ cells (depleted) or that were not depleted of CD57+ cells (undepleted). **FIG. 5F** shows the total number of T cells over time in depleted and undepleted cell compositions, following stimulation. **FIG. 5G** shows the percentage of cells expressing a chimeric antigen receptor (CAR) following transduction, in depleted and undepleted cell compositions.

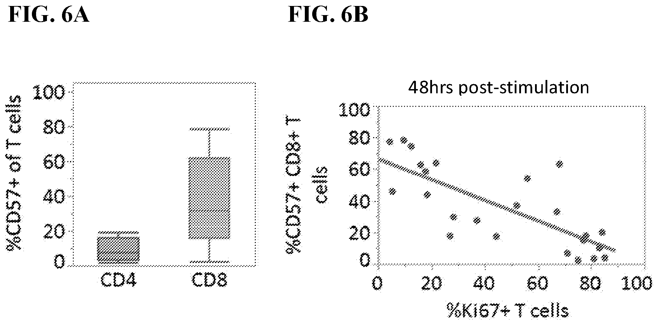

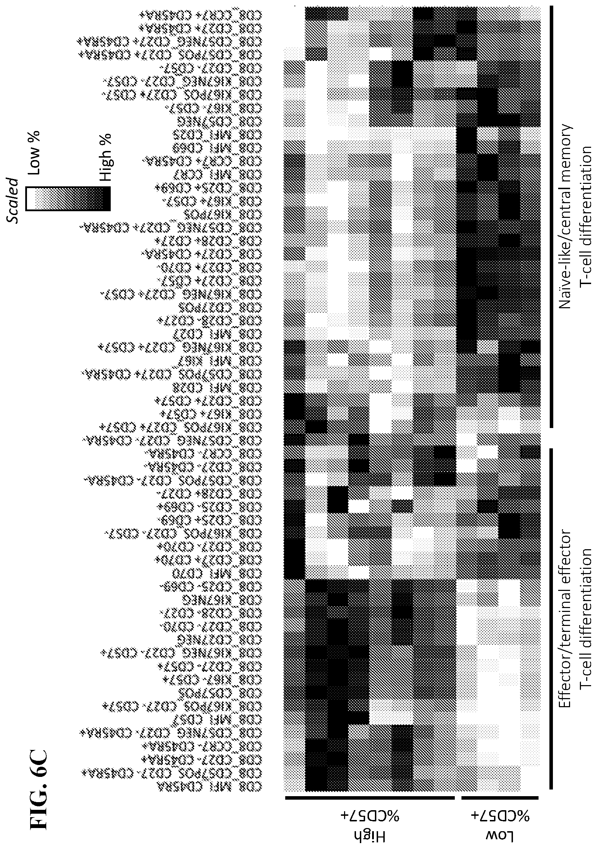

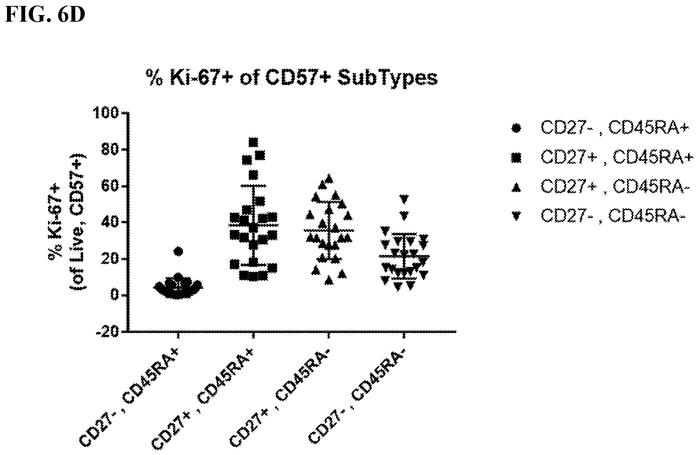

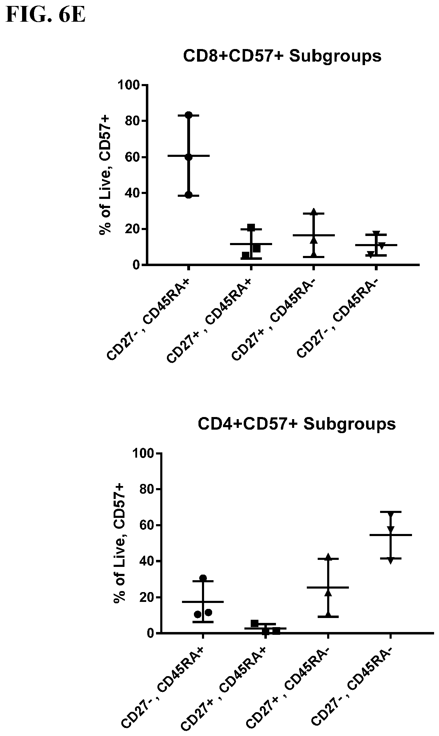

**FIG. 6A** shows a whisker plot indicating the percentage of CD57+ T cells in CD4+ and CD8+ T cells obtained from non-Hodgkins lymphoma (NHL) patients for engineering the cells to express a chimeric antigen receptor (CAR) using an exemplary manufacturing process. Boxes represent interquartile range and whiskers represent full range of the data set. **FIG. 6B** shows the relationship between the percentage of CD57+CD8+ cells and the percentage of Ki-67+ cells in the various cell populations obtained from NHL patients. **FIG. 6C** shows a hierarchical clustering analysis for expression of various surface markers associated with T cell differentiation phenotypes (e.g., naïve-like T cells, effector T ($T_{EFF}$) cells, memory T cells, central memory T cells ($T_{CM}$), terminal effector cells), and the expression of CD57 in the cell populations obtained from NHL patients. Each row represents an individual patient, columns represent markers or combinations of markers that are associated with T cell differentiation phenotypes. **FIG. 6D** shows the percentage of live, CD57+ cells expressing Ki67 in subsets of cell poulations, grouped by CD27 and CD45RA expression status. **FIG. 6E** shows the percentage of live, CD8+CD57+ cells in subsets of cells populations, sorted by CD27 and CD45RA expression (top panel), and the percentage of live, CD4+CD57+ cells in subsets of cell populations, sorted by CD27 and CD45RA expression (bottom panel).

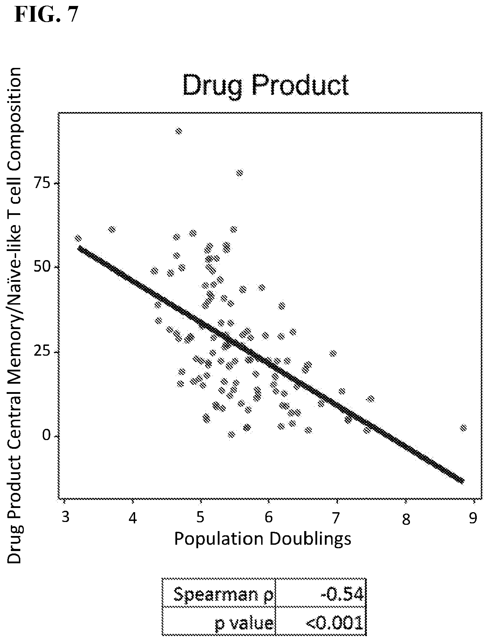

**FIG. 7** shows the relationship between the percentage of central memory /naïve-like CD4+ T cells in the therapeutic output T cell composition (e.g., drug product) and the number of population doublings to achieve harvest criterion (Spearman $\rho$: -0.54; p-value: <0.001). A similar result was observed for CD8+ T cells in the therapeutic output T cell composition (e.g., drug product).

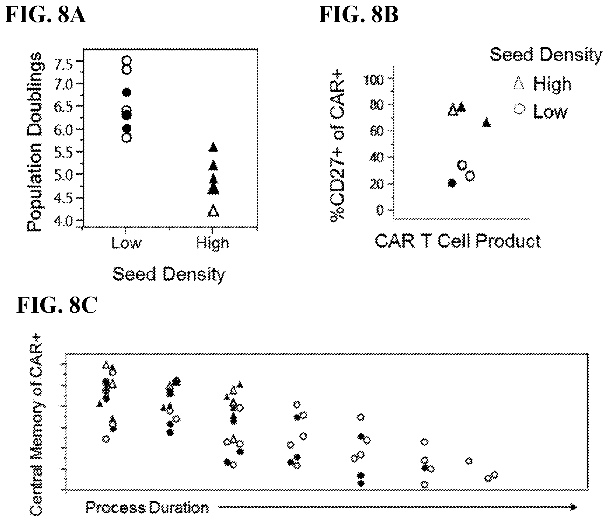

**FIG. 8A** shows the number of population doublings to reach harvest criterion for high ($0.35\times10^6$ cells/mL) and low ($0.05\times10^6$ cells/mL) seed density during the expansion step of the production process. **FIG. 8B** shows the percentage of CD27+CAR+CD8+ T cells in the output therapeutic composition as a function of seed density during the expansion step of the production process. **FIG. 8C** shows the impact of processing duration on the amount of central memory T cells in the output T cell composition.

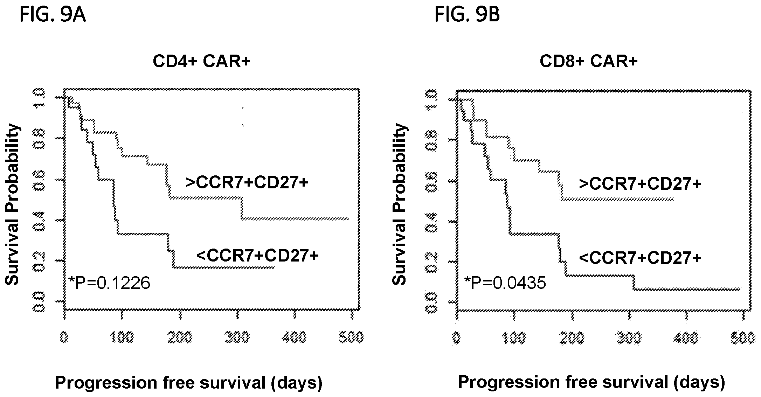

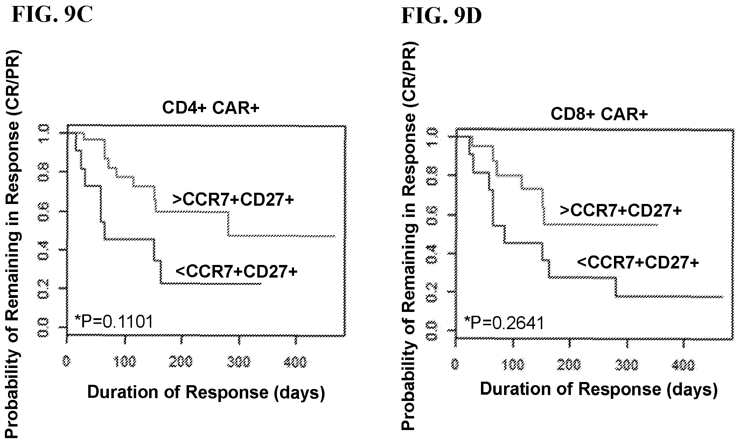

**FIGS. 9A-9D** show the Kaplan-Meier survival curves for subjects who were administered CAR[+] T cell compositions, divided into groups that were administered compositions containing a percentage of CCR7[+]CD27[+] CAR[+] T cells among CD4[+] CAR[+] T cells (**FIG. 9A** for progression free survival, **FIG. 9C** for duration of response) and among CD8[+] CAR[+] T cells (**FIG. 9B** for progression free survival, **FIG. 9D** for duration of response) that is above or below a certain threshold level.

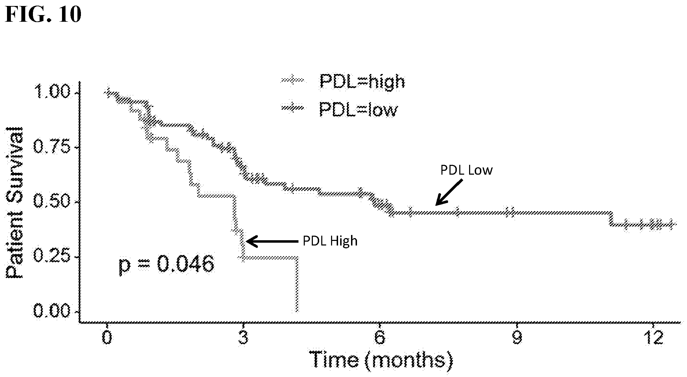

**FIG. 10** shows a PFS curve based on an optimal-split log-rank test for patients with "high" or "low" numbers of population doublings (PDL) in CD8+/CAR+ T cells. Low PDL refers to <6 PDL and >6 PDL refers to high PDL.

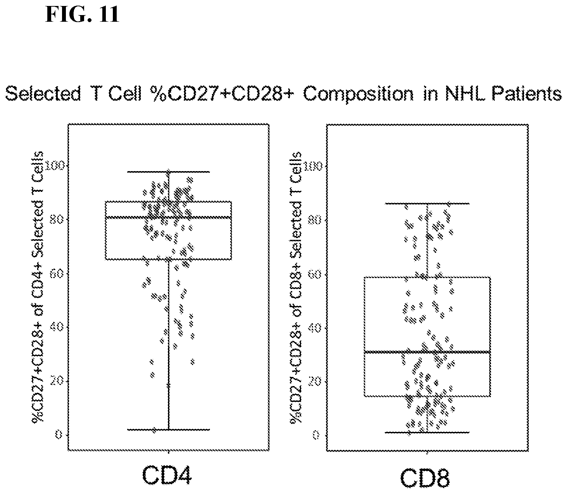

**FIG. 11** shows the percentage of CD27+CD28+ T cells in enriched CD4+ (left panel) and CD8+ (right panel) input compositions derived from Non-Hodgkin's lymphoma patients.

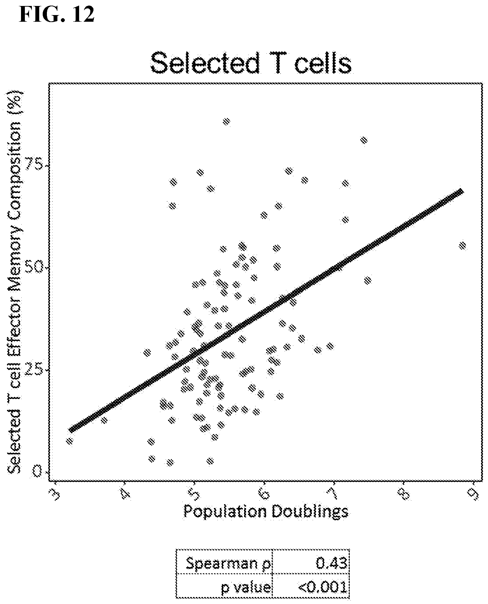

**FIG. 12** shows the relationship between the percentage of effector memory T cells in enriched CD4+ input compositions and the number of population doublings needed to achieve harvest criterion (Spearman ρ: 0.43; p-value: <0.001). A similar result was observed for enriched CD8+ input compositions.

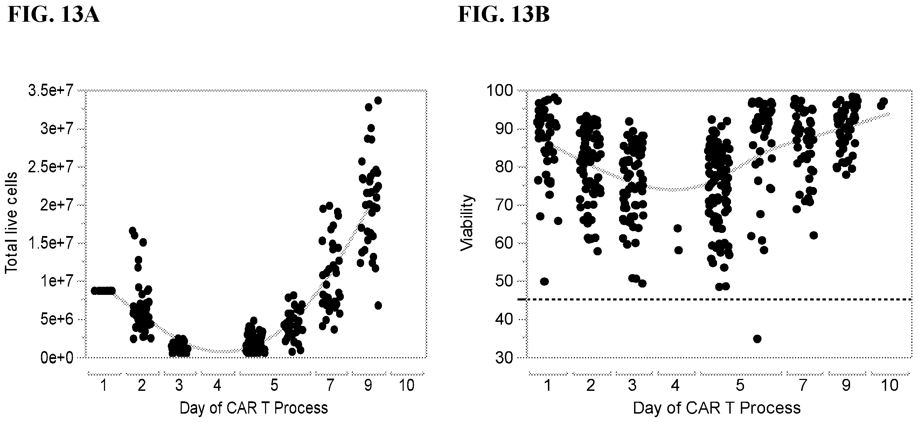

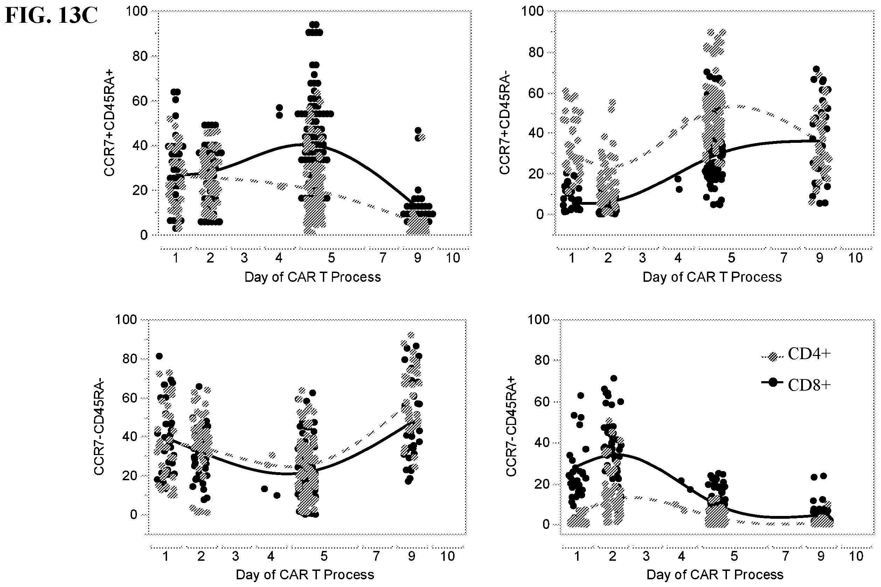

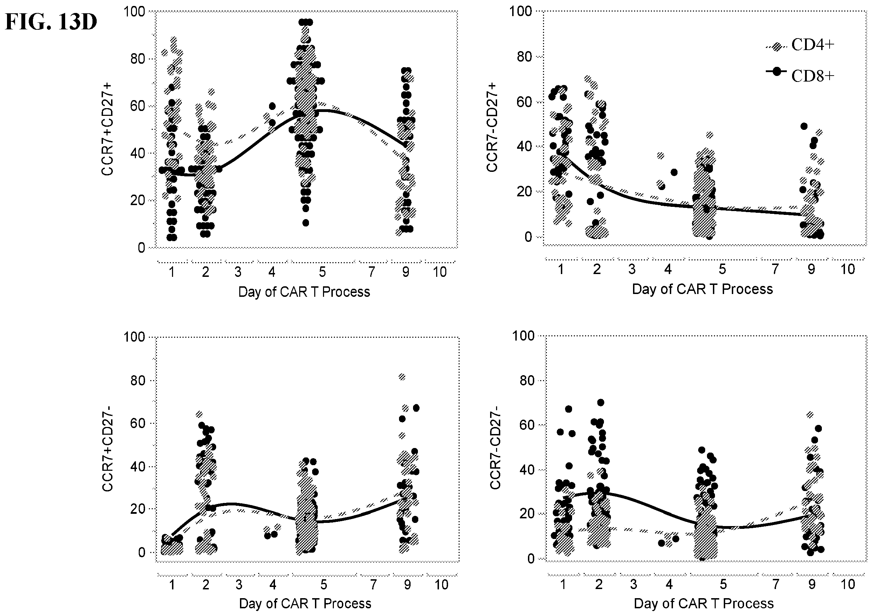

FIGS. 13A-D show total live cells (FIG. 13A), viability (FIG. 13B), and phenotype (FIGS. 13C and 13D) during and after manufacturing runs, including a variety of non-expanded and expanded engineering processes.

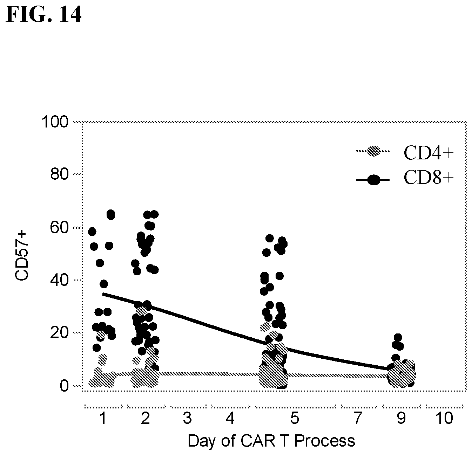

**FIG. 14** shows the percentage of CD57+ cells in CD4+ and CD8+ cell populations during and after manufacturing runs, including a variety of non-expanded and expanded engineering processes.

Detailed Description

Embodiments of the invention fall within some of the instances disclosed below. Any references herein to methods of treatment are intended to refer to products for use in said methods.

Disclosed herein are methods and compositions useful for, *inter alia,* selecting, isolating, enriching, stimulating, activating, genetically engineering, or expanding sample, populations, or compositions of cells having a reduced frequency of CD57+ T cells, such as compared to starting cellular material or biological samples. In some instances, disclosed herein is a method for enriching T cells that is or includes selecting, isolating, or removing CD57+ T cells from a biological sample, such as to generate a population of enriched CD57- T cells, or a population depleted for CD57+ cells. In some instances, disclosed herein is a method for enriching T cells that is or includes selecting, isolating, or removing CD57+ T cells from a biological sample, such as to generate a population of enriched CD57- T cells, or a population depleted for CD57+ T cells.

In some instances, disclosed herein is a method for stimulating a population of CD57- T cells by incubating the cells of the population under stimulatory conditions. Also disclosed herein is a method for genetically engineering T cells by introducing a heterologous polypeptide into cells, such as cells of or originating from a population of enriched CD57- T cells.

Particular disclosures contemplate that existing methods for generating engineered T cells, e.g., engineered T cells expressing chimeric antigen receptors (CARs) may include steps, stages, or phases where populations or compositions of T cells proliferate or expand. However, in some cases disclosed herein, a portion of populations or compositions may not display any proliferation or expansion, or, in some cases disclosed herein, may expand slowly are thus require extra days to complete the engineering process.

In some disclosures, existing methods or processes for generating or manufacturing engineered cell compositions can result in heterogeneity in the manufacturing process. In some disclosures, phenotypes associated with memory T cells can affect clinical outcome (see, e.g., Fraietta et al., Nat Med. 2018; 24(5):563-571; and Larson et al., Cancer Res. 2018; 78(13 Suppl):Abstract nr 960). In some cases disclosed herein, enrichment for cells exhibiting early memory T cell phenotypes can improve the manufacturing of engineered cell compositions (see, e.g., Singh et al., Sci Trans Med. 2016; 8(320):320ra3).

The disclosed methods and compositions address these issues. The disclosed methods and compositions are directed, at least in part, to populations of cells, e.g., populations of enriched CD57- T cells that undergo improved or more rapid proliferation and expansion, such as during processes for stimulating or engineering T cells. CD57 (which is also known as HNK1 and LEU7) is a beta-1,3-glucuronyltransferase that may be expressed on the surface of T and NK lymphocytes. In some disclosures, CD57 expression, e.g., surface expression, is associated with mature, effector-differentiated sub-populations of T and NK cells. In some disclosures, CD57 expression corresponds with T cells or T cell populations that lack expression of co-stimulatory receptors CD28 and CD27, which, in certain disclosures, can affect sustained proliferation and cell survival. In particular disclosures, CD57 expression may also identify cells with less or reduced proliferative capacity. In some disclosures, CD57+CD28- cell populations may demonstrate shortened telomere length and reduced proliferative capacity as compared to CD57- cell populations (Reviewed in Strioga, Pasukoniene, & Characiejus, Immunol. (2011)).

Particular disclosures contemplate that a reduction in the frequency of CD57+ T cells from starting cellular material used in genetic engineering processes will enrich for cells with greater proliferative capacity. In some disclosures, negative selection of CD57+ T cells improves manufacturing success and drug product consistency by pre-enriching for cells that are better poised to expand. Additionally, in some disclosures, enriching the populations or compositions of cells with cells exhibiting increased proliferative capacity may reduce the extent of effector cell differentiation, which should aid in improved target product profile consistency across subjects in an autologous setting or across batches in an allogeneic setting (CD27+, CCR7+ T cells).

In some disclosures, the methods are used in connection with a process that generates or produces genetically engineered cells that are suitable for cell therapy in a manner that may be faster and more efficient than alternative processes. In certain disclosures, the methods disclosed herein have a high rate of success for generating or producing compositions of engineered cells from a broader population of subjects than what may be possible from alternative processes. Thus, in some disclosures, the speed and efficiency of the disclosed methods for generating engineered cells for cell therapy allow for easier planning and coordination of cell therapy treatments, such as autologous therapy, to a broader population of subjects than what may be possible by some alternative methods. In some disclosures, it is contemplated that depletion of CD57+ cells (e.g. CD57+ T cells) is advantageous, such as by improving the consistency of the cell populations in downstream processes. For example, it is observed herein that depleting CD57+ cells may deplete cells with less or reduced proliferative capacity, such that depleted compositions exhibited improved consistency in cell proliferation rates. Relatedly, improving consistency in cell proliferation rates may improve consistency in the duration required for cell populations to reach a harvest criterion. It is additionally observed herein that depleting CD57+ cells prior to tranducing the cell population with a vector encoding a chimeric antigen receptor (CAR) may improve consistency in the CAR expression of the transduced cells.

In some disclosures, pre-selecting incoming donor cells with improved proliferative capacity, e.g., by removing CD57+ T cells or screening for low amounts of CD57 + T cells, can offer improved process control over the number of cells used in a process to generate a cell therapy. In certain disclosures, expression of CD57 may serve as a biomarker indicating cells that exhibit delayed or poor growth. Thus, some of thedisclosed examples are directed to methods that utilize one or more selection reagents or process steps to selectively remove CD57+ cells prior to a process for stimulating, genetically engineering, or expanding cells. In some disclosures, such reagents and process steps may be used in conjunction with CD8+ and CD4+ selection strategies. For example, in some disclosures, selection of CD57+ cells is employed to remove or deplete CD57+ cells from a sample, composition, or population of cells prior to any steps for CD8+ or CD4+ selection. For example, in some disclosures, selection of CD57+ T cells is employed to remove or deplete CD57+ T cells from a sample, composition, or population of cells prior to any steps for CD8+ or CD4+ selection, thereby generating a CD57-depleting population. In some disclosures, it may be advantageous to deplete CD57+ cells by negative selection (as opposed to positive selection), such as prior to any steps for CD8+ or CD4+ selection. In some disclosures, depleting CD57+ cells by negative selection, such as prior to any steps for CD8+ or CD4+ selection, reduces the likelihood of the CD57-depleted population being contaminated by one or more reagents or solutions used in the CD57 selection step.

In some disclosures, selection of CD57+ T cells is employed to remove or deplete CD57+ T cells from a sample, composition, or population of cells following any steps for CD8+ or CD4+ selection. In some disclosures, such reagents and process steps may be used in conjunction with CD3+ selection strategies. For example, in some disclosures, selection of CD57+ T cells is employed to remove or deplete CD57+ T cells from a sample, composition, or population of cells prior to any steps for CD3+ selection. In some disclosures, selection of CD57+ T cells is employed to remove or deplete CD57+ T cells from a sample, composition, or population of cells following any steps for CD3+ selection. In some disclosures, CD57+ cells are selected or removed with CD57-directed magnetic beads that bind CD57+ cells, such as in a column, and the column flow-through (unbound fractions) would then contain a CD57+ depleted cell source, e.g., a population of cells enriched for CD57- T cells. In some disclosures, CD57+ T cells are selected or removed with CD57-directed magnetic beads that bind CD57+ T cells, such as in a column, and the column flow-through (unbound fractions) would then contain a CD57+ depleted cell source, e.g., a population of cells enriched for CD57- T cells.

Particular disclosures contemplate that some existing *ex vivo* T-cell activation or expansion protocols are likely to reduce the presence of CD57 + T cell after an amount of time, such as after the first 48 hours of the process, since CD57+ T cells are less likely to proliferate. In some disclosures, the CD57+ cells (e.g. CD57+ T cells) either die during such processes or their frequency is diminished by subsets of cells that are capable of robust expansion. However, while a natural reduction of CD57+ T cells may occur during such processes, the natural reduction does not control for the quantity of cells entering process that exhibit high proliferative capacity (e.g., CD57- cells). Thus, in some disclosures, since CD57+ cells (e.g. CD57+ T cells) are viable but less proliferative cells, they contribute to the overall starting cell number input to the process. Thus, in some disclosures, an advantage of removing CD57+ T cells or verifying low CD57+ T cell content in a sample, composition, or population insures that the CD57- T cells, e.g., cells with a capacity to proliferate, do not make up a minority population in incoming material. Thus, in some disclosures, insuring a low frequency of CD57+ T cells or a reduced fraction of proliferating cells could reduce incidences relating to prolonged process times, increased cellular differentiation, and/ or failure to meet harvest criteria during the engineering processes.

In some disclosures, the disclosed examples are based on the observation that during a process for engineering cells, such as a manufacturing process for generating compositions containing recombinant receptor-expressing T cells for cell therapy, many cells express or upregulate markers that are associated with stimulation or activation of T cells, including CD25 and CD69 following stimulation, such as incubation of cells in the presence of anti-CD3/anti-CD8 antibodies. In some disclosures, only a subset of cells are observed to enter the cell cycle, as shown based on expression of Ki-67. In some cases disclosed herein, Ki-67+ cells primarily include cells expressing CD27 and CD28, whereas Ki67- populations were observed to be enriched for CD57+ cells and exhibited CD27-CD28- phenotypes. In some disclosures, CD57+ cells were observed herein to exhibit phenotypes associated with stimulation or activation, and persisted throughout early stage of the manufacturing process. In some disclosures, the frequency of CD57+ T cells decreased after approximately 48 hours after stimulation, which typically which coincided with T-cell expansion and increased viability. In some disclosures, particular types of cells, such as CD57+ cells (e.g. CD57+ T cells), exhibited low or no expansion during stimulation or cultivation, while continuing to use growth factors and/or activation reagents, resulting in process and product heterogeneity.

In some disclosures, it was observed that when CD57+ cells (e.g. CD57+ T cells) were selected and combined with CD57- cells (e.g. CD57+ T cells) at various ratios prior to stimulation, the frequency of CD57+ cells in the cell composition prior to stimulation (e.g, input composition) was associated with longer process duration. In some disclosures, it was also observed that cell compositions, such as compositions containing CAR+ T cells, did not expand or proliferate when 95% or more, such as 100% of the T cells in the composition were CD57+ T cells.

In some disclosures, while CD27+ T cells can contribute to expanding cells during a manufacturing process for generating engineered T cells, CD57+ cells e.g. CD57+ T cells) in general did not expand and were observed to contribute minimally to the cells in the engineered cell composition (e.g., cell composition for administration). Thus, the presence of CD57+ T cells can impact the manufacturing process, and also was observed to contribute to variability in the process, e.g., during cultivation for expansion, and other cell composition attributes. In some cases, as disclosed herein, selective depletion of CD57+ T cells in the beginning of a manufacturing process, e.g., prior to stimulation of cells, can improve the consistency, quality and potency of the engineered cell composition.

Thus, in some disclosures, the disclosed methods can decrease process duration for generating a cell therapy and thus, in certain disclosures, improve consistency of manufacturing schedules. Further, in some disclosures, the speed and efficiency of the disclosed methods for generating engineered cells for cell therapy allow for easier planning and coordination of cell therapy treatments, such as autologous therapy, to a broader population of subjects than what may be possible by some alternative methods.

In certain disclosures, the disclosed methods successfully remove at least a portion of non-proliferative cells at the initiation of process generating cells useful for a cell therapy, e.g., populations or compositions of engineered T cells. In some disclosures, this is achieved through selecting out CD57+ cells (e.g. CD57+ T cells) prior to the initiation or such processes, or by screening to insure that only cell compositions or population having no or low CD57+ T cell content are used for such processes, improve the success the processes, e.g., the rate or frequency of successfully generating cell population suitable for use in a cell therapy. In some disclosures, the disclosed methods decrease the required duration and number of doublings of the cells, e.g., during proliferation, cultivation, or expansion, to harvesting to yield a requisite number of T cells for use as a cell therapy. Thus, without wishing to be bound by theory, some disclosures contemplate that the disclosed methods increase or verify a sufficient number of incoming donor cells that exhibit improved proliferative capacity.

In some disclosures, the cell therapies generated from populations of enriched CD57-T cells contain T cells with a lesser degree of differentiation than cell therapies generated from alternative processes. In certain disclosures, the reduced cell differentiation of the cell therapies improves the consistency among the cell therapies generated by the disclosed processes (e.g., as compared to alternative processes, e.g., cell therapies that are generated from populations of cells containing variable amount of CD57+ T cells. In certain disclosures, the reduced cell differentiation of the cell therapies improves the product quality profiles of the cell therapies.

Particular disclosurescontemplate that CD57 is expressed by NK and NKT cells in addition to T cells, all of which may be present in a biological sample, e.g., leukapheresis material. Thus, in some disclosures, negative selection for CD57+ cells (e.g. CD57+ T cells) reduces residual non-T cells and improves T cell purity. Thus, the disclosed methods increase the purity of populations of T cells that are processed, such as by stimulation, transduction, or expansion, as well as the T cell purity of resulting cell therapies.

The section headings used herein are for organizational purposes only and are not to be construed as limiting the subject matter described.

## I. POPULATIONS OF ENRICHED CD57- T CELLS

In certain examples, disclosed herein are populations of enriched CD57- T cells (also referred to herein as CD57- T cell populations, compositions of enriched CD57- T cells, or CD57- T cell compositions). In certain disclosures, the disclosed populations of enriched CD57-T cells are used in connection with methods for stimulating, activating, engineering, transducing, cultivating, or expanding T cells, e.g., T cells of or originating from a population of enriched CD57- T cells. In some disclosures, the populations of enriched CD57- T cells result from or are products of isolation, selection, or enrichment, e.g., of a biological sample, such as a biological sample containing one or more immune cells. In certain disclosures, the population of enriched CD57- T cells is or includes viable T cells, CD3+ T cells, CD4+ T cells, and/or CD8+ T cells. In particular disclosures, the cells of the population of enriched CD57- T cells are or include viable T cells, CD3+ T cells, CD4+ T cells, and/or CD8+ T cells or a combination of any of the foregoing. In various disclosures, the cells of the population of enriched CD57- T cells are or include viable CD57- T cells, CD57- CD3+ T cells, CD57- CD4+ T cells, CD57-CD8+ T cells, or a combination of any of the foregoing.

Particular disclosures contemplate that the amount of CD57 expression, e.g., amount of CD57+ T cells, in a sample, population, or composition containing cells may be measured by any suitable known means. In some disclosures, CD57 expression is measured in a sample, population, or composition to measure, assess, or determine the amount, frequency, or percentage of CD57+ cells, e.g., CD57+ T cells in the sample, population, or composition. In certain disclosures, CD57 expression is measured in a sample, population, or composition to measure, assess, or determine the amount, frequency, or percentage of CD57+ cells, e.g., CD57+ T cells in the sample, population, or composition.

In some disclosures, cell compositions having a higher percentage of CD57+ cells can result in a lower percentage of cells capable of proliferative expansion. In some cases disclosed herein, an engineered cell composition with a high percentage of CD57+ cells is associated with a reduced proliferative capacity and may result in prolonged process times, higher doublings to achieve threshold cell numbers, increased cellular differentiation and/or failure to meet a harvest criterion in a manufacturing process for producing an engineered T cell composition for cell therapy.

Also disclosed in some examples are methods for identifying a population of cells capable of expansion, the method including measuring the frequency of CD57+ cells in the population, wherein the population of cells is identified as capable of expansion if frequency of CD57+ cells are below a threshold frequency. In some of any such disclosures, the threshold frequency is a percentage that is less than at or about 35%, 30%, 20%, 10%, 5%, 1%, or 0.1%. In some of any such disclosures, a population that is capable of expansion expands at least at or about 2-fold, 4-fold, 8-fold, or 16-fold within 4, 5, 6, 7 or 8 days of cultivation under conditions that promote proliferation or expansion.

Also disclosed in some casesare methods for determining the capacity of expansion of a population of T cells, the method including measuring a value of a trait associated with CD57 expression in a population of T cells, wherein the if a population of T cells is determined as capable of expansion if the value of the trait is less than at or about a threshold value of the trait.

In some disclosures, the threshold the threshold value: i) is at, at about, or within 25%, within 20%, within 15%, within 10%, or within 5% below a mean or median measurement of the trait associated with CD57 expression, and/or is below one standard deviation less than at or about the mean or median measurement, in a plurality of reference T cell populations; ii) is below a lowest measurement of the trait associated with CD57 expression, optionally within 50%, within 25%, within 20%, within 15%, within 10%, or within 5% below the lowest measurement, in a population from among a plurality of reference T cell populations; iii) is below a mean or median measurement of the trait associated with CD57 expression calculated from among more than 65%, 75%, 80%, 85% of samples from a plurality of reference T cell compositions; wherein the plurality of reference T cell populations are a plurality of populations that did not expand when cultivated under conditions that promote proliferation or expansion of T cells, optionally wherein the cells did not expand by at least at or about 2-fold, 4-fold, 8-fold, or 16-fold within 4, 5, 6, 7 or 8 days of cultivation.

In some disclosures, the trait is a level or amount of CD57 polypeptide expressed in the total T cells, CD4+ T cells, or CD8+ T cells. In some disclosures, the trait is a frequency, percentage, or amount of CD57+ T cells, CD57+CD4+ T cells, or CD57+CD8+ T cells present in the cell population. In some disclosures, the trait is a level or amount of CD57 polypeptide expressed in the CD3+ T cells. In some disclosures, the trait is a frequency, percentage, or amount of CD57+CD3+ T cells T cells present in the cell population. In some disclosures, the trait is a level or amount of CD57 mRNA present in the T cells in the cell population. In some disclosures, the trait is a level or amount of chromatin accessibility of the gene encoding CD57 (B3GAT1).

In some disclosures, wherein the method further includes measuring a second value of second a trait associated with the expression of one or more second gene products in a population of T cells, wherein the population is capable of expanding if the value of the trait is less than at or about the threshold value of the trait and if the second value of the second trait is greater than at or about a second threshold of the second trait.

In some disclosures, the second gene product a marker associated with a naïve-like T cell. In some disclosures, the one or more second gene product is selected from CD27, CD28, CCR7, or CD45RA. In some disclosures, the one or more second gene product is CD27 and CD28.

In certain disclosures, negative expression, e.g., negative expression of CD57 or CD57-, is an expression equal to or less than the level of background expression, e.g., as detected using a standard technique, such as a technique involving antibody-staining. In certain disclosures, negative expression is equal to or less than the level of background expression as detected by suitable techniques for assessing protein or gene expression, such as but not limited to immunohistochemistry, immunofluorescence, or flow cytometry based techniques. In some disclosures, positive expression, e.g., of a particular protein, is or includes surface expression of the protein in an amount, level, or concentration above background. In particular disclosures, negative expression, e.g., of a particular protein, is or includes surface expression of the protein in an amount, level, or concentration at or below background.

In certain disclosures, the methods disclosed herein include one or more steps of assessing, measuring, determining, and/or quantifying the expression of one or more proteins or genes (e.g., CD57) in a sample, population, or composition, such as to quantify cells in the sample, composition, or population with positive or negative expression for the protein or gene (e.g., CD57). Such steps may include assessing, measuring, determining, and/or quantifying any suitable trait associated with expression, such as measuring levels of protein, surface protein, mRNA, or gene accessibility, e.g., epigenetic gene accessibility.

In some disclosures, the expression of a protein (e.g., CD57) is or includes assessing, measuring, determining, and/or quantifying a level, amount, or concentration of the protein, or a protein encoded by the gene, expressed on the surface of cells. In particular disclosures, the expression of a protein (e.g., CD57) is assessed by assessing, measuring, determining, and/or quantifying the surface expression of the protein, e.g., the level, amount, or concentration of the protein on the surface of the cells. In particular disclosures, the amount, frequency, or percentage of cells positive for surface expression of the protein, e.g., cells with surfaces having a greater amount, concentration, or density of proteins on the surface that is greater than the background signal of the technique used to measure the surface protein. In particular disclosures, the surface expression of a protein (e.g., CD57) is measured by immunohistochemistry, immunofluorescence, or flow cytometry based techniques. In some disclosures, the amount, frequency, or percentage of cells positive for surface expression of a protein is determined by a suitable known technique such as an immunohistochemistry, immunofluorescence, or flow cytometry based technique.

In particular disclosures, the amount, frequency, or percentage of cells that are negative or positive for protein expression, e.g., surface expression, in the sample, composition, or population is determined by flow cytometry. In some disclosures, the protein is CD3, CD4, CD8, CD25, CD27, CD28, CD57, CCR7, or CD45RA. In particular disclosures, the protein is CD57.

In particular disclosures, the expression of a protein (e.g., CD57) in a sample, population, or composition is or includes any suitable method for assessing, measuring, determining, and/or quantifying the level, amount, or concentration of protein. Such methods include, but are not limited to, detection with immunoassays, nucleic acid-based or protein-based aptamer techniques, HPLC (high precision liquid chromatography), peptide sequencing (such as Edman degradation sequencing or mass spectrometry (such as MS/MS), optionally coupled to HPLC), and microarray adaptations of any of the foregoing (including nucleic acid, antibody or protein-protein (i.e., non- antibody) arrays). In some disclosures, the immunoassay is or includes methods or assays that detect proteins based on an immunological reaction, e.g., by detecting the binding of an antibody or antigen binding antibody fragment to a gene product. Immunoassays include, but are not limited to, quantitative immunocytochemistry or immunohistochemistry, ELISA (including direct, indirect, sandwich, competitive, multiple and portable ELISAs (see, e.g., U.S. Patent No. 7,510,687), western blotting (including one, two or higher dimensional blotting or other chromatographic means, optionally including peptide sequencing), enzyme immunoassay (EIA), RIA (radioimmunoassay), and SPR (surface plasmon resonance).

In certain disclosures, the expression of a protein or its corresponding gene is measured, assessed, or quantified by measuring an mRNA (or cDNA product derived from the mRNA) that encodes the protein (e.g., CD57). In particular disclosures, the amount or level of the mRNA (or corresponding cDNA) is assessed, measured, determined, and/or quantified by any suitable means (PCR), including reverse transcriptase (rt) PCR, droplet digital PCR, real-time and quantitative PCR methods (including, e.g., TAQMAN®, molecular beacon, LIGHTUP™, SCORPION™, SIMPLEPROBES®; see, e.g., U.S. Pat. Nos.5,538,848; 5,925,517; 6,174,670; 6,329,144; 6,326,145 and 6,635,427); northern blotting; Southern blotting, e.g., of reverse transcription products and derivatives; array based methods, including blotted arrays, microarrays, or in situ-synthesized arrays; and sequencing, e.g., sequencing by synthesis, pyrosequencing, dideoxy sequencing, or sequencing by ligation, or any other known assay methods such as discussed in Shendure et al., Nat. Rev. Genet. 5:335-44 (2004) or Nowrousian, Euk. Cell 9(9): 1300-1310 (2010), including such specific platforms as HELICOS®, ROCHE® 454, ILLUMINA®/SOLEXA®, ABI SOLiD®, and POLONATOR® sequencing. In some disclosures, the expression of mRNA is determined by a next generation sequencing method such as RNA sequencing (RNA-Seq). RNA sequencing methods have been adapted for the most common DNA sequencing platforms (HiSeq systems (Illumina), 454 Genome Sequencer FLX System (Roche), Applied Biosystems SOLiD (Life Technologies), IonTorrent (Life Technologies)). These platforms require initial reverse transcription of RNA into cDNA. Conversely, the single molecule sequencer HeliScope (Helicos BioSciences) is able to use RNA as a template for sequencing.

In some disclosures, the expression of a protein or its corresponding gene is or includes an epigenetic analysis of the protein. In some disclosures, a population of cells is assessed for the accessibility of a gene, e.g., the accessibility of B3GAT1 which encodes CD57. The epigenetic analysis may be performed by any suitable known means, including but not limited to Assay for Transposase-Accessible Chromatin using sequencing (ATAC-seq) to examine chromatin accessibility.

In some disclosures, the population of enriched CD57- cells contains, contains about, or contains less than at or about 25%, 20%, 15%, 12%, 10%, 9%, 8%, 7%, 6%, 5%, 4%, 3%, 2%, 1%, 0.5%, 0.1%, 0.01%, or 0.001%, CD57+ T cells. In certain disclosures, the population of enriched CD57- cells is essentially free of CD57+ cells. In certain disclosures, the population of enriched CD57- cells is essentially free of CD57+ cells. In particular disclosures, the population of enriched CD57- cells contains less than at or about 20% CD57+ cells. In certain disclosures, the population of enriched CD57- cells contains less than at or about 10% CD57+ cells. In some disclosures, the population of enriched CD57- cells contains less than at or about 5% CD57+ cells. In various disclosures, the population of enriched CD57- cells contains less than at or about 1%, 0.1%, or 0.01% CD57+ cells.

In certain disclosures, the population of enriched CD57- cells contains, contains about, or contains at least at or about 60%, 65%, 70%, 75%, 80%, 85%, 90%, 95%, 97%, 98%, 99%, 99.5%, 99.9%, 99.99%, or 100% or about 100% CD57- T cells. In certain disclosures, the population of enriched CD57- T cells contains at least at or about 80% CD57- T cells. In certain disclosures, the population of enriched CD57- T cells contains at least at or about 95% CD57- T cells. In various disclosures, the population of enriched CD57- T cells contains at least at or about 99%, 99.9%, or 99.99% CD57- T cells. In particular disclosures, all or essentially all of the cells of the population of enriched CD57- T cells are CD57- T cells. In some disclosures, the population of enriched CD57- cells contains, contains about, or contains at least at or about 60%, 65%, 70%, 75%, 80%, 85%, 90%, 95%, 97%, 98%, 99%, 99.5%, 99.9%, 99.99%, or 100% or about 100% CD57-CD3+ T cells. In certain disclosures, the population of enriched CD57- T cells contains at least at or about 80% CD57- CD3+ T cells. In certain disclosures, the population of enriched CD57- T cells contains at least at or about 95% CD57- CD3+ T cells. In various disclosures, the population of enriched CD57- T cells contains at least at or about 99%, 99.9%, or 99.99% CD57- CD3+ T cells. In particular disclosures, all or essentially all of the cells of the population of enriched CD57- T cells are CD57- CD3+ T cells.

In particular disclosures, the cells of the population of enriched CD57- T cells are or include viable cells. In some disclosures, cell viability is assessed with an assay that may include, but is not limited to, dye uptake assays (e.g., calcein AM assays), XTT cell viability assays, and dye exclusion assays (e.g., trypan blue, Eosin, or propidium dye exclusion assays). In particular disclosures, a viable cell has negative expression of one or more apoptotic markers, e.g., Annexin V or active Caspase 3. In some disclosures, the viable cell is negative for the expression of one or more apoptosis marker that may include, but are not limited to, a caspase or an active caspase, e.g., caspase 2, caspase 3, caspase 6, caspase 7, caspase 8, caspase 9, or caspase 10, Bcl-2 family members, e.g., Bax, Bad, and Bid, Annexin V, or TUNEL staining. In particular disclosures, the viable cells are active caspase 3 negative. In certain disclosures, the viable cells are Annexin V negative. In certain disclosures, at least at or about 60%, at least at or about 65%, at least at or about 70%, at least at or about 75%, at least at or about 80%, at least at or about 85%, at least at or about 90%, at least at or about 95%, at least at or about 97%, at least at or about 99%, at least at or about 99.5%, at least at or about 99.9%, or 100% or about 100% of the cells of the population of enriched CD57- T cells are viable cells. In some disclosures, the viable cells are or include viable CD3+, viable CD4+, viable CD8+, viable CD57-, viable CD57- CD3+, viable CD57-CD4+, or viable CD57-CD8+ T cells, or a combination of any of the foregoing. In some disclosures, the viable cells are active caspase 3 negative. In particular disclosures, the viable cells are Annexin V negative.

In certain disclosures, the population of enriched CD57- cells contains, contains about, or contains at least at or about 60%, 65%, 70%, 75%, 80%, 85%, 90%, 95%, 97%, 98%, 99%, 99.5%, 99.9%, 99.99%, or 100% or about 100% CD57- CD4+ T cells. In certain disclosures, the population of enriched CD57- T cells contains at least at or about 80% CD57-CD4+ T cells. In certain disclosures, the population of enriched CD57- T cells contains at least at or about 90% CD57- T cells. In various disclosures, the population of enriched CD57- T cells contains at least at or about 95% CD57-CD4+ T cells. In particular disclosures, all or essentially all of the cells of the population of enriched CD57- T cells are CD57- CD4+ T cells. In some disclosures, the population of enriched CD57- cells contains, contains about, or contains at least at or about 60%, 65%, 70%, 75%, 80%, 85%, 90%, 95%, 97%, 98%, 99%, 99.5%, 99.9%, 99.99%, or 100% or about 100% CD57-CD8+ T cells. In certain disclosures, the population of enriched CD57- T cells contains at least at or about 80% CD57- CD8+ T cells. In certain disclosures, the population of enriched CD57- T cells contains at least at or about 90% CD57- CD8+ T cells. In various disclosures, the population of enriched CD57- T cells contains at least at or about 95%, CD57- CD8+ T cells. In particular disclosures, all or essentially all of the cells of the population of enriched CD57- T cells are CD57- CD8+ T cells. In some disclosures, the population of enriched CD57- cells contains, contains about, or contains at least at or about 60%, 65%, 70%, 75%, 80%, 85%, 90%, 95%, 97%, 98%, 99%, 99.5%, 99.9%, 99.99%, or 100% or about 100% CD57-CD3+ T cells. In certain disclosures, the population of enriched CD57- T cells contains at least at or about 80% CD57- CD3+ T cells. In certain disclosures, the population of enriched CD57- T cells contains at least at or about 90% CD57-CD3+ T cells. In various disclosures, the population of enriched CD57- T cells contains at least at or about 95%, CD57- CD3+ T cells. In particular disclosures, all or essentially all of the cells of the population of enriched CD57- T cells are CD57- CD3+ T cells.

In particular disclosures, a frequency of the cells of the population of enriched CD57-cells are naïve-like cells. In some disclosures, a naïve-like T cell is a T cell that is positive for the expression of one or more markers that indicate that the cell is naive and/or is a naïve-like cell. In certain disclosures, a naïve-like T cell is a cell that is positive for the expression of a marker that is associated with a naive or naïve-like state in T cells. In particular disclosures, a naïve-like T cell is a T cell that is negative for the expression of one or more markers that indicates that the cell is not naive and/or is a not a naïve-like cell. In certain disclosures, a non-naive or non-naïve-like state in a T cells includes, for example but not limited to, effector T ($T_{EFF}$) cells, memory T cells, central memory T cells ($T_{CM}$), effector memory T ($T_{EM}$) cells, and combinations thereof.

In some disclosures, a naive-like T cell is positive for the expression of at least one or more markers that indicate that the cell is naive and/or is a naïve-like cell, and/or is associated with a naive or naïve-like state in T cells. In some disclosures, the markers are expressed on the cell surface. In certain disclosures, the naïve-like T cell is negative for the expression of at least one or more markers that indicate that the cell is non-naive and/or is a non-naïve-like cell, and/or is associated with a non-naive or non-naïve-like state in T cells.

Markers that indicate that the T cell is naive and/or is a naïve-like T cell, and/or are associated with a naive or naïve-like state in T cells include, but are not limited to, CD27, CD28, CD45RA, CD62L, and/or CCR7. In some disclosures, the naïve-like T cell, e.g., the naïve-like CD4+ and/or CD8+ T cell, is positive for expression of CD27, CD28, CD45RA, and/or CCR7. In certain disclosures, the naïve-like T cell is positive for the surface expression of one or more of CD27, CD28, CD45RA, and/or CCR7. In some disclosures, the naïve-like T cell, e.g., the naïve-like CD4+ and/or CD8+ T cell, is negative for expression of CD62L. In some disclosures, the naïve-like T cell, e.g., the naïve-like CD3+ T cell, is negative for expression of CD62L. In some disclosures, the naïve-like T cell, e.g., the naïve-like CD3+, CD4+, and/or CD8+ T cell, is negative for expression of CD62L.

Markers that indicate that the cell is a non-naive and/or is a non-naïve-like T cell, and/or are associated with a non-naive or non-naïve-like state in T cells include, but are not limited to, CD25, CD45RO, CD56, KLRG1, and/or CD95. In some disclosures, the naïve-like T cell, e.g., a naïve-like CD4+ and/or CD8+ T cell, is negative for expression of CD25, CD45RO, CD56, and/or KLRG1. In particular disclosures, the naïve-like T cell, e.g., a naïve-like CD4+ and/or CD8+ T cell, has low expression of a marker associated with non-naive or non-naïve-like cells. In some disclosures, the naïve-like T cell, e.g., a naïve-like CD3+ T cell, is negative for expression of CD25, CD45RO, CD56, and/or KLRG1. In particular disclosures, the naïve-like T cell, e.g., a naïve-like CD3+ T cell, has low expression of a marker associated with non-naive or non-naïve-like cells. In particular disclosures, the naïve-like T cell has low expression of CD95. In certain disclosures, the naïve-like T cell is negative for the surface expression of one or more of CD25, CD45RO, CD56, and/or KLRG1.

In some disclosures, low expression of a marker associated with non-naïve or non-naïve-like cells is or includes at least 10%, at least 20%, at least 30%, at least 40%, at least 50%, at least 60%, at least 70%, at least 80%, at least 90%, at least 95%, or at least 99% less expression than the expression of the marker in a cell that is a non-naïve-like cells, and/or a cell that is positive for one or more markers that indicate that the cell is a non-naive and/or is a non-naïve-like T cell, and/or are associated with a non-naive or non-naive-like state in T cells. In certain disclosures, low expression of a marker associated with non-naive or non-naïve-like cells is or includes at least 10%, at least 20%, at least 30%, at least 40%, at least 50%, at least 60%, at least 70%, at least 80%, at least 90%, at least 95%, or at least 99% less expression than the expression of the marker in an effector T ($T_{EFF}$) cell, a memory T cell, a central memory T cell ($T_{CM}$), and/or an effector memory T ($T_{EM}$) cell.

In some disclosures, markers that indicate that the cell is a non-naïve and/or is a non-naïve-like T cell, and/or are associated with a non-naive or non-naive-like state in T cells include one or more cytokines. For example, in certain disclosures, a non-naive or non-naïve-like T cell is negative for the expression and/or the production of one or more of IL-2, IFN-γ, IL-4, and IL-10. In some disclosures, the one or more cytokines are secreted. In particular disclosures, the one or more cytokines are expressed internally by the non-naïve-like T cells, for example, during or after treatment with an agent that prevents, inhibits, or reduces secretion.

In certain disclosures, a naïve-like T cell, e.g., a naïve-like CD57- T cell, is positive for the expression, e.g., surface expression, of CD45RA and CCR7. In particular disclosures, a naïve-like CD4+ T cell is positive for the expression, e.g., surface expression, of CD45RA and CCR7. In some disclosures, a naive-like CD8+ T cell is positive for the expression, e.g., surface expression, of CD45RA and CCR7. In some disclosures, a naive-like CD3+ T cell is positive for the expression, e.g., surface expression, of CD45RA and CCR7. In particular disclosures, a naive-like T cell is positive for the expression, e.g., surface expression, of CD45RA, CD27, and CCR7 and is negative for the expression, e.g., surface expression of CD45RO. In particular disclosures, a naive-like CD4+ T cell is positive for the expression, e.g., surface expression, of CD45RA, CD27, and CCR7 and is negative for the expression, e.g., surface expression of CD45RO. In some disclosures, a naive-like CD8+ T cell is positive for the expression, e.g., surface expression, of CD45RA, CD27, and CCR7 and is negative for the expression, e.g., surface expression of CD45RO. In some disclosures, a naive-like CD3+ T cell is positive for the expression, e.g., surface expression, of CD45RA, CD27, and CCR7 and is negative for the expression, e.g., surface expression of CD45RO.

In certain disclosures, the population of enriched CD57- T cells contains, contains about, or contains at least at or about 10%, 15%, 20%, 25%, 30%, 35%, 40%, 45%, or 50% T cells that are positive for CD25 expression. In various disclosures, the population of enriched CD57- T cells contains, contains about, or contains at least at or about 10%, 15%, 20%, 25%, 30%, 35%, 40%, 45%, or 50% CD57- CD25+ T cells. In various disclosures, the population of enriched CD57- cells contains between or between about 10% and 60%, 20% and 50%, or 25% and 40% CD25+ T cells, each inclusive. In some disclosures, the population of enriched CD57-cells contains between or between about 10% and 60%, 20% and 50%, or 25% and 40% CD57-CD25+ T cells, each inclusive.

In certain disclosures, the population of enriched CD57- T cells contains, contains about, or contains at least at or about 10%, 15%, 20%, 25%, 30%, 35%, 40%, 45%, or 50% T cells that are positive for CD27 expression. In various disclosures, the population of enriched CD57- T cells contains, contains about, or contains at least at or about 10%, 15%, 20%, 25%, 30%, 35%, 40%, 45%, or 50% CD57- CD27+ T cells. In various disclosures, the population of enriched CD57- cells contains between or between about 10% and 60%, 20% and 50%, or 25% and 40% CD27+ T cells, each inclusive. In some disclosures, the population of enriched CD57-cells contains between or between about 10% and 60%, 20% and 50%, or 25% and 40% CD57-CD27+ T cells, each inclusive. In certain disclosures, the population of enriched CD57- T cells contains at least at or about 25% CD27+ T cells.

In particular disclosures, the population of enriched CD57- T cells contains, contains about, or contains at least at or about 10%, 15%, 20%, 25%, 30%, 35%, 40%, 45%, or 50% T cells that are positive for CD28 expression. In various disclosures, the population of enriched CD57- T cells contains, contains about, or contains at least at or about 5%, 10%, 15%, 20%, 25%, 30%, 35%, 40%, 45%, or 50% CD57- CD28+ T cells. In certain disclosures, the population of enriched CD57- cells contains between or between about at or about 5% and at or about 50%, at or about 5% and at or about 35%, or at or about 10% and at or about 25% CD27+ T cells, each inclusive. In some disclosures, the population of enriched CD57- cells contains between or between at or about 5% and at or about 50%, at or about 5% and at or about 35%, or at or about 10% and at or about 25% CD57- CD27+ T cells, each inclusive. In certain disclosures, the population of enriched CD57- T cells contains at least at or about 25% CD27+ T cells.

In some disclosures, the population of enriched CD57- T cells contains, contains about, or contains at least at or about 10%, 15%, 20%, 25%, 30%, 35%, 40%, 45%, or 50% T cells that are positive for CCR7 expression. In certain disclosures, the population of enriched CD57- T cells contains, contains about, or contains at least at or about 5%, 10%, 15%, 20%, 25%, 30%, 35%, 40%, 45%, or 50% CD57- CCR7+ T cells. In come disclosures, the population of enriched CD57- cells contains between or between about at or about 5% and at or about 50%, at or about 5% and at or about 35%, or at or about 10% and at or about 25% CCR7+T cells, each inclusive. In particular disclosures, the population of enriched CD57- cells contains between or between about at or about 5% and at or about 50%, at or about 5% and at or about 35%, or at or about 10% and at or about 25% CD57- CCR7+ T cells, each inclusive. In some disclosures, the population of enriched CD57- T cells contains at least at or about 25% CCR7+ T cells.

In some disclosures, the population of enriched CD57- T cells contains, contains about, or contains at least at or about 10%, 15%, 20%, 25%, 30%, 35%, 40%, 45%, or 50% T cells that are positive for CD45RA expression. In certain disclosures, the population of enriched CD57- T cells contains, contains about, or contains at least at or about 5%, 10%, 15%, 20%, 25%, 30%, 35%, 40%, 45%, or 50% CD57- CD45RA + T cells. In come disclosures, the population of enriched CD57- cells contains between or between about at or about 5% and at or about 50%, at or about 5% and at or about 35%, or at or about 10% and at or about 25% CD45RA+ T cells, each inclusive. In particular disclosures, the population of enriched CD57-cells contains between or between about at or about 5% and at or about 50%, at or about 5% and at or about 35%, or at or about 10% and at or about 25% CD57- CD45RA + T cells, each inclusive. In some disclosures, the population of enriched CD57- T cells contains at least at or about 25% CD45RA + T cells.

In some disclosures, the frequency of the naïve-like cells in the depleted population is at least at or about 10%, 20%, 30%, 40%, or 50% greater than at or about the frequency of naïve-like cells in the biological sample. In some disclosures, the frequency of one or more of CD25+ T cells, CD27+ T cells, CD28+ T cells, CCR7+ T cells, or CD45RA+ T cells in the depleted population is at least at or about 10%, 20%, 30%, 40%, or 50% greater than at or about the frequency of the respective cells in the biological sample. In some disclosures, the depleted population comprises at least at or about 15%, 20%, 25%, 30%, 35%, or 40% CD27+ T cells. In some disclosures, the depleted population comprises at least at or about 10%, 15%, 20%, 25%, 25%, 30%, 35%, or 40% CD28+ T cells. In some disclosures, the depleted population comprises at least at or about 10%, 15%, 20%, 25%, 30%, 35%, 40%, 50%, 60%, 70% or 80% CD27+CD28+ T cells. In some disclosures, the depleted population comprises at least at or about 70% or 80% CD27+CD28+ T cells. In some disclosures, the depleted population comprises at least at or about 10%, 15%, 20%, or 25% CCR7+ T cells.

## II. SELECTION OF CD57- T CELLS AND/OR DEPLETION OF CD57+ T CELLS

In some disclosures, the population of enriched CD57- T cells is obtained from a biological sample. In particular disclosures, the population of enriched CD57- T cells are selected, isolated, or enriched from a biological sample. In particular disclosures, CD57+ T cells are removed, separated, or depleted from a biological sample. In certain disclosures, at least 25%, 30%, 35%, 40%, 45%, 50%, 55%, 60%, 65%, 70%, 75%, 80%, 85%, 90%, 95%, 99%, or 99.9% CD57+ T cells are removed, separated, or depleted from the biological sample.

In particular disclosures, subsets of cells, e.g., subsets of T cells, are selected, isolated, or enriched from the biological sample prior to selecting, isolating, or enriching CD57-T cells from the biological sample. In some disclosures, subsets of cells, e.g., T cells are selected, isolated, or enriched from the population of enriched CD57- T cells.

In some disclosures, the population of enriched CD57- T cells contains, contains about, or contains less than 50%, 45%, 40%, 35%, 30%, 25%, 20%, 15%, 10%, 5%, 1%, 0.1%, 0.01%, or 0.001% of the CD57+ T cells of the biological sample, e.g., prior the selection, isolation, or enrichment. In particular disclosures, the population of enriched CD57- T cells contains, contains about, or contains less than 20% of the CD57+ T cells of the biological sample. In certain disclosures, the population of enriched CD57- T cells contains, contains about, or contains less than 5% of the CD57+ T cells of the biological sample. In some disclosures, the population of enriched CD57- T cells contains, contains about, or contains less than 1% of the CD57+ T cells of the biological sample e.g., prior the selection, isolation, or enrichment. In various disclosures, the population of enriched CD57- T cells contains, contains about, or contains less than 0.1% of the CD57+ T cells of the biological sample. In particular disclosures, the population of enriched CD57- T cells contains, contains about, or contains less than 0.01% of the CD57+ T cells of the biological sample. In some disclosures, the frequency of the CD57+ T cells in the depleted population is less than at or about 35%, 30%, 20%, 10%, 5%, 1%, or 0.1% of the frequency of CD57+ T cells in the biological sample. In some disclosures, the depleted population comprises less than at or about 3%, less than at or about 2%, less than at or about 1%, less than at or about 0.1%, or less than at or about 0.01% CD57+ T cells. In some disclosures, the depleted population is free or is essentially free of CD57+ T cells.

In particular disclosures, the cells of the population of enriched CD57- T cells are less differentiated than the cells of the biological sample, e.g., prior the selection, isolation, or enrichment. In certain disclosures, the population of enriched CD57- T cells contains a greater frequency of naïve-like cells than the biological sample. In certain disclosures, the population of enriched CD57+ T cells includes, includes about, or includes at least at or about 25%, 30%, 35%, 40%, 45%, 50%, 55%, 60%, 65%, 70%, 75%, 80%, 85%, 90%, 95%, 100%, 125%, 150%, 1-fold, 2-fold, 3-fold, 4-fold, 5-fold, or 10 fold more naïve-like cells than the biological sample e.g., prior the selection, isolation, or enrichment.

In some disclosures, naive-like cells include naive T cells or central memory T cells. In some disclosures, naïve-like cells can include cells positive or expressing high levels of one or more surface markers, e.g., CD28+, CD62L+, CCR7+, CD27+, CD127+, CD4+, CD8+, CD45RA+, and/or CD45RO+ T cells. In some disclosures, the cells are CD27+. In some disclosures, the cells are CD28+. In some disclosures, the cells are CCR7+. In particular disclosures, CCR7 is expressed by naive or naïve-like T cells (e.g. CCR7+CD45RA+ or CCR7+CD27+) and central memory T cells (CCR7+CD45RA-). In certain disclosures, naive-like T cells or the T cells that are surface positive for a marker expressed on naïve-like T cells are CCR7+CD45RA+, where the cells are CD27+ or CD27-. In certain disclosures, naive-like T cells or the T cells that are surface positive for a marker expressed on naïve-like T cells are CD27+CCR7+, where the cells are CD45RA+ or CD45RA-. In certain disclosures, naïve-like T cells or the T cells that are surface positive for a marker expressed on naïve-like T cells are CD62L-CCR7+. In certain disclosures, naïve-like cells include cells at an early stage of differentiation (e.g., cells that are CCR7+CD27+).

In certain disclosures, central memory T cells may include cells in various differentiation states and may be characterized by positive or high expression (e.g., surface expression) of certain cell markers and/or negative or low expression (e.g., surface expression) of other cell markers. In some disclosures, less differentiated cells, e.g., central memory cells, are longer lived and exhaust less rapidly, thereby increasing persistence and durability. In some disclosures, a responder to a cell therapy, such as a CAR-T cell therapy, has increased expression of central memory genes. *See,e.g.,* Fraietta et al. (2018) Nat Med. 24(5):563-571. In some disclosures, central memory T cells are characterized by positive or high expression of CD45RO, CD62L, CCR7, CD28, CD3, and/or CD127. In some disclosures, central memory T cells are characterized by negative or low expression of CD45RA and/or granzyme B. In certain disclosures, central memory T cells or the T cells that are surface positive for a marker expressed on central memory T cells are CCR7+CD45RA-.

In particular disclosures, the population of enriched CD57- T cells includes, includes about, or includes at least at or about 25%, 30%, 35%, 40%, 45%, 50%, 55%, 60%, 65%, 70%, 75%, 80%, 85%, 90%, 95%, 100%, 125%, 150%, 1-fold, 2-fold, 3-fold, 4-fold, 5-fold, or 10 fold more CD27+ T cells than the biological sample e.g., prior the selection, isolation, or enrichment. In particular disclosures, the population of enriched CD57- T cells includes, includes about, or includes at least at or about 25%, 30%, 35%, 40%, 45%, 50%, 55%, 60%, 65%, 70%, 75%, 80%, 85%, 90%, 95%, 100%, 125%, 150%, 1-fold, 2-fold, 3-fold, 4-fold, 5-fold, or 10 fold more CD28+ T cells than the biological sample e.g., prior the selection, isolation, or enrichment. In various disclosures, the population of enriched CD57- T cells includes, includes about, or includes at least at or about 25%, 30%, 35%, 40%, 45%, 50%, 55%, 60%, 65%, 70%, 75%, 80%, 85%, 90%, 95%, 100%, 125%, 150%, 1-fold, 2-fold, 3-fold, 4-fold, 5-fold, or 10 fold more CD25+ T cells than the biological sample e.g., prior the selection, isolation, or enrichment. In certain disclosures, the population of enriched CD57- T cells includes, includes about, or includes at least at or about 25%, 30%, 35%, 40%, 45%, 50%, 55%, 60%, 65%, 70%, 75%, 80%, 85%, 90%, 95%, 100%, 125%, 150%, 1-fold, 2-fold, 3-fold, 4-fold, 5-fold, or 10 fold more CCR7+ T cells than the biological sample e.g., prior the selection, isolation, or enrichment. In certain disclosures, the population of enriched CD57- T cells includes, includes about, or includes at least at or about 25%, 30%, 35%, 40%, 45%, 50%, 55%, 60%, 65%, 70%, 75%, 80%, 85%, 90%, 95%, 100%, 125%, 150%, 1-fold, 2-fold, 3-fold, 4-fold, 5-fold, or 10 fold more CD45RA+ cells than the biological sample e.g., prior the selection, isolation, or enrichment.

In certain disclosures, T cells, e.g., CD3+ T cells, are selected, isolated, or enriched from the biological sample prior to selecting, isolating, or enriching CD57- T cells from the biological sample. In some disclosures, T cells, e.g., CD3+ T cells, are selected, isolated, or enriched from the population of enriched CD57- T cells. In particular disclosures, selecting, isolating or enriching T cells, e.g. CD3+ T cells, involves positive selection of the cells from the sample.

In some disclosures, CD57+ cells are selected, isolated, or enriched from a sample, cell composition, or cell population, thereby producing isolated or selected CD57+ cells and a population of enriched CD57- cells, e.g., T cells. In certain disclosures, CD57+ cells are selected, isolated, or enriched from a biological sample, thereby producing isolated or selected CD57+ cells and a population of enriched CD57- cells, e.g., T cells. In certain disclosures, CD3+T cells are enriched, selected, or isolated from the population of enriched CD57- cells, thereby generating a population of enriched CD57- CD3+ T cells and a non-selected population of enriched CD57- cells. In certain disclosures, CD3+T cells are enriched, selected, or isolated from the non-selected population of enriched CD57- cells, thereby generating a population of enriched CD57-CD3+ T cells. In various disclosures, CD3+ T cells are enriched, selected, or isolated from the population of enriched CD57- cells, thereby generating a population of enriched CD57- CD3+ T cells and a non-selected population enriched for CD57- cells, and then CD4+ or CD8+ T cells are enriched, selected, or isolated from the non-selected population of enriched CD57- cells, thereby generating a population of enriched CD57-CD4+ T cells or CD57-CD8+ T cells.

In certain disclosures, subsets of T cells, e.g., CD4+ or CD8+ T cells, are selected, isolated, or enriched from the biological sample prior to selecting, isolating, or enriching CD57-T cells from the biological sample. In some disclosures, subsets of T cells, e.g., CD4+ or CD8+ T cells, are selected, isolated, or enriched from the population of enriched CD57- T cells. In particular disclosures, the selecting, isolating or enriching a subset of T cells, e.g. CD4+ or CD8+ T cells, involves positive selection of the cells from the sample.

In some disclosures, CD57+ cells are selected, isolated, or enriched from a sample, cell composition, or cell population, thereby producing isolated or selected CD57+ cells and a population of enriched CD57- cells, e.g., T cells. In certain disclosures, CD57+ cells are selected, isolated, or enriched from a biological sample, thereby producing isolated or selected CD57+ cells and a population of enriched CD57- cells, e.g., T cells. In particular disclosures, CD4+ T cells are enriched, selected, or isolated from the population of enriched CD57- cells, thereby generating a population of enriched CD57- CD4+ T cells and a non-selected population enriched for CD57- cells. In certain disclosures, CD8+ T cells are enriched, selected, or isolated from the population of enriched CD57- cells, thereby generating a population of enriched CD57-CD8+ T cells and a non-selected population of enriched CD57- cells. In certain disclosures, CD8+ T cells are enriched, selected, or isolated from the non-selected population of enriched CD57- cells, thereby generating a population of enriched CD57-CD8+ T cells. In particular disclosures, CD4+ T cells are enriched, selected, or isolated from the non-selected population of enriched CD57- cells, thereby generating a population of enriched CD57-CD4+ T cells.

In particular disclosures, CD4+ T cells are enriched, selected, or isolated from the population of enriched CD57- cells, thereby generating a population of enriched CD57- CD4+ T cells and a non-selected population enriched for CD57- cells, and then CD8+ T cells are enriched, selected, or isolated from the non-selected population of enriched CD57- cells, thereby generating a population of enriched CD57-CD8+ T cells. In various disclosures, CD4+ T cells are enriched, selected, or isolated from the population of enriched CD57- cells, thereby generating a population of enriched CD57- CD4+ T cells and a non-selected population enriched for CD57- cells, and then CD8+ T cells are enriched, selected, or isolated from the non-selected population of enriched CD57- cells, thereby generating a population of enriched CD57-CD8+ T cells.

In particular disclosures, (1) CD4+ T cells are enriched, selected, or isolated from a biological sample, thereby generating a population of enriched CD4+ T cells and a non-selected population enriched for CD4- cells; (2) CD8+ T cells are enriched, selected, or isolated from the non-selected population of enriched CD4- cells, thereby generating a population of enriched CD8+ T cells; and (3) CD57+ T cells are depleted from the enriched CD4+ and CD8+ T cell populations, generating populations of enriched CD57-CD4+ and CD57-CD8+ T cells. In particular disclosures, (1) CD8+ T cells are enriched, selected, or isolated from a biological sample, thereby generating a population of enriched CD8+ T cells and a non-selected population enriched for CD8- cells; (2) CD4+ T cells are enriched, selected, or isolated from the non-selected population of enriched CD4- cells, thereby generating a population of enriched CD4+ T cells; and (3) CD57+ T cells are depleted from the enriched CD4+ and CD8+ T cell populations, generating populations of enriched CD57-CD4+ and CD57-CD8+ T cells.

In particular disclosures, CD4+ T cells are enriched, selected, or isolated from a biological sample, thereby generating an enriched population of CD4+ T cells, and then CD57+ cells are removed from the enriched population of CD4+ T cells, thereby generating a population of enriched CD57-CD4+ T cells. In particular disclosures, CD8+ T cells are enriched, selected, or isolated from a biological sample, thereby generating an enriched population of CD8+ T cells, and then CD57+ cells are removed from the enriched population of CD8+ T cells, thereby generating a population of enriched CD57-CD8+ T cells.

In some disclosures, the one or more populations enriched CD57- T cells are frozen, e.g., cryopreserved and/or cryoprotected, after isolation, selection and/or enrichment. In particular disclosures, a population of enriched CD57-CD4+ T cells are frozen, e.g., cryopreserved and/or cryoprotected, after isolation, selection and/or enrichment. In certain disclosures, a population of enriched CD57- CD8+ T cells are frozen, e.g., cryopreserved and/or cryoprotected, after isolation, selection and/or enrichment. In certain disclosures, a population of enriched CD57- CD3+ T cells are frozen, e.g., cryopreserved and/or cryoprotected, after isolation, selection and/or enrichment. In some disclosures, the one or more populations of enriched T cells are frozen e.g., cryopreserved and/or cryoprotected, prior to any steps of incubating, activating, stimulating, engineering, transducing, transfecting, cultivating, expanding, harvesting, and/or formulating the population of cells. In particular disclosures, a population of enriched CD57-CD4+ T cells are frozen e.g., cryopreserved and/or cryoprotected, prior to any steps of incubating, activating, stimulating, engineering, transducing, transfecting, cultivating, expanding, harvesting, and/or formulating the population of cells. In some disclosures, a population of enriched CD57-CD8+ T cells are frozen e.g., cryopreserved and/or cryoprotected, prior to any steps of incubating, activating, stimulating, engineering, transducing, transfecting, cultivating, expanding, harvesting, and/or formulating the population of cells. In some disclosures, a population of enriched CD57-CD3+ T cells are frozen e.g., cryopreserved and/or cryoprotected, prior to any steps of incubating, activating, stimulating, engineering, transducing, transfecting, cultivating, expanding, harvesting, and/or formulating the population of cells. In particular disclosures, the one or more cryoprotected input compositions are stored, e.g., at or at about -80°C, for between 12 hours and 7 days, between 24 hours and 120 hours, or between 2 days and 5 days. In particular disclosures, the one or more cryoprotected input compositions are stored at or at about -80°C, for an amount of time of less than 10 days, 9 days, 8 days, 7 days, 6 days, or 5 days, 4 days, 3 days, 2 days, or 1 day. In some disclosures, the one or more cryoprotected input compositions are stored at or at about -70°C or -80°C for less than 3 days, such as for about 2 days.

In some disclosures, "depleting" or "removing" when referring to one or more particular cell type or cell population, refers to decreasing the number or percentage of the cell type or population, e.g., compared to the total number of cells in or volume of the composition, or relative to other cell types, such as by negative selection based on markers expressed by the population or cell, or by positive selection based on a marker not present on the cell population or cell to be depleted. In general, the terms depleting or removing does not require complete removal of the cell, cell type, or population from the composition.

In some disclosures, "enriching" when referring to one or more particular cell type or cell population, refers to increasing the number or percentage of the cell type or population, e.g., compared to the total number of cells in or volume of the composition, or relative to other cell types, such as by positive selection based on markers expressed by the population or cell, or by negative selection based on a marker not present on the cell population or cell to be depleted. In general, the term enriching does not require complete removal of other cells, cell type, or populations from the composition and does not require that the cells so enriched be present at or even near 100 % in the enriched composition.

In some disclosures, cell populations or cell compositions obtained from a subject, such as a human subject, for cell therapy, e.g., adoptive cell therapy, can exhibit low growth or slow growth, such that they do not reach (e.g., no growth) the threshold for harvesting cells (e.g., harvest criterion) for generating a therapeutic composition, or do not reach the threshold for harvesting cells (e.g., harvest criterion) for generating a therapeutic composition within a specific period of time (e.g., slow growth). In some disclosures, some of such cell populations can contain a high frequency of CD57+ cells, such as a frequency of CD57+ above a threshold value. In other disclosures, cell populations or cell compositions obtained from a subject, such as a human subject, for cell therapy, e.g., adoptive cell therapy, can exhibit improved growth compared to the populations exhibiting no growth or slow growth. In some disclosures, such cell populations or cell compositions can contain a low frequency of CD57+ cells, such as a frequency of CD57+ cells less than a threshold value. In some disclosures, cell populations or cell compositions that exhibit improved growth can exhibit phenotypes or express markers associated with naïve-like or central memory-like phenotypes, such as CD27+, CD28+ and/or CCR7+. In some disclosures, the disclosed methods are based on observations that there is variability or heterogeneity in CD57+ T cell expression among T cells in a biological sample (e.g. leukapheresis or apheresis sample) from human subjects, which, in some disclosures, can results in variability in the phenotype and function of engineered T cell compositions produced for use in adoptive cell therapy from a plurality of different subjects, even using the same manufacturing process. In particular disclosures, the disclosed methods control for or reduce such variability by selecting, isolating, or enriching CD57- T cells from a biological sample, such as by removing, separating, or depleting CD57+ T cells from the biological sample. Such cells can then be used in processes to engineer or manufacture cells for cell therapy to minimize variability among products, while also improving particular produt attributes and features such as the ability to expand and persist upon administration to a subject.

## A. Samples and Cell Preparation

In particular disclosures, the disclosed methods are used in connection with isolating, selecting, or enriching cells from a biological sample to generate one or more populations of enriched cells, e.g., CD57- T cells. In some disclosures, the disclosed methods include isolation of cells or populations thereof from biological samples, such as those obtained from or derived from a subject, such as one having a particular disease or condition or in need of a cell therapy or to which cell therapy will be administered. In some disclosures, the subject is a human, such as a subject who is a patient in need of a particular therapeutic intervention, such as the adoptive cell therapy for which cells are being isolated, processed, and/or engineered. Accordingly, the cells in some disclosures are primary cells, e.g., primary human cells. The samples include tissue, fluid, and other samples taken directly from the subject. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some disclosures, the sample is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources. In some disclosures, the sample is or comprises a whole blood sample, a buffy coat sample, a peripheral blood mononuclear cells (PBMC) sample, an unfractionated T cell sample, a lymphocyte sample, a white blood cell sample, an apheresis product, or a leukapheresis product.

In some examples disclosed herein, cells from the circulating blood of a subject are obtained, e.g., by apheresis or leukapheresis. The samples, in some disclosures, contain lymphocytes, including T cells, monocytes, granulocytes, B cells, other nucleated white blood cells, red blood cells, and/or platelets, and in some disclosurescontains cells other than red blood cells and platelets.

In some disclosures, the blood cells collected from the subject are washed, e.g., to remove the plasma fraction and to place the cells in an appropriate buffer or media for subsequent processing steps. In some disclosures, the cells are washed with phosphate buffered saline (PBS). In some disclosures, the wash solution lacks calcium and/or magnesium and/or many or all divalent cations. In some disclosures, a washing step is accomplished a semi-automated "flow-through" centrifuge (for example, the Cobe 2991 cell processor, Baxter) according to the manufacturer's instructions. In some disclosures, a washing step is accomplished by tangential flow filtration (TFF) according to the manufacturer's instructions. In some disclosures, the cells are resuspended in a variety of biocompatible buffers after washing, such as, for example, Ca++/Mg++ free PBS. In certain disclosures, components of a blood cell sample are removed and the cells directly resuspended in culture media.

In some disclosures, the sample containing cells (e.g., an apheresis product or a leukapheresis product) is washed in order to remove one or more anti-coagulants, such as heparin, added during apheresis or leukapheresis.

In some disclosures, the sample containing cells (e.g., a whole blood sample, a buffy coat sample, a peripheral blood mononuclear cells (PBMC) sample, an unfractionated T cell sample, a lymphocyte sample, a white blood cell sample, an apheresis product, or a leukapheresis product) is cryopreserved and/or cryoprotected (e.g., frozen) and then thawed and optionally washed prior to any steps for isolating, selecting, activating, stimulating, engineering, transducing, transfecting, incubating, culturing, harvesting, formulating a population of the cells, and/or administering the formulated cell population to a subject.

In some disclosures, a sample containing autologous Peripheral Blood Mononuclear Cells (PBMCs) from a subject is collected in a method suitable to ensure appropriate quality for manufacturing. In one disclosure, the sample containing PBMCs is derived from fractionated whole blood. In some disclosures, whole blood from a subject is fractionated by leukapheresis using a centrifugal force and making use of the density differences between cellular phenotypes, when autologous mononuclear cells (MNCs) are preferentially enriched while other cellular phenotypes, such as red blood cells, are reduced in the collected cell composition. In some disclosures, autologous plasma is concurrently collected during the MNC collection, which in some disclosurescan allow for extended leukapheresis product stability. In one disclosure, the autologous plasma is added to the leukapheresis product to improve the buffering capacity of the leukapheresis product matrix. In some disclosures, a total volume of whole blood processed in order to generate the leukapheresis product is or is about 2L, 4L, 6L, 8L, 10L, 12L, 14L, 16L, 18L, or 20L, or is any value between any of the foregoing. In some disclosures, the volume of autologous plasma collected is or is about 10mL, 50mL, 100mL, 150mL, 200mL, 250mL, or 300mL, or more, or is a volume between any of the foregoing. In some disclosures, the leukapheresis product is subjected to a procedure, e.g., washing and formulation for in-process cryopreservation, within about 48 hours of the leukapheresis collection completion. In some disclosures, the leukapheresis product is subjected to one or more wash steps, e.g., within about 2 hours, 6 hours, 12 hours, 18 hours, 24 hours, 36 hours, or 48 hours of the leukapheresis collection completion. In some disclosures, the one or more wash step removes the anticoagulant during leukapheresis collection, cellular waste that may have accumulated in the leukapheresis product, residual platelets and/or cellular debris. In some disclosures, one or more buffer exchange is performed during the one or more wash step.

In particular disclosures, an apheresis product or a leukapheresis product is cryopreserved and/or cryoprotected (e.g., frozen) and then thawed before being subject to a cell enrichment, selection or isolation step (e.g., a T cell selection or isolation step) as described *infra*. In some disclosures, after a cryopreserved and/or cryoprotected apheresis product or leukapheresis product is subject to a T cell selection or isolation step, no additional cryopreservation and/or cryoprotection step is performed during or between any of the subsequent steps, such as the steps of activating, stimulating, engineering, transducing, transfecting, incubating, culturing, harvesting, formulating a population of the cells, and/or administering the formulated cell population to a subject. For example, T cells selected from a thawed cryopreserved and/or cryoprotected apheresis product or leukapheresis product are not again cryopreserved and/or cryoprotected before being thawed and optionally washed for a downstream process, such as T cell activation/stimulation or transduction.

In particular disclosures, an apheresis product or a leukapheresis product is cryopreserved and/or cryoprotected (e.g., frozen) at a density of, of about, or at least $5 \times 10^6$ cells/mL, $10 \times 10^6$ cells/mL, $20 \times 10^6$ cells/mL, $30 \times 10^6$ cells/mL, $40 \times 10^6$ cells/mL, $50 \times 10^6$ cells/mL, $60 \times 10^6$ cells/mL, $70 \times 10^6$ cells/mL, $80 \times 10^6$ cells/mL, $90 \times 10^6$ cells/mL, $100 \times 10^6$ cells/mL, $110 \times 10^6$ cells/mL, $120 \times 10^6$ cells/mL, $130 \times 10^6$ cells/mL, $140 \times 10^6$ cells/mL, or $150 \times 10^6$ cells/mL, or any value between any of the foregoing, in a cryopreservation solution or buffer. In some disclosures, the cryopreservation solution or buffer is or contains, for example, a DMSO solution optionally comprising human serum albumin (HSA), or other suitable cell freezing media.

In particular disclosures, the cryopreserved and/or cryoprotected apheresis product or leukapheresis product is banked (e.g., without T cell selection before freezing the sample), which, in some disclosures, can allow more flexibility for subsequent manufacturing steps. In some disclosures, the cryopreserved and/or cryoprotected apheresis product or leukapheresis product is aliquoted into multiple cryopreservation container such as bags, which can each invidually or in combination be used in processing of the product. For example, when the total number of viable cells in the apheresis product or leukapheresis product is less than $15 \times 10^9$ cells, the cryopreserved and/or cryoprotected apheresis product or leukapheresis product is aliquoted into four cryopreservation container such as bags. In some disclosures, when the total number of viable cells in the apheresis product or leukapheresis product is $15\text{-}30 \times 10^9$ cells, the cryopreserved and/or cryoprotected apheresis product or leukapheresis product is aliquoted into eight cryopreservation container such as bags.

In one disclosure, banking cells before selection increases cell yields for a downstream process, and banking cells earlier may mean they are healthier and may be easier to meet manufacturing success criteria. In another disclosure, once thawed, the cryopreserved and/or cryoprotected apheresis product or leukapheresis product can be subject to one or more different selection methods. Advantages of this approach are, among other things, to enhance the availability, efficacy, and/or other aspects of cells of a cell therapy for treatment of a disease or condition of a subject, such as in the donor of the sample and/or another recipient.

In some disclosures, the sample (e.g. apheresis or leukapheresis sample) is collected and cryopreserved and/or cryoprotected prior to or without prior cell selection (e.g., without prior T cell selection, such as selection by chromatography), at a time after the donor is diagnosed with a disease or condition. In some disclosures, the time of cryopreservation also is before the donor has received one or more of the following: any initial treatment for the disease or condition, any targeted treatment or any treatment labeled for treatment for the disease or condition, or any treatment other than radiation and/or chemotherapy. In some disclosures, the sample is collected after a first relapse of a disease following initial treatment for the disease, and before the donor or subject receives subsequent treatment for the disease. The initial and/or subsequent treatments may be a therapy other than a cell therapy. In some disclosures, the collected cells may be used in a cell therapy following initial and/or subsequent treatments. In one disclosure, the cryopreserved and/or cryoprotected sample without prior cell selection may help reduce up-front costs, such as those associated with non-treatment patients in a randomized clinic trial who may crossover and require treatment later.

In some disclosures, the sample (e.g. apheresis or leukapheresis sample) is collected and cryopreserved and/or cryoprotected prior to or without prior cell selection (e.g., without prior T cell selection, such as selection by chromatography), at a time after a second relapse of a disease following a second line of treatment for the disease, and before the donor or subject receives subsequent treatment for the disease. In some disclosures, patients are identified as being likely to relapse after a second line of treatment, for example, by assessing certain risk factors. In some disclosures, the risk factors are based on disease type and/or genetics, such as double-hit lymphoma, primary refractory cancer, or activated B-cell lymphoma. In some disclosures, the risk factors are based on clinical presentation, such as early relapse after first-line treatment, or other poor prognostic indicators after treatment (e.g., IPI (International Prognostic Index) > 2).

In some disclosures, the sample (e.g. apheresis or leukapheresis sample) is collected and cryopreserved and/or cryoprotected prior to or without prior cell selection (e.g., without prior T cell selection, such as selection by chromatography), at a time before the donor or subject is diagnosed with a disease. In some disclosures, the donor or subject may be determined to be at risk for developing a disease. In some disclosures, the donor or subject may be a healthy subject. In certain cases disclosed herein, the donor or subject may elect to bank or store cells without being deemed at risk for developing a disease or being diagnosed with a disease in the event that cell therapy is required at a later stage in life. In some disclosures, a donor or subject may be deemed at risk for developing a disease based on factors such as genetic mutations, genetic abnormalities, genetic disruptions, family history, protein abnormalities (such as deficiencies with protein production and/or processing), and lifestyle choices that may increase the risk of developing a disease. In some disclosures, the cells are collected as a prophylactic.

In some disclosures, the cryopreserved and/or cryoprotected sample of cells (e.g. apheresis or leukapheresis sample), such as a sample of cells that has not been subjected to a prior cell selection (e.g., without prior T cell selection, such as selection by chromatography) is stored, or banked, for a period of time greater than or equal to 12 hours, 24 hours, 36 hours, or 48 hours, or greater than or equal to 0.5 days, one day, 1.5 days, or two days. In some disclosures, the sample is stored or banked for a period of time greater than or equal to 1 week, 2 weeks, 3 weeks, or 4 weeks. In some disclosures, the sample is placed into long-term storage or long-term banking. In some disclosures, the sample is stored for a period of time greater than or equal to 1 month, 2 months, 3 months, 4 months, 5 months, 6 months, 7 months, 8 months, 9 months, 10 months, 1 1 months, 1 year, 2 years, 3 years, 4 years, 5 years, 6 years, 7 years, 8 years, 9 years, 10 years, 1 1 years, 12 years, 13 years, 14 years, 15 years, 16 years, 17 years, 18 years, 19 years, 20 years, 25 years, 30 years, 35 years, 40 years, or more.

In some disclosures, an apheresis or leukapheresis sample taken from a donor is shipped in a cooled environment to a storage or processing facility, and/or cryogenically stored at the storage facility or processed at the processing facility. In some disclosures, before shipping, the sample is processed, for example, by selecting T cells, such as CD3+ T cells, CD4+ T cells, and/or CD8+ T cells. In some disclosures, such processing is performed after shipping and before cryogenically storing the sample. In some disclosures, the processing is performed after thawing the sample following cryogenically storage.

By allowing donors to store their cells at a stage when the donors, and thus their cells, have not undergone extensive treatment for a disease and/or prior to contracting of a disease or condition or diagnosis thereof, such cells may have certain advantages for use in cell therapy compared to cells harvested after one or after multiple rounds of treatment. For example, cells harvested before one or more rounds of treatment may be healthier, may exhibit higher levels of certain cellular activities, may grow more rapidly, and/or may be more receptive to genetic manipulation than cells that have undergone several rounds of treatment. Another example of an advantage according to instances disclosed herein may include convenience. For example, by collecting, optionally processing, and storing a donor's cells before they are needed for cell therapy, the cells would be readily available if and when a recipient later needs them. This could increase apheresis lab capacity, providing technicians with greater flexibility for scheduling the apheresis collection process.

Exemplary methods and systems for cryogenic storage and processing of cells from a sample, such as an apheresis sample, can include those described in WO2018170188. In some disclosures, the method and systems involve collecting apheresis before the patient needs cell therapy, and then subjecting the apheresis sample to cryopreservation for later use in a process for engineering the cells, e.g. T cells, with a recombinant receptor (e.g. CAR). In some cases disclosed herein, such processes can include those described herein. In some disclosures, an apheresis sample is collected from a subject and cryopreserved prior to subsequent T cell selection, activation, stimulation, engineering, transduction, transfection, incubation, culturing, harvest, formulation of a population of the cells, and/or administration of the formulated cell population to a subject. In such examples disclosed herein, the cryopreserved apheresis sample is thawed prior to subjecting the sample to one or more selection steps, such as any as described herein.

In some disclosures, the cryopreserved and/or cryoprotected sample of cells (e.g. apheresis or leukapheresis sample), such as a sample of cells that has not been subject to a prior cell selection (e.g., without prior T cell selection, such as selection by chromatography) is thawed prior to its use for downstream processes for manufacture of a cell population for cell therapy, for example, a T cell population containing CAR+ T cells. In some disclosures, such a cryopreserved and/or cryoprotected sample of cells (e.g. apheresis or leukapheresis sample) is used in connection with the process disclosed herein for engineered a T cell therapy, such as a CAR+ T cell therapy. In particular examples disclosed herein, no further step of cryopreservation is carried out prior to or during the harvest/formuation steps.

n some disclosures, a cryopreserved and/or cryoprotected apheresis product or leukapheresis product is thawed. In some disclosures, the thawed cell composition is subjected to dilution (e.g., with a serum-free medium) and/or wash (e.g., with a serum-free medium), which in some cases disclosed herein can remove or reduce unwanted or undesired components. In some cases disclosed herein, the dilution and/or wash removes or reduces the presence of a cryoprotectant, e.g. DMSO, contained in the thawed sample, which otherwise may negatively impact cellular viability, yield, recovery upon extended room temperature exposure. In some disclosures, the dilution and/or wash allows media exchange of a thawed cryopreserved product into a serum-free medium, such as in PCT/US2018/064627.

In some disclosures, the serum-free medium comprises a basal medium (e.g.OpTmizer™ T-Cell Expansion Basal Medium (ThermoFisher), supplemented with one or more supplement. In some disclosures, the one or more supplement is serum-free. In some disclosures, the serum-free medium comprises a basal medium supplemented with one or more additional components for the maintenance, expansion, and/or activation of a cell (e.g., a T cell), such as provided by an additional supplement (e.g. OpTmizer™ T-Cell Expansion Supplement (ThermoFisher)). In some disclosures, the serum-free medium further comprises a serum replacement supplement, for example, an immune cell serum replacement, e.g., ThermoFisher, #A2596101, the CTS™ Immune Cell Serum Replacement, or the immune cell serum replacement described in Smith et al. Clin Transl Immunology. 2015 Jan; 4(1): e31. In some disclosures, the serum-free medium further comprises a free form of an amino acid such as L-glutamine. In some disclosures, the serum-free medium further comprises a dipeptide form of L-glutamine (e.g., L-alanyl-L-glutamine), such as the dipeptide in Glutamax™ (ThermoFisher). In some disclosures, the serum-free medium further comprises one or more recombinant cytokines, such as recombinant human IL-2, recombinant human IL-7, and/or recombinant human IL-15.

**B. T Cell Selection**

In some disclosures, selection, isolation, or enrichment of the cells, e.g., CD57+ or CD57- cells (e.g. CD57+ T cells), includes one or more preparation and/or non-affinity based cell separation steps. In some examples disclosed herein, cells are washed, centrifuged, and/or incubated in the presence of one or more reagents, for example, to remove unwanted components, enrich for desired components, lyse or remove cells sensitive to particular reagents. In some examples disclosed herein, cells are separated based on one or more property, such as density, adherent properties, size, sensitivity and/or resistance to particular components. In some disclosures, the methods include density-based cell separation methods, such as the preparation of white blood cells from peripheral blood by lysing the red blood cells and centrifugation through a Percoll or Ficoll gradient. In certain disclosures, methods, techniques, and reagents for selection, isolation, and enrichment are described, for example, in PCT Application Nos. WO2013124474 andWO2015164675.

In certain disclosures, CD57- cells are isolated, enriched, or selected in a process or procedure that involves one or more selection steps. In some disclosures, the one or more selection steps are or involve negative selection. In certain disclosures, CD57- cells are isolated, enriched, or selected by separation or removal of CD57+ cells. In certain disclosures, a cell population enriched for CD57- cells results from negative selection of CD57+ cells from the population.

In certain disclosures, a bivalent antibody to link CD57+ cells to a large density cell or bead. This technology has been used most prominently with red blood cells (e.g. RosetteSep™ STEMCELL Technologies), or any other similar or suitable technology to couple target cells, e.g., CD57+ T cells, to density gradients for removal.

In some disclosures, at least a portion of the selection step includes incubation of cells with a selection reagent. The incubation with a selection reagent or reagents, e.g., as part of selection methods which may be performed using one or more selection reagents for selection of one or more different cell types based on the expression or presence in or on the cell of one or more specific molecules, such as surface markers, e.g., surface proteins, intracellular markers, or nucleic acid. In certain disclosures, such surface proteins may include CD57, CD4, or CD8. In certain disclosures, such surface proteins may include CD3. In some disclosures, any known method using a selection reagent or reagents for separation based on such markers may be used. In some disclosures, the selection reagent or reagents result in a separation that is affinity- or immunoaffinity-based separation. For example, the selection in some disclosure includes incubation with a reagent or reagents for separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner. In some disclosures, the reagent or reagents for separation of cells is or include antibodies or antigen binding fragments thereof that bind to or recognize CD4, CD8, or CD57. In some disclosures, the reagent or reagents for separation of cells is or include antibodies or antigen binding fragments thereof that bind to or recognize CD3.

In some disclosures of such processes, a volume of cells is mixed with an amount of a desired affinity-based selection reagent. The immunoaffinity-based selection can be carried out using any system or method that results in a favorable energetic interaction between the cells being separated and the molecule specifically binding to the marker on the cell, e.g., the antibody or other binding partner on the solid surface, e.g., particle. In some disclosures, methods are carried out using particles such as beads, e.g. magnetic beads,that are coated with a selection agent (e.g. antibody) specific to the marker of the cells. The particles (e.g. beads) can be incubated or mixed with cells in a container, such as a tube or bag, while shaking or mixing, with a constant cell density-to-particle (e.g., bead) ratio to aid in promoting energetically favored interactions. In other cases disclosed herein, the methods include selection of cells in which all or a portion of the selection is carried out in the internal cavity of a centrifugal chamber, for example, under centrifugal rotation. In some disclosures, incubation of cells with selection reagents, such as immunoaffinity-based selection reagents, is performed in a centrifugal chamber. In certain disclosures, the isolation or separation is carried out using a system, device, or apparatus described in International Patent Application, Publication Number WO2009/072003, or US 20110003380 A1. In one example disclosed herein, the system is a system as described in International Publication Number WO2016/073602.

In some disclosures, by conducting such selection steps or portions thereof (e.g., incubation with antibody-coated particles, e.g., magnetic beads) in the cavity of a centrifugal chamber, the user is able to control certain parameters, such as volume of various solutions, addition of solution during processing and timing thereof, which can provide advantages compared to other available methods. For example, the ability to decrease the liquid volume in the cavity during the incubation can increase the concentration of the particles (e.g. bead reagent) used in the selection, and thus the chemical potential of the solution, without affecting the total number of cells in the cavity. This in turn can enhance the pairwise interactions between the cells being processed and the particles used for selection. In some disclosures, carrying out the incubation step in the chamber, e.g., when associated with the systems, circuitry, and control as described herein, permits the user to effect agitation of the solution at desired time(s) during the incubation, which also can improve the interaction.

In some disclosures, at least a portion of the selection step is performed in a centrifugal chamber, which includes incubation of cells with a selection reagent. In some disclosures of such processes, a volume of cells is mixed with an amount of a desired affinity-based selection reagent that is far less than is normally employed when performing similar selections in a tube or container for selection of the same number of cells and/or volume of cells according to manufacturer's instructions. In some disclosures, an amount of selection reagent or reagents that is/are no more than 5%, no more than 10%, no more than 15%, no more than 20%, no more than 25%, no more than 50%, no more than 60%, no more than 70% or no more than 80% of the amount of the same selection reagent(s) employed for selection of cells in a tube or container-based incubation for the same number of cells and/or the same volume of cells according to manufacturer's instructions is employed.

In some disclosures, for selection, e.g., immunoaffinity-based selection of the cells, the cells are incubated in the cavity of the chamber in a population that also contains the selection buffer with a selection reagent, such as a molecule that specifically binds to a surface marker on a cell that it desired to enrich and/or deplete, but not on other cells in the population, such as an antibody, which optionally is coupled to a scaffold such as a polymer or surface, e.g., bead, e.g., magnetic bead, such as magnetic beads coupled to monoclonal antibodies specific for CD4 and CD8. In some disclosures, for selection, e.g., immunoaffinity-based selection of the cells, the cells are incubated in the cavity of the chamber in a population that also contains the selection buffer with a selection reagent, such as a molecule that specifically binds to a surface marker on a cell that it desired to enrich and/or deplete, but not on other cells in the population, such as an antibody, which optionally is coupled to a scaffold such as a polymer or surface, e.g., bead, e.g., magnetic bead, such as magnetic beads coupled to monoclonal antibodies specific for CD3. In some disclosures, as described, the selection reagent is added to cells in the cavity of the chamber in an amount that is substantially less than (e.g. is no more than 5%, 10%, 20%, 30%, 40%, 50%, 60%, 70% or 80% of the amount) as compared to the amount of the selection reagent that is typically used or would be necessary to achieve about the same or similar efficiency of selection of the same number of cells or the same volume of cells when selection is performed in a tube with shaking or rotation. In some disclosures, the incubation is performed with the addition of a selection buffer to the cells and selection reagent to achieve a target volume with incubation of the reagent of, for example, 10 mL to 200 mL, such as at least or about at least or about or 10 mL, 20 mL, 30 mL, 40 mL, 50 mL, 60 mL, 70 mL, 80 mL, 90 mL, 100 mL, 150 mL or 200 mL. In some disclosures, the selection buffer and selection reagent are pre-mixed before addition to the cells. In some disclosures, the selection buffer and selection reagent are separately added to the cells. In some disclosures, the selection incubation is carried out with periodic gentle mixing condition, which can aid in promoting energetically favored interactions and thereby permit the use of less overall selection reagent while achieving a high selection efficiency.

In some disclosures, the total duration of the incubation with the selection reagent is from or from about 5 minutes to 6 hours, such as 30 minutes to 3 hours, for example, at least or about at least 30 minutes, 60 minutes, 120 minutes or 180 minutes.

In some disclosures, the incubation generally is carried out under mixing conditions, such as in the presence of spinning, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm), such as at an RCF at the sample or wall of the chamber or other container of from or from about 80g to 100g (e.g. at or about or at least 80 g, 85 g, 90 g, 95 g, or 100 g). In some disclosures, the spin is carried out using repeated intervals of a spin at such low speed followed by a rest period, such as a spin and/or rest for 1, 2, 3, 4, 5, 6, 7, 8, 9, or 10 seconds, such as a spin at approximately 1 or 2 seconds followed by a rest for approximately 5, 6, 7, or 8 seconds.

In some disclosures, such process is carried out within the entirely closed system to which the chamber is integral. In some disclosures, this process (and in some disclosures also one or more additional step, such as a previous wash step washing a sample containing the cells, such as an apheresis sample) is carried out in an automated fashion, such that the cells, reagent, and other components are drawn into and pushed out of the chamber at appropriate times and centrifugation effected, so as to complete the wash and binding step in a single closed system using an automated program.

In some disclosures, after the incubation and/or mixing of the cells and selection reagent and/or reagents, the incubated cells are subjected to a separation to select for cells based on the presence or absence of the particular reagent or reagents. In some disclosures, the separation is performed in the same closed system in which the incubation of cells with the selection reagent was performed. In some disclosures, after incubation with the selection reagents, incubated cells, including cells in which the selection reagent has bound are transferred into a system for immunoaffinity-based separation of the cells. In some disclosures, the system for immunoaffinity-based separation is or contains a magnetic separation column.

Such separation steps can be based on positive selection, in which the cells having bound the reagents, e.g. antibody or binding partner, are retained for further use, and/or negative selection, in which the cells having not bound to the reagent, e.g., antibody or binding partner, are retained. In some examples disclosed herein, both fractions are retained for further use. In some disclosures, negative selection can be particularly useful where no antibody is available that specifically identifies a cell type in a heterogeneous population, such that separation is best carried out based on markers expressed by cells other than the desired population.

In some disclosures, the process steps further include negative and/or positive selection of the incubated and cells, such as using a system or apparatus that can perform an affinity-based selection. In some disclosures, isolation is carried out by enrichment for a particular cell population by positive selection, or depletion of a particular cell population, by negative selection. In some disclosures, positive or negative selection is accomplished by incubating cells with one or more antibodies or other binding agent that specifically bind to one or more surface markers expressed or expressed (marker+) at a relatively higher level (markerhigh) on the positively or negatively selected cells, respectively.

The separation need not result in 100 % enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells.

In some examples, multiple rounds of separation steps are carried out, where the positively or negatively selected fraction from one step is subjected to another separation step, such as a subsequent positive or negative selection. In some examples, a single separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types. In certain disclosures, separation steps are repeated and or performed more than once, where the positively or negatively selected fraction from one step is subjected to the same separation step, such as a repeated positive or negative selection. In some examples, a single separation step is repeated and/or performed more than once, for example to increase the purity of the selected cells and/or to further remove and/or deplete the negatively selected cells from the negatively selected fraction. In certain disclosures, one or more separation steps are performed two times, three times, four times, five times, six times, seven times, eight times, nine times, ten times, or more than ten times. In certain disclosures, the one or more selection steps are performed and/or repeated between one and ten times, between one and five times, or between three and five times.

For example, in some disclosures, specific subpopulations of T cells, such as cells positive or expressing high levels of one or more surface markers, e.g., CD3+, CD4+, CD8+, or CD57+ T cells, are isolated by positive or negative selection techniques. In some disclosures, such cells are selected by incubation with one or more antibody or binding partner that specifically binds to such markers. In some disclosures, the antibody or binding partner can be conjugated, such as directly or indirectly, to a solid support or matrix to effect selection, such as a magnetic bead or paramagnetic bead. For example, in some disclosures, CD4+ T cells, CD8+ T cells, or CD57+ T cells may be selected, e.g., positively selected, with CD4 Microbeads, CD8 Microbeads, or CD57 Microbeads (Miltenyl Biotec). For example, in some disclosures, CD3+ T cells may be selected, e.g., positively selected, with CD3 Microbeads (Miltenyl Biotec).

In certain disclosures, CD57- cells are separated from a PBMC sample by negative selection of cells positive for CD57 expression. In various disclosures, T cells are separated from a PBMC sample by negative selection of markers expressed on non-T cells, such as B cells, monocytes, or other white blood cells, such as CD14. In some disclosures, a CD3+ selection step is used to separate T cells from non-T cells. Such a CD3+ population can be further sorted into sub-populations by positive or negative selection for CD4+ or CD8+, and/or markers expressed or expressed to a relatively higher degree on one or more naïve-like, memory, and/or effector T cell subpopulations. In some disclosures, a CD4+ or CD8+ selection step is used to separate CD4+ helper and CD8+ cytotoxic T cells. Such CD4+ and CD8+ populations can be further sorted into sub-populations by positive or negative selection for markers expressed or expressed to a relatively higher degree on one or more naïve-like, memory, and/or effector T cell subpopulations.

In some disclosures, CD8+ cells are further enriched for or depleted of CD57- T cells, such as by positive or negative selection based on surface expression of CD57. In certain disclosures, CD4+ cells are further enriched for or depleted of CD57- T cells, such as by positive or negative selection based on surface expression of CD57. In certain disclosures, CD3+ cells are further enriched for or depleted of CD57- T cells, such as by positive or negative selection based on surface expression of CD57.

In some disclosures, CD8+ cells are further enriched for or depleted of naive, central memory, effector memory, and/or central memory stem cells, such as by positive or negative selection based on surface antigens associated with the respective subpopulation. In some disclosures, enrichment for central memory T (TCM) cells is carried out to increase efficacy, such as to improve long-term survival, expansion, and/or engraftment following administration, which in some disclosures is particularly robust in such sub-populations. See Terakura et al., (2012) Blood.1:72-82; Wang et al. (2012) J Immunother. 35(9):689-701. In some disclosures, combining TCM-enriched CD8+ T cells and CD4+ T cells further enhances efficacy.

In some disclosures, the same CD4 expression-based selection step used in preparing the CD8+ cell population or subpopulation, also is used to generate the CD4+ cell population or sub-population, such that both the positive and negative fractions from the CD4-based separation are retained and used in subsequent steps of the methods, optionally following one or more further positive or negative selection steps. In some disclosures, the selection for the CD4+ cell population and the selection for the CD8+ cell population are carried out simultaneously. In some disclosures, the CD4+ cell population and the selection for the CD8+ cell population are carried out sequentially, in either order. In some disclosures, methods for selecting cells can include those as described in published U.S. App. No. US20170037369. In some disclosures, the selected CD4+ cell population and the selected CD8+ cell population may be combined subsequent to the selecting. In some disclosures, the selected CD4+ cell population and the selected CD8+ cell population may be combined in a bioreactor bag as described herein.

In various disclosures, a biological sample, e.g., a sample of PBMCs or other white blood cells, are subjected to selection of CD57+ T cells, wherein the negative fractions containing enriched CD57- cells are retained. In some disclosures, the negative fraction enriched with CD57- cells is subjected to selection of CD3+ T cells, where the positive fraction is retained. In certain disclosures, CD8+ T cells are selected from the negative fraction enriched with CD57- cells. In some disclosures, the negative fraction enriched with CD57- cells is subjected to selection of CD8+ T cells, where both the negative and positive fractions are retained. In certain disclosures, CD4+ T cells are selected from the negative fraction. In particular disclosures, from the negative fraction enriched with CD57- cells, are subjected to selection of CD4+ T cells, where both the negative and positive fractions are retained. In certain disclosures, CD8+ T cells are selected from the negative fraction.

In some disclosures, the incubated sample or population of cells to be separated is incubated with a selection reagent containing small, magnetizable or magnetically responsive material, such as magnetically responsive particles or microparticles, such as paramagnetic beads (e.g., such as Dynalbeads or MACS® beads). The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select. In some disclosures, the selection agent is or includes a paramagnetic bead and an attached antibody or antigen binding fragment thereof that binds to or recognizes CD3, CD4, CD8, or CD57. In some disclosures, the selection agent is a CD3, CD4, CD8, or CD57 MACS® microbead.

In some disclosures, the magnetic particle or bead comprises a magnetically responsive material bound to a specific binding member, such as an antibody or other binding partner. Many well-known magnetically responsive materials for use in magnetic separation methods are known, e.g., those described in Molday, U.S. Pat. No. 4,452,773, and in European Patent Specification EP 452342 B. Colloidal sized particles, such as those described in Owen U.S. Pat. No. 4,795,698, and Liberti et al., U.S. Pat. No. 5,200,084 also may be used.

The incubation generally is carried out under conditions whereby the antibodies or binding partners, or molecules, such as secondary antibodies or other reagents, which specifically bind to such antibodies or binding partners, which are attached to the magnetic particle or bead, specifically bind to cell surface molecules if present on cells within the sample.

In certain disclosures, the magnetically responsive particles are coated in primary antibodies or other binding partners, secondary antibodies, lectins, enzymes, or streptavidin. In certain disclosures, the magnetic particles are attached to cells via a coating of primary antibodies specific for one or more markers. In certain disclosures, the cells, rather than the beads, are labeled with a primary antibody or binding partner, and then cell-type specific secondary antibody- or other binding partner (e.g., streptavidin)-coated magnetic particles, are added. In certain disclosures, streptavidin-coated magnetic particles are used in conjunction with biotinylated primary or secondary antibodies.

In some disclosures, separation is achieved in a procedure in which the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some disclosures, a combination of positive and negative selection is performed during the same selection step, where the positive and negative fractions are retained and further processed or subject to further separation steps.

In some disclosures, the affinity-based selection is via magnetic-activated cell sorting (MACS) (Miltenyi Biotech, Auburn, CA). Magnetic Activated Cell Sorting (MACS), e.g., CliniMACS systems are capable of high-purity selection of cells having magnetized particles attached thereto. In certain disclosures, MACS operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain disclosures, the non-target cells are labelled and depleted from the heterogeneous population of cells. In various disclosures, the selection agent is a CD3, CD4, CD8, or CD57 MACS® microbead.

In some disclosures, the suboptimal yield concentration of the affinity reagent is a concentration below a concentration used or required to achieve an optimal or maximal yield of bound cells in a given selection or enrichment involving incubating cells with the reagent and recovering or separating cells having bound to the reagent ("yield," for example, being the number of the cells so-recovered or selected compared to the total number of cells in the incubation that are targeted by the reagent or to which the reagent is specific or that have a marker for which the reagent is specific and capable of binding). The suboptimal yield concentration generally is a concentration or amount of the reagent that in such process or step achieves less than all, e.g., no more than 70 % yield of bound cells, e.g., CD57+, CD4+, or CD8+ T cells, upon recovery of the cells having bound to the reagent. In some disclosures, the suboptimal yield concentration generally is a concentration or amount of the reagent that in such process or step achieves less than all, e.g., no more than 70 % yield of bound cells, e.g., CD3+ T cells, upon recovery of the cells having bound to the reagent. In some disclosures, no more than at or about 50 %, 45 %, 40 %, 30 %, or 25 % yield is achieved by the suboptimal concentration of the affinity reagent. The concentration may be expressed in terms of number or mass of particles or surfaces per cell and/or number of mass or molecules of agent (e.g., antibody, such as antibody fragment) per cell.

In some disclosures, e.g., when operating in a suboptimal yield concentration for each or one or more of two or more selection reagents with affinity to CD57+, CD4+, or CD8+ T cells, one or more of such reagents is used at a concentration that is higher than one or more of the other such reagent(s), in order to bias the ratio of the cell type recognized by that reagent as compared to the cell type(s) recognized by the other(s). In some disclosures, e.g., when operating in a suboptimal yield concentration for each or one or more of two or more selection reagents with affinity to CD57+, CD3+, CD4+, or CD8+ T cells, one or more of such reagents is used at a concentration that is higher than one or more of the other such reagent(s), in order to bias the ratio of the cell type recognized by that reagent as compared to the cell type(s) recognized by the other(s). For example, the reagent specifically binding to the marker for which it is desired to bias the ratio may be included at a concentration (e.g., agent or mass per cells) that is increased by half, 1-fold, 2-fold, 3-fold, 4-fold, 5-fold, 10-fold, or more, compared to other(s), depending on how much it is desired to increase the ratio.In some disclosures, when operating in the suboptimal range and/or with enough cells to achieve saturation of reagents, the amount of immunoaffinity reagent is proportional to the approximate yield of enriched cells. In certain disclosures, an appropriate amount or concentration of immunoaffinity reagents that depend on the desired ratio of the generated population containing the enriched or selected cells, e.g., CD57-, CD4+, or CD8+ T cells, can be determined as a matter of routine. In certain disclosures, an appropriate amount or concentration of immunoaffinity reagents that depend on the desired ratio of the generated population containing the enriched or selected cells, e.g., CD3+ T cells, can be determined as a matter of routine.

In some disclosures, the separation and/or isolation steps are carried out using magnetic beads in which immunoaffinity reagents are reversibly bound, such as via a peptide ligand interaction with a streptavidin mutein as described in WO 2015/164675. Exemplary of such magnetic beads are Streptamers®. In some disclosures, the separation and/or steps is carried out using magnetic beads, such as those commercially available from Miltenyi Biotec.

In some disclosures, the magnetically responsive particles are left attached to the cells that are to be subsequently incubated, cultured and/or engineered; in some disclosures, the particles are left attached to the cells for administration to a patient. In some disclosures, the magnetizable or magnetically responsive particles are removed from the cells. Methods for removing magnetizable particles from cells are known and include, e.g., the use of competing non-labeled antibodies, magnetizable particles or antibodies conjugated to cleavable linkers, etc. In some disclosures, the magnetizable particles are biodegradable.

In some disclosures, the isolation and/or selection results in one or more populations of enriched T cells, e.g., CD57- T cells, CD3+ T cells, CD4+ T cells, and/or CD8+ T cells. In some disclosures, two or more separate population of enriched T cells are isolated, selected, enriched, or obtained from a single biological sample. In some disclosures, separate populations are isolated, selected, enriched, and/or obtained from separate biological samples collected, taken, and/or obtained from the same subject.

In certain disclosures, the isolation and/or selection results in one or more populations of enriched T cells that includes at least 60%, at least 65%, at least 70%, at least 75%, at least 80%, at least 85%, at least 90%, at least 95%, at least 98%, at least 99%, at least 99.5%, at least 99.9%, or at or at about 100% CD57- CD3+ T cells. In particular disclosures, the population of enriched T cells consists essentially of CD57- CD3+ T cells.

In certain disclosures, the isolation and/or enrichment results in a populations of enriched CD4+ T cells that includes at least 60%, at least 65%, at least 70%, at least 75%, at least 80%, at least 85%, at least 90%, at least 95%, at least 98%, at least 99%, at least 99.5%, at least 99.9%, or at or at about 100% CD57- CD4+ T cells. In certain disclosures, the input composition of CD4+ T cells includes less than 40%, less than 35%, less than 30%, less than 25%, less than 20%, less than 15%, less than 10%, less than 5%, less than 1%, less than 0.1%, or less than 0.01% CD8+ T cells, and/or contains no CD8+ T cells, and/or is free or substantially free of CD8+ T cells. In some disclosures, the population of enriched T cells consists essentially of CD57- CD4+ T cells.

In certain disclosures, the isolation and/or enrichment results in a populations of enriched CD57- CD8+ T cells that includes at least 60%, at least 65%, at least 70%, at least 75%, at least 80%, at least 85%, at least 90%, at least 95%, at least 98%, at least 99%, at least 99.5%, at least 99.9%, or at or at about 100% CD57- CD8+ T cells. In certain disclosures, the population of CD8+ T cells contains less than 40%, less than 35%, less than 30%, less than 25%, less than 20%, less than 15%, less than 10%, less than 5%, less than 1%, less than 0.1%, or less than 0.01% CD4+ T cells, and/or contains no CD4+ T cells, and/or is free of or substantially free of CD4+ T cells. In some disclosures, the population of enriched T cells consists essentially of CD57- CD8+ T cells.

## C. Cell Selection by Chromatography

In disclosures of the methods disclosed herein, cells of a sample, e.g., T cells, are selected by chromatographic isolation, such as by column chromatography including affinity chromatography or gel permeation chromatography. In some disclosures, cells, e.g., CD57- T cells, are isolated, selected, or enriched by chromatographic isolation, such as by column chromatography including affinity chromatography or gel permeations chromatography. In some disclosures, the method employs a receptor binding reagent that binds to a receptor molecule (e.g., CD57) that is located on the surface of a target cell, such as the cell to be isolated, selected, or enriched (e.g., CD57+ cells). Such methods may be described as (traceless) cell affinity chromatography technology (CATCH) and may include any of the methods or techniques described in PCT Application Nos. WO2013124474 and WO2015164675. In particular disclosures, CD57+ cells are negatively selected by chromatographic isolation.

In some disclosures, the target cells (e.g., CD57+ cells), have or express a receptor molecule on the cell surface, such that the cells to be isolated, selected, or enriched are defined by the presence of at least one common specific receptor molecule (e.g., CD57). In some disclosures, the sample containing the target cell may also contain additional cells that are devoid of the receptor molecule. For example, in some disclosures, T cells are isolated, enriched, and or elected from a sample containing multiple cells types, e.g., red blood cells or B cells. In certain disclosures, CD57+ cells are isolated, enriched, and or elected from a sample containing multiple cells types, e.g., red blood cells or B cells, thereby providing isolated CD57+ cells and a non-selected population of cells, e.g., a population of enriched CD57- T cells.

In some disclosures, the receptor binding reagent is comprised in a chromatography column, e.g., bound directly or indirectly to the chromatography matrix (e.g., stationary phase). In some disclosures, the receptor binding reagent is present on the chromatography matrix (e.g., stationary phase) at the time the sample is added to the column. In some disclosures, the receptor binding reagent is capable of being bound indirectly to the chromatography matrix (e.g., stationary phase) through a reagent, e.g., an affinity reagent as described herein. In some disclosures, the affinity reagent is bound covalently or non-covalently to the stationary phase of the column. In some disclosures, the affinity reagent is reversibly immobilized on the chromatography matrix (e.g., stationary phase). In some cases disclosed herein, the affinity reagent is immobilized on the chromatography matrix (e.g., stationary phase) via covalent bonds. In some disclosures, the affinity reagent is reversibly immobilized on the chromatography matrix (e.g., stationary phase) non-covalently.

In some disclosures, the receptor binding reagent may be present, for example bound directly to (e.g., covalently or non-covalently) or indirectly via an affinity reagent, on the chromatography matrix (e.g., stationary phase) at the time the sample is added to the chromatography column (e.g., stationary phase). Thus, upon addition of the sample, target cells can be bound by the receptor binding reagent and immobilized on the chromatography matrix (e.g., stationary phase) of the column. Alternatively, in some disclosures, the receptor binding reagent can be added to the sample. In this way, the receptor binding reagent binds to the target cells (e.g., T cells) in the sample, and the sample can then be added to a chromatography matrix (e.g., stationary phase) comprising the affinity reagent, where the receptor binding reagent, already bound to the target cells, binds to the affinity reagent, thereby immobilizing the target cells on the chromatography matrix (e.g., stationary phase). In some disclosures, the receptor binding reagent binds to the affinity reagent as described herein, for example as described herein, via binding partner C, as described herein, comprised in the receptor binding reagent.

In some disclosures, a receptor binding reagent is added to the sample. In certain disclosures, the receptor binding reagent has a binding site B, which specifically binds to the receptor molecule on the surface of the cell, e.g., the target cell. In some disclosures, the receptor binding reagent also includes a binding partner C, which can specifically and reversibly bind to a binding site Z of an affinity reagent. For example, in certain disclosures, a receptor binding reagent that binds to or recognizes CD57 is added to the sample, which binds to CD57 on the surface of cells positive for CD57 expression at binding site B.

In certain disclosures, the affinity reagent may also contain two or more binding sites Z that can be bound by the binding partner C, thereby providing a multimerization of the receptor binding reagent. This affinity reagent used herein can thus also be a multimerization reagent. The affinity reagent may, for example, be streptavidin, a streptavidin mutein, avidin, an avidin mutein or a mixture thereof. In some disclosures, different chromatography matrices are coupled to different affinity reagents, and may be layered into a column forming a multicomponent system for separation.

In some disclosures, two or more receptor binding reagents associate with, such as are reversibly or irreversibly bound to, the affinity reagent, such as via the one or plurality of binding sites Z present on the affinity reagent. In some cases disclosed herein, this results in the receptor binding reagents being closely arranged to each other such that an avidity effect can take place if a target cell having (at least two copies of) a cell surface molecule (e.g., selection marker) is brought into contact with the receptor binding reagent that is able to bind the particular molecule (e.g., selection marker).

In some disclosures, two or more different receptor binding reagents that are the same, i.e. have the same selection marker binding specificity, can be reversibly bound to the affinity reagent. In some disclosures, it is possible to use at least two different receptor binding reagents, and in some cases disclosed herein, three or four different receptor binding reagents that bind to different selection markers. In some disclosures, each of the at least two receptor binding reagents can bind to a different molecule (e.g., selection marker), such as a first molecule, second molecule and so on. In some cases disclosed herein, the different molecules (e.g., selection markers), such as cell surface molecules, can be present on the same target cell. In other cases disclosed herein, the different molecules (e.g., selection markers), such as cell surface molecules, can be present on different target cells that are present in the same population of cells. In some cases disclosed herein, a third, fourth and so on receptor binding reagents can be associated with the same reagent, each containing a further different binding site.

In some disclosures, the two or more different receptor binding reagents contain the same binding partner C. In some disclosures, the two or more different receptor binding reagents contain different binding partners. In some disclosures, a first receptor binding reagent can have a binding partner C1 that can specifically bind to a binding site Z1 present on the affinity reagent and a second receptor binding reagent can have a binding partner C2 that can specifically bind to the binding site Z1 or to a binding site Z2 present on the affinity reagent. Thus, in some instances, the plurality of binding sites Z comprised by the affinity reagent includes binding sites Z1 and Z2, which are capable of reversibly binding to binding partners C1 and C2, respectively, comprised by the receptor binding reagent. In some disclosures, C1 and C2 are the same, and/or Z 1 and Z2 are the same. In other disclosures, one or more of the plurality of binding sites Z can be different. In other instances, one or more of the plurality of binding partners C may be different. It is within a level of a skilled artisan to choose any combination of different binding partners C that are compatible with an affinity reagent containing the binding sites Z, as long as each of the binding partners C are able to interact, such as specifically bind, with one of the binding sites Z.

In certain disclosures the sample, e.g., the sample containing the cells and the receptor binding reagent (e.g. antibody), is loaded on or contacted with a chromatography matrix containing an attached or immobilized affinity reagent (e.g. binding reagent). In particular disclosures, the affinity reagent has a plurality of binding sites Z that specifically bind to the binding partner C of the receptor binding reagent. In certain disclosures, the receptor binding reagent binds to the affinity reagent by the interaction between the binding partner C and the binding site Z. Thus, in some disclosures, the cell, e.g., the target cell, is immobilized via the complex that is formed by the one or more binding sites Z of the affinity reagent and the binding site Z of receptor binding reagent on the chromatography matrix. In further disclosures, the cells, e.g., the target cell, may be depleted from the sample, such as by rinsing, releasing, or washing the remaining sample from the chromatography matrix. In particular disclosures, the receptor binding reagent may either be included in the sample that contains the cells or it may applied or contacted to the chromatography matrix for binding to the attached affinity or multimerization reagent, such as before the sample is added to the chromatography matrix.

In some disclosures, the chromatography matrix is used to remove or separate target cells from a sample, e.g., by negative selection. For example, in certain disclosures, a sample containing CD57+ cells and CD57- cells is contacted or incubated with a receptor binding reagent that binds to and or recognizes CD57. In certain disclosures, the sample and the receptor binding reagents are loaded onto the matrix, where, in some disclosures, a complex is formed by the immobilized or attached affinity reagent, the receptor binding reagent, and a CD57+ T cell. In some disclosures, unbound cells are removed or rinsed from the chromatography matrix, thereby removing the bound CD57+ cells and providing a sample, e.g., a population, enriched for CD57- cells.

In certain disclosures, the chromatography matrix is used to isolate, select, or enrich target cells from a sample, e.g., by positive selection. For example, in some disclosures, a sample containing CD4+ or CD8+ T cells and other cells, e.g., non-T cell immune cells, is contacted or incubated with a receptor binding reagent that binds to and or recognizes CD4 or CD8. In certain disclosures, the sample and the receptor binding reagents are loaded onto the matrix, where, in some disclosures, a complex is formed by the immobilized or attached affinity reagent, the receptor binding reagent, and CD4+ or CD8+ T cell. In certain disclosures, unbound cells are removed or rinsed from the chromatography matrix. In particular disclosures, the immobilized CD4+ or CD8+ cells may be removed or released by the addition of the competition reagent, such as by disrupting the complex. In some disclosures, the separated, released, or eluted CD4+ or CD8+ T cells are thus a sample, composition, or population of cells enriched for CD4+ or CD8+ T cells.

In certain disclosures, the chromatography matrix is used to isolate, select, or enrich target cells from a sample, e.g., by positive selection. For example, in some disclosures, a sample containing CD3+ T cells and other cells, e.g., non-T cell immune cells, is contacted or incubated with a receptor binding reagent that binds to and or recognizes CD3. In certain disclosures, the sample and the receptor binding reagents are loaded onto the matrix, where, in some disclosures, a complex is formed by the immobilized or attached affinity reagent, the receptor binding reagent, and CD3+ T cell. In certain disclosures, unbound cells are removed or rinsed from the chromatography matrix. In particular disclosures, the immobilized CD3+ cells may be removed or released by the addition of the competition reagent, such as by disrupting the complex. In some disclosures, the separated, released, or eluted CD3+ T cells are thus a sample, composition, or population of cells enriched for CD3+ T cells.

In some disclosures, a competition reagent is loaded onto the chromatography column. In some disclosures, a reversible bond formed between binding partner C and binding site Z can be disrupted by a competition reagent. In particular disclosures, the competition reagent has a binding site that is able to bind to the binding site Z of the affinity reagent. In some disclosures, a competition reagent can be a biotin, a biotin derivative or analog or a streptavidin-binding peptide capable of competing for binding with the binding partner C for the one or more binding sites Z. In certain disclosures, the competition reagent forms a complex with the affinity reagent, and is thereby immobilized on the chromatography matrix. In some disclosures, the binding partner C and the competition reagent are different, and the competition reagent exhibits a higher binding affinity for the one or more binding sites Z compared to the affinity of the binding partner. In particular disclosures of any of the methods disclosed herein, addition of a competition reagent to the stationary phase of the chromatography column to disrupt the binding of the selection agent (e.g., the receptor-binding agent) to the affinity reagent is not required to detach the target cells (e.g., T cells) from the chromatography matrix (e.g., stationary phase).

As a result of this competitive binding, the binding between the receptor binding reagent and the affinity reagent at binding partner C and binding site Z is displaced. In particular disclosures, adding or loading the competition reagent to a chromatography matrix with an attached complex containing the affinity reagent, receptor binding reagent, and the cell, e.g., the target cell, elutes the cell from the chromatography matrix. In some disclosures, the receptor binding reagent has a low affinity towards the receptor molecule of the cell at binding site B, such that the receptor binding reagent dissociates from the cell in the presence of the competition reagent. Thus, in some disclosures, the cells, e.g., the target cells, are eluted from the chromatography matrix free or essentially free of bound receptor binding molecules.

In some disclosures, an elution sample from the eluate of the first chromatography column, which includes the cells, e.g., the target cells, the competition reagent, and the receptor binding reagent, is collected. In certain disclosures, the elution sample is loaded onto a second chromatography column, which has a suitable stationary phase that is both an affinity chromatography matrix and, at the same time, can act as gel permeation matrix. In particular disclosures, the affinity chromatography matrix has an affinity reagent immobilized thereon. In some disclosures, the receptor binding reagent and the competition reagent bind to a binding site Z on the affinity reagent, and are thereby immobilized on the chromatography matrix. As a result, in certain disclosures, the elution sample containing the isolated target cells is depleted of the receptor binding reagent and the competition reagent. Thus, in some disclosures, the target cells, being freed of any reactants, are now in a condition for further use, for example, for processing by any of the methods described herein.

In some disclosures, the cells, e.g., the target cells of the sample, may be depleted from the sample, such as by rinsing, releasing, or washing the remaining sample from the chromatography matrix (e.g., stationary phase). In some disclosures, one or more (e.g., 2, 3, 4, 5, 6) wash steps are used to remove unbound cells and debris from the chromatography matrix (e.g., stationary phase). In some disclosures, at least two wash steps are performed. In some disclosures, the sample is allowed to penetrate the matrix for at least or about 5, 10, 15, 16, 20, 25, 30, 35, 40, 45, 50, 55, 60, 70, 80, 90, 100, or 120 minutes before one or more wash steps are performed. In some disclosures, a wash step is performed at, about, or at least 5, 10, 15, 20, 25, 30, 35, 40, 45, 50, 55, 60, 70, 80, 90, 100, or 120 minutes after the sample is added to the chromatography column (e.g., stationary phase). In some disclosures, a wash step is performed at, about, or at least 5, 10, 15, 20, 25, 30, 35, 40, 45, 50, 55, or 60 minutes after the sample is added to the chromatography column (e.g., stationary phase). In some disclosures, one or more wash steps are performed within or within about 120, 100, 90, 80, 70, 60, 55, 50, 45, 40, 35, 30, 25, 20, 15, 10, or 5 minutes following addition of the sample to the chromatography column (e.g., stationary phase). In some disclosures, one or more wash steps are performed within or within about 60, 55, 50, 45, 40, 35, 30, 25, 20, 15, 10, or 5 minutes following addition of the sample to the chromatography column (e.g., stationary phase). In some disclosures, one or more wash steps are performed within or within about 5 to 60 minutes following addition of the sample to the chromatography column (e.g., stationary phase). In some disclosures, one or more wash steps are performed within or within about 5 to 50 minutes following addition of the sample to the chromatography column (e.g., stationary phase). In some disclosures, one or more wash steps are performed within or within about 5 to 40 minutes following addition of the sample to the chromatography column (e.g., stationary phase). In some disclosures, one or more wash steps are performed within or within about 5 to 30 minutes following addition of the sample to the chromatography column (e.g., stationary phase). In some disclosures, one or more wash steps are performed within or within about 5 to 20 minutes following addition of the sample to the chromatography column (e.g., stationary phase). In some disclosures, one or more wash steps are performed within or within about 5 to 10 minutes following addition of the sample to the chromatography column (e.g., stationary phase). In some disclosures, one or more wash steps are performed within or within about 10 to 60 minutes following addition of the sample to the chromatography column (e.g., stationary phase). In some disclosures, one or more wash steps are performed within or within about 20 to 60 minutes following addition of the sample to the chromatography column (e.g., stationary phase). In some disclosures, one or more wash steps are performed within or within about 30 to 60 minutes following addition of the sample to the chromatography column (e.g., stationary phase). In some disclosures, one or more wash steps are performed within or within about 40 to 60 minutes following addition of the sample to the chromatography column (e.g., stationary phase). In some disclosures, one or more wash steps are performed within or within about 50 to 60 minutes following addition of the sample to the chromatography column (e.g., stationary phase).

In some disclosures, multiple rounds of cell selection steps are carried out, where the positively or negatively selected fraction from one step is subjected to another selection step, such as a subsequent positive or negative selection. In certain disclosures, methods, techniques, and reagents for selection, isolation, and enrichment are described, for example, in PCT Application No. WO2015164675.

In some disclosures, a single selection step can be used to isolate target cells (e.g., CD57- T cells) from a sample. In some disclosures, the single selection step can be performed on a single chromatography column. In some examples disclosed herein, a single selection step can deplete cells expressing multiple markers simultaneously. Likewise, multiple cell types can simultaneously be positively selected. In certain disclosures, selection steps are repeated and or performed more than once, where the positively or negatively selected fraction from one step is subjected to the same selection step, such as a repeated positive or negative selection. In some examples disclosed herein, a single selection step is repeated and/or performed more than once, for example to increase the purity of the selected cells and/or to further remove and/or deplete the negatively selected cells from the negatively selected fraction. In certain disclosures, one or more selection steps are performed two times, three times, four times, five times, six times, seven times, eight times, nine times, ten times, or more than ten times. In certain disclosures, the one or more selection steps are performed and/or repeated between one and ten times, between one and five times, or between three and five times. In some disclosures, two selection steps are performed.

Cell selection may be performed using one or more chromatography columns. In some disclosures, the one or more chromatography columns are included in a closed system. In some disclosures, the closed system is an automated closed system, for example requiring minimal or no user (e.g., human) input. In some disclosures, cell selection is performed sequentially (e.g., a sequential selection technique). In some disclosures, the one or more chromatography columns are arranged sequentially. For example, a first column may be oriented such that the output of the column (e.g., eluent) can be fed, e.g., via connected tubing, to a second chromatography column. In some disclosures, a plurality of chromatography columns may be arranged sequentially. In some disclosures, cell selection may be achieved by carrying out sequential positive and negative selection steps, the subsequent step subjecting the negative and/or positive fraction from the previous step to further selection, where the entire process is carried out in the same tube or tubing set.

In some disclosures, a sample containing target cells is subjected to a sequential selection in which a first selection is effected to enrich for one of the CD4+ or CD8+ populations, and the non-selected cells from the first selection are used as the source of cells for a second selection to enrich for the other of the CD4+ or CD8+ populations. In some disclosures, a further selection or selections can be effected to enrich for sub-populations of one or both of the CD4+ or CD8+ population, for example, central memory T ($T_{CM}$) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. In some disclosures, a sample containing target cells is subjected to a sequential selection in which a first selection is effected to enrich for a CD3+ population, and the selected cells are used as the source of cells for a second selection to enrich for CD3+ populations. In some disclosures, a sample containing target cells is subjected to a sequential selection in which a first selection is effected to enrich for a CD3+ population on a first stationary phase (e.g., in a first chromatograph column), and the flow through containing unbound cells is used as the source of cells for a second selection to enrich for a CD3+ population on a second stationary phase (e.g., in a second chromatograph column), wherein the first and second stationary phases are arranged sequentially. In some disclosures, a further selection or selections can be effected to enrich for sub-populations of the CD3 + population, for example, central memory T ($T_{CM}$) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. In some disclosures, a sample containing target cells is subjected to a sequential selection in which a first selection is effected to enrich for a CD3+ population, and the selected cells are used as the source of cells for a second selection to enrich for CD4+ populations. In some disclosures, a further selection or selections can be effected to enrich for sub-populations of the CD3+CD4+ population, for example, central memory T ($T_{CM}$) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. In some disclosures, a sample containing target cells is subjected to a sequential selection in which a first selection is effected to enrich for a CD3+ population, and the selected cells are used as the source of cells for a second selection to enrich for CD8+ populations. In some disclosures, a further selection or selections can be effected to enrich for sub-populations of the CD3+CD8+ population, for example, central memory T ($T_{CM}$) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. It is contemplated that in some disclosures, specific subpopulations of T cells (e.g., CD3+ cells), such as cells positive or expressing high levels of one or more surface markers, e.g., CD28+, CD62L+, CCR7+, CD27+, CD127+, CD4+, CD8+, CD45RA+, and/or CD45RO+ T cells, are selected by positive or negative sequential selection techniques.

In some disclosures, a sample containing target cells is subjected to a sequential selection in which a first selection is effected to enrich for a CD57- population, and the selected cells are used as the source of cells for a second selection to enrich for CD3+ populations. In some disclosures, a sample containing target cells is subjected to a sequential selection in which a first selection is effected to enrich for a CD57- population on a first stationary phase (e.g., in a first chromatograph column), and the flow through containing unbound cells is used as the source of cells for a second selection to enrich for a CD3+ population on a second stationary phase (e.g., in a second chromatograph column), wherein the first and second stationary phases are arranged sequentially. In some disclosures, a further selection or selections can be effected to enrich for sub-populations of the CD57- population, for example, central memory T ($T_{CM}$) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. In some disclosures, a sample containing target cells is subjected to a sequential selection in which a first selection is effected to enrich for a CD57- population, and the selected cells are used as the source of cells for a second selection to enrich for CD3+ populations. In some disclosures, a further selection or selections can be effected to enrich for sub-populations of the CD57-CD3+ population, for example, central memory T ($T_{CM}$) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. It is contemplated that in some disclosures, specific subpopulations of T cells (e.g., CD57- cells), such as cells positive or expressing high levels of one or more surface markers, e.g., CD28+, CD62L+, CCR7+, CD27+, CD127+, CD4+, CD8+, CD45RA+, and/or CD45RO+ T cells, are selected by positive or negative sequential selection techniques.

In some disclosures, a sample containing target cells is subjected to a sequential selection in which a first selection is effected to enrich for a CD3+ population, and the selected cells are used as the source of cells for a second selection to enrich for CD57- populations. In some disclosures, a sample containing target cells is subjected to a sequential selection in which a first selection is effected to enrich for a CD3+ population on a first stationary phase (e.g., in a first chromatograph column), and the flow through containing unbound cells is used as the source of cells for a second selection to enrich for a C57- population on a second stationary phase (e.g., in a second chromatograph column), wherein the first and second stationary phases are arranged sequentially. In some disclosures, a further selection or selections can be effected to enrich for sub-populations of the CD3+ population, for example, central memory T ($T_{CM}$) cells, naïve T cells, and/or cells positive for or expressing high levels of one or more surface markers, e.g., CD28+, CD62L+, CCR7+, CD27+, CD127+, CD4+, CD8+, CD45RA+, and/or CD45RO+. In some disclosures, a sample containing target cells is subjected to a sequential selection in which a first selection is effected to enrich for a CD3+ population, and the selected cells are used as the source of cells for a second selection to enrich for CD57- populations. In some disclosures, a further selection or selections can be effected to enrich for sub-populations of the CD3+CD57- population, for example, central memory T ($T_{CM}$) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. It is contemplated that in some disclosures, specific subpopulations of T cells (e.g., CD57- cells), such as cells positive or expressing high levels of one or more surface markers, e.g., CD28+, CD62L+, CCR7+, CD27+, CD127+, CD4+, CD8+, CD45RA+, and/or CD45RO+ T cells, are selected by positive or negative sequential selection techniques.

In some disclosures, cell selection is performed in parallel (e.g., parallel selection technique). In some disclosures, the one or more chromatography columns are arranged in parallel. For example, two or more columns may be arranged such that a sample is loaded onto two or more columns at the same time via tubing that allows for the sample to be added to each column, for example, without the need for the sample to traverse through a first column. For example, using a parallel selection technique, cell selection may be achieved by carrying out positive and/or negative selection steps simultaneously, for example in a closed system where the entire process is carried out in the same tube or tubing set. In some disclosures, a sample containing target cells is subjected to a parallel selection in which the sample is load onto two or more chromatography columns, where each column effects selection of a cell population. In some disclosures, the two or more chromatography columns effect selection of CD57-, CD3+, CD4+, or CD8+ populations individually. In some disclosures, the two or more chromatography columns, including affinity chromatography or gel permeation chromatography, independently effect selection of the same cell population. For example, the two or more chromatography columns may effect selection of CD57- cells. In some disclosures, the two or more chromatography columns, including affinity chromatography or gel permeation chromatography, independently effect selection of different cell populations. For example, the two or more chromatography columns independently may effect selection of CD57- cells, CD4+ cells, CD3+ and/or CD8+ cells. In some disclosures, a further selection or selections, for example using sequential selection techniques, can be effected to enrich for sub-populations of one or all cell populations selected via parallel selection. For example, selected cells may be further selected for central memory T ($T_{CM}$) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. In some disclosures, a sample containing target cells is subjected to a parallel selection in which parallel selection is effected to enrich for a CD57- population on the two or more columns. In some disclosures, a further selection or selections can be effected to enrich for sub-populations of the CD57- population, for example, central memory T ($T_{CM}$) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. In some disclosures, a sample containing target cells is subjected to a parallel selection in which a selection is effected to enrich for a CD57- population and a CD3+ population on the two or more columns, independently. In some disclosures, a further selection or selections can be effected to enrich for sub-populations of the CD57- and CD3+ populations, for example, central memory T (T$_{CM}$) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. In some disclosures, a sample containing target cells is subjected to a parallel selection in which a selection is effected to enrich for a CD57- population and a CD4+ population on the two or more columns, independently. In some disclosures, a further selection or selections can be effected to enrich for sub-populations of the CD57- and CD4+ populations, for example, central memory T (T$_{CM}$) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. In some disclosures, a sample containing target cells is subjected to a parallel selection in which parallel selection is effected to enrich for a CD57- population and a CD8+ population. In some disclosures, a further selection or selections can be effected to enrich for sub-populations of the CD57- and CD8+ populations, for example, central memory T (T$_{CM}$) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. In some disclosures, a sample containing target cells is subjected to a parallel selection in which parallel selection is effected to enrich for a CD4+ population and a CD8+ population. In some disclosures, a further selection or selections can be effected to enrich for sub-populations of the CD4+ and CD8+ populations, for example, central memory T (T$_{CM}$) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. It is contemplated that in some disclosures, specific subpopulations of T cells (e.g., CD3+, CD4+, CD8+ T cells), such as cells positive or expressing high levels of one or more surface markers, e.g., CD28+, CD62L+, CCR7+, CD27+, CD127+, CD4+, CD8+, CD45RA+, and/or CD45RO+ T cells, are selected by positive or negative parallel selection techniques. In some disclosures, sequential and parallel selection techniques can be used in combination.

In some disclosures, two columns are used for parallel selection. In some disclosures, the two columns select for the same cell type (e.g., same selection marker). In some disclosures, the two columns each select for CD57- T cells.

In general, binding capacity of a stationary phase (e.g., selection resin) affects how much stationary phase is needed in order to select a certain number of target moieties, e.g., target cells such as T cells. The binding capacity, e.g., the number of target cells that can be immobilized per mL of the stationary phase (e.g., selection resin), can be used to determine or control the number of captured target cells on one or more columns. One or more chromatography column can be used for the on-column cell selection and stimulation disclosed herein. When multiple columns are used, they can be arranged sequentially, in parallel, or in a suitable combination thereof. Thus, the binding capacity of a stationary phase (e.g., selection resin) can be used to standardize the reagent amount in a single-column approach or the reagent amount for each column in a multiple-column approach.

In some disclosures, the binding capacity of the stationary phase used herein is the maximum number of target cells bound to the stationary phase at given solvent and cell concentration conditions, when an excess of target cells are loaded onto the stationary phase. In some disclosures, the binding capacity is or is about 100 million ± 25 million target cells (e.g., T cells) per mL of stationary phase. In some disclosures, the static binding capacity of the stationary phase (e.g., selection resin) disclosed herein ranges between about 75 million and about 125 million target cells per mL of stationary phase. In one disclosure, the binding capacity of the stationary phase used herein for on-column cell selection and stimulation is a static binding capacity. In some disclosures, the static binding capacity is the maximum amount of cells capable of being immobilized on the stationary phase, e.g., at certain solvent and cell concentration conditions. In some disclosures, the static binding capacity of the stationary phase (e.g., selection resin) disclosed herein ranges between about 50 million and about 100 million target cells per mL of stationary phase. In some disclosures, the static binding capacity is or is about 100 million ± 25 million target cells (e.g., T cells) per mL of stationary phase. In some disclosures, the static binding capacity of the stationary phase (e.g., selection resin) disclosed herein ranges between about 75 million and about 125 million target cells per mL of stationary phase. In some disclosures, the static binding capacity of the stationary phase (e.g., selection resin) is between about 10 million and about 20 million, between about 20 million and about 30 million, between about 30 million and about 40 million, between about 40 million and about 50 million, between about 50 million and about 60 million, between about 60 million and about 70 million, between about 70 million and about 80 million, between about 80 million and about 90 million, between about 90 million and about 100 million, between about 110 million and about 120 million, between about 120 million and about 130 million, between about 130 million and about 140 million, between about 140 million and about 150 million, between about 150 million and about 160 million, between about 160 million and about 170 million, between about 170 million and about 180 million, between about 180 million and about 190 million, or between about 190 million and about 200 million target cells per mL of stationary phase.

In some disclosures, the binding capacity of the stationary phase used herein is the number of target cells that bind to the stationary phase under given flow conditions before a significant breakthrough of unbound target cells occurs. In one disclosure, the binding capacity of the stationary phase used herein for on-column cell selection is a dynamic binding capacity, i.e., the binding capacity under operating conditions in a packed chromatography column during sample application. In some disclosures, the dynamic binding capacity is determined by loading a sample containing a known concentration of the target cells and monitoring the flow-through, and the target cells will bind the stationary phase to a certain break point before unbound target cells will flow through the column. In some disclosures, the dynamic binding capacity is or is about 100 million ± 25 million target cells (e.g., T cells) per mL of stationary phase. In some disclosures, the dynamic binding capacity of the stationary phase (e.g., selection resin) disclosed herein is between or is between about 75 million and about 125 million target cells per mL of stationary phase. In some disclosures, the dynamic binding capacity of the stationary phase (e.g., selection resin) disclosed herein ranges between about 50 million and about 100 million target cells per mL of stationary phase. In some disclosures, the dynamic binding capacity of the stationary phase (e.g., selection resin) is between about 10 million and about 20 million, between about 20 million and about 30 million, between about 30 million and about 40 million, between about 40 million and about 50 million, between about 50 million and about 60 million, between about 60 million and about 70 million, between about 70 million and about 80 million, between about 80 million and about 90 million, between about 90 million and about 100 million, between about 110 million and about 120 million, between about 120 million and about 130 million, between about 130 million and about 140 million, between about 140 million and about 150 million, between about 150 million and about 160 million, between about 160 million and about 170 million, between about 170 million and about 180 million, between about 180 million and about 190 million, or between about 190 million and about 200 million target cells per mL of stationary phase.

In some disclosures, the stationary phase is 20 mL. In some disclosures, the stationary phase has a binding capacity of 2 billion ± 0.5 billion cells.

Generally, a chromatographic method is a fluid chromatography, typically a liquid chromatography. In some disclosures, the chromatography can be carried out in a flow through mode in which a fluid sample containing the cells, e.g., the target cells, is applied, for example, by gravity flow or by a pump on one end of a column containing the chromatography matrix and in which the fluid sample exists the column at the other end of the column. In addition the chromatography can be carried out in an "up and down" mode in which a fluid sample containing the cells to be isolated is applied, for example, by a pipette on one end of a column containing the chromatography matrix packed within a pipette tip and in which the fluid sample enters and exists the chromatography matrix /pipette tip at the other end of the column. Alternatively, the chromatography can also be carried out in a batch mode in which the chromatography material (stationary phase) is incubated with the sample that contains the cells, for example, under shaking, rotating or repeated contacting and removal of the fluid sample, for example, by means of a pipette

In some disclosures, any material may be employed as chromatography matrix in the context of the invention, as long as the material is suitable for the chromatographic isolation of cells. In particular disclosures, a suitable chromatography material is at least innocuous or essentially innocuous, e.g., not detrimental to cell viability, when used in a packed chromatography column under desired conditions for cell isolation and/or cell separation. In some disclosures, the chromatography matrix remains in a predefined location, typically in a predefined position, whereas the location of the sample to be separated and of components included therein is being altered. Thus, in some disclosures, the chromatography matrix is a "stationary phase."

Typically, the respective chromatography matrix has the form of a solid or semi-solid phase, whereas the sample that contains the target cell to be isolated/separated is a fluid phase. The mobile phase used to achieve chromatographic separation is likewise a fluid phase. The chromatography matrix can be a particulate material (of any suitable size and shape) or a monolithic chromatography material, including a paper substrate or membrane (cf. the Example Section). Thus, the chromatography can be both column chromatography as well as planar chromatography. In addition to standard chromatography columns, columns allowing a bidirectional flow or pipette tips can be used for column based/flow through mode based chromatographic separation of cells as described here. Thus, in some cases disclosed herein, pipette tips or columns allowing a bidirectional flow are also comprised by chromatography columns useful in the present methods. In some disclosures, a particulate matrix material is used, and the particulate matrix material may, for example, have a mean particle size of about 5 μm to about 200 μm, or from about 5 μm to about 400 μm, or from about 5 μm to about 600 μm. In some disclosures, planar chromatography is used, and the matrix material may be any material suitable for planar chromatography, such as conventional cellulose-based or organic polymer based membranes (for example, a paper membrane, a nitrocellulose membrane or a polyvinylidene difluoride (PVDF) membrane) or silica coated glass plates. In one disclosure, the chromatography matrix/stationary phase is a non-magnetic material or non-magnetizable material.

In some disclosures, the chromatography matrix/stationary phase is a non-magnetic material or non-magnetisable material. Such material may include derivatized silica or a crosslinked gel. A crosslinked gel (which is typically manufactured in a bead form) may be based on a natural polymer, such as a crosslinked polysaccharide. An example of a polysaccharide matrix includes, but is not limited to, an agarose gel (for example, Superflow™ agarose or a Sepharose® material such as Superflow™ Sepharose® that are commercially available in different bead and pore sizes) or a gel of crosslinked dextran(s). A further illustrative example is a particulate cross-linked agarose matrix, to which dextran is covalently bonded, that is commercially available (in various bead sizes and with various pore sizes) as Sephadex® or Superdex®, both available from GE Healthcare. Another illustrative example of such a chromatography material is Sephacryl® which is also available in different bead and pore sizes from GE Healthcare.

In some disclosures, a crosslinked gel may also be based on a synthetic polymer, such as on a polymer class that does not occur in nature. Suitable examples disclosed herein include but are not limited to agarose gels or a gel of crosslinked dextran(s). A crosslinked gel may also be based on a synthetic polymer, i.e. on a polymer class that does not occur in nature. Usually such a synthetic polymer on which a chromatography stationary phase for cell separation is based is a polymer that has polar monomer units, and which is therefore in itself polar. Thus, in some cases disclosed herein, such a polar polymer is hydrophilic. Hydrophilic molecules, also termed lipophobic, in some disclosures contain moieties that can form dipole-dipole interactions with water molecules. In general, hydrophobic molecules, also termed lipophilic, have a tendency to separate from water.

Illustrative examples of suitable synthetic polymers are polyacrylamide(s), a styrenedivinylbenzene gel and a copolymer of an acrylate and a diol or of an acrylamide and a diol. An illustrative example is a polymethacrylate gel, commercially available as a Fractogel®. A further example is a copolymer of ethylene glycol and methacrylate, commercially available as a Toyopearl®. In some disclosures a chromatography stationary phase may also include natural and synthetic polymer components, such as a composite matrix or a composite or a co-polymer of a polysaccharide and agarose, e.g. a polyacrylamide/agarose composite, or of a polysaccharide and N,N'-methylenebisacrylamide. An illustrative example of a copolymer of a dextran and N,N'-methylenebisacryl¬amide is the above-mentioned Sephacryl® series of material. A derivatized silica may include silica particles that are coupled to a synthetic or to a natural polymer. Examples of such disclosures include, but are not limited to, polysaccharide grafted silica, polyvinyl¬pyrrolidone grafted silica, polyethylene oxide grafted silica, poly(2-hydroxyethylaspartamide) silica and poly(N-isopropylacrylamide) grafted silica.

A chromatography matrix employed in the present invention is in some disclosures a gel filtration (also known as size exclusion) matrix, for example, when used in a removal cartridge as described herein. A gel filtration can be characterized by the property that it is designed to undergo, at least essentially, no interaction with the cells to be separated. Hence, a gel filtration matrix allows the separation of cells or other biological entities as defined herein largely on the basis of their size. A respective chromatography matrix is typically a particulate porous material as mentioned above. The chromatography matrix may have a certain exclusion limit, which is typically defined in terms of a molecular weight above which molecules are entirely excluded from entering the pores. The respective molecular weight defining the size exclusion limit may be selected to be below the weight corresponding to the weight of a target cell (or biological entity) to be isolated. In such a disclosurethe target cell is prevented from entering the pores of the size exclusion chromatography matrix. Likewise, a stationary phase that is an affinity chromatography matrix may have pores that are of a size that is smaller than the size of a chosen target cell. In illustrative disclosures the affinity chromatography matrix and/or the gel filtration matrix has a mean pore size of 0 to about 500 nm.

Other components present in a sample such as receptor binding molecules or a competition reagent may have a size that is below the exclusion limit of the pores and this can enter the pores of the size exclusion chromatography matrix. Of such components that are able to partially or fully enter the pore volume, larger molecules, with less access to the pore volume will usually elute first, whereas the smallest molecules elute last. In some disclosures the exclusion limit of the size exclusion chromatography matrix is selected to be below the maximal width of the target cell. Hence, components that have access to the pore volume will usually remain longer in/on the size exclusion chromatography matrix than target cell. Thus, target cells can be collected in the eluate of a chromatography column separately from other matter/components of a sample. Therefore components such as a receptor binding reagent, or where, applicable a competition reagent, elute at a later point of time from a gel filtration matrix than the target cell. This separation effect will be further increased, if the gel permeation matrix comprises an affinity reagent (usually covalently bound thereon) that comprises binding sites, for example binding sites Z that are able to bind reagents such as a receptor binding reagent and/or a competition reagent present in a sample. The receptor binding reagent and/or the competition reagent will be bound by the binding sites Z of the affinity reagent and thereby immobilized on the gel permeation matrix. This method is usually carried out in a removal cartridge as used in the present invention and in some disclosures a method, a combination and a kit according to the invention include and/or employ such a gel filtration matrix. In a respective method cells are accordingly separated on the basis of size.

A chromatography matrix employed in the present invention may also include magnetically attractable matter such as one or more magnetically attractable particles or a ferrofluid. A respective magnetically attractable particle may comprise a multimerization reagent or an affinity reagent with binding site that is capable of binding a target cell. Magnetically attractable particles may contain diamagnetic, ferromagnetic, paramagnetic or superparamagnetic material. Superparamagnetic material responds to a magnetic field with an induced magnetic field without a resulting permanent magnetization. Magnetic particles based on iron oxide are for example commercially available as Dynabeads® from Dynal Biotech, as magnetic MicroBeads from Miltenyi Biotec, as magnetic porous glass beads from CPG Inc., as well as from various other sources, such as Roche Applied Science, BIOCLON, BioSource International Inc., micromod, AMBION, Merck, Bangs Laboratories, Polysciences, or Novagen Inc., to name only a few. Magnetic nanoparticles based on superparamagnetic Co and FeCo, as well as ferromagnetic Co nanocrystals have been described, for example by Hütten, A. et al. (J. Biotech. (2004), 112, 47-63). However, in some disclosures a chromatography matrix employed in the present invention is void of any magnetically attractable matter.

**Receptor Binding Reagent**

As described above, in certain disclosures, the methods disclosed herein employ a receptor binding reagent. In some disclosures, the reagent, as described in this Section, is a receptor binding reagent. In some disclosures, the receptor binding reagent binds to a molecule on the surface of a cell, such as a cell surface molecule. In some instances, the cell surface molecule is a selection marker. In some disclosures, the receptor binding reagent is capable of specifically binding to a selection marker expressed by one or more of the cells in a sample. In some disclosures, reference to specific binding to a molecule, such as a cell surface molecule or cell surface receptor, throughout the disclosure does not necessarily mean that the agent binds only to such molecule. For example, a reagent that specifically binds to a molecule may bind to other molecules, generally with much lower affinity as determined by, e.g., immunoassays, BIAcore®, KinExA 3000 instrument (Sapidyne Instruments, Boise, ID), or other assays. In some cases disclosed herein, the ability of a reagent, under specific binding conditions, to bind to a target molecule such that its affinity or avidity is at least 5 times as great, such as at least 10, 20, 30, 40, 50, 100, 250 or 500 times as great, or even at least 1000 times as great as the average affinity or avidity of the same agent to a collection of random peptides or polypeptides of sufficient statistical size.

In some disclosures, the cells, e.g., target cells (e.g., T cells), have or express a molecule on the cell surface, e.g., a selection marker, such that the cells to be selected are defined by the presence of at least one common specific molecule (e.g., selection marker). In some disclosures, the sample containing the target cell may also contain additional cells that are devoid of the molecule (e.g., selection marker). For example, in some disclosures, T cells may be selected from a sample containing multiple cells types, e.g., red blood cells or B cells. Selection marker and receptor molecule may be used interchangeably herein to refer to a cell surface molecule.

In some disclosures, the receptor molecule that is located on the cell surface, e.g., the target cell surface may be any molecule as long as it remains covalently or non-covalently bonded to the cell surface during a chromatographic separation process in a method according to the invention. The receptor molecule is a molecule against which a receptor binding reagent may be directed. In some disclosures the receptor is a peptide or a protein, such as a membrane receptor protein. In some disclosures the receptor is a lipid, a polysaccharide or a nucleic acid. A receptor that is a protein may be a peripheral membrane protein or an integral membrane protein. It may in some disclosures have one or more domains that span the membrane. In certain disclosures, the receptor molecule is a surface protein of an immune cell, e.g., CD4, CD8, or CD57. In some cases disclosed herein, for T cells the receptor molecule is CD3. In some cases disclosed herein, for T cells the receptor molecule is CD4 or CD8. In some disclosures the receptor molecule may be an antigen defining a desired cell population or subpopulation, for instance a population or subpopulation of blood cells, e. g. lymphocytes (e.g. T cells, CD57+ T cells, CD4+ T cells, or CD8+ T cells).

In some disclosures, the cell surface molecule, e.g., selection marker, may be an antigen defining a desired cell population or subpopulation, for instance a population or subpopulation of blood cells, e. g. lymphocytes (e.g. T cells, T-helper cells, for example, CD57-T cells, CD3+ T cells, CD8+ Tcells, CD4+ T-helper cells, B cells or natural killer cells), monocytes, or stem cells, e.g. CD34-positive peripheral stem cells or Nanog or Oct-4 expressing stem cells. In some disclosures, the selection marker can be a marker expressed on the surface of T cells or a subset of T cells, such as CD57, CD25, CD28, CD62L, CCR7, CD27, CD127, CD3, CD4, CD8, CD45RA, and/or CD45RO. Examples of T-cells include cells such as CMV-specific CD8+ T-lymphocytes, cytotoxic T-cells, memory T-cells and regulatory T-cells (Treg). An illustrative example of Treg includes CD4 CD25 CD45RA Treg cells and an illustrative example of memory T-cells includes CD62L CD8+ specific central memory T-cells.

In some disclosures, the receptor binding reagent has or contains a binding site B. In certain disclosures, the binding site B is monovalent. In some disclosures, a monovalent binding site B is or contains a monovalent antibody fragment or a proteinaceous binding molecule with immunoglobulin-like functions, an aptamer or an MHC molecule. Examples of monovalent antibody fragments include, but are not limited to a Fab fragment, an Fv fragment, and a single-chain Fv fragment (scFv), including a divalent single-chain Fv fragment. Examples of (recombinant) antibody fragments are Fab fragments, Fv fragments, single-chain Fv fragments (scFv), a divalent antibody fragment such as an (Fab)2'-fragment, diabodies, triabodies (Iliades, P., et al., FEBS Lett (1997) 409, 437-441), decabodies (Stone, E., et al., Journal of Immunological Methods (2007) 318, 88-94) and other domain antibodies (Holt, L.J., et al., Trends Biotechnol. (2003), 21, 11, 484-490). In some disclosures one or more binding sites of the receptor molecule binding reagent may be a bivalent proteinaceous artificial binding molecule such as a dimeric lipocalin mutein that is also known as "duocalin". In some disclosures the receptor binding reagent may have a single second binding site, i.e., it may be monovalent. Examples of monovalent receptor binding reagents include, but are not limited to, a monovalent antibody fragment, a proteinaceous binding molecule with antibody-like binding properties or an MHC molecule.

Yet further examples of suitable proteinaceous binding molecules are an EGF-like domain, a Kringle-domain, a fibronectin type I domain, a fibronectin type II domain, a fibronectin type III domain, a PAN domain, a G1a domain, a SRCR domain, a KunitzBovine pancreatic trypsin Inhibitor domain, tendamistat, a Kazal-type serine protease inhibitor domain, a Trefoil (P-type) domain, a von Willebrand factor type C domain, an Anaphylatoxin-like domain, a CUB domain, a thyroglobulin type I repeat, LDL-receptor class A domain, a Sushi domain, a Link domain, a Thrombospondin type I domain, an immunoglobulin domain or a an immunoglobulin-like domain (for example, domain antibodies or camel heavy chain antibodies), a C-type lectin domain, a MAM domain, a von Willebrand factor type A domain, a Somatomedin B domain, a WAP-type four disulfide core domain, a F5/8 type C domain, a Hemopexin domain, an SH2 domain, an SH3 domain, a Laminin-type EGF-like domain, a C2 domain, "Kappabodies" (cf. Ill. Et al., Protein Eng (1997) 10, 949-57, a so called "minibody" (Martin et al., EMBO J (1994) 13, 5303-5309), a diabody (cf. Holliger et al., PNAS USA (1993)90, 6444-6448), a so called "Janusis" (cf. Traunecker et al., EMBO J (1991) 10, 3655-3659, or Traunecker et al., Int J Cancer (1992) Suppl 7, 51-52), a nanobody, a microbody, an affilin, an affibody, a knottin, ubiquitin, a zinc-finger protein, an autofluorescent protein or a leucine-rich repeat protein. An example of a nucleic acid molecule with antibody-like functions is an aptamer. An aptamer folds into a defined three-dimensional motif and shows high affinity for a given target structure.

In particular disclosures, the receptor binding protein contains a binding partner C. In some disclosures, the binding partner C included in the receptor binding reagent may for instance be hydrocarbon-based (including polymeric) and include nitrogen-, phosphorus-, sulphur-, carben-, halogen- or pseudohalogen groups. It may be an alcohol, an organic acid, an inorganic acid, an amine, a phosphine, a thiol, a disulfide, an alkane, an amino acid, a peptide, an oligopeptide, a polypeptide, a protein, a nucleic acid, a lipid, a saccharide, an oligosaccharide, or a polysaccharide. As further examples, it may also be a cation, an anion, a polycation, a polyanion, a polycation, an electrolyte, a polyelectrolyte, a carbon nanotube or carbon nanofoam. Generally, such a binding partner has a higher affinity to the binding site of the multimerization reagent than to other matter. Examples of a respective binding partner include, but are not limited to, a crown ether, an immunoglobulin, a fragment thereof and a proteinaceous binding molecule with antibody-like functions.

In some disclosures the binding partner C that is included in the receptor binding reagent includes biotin and the affinity reagent includes a streptavidin analog or an avidin analog that reversibly binds to biotin. In some disclosures the binding partner C that is included in the receptor binding reagent includes a biotin analog that reversibly binds to streptavidin or avidin, and the affinity reagent includes streptavidin, avidin, a streptavidin analog or an avidin analog that reversibly binds to the respective biotin analog. In some disclosures the binding partner C that is included in the receptor binding reagent includes a streptavidin or avidin binding peptide and the affinity reagent includes streptavidin, avidin, a streptavidin analog or an avidin analog that reversibly binds to the respective streptavidin or avidin binding peptide.

In some disclosures the binding partner that is included in the receptor binding reagent may include a streptavidin-binding peptide In some disclosures, the peptide sequence contains a sequence with the general formula His-Pro-Xaa, where Xaa is glutamine, asparagine, or methionine, such as contained in the sequence set forth in SEQ ID NO: 78. In some disclosures, the peptide sequence has the general formula set forth in SEQ ID NO: 69, such as set forth in SEQ ID NO: 79. In one example disclosed herein, the peptide sequence is Trp-Arg-His-Pro-Gln-Phe-Gly-Gly (also called Strep-tag®, set forth in SEQ ID NO: 75). In one example disclosed herein, the peptide sequence is Ala-Trp-Arg-His-Pro-Gln-Phe-Gly-Gly (also called Strep-tag®, set forth in SEQ ID NO: 90). In one example disclosed herein, the peptide sequence is Trp-Ser-His-Pro-Gln-Phe-Glu-Lys (also called Strep-tag® II, set forth in SEQ ID NO: 69), which is described in US patent 6,103,493, for example, and is commercially available under the trademark Strep-Tactin®. The streptavidin binding peptides might, for example, be single peptides such as the "Strep-tag®" described in US patent 5,506,121, for example, or streptavidin binding peptides having a sequential arrangement of two or more individual binding modules as described in International Patent Publication WO 02/077018 or US patent 7,981,632.

In some disclosures, the binding partner C of the receptor binding reagent includes a moiety known to the skilled artisan as an affinity tag. In such a disclosure the affinity reagent includes a corresponding binding partner, for example, an antibody or an antibody fragment, known to bind to the affinity tag. As a few illustrative examples of known affinity tags, the binding partner that is included in the receptor binding reagent may include dinitrophenol or digoxigenin, oligohistidine, polyhistidine, an immunoglobulin domain, maltose-binding protein, glutathione-S-transferase (GST), chitin binding protein (CBP) or thioredoxin, calmodulin binding peptide (CBP), FLAG'-peptide, the HA-tag, the VSV-G-tag, the HSV-tag, the T7 epitope, maltose binding protein (MBP), the HSV epitope of the sequence of herpes simplex virus glycoprotein D, the "myc" epitope of the transcription factor c-myc of the sequence, the V5-tag, or glutathione-S-transferase (GST). In such a disclosure the complex formed between the one or more binding sites of the affinity reagent, in this case an antibody or antibody fragment, and the antigen can be disrupted competitively by adding the free antigen, i.e. the free peptide (epitope tag) or the free protein (such as MBP or CBP). The affinity tag might also be an oligonucleotide tag. Such an oligonucleotide tag may, for instance, be used to hybridize to an oligonucleotide with a complementary sequence, linked to or included in the affinity reagent.

In line with International Patent Application Publication No. WO 2013/011011 the strength of the binding between the receptor binding reagent and a receptor molecule on a target cell may not be not essential for the reversibility of the binding of the target cell to the affinity reagent via the receptor binding reagent. Rather, irrespective of the strength of the binding, meaning whether the equilibrium dissociation constant ($K_D$) for the binding between the receptor binding reagent via the binding site B and the receptor molecule is of low affinity, for example, in the range of a $K_D$ of about $10^{-3}$ to about $10^{-7}$ M, or of high affinity, for example, in the range of a $K_D$ of about $10^{-7}$ to about $1 \times 10^{-10}$ M, a target cell can be reversibly stained as long as the dissociation of the binding of the receptor binding reagent via the binding site B and the receptor molecule occurs sufficiently fast. In this regard the dissociation rate constant ($k_{off}$) for the binding between the receptor binding reagent via the binding site B and the receptor molecule may have a value of about $3 \times 10^{-5}$ sec$^{-1}$ or greater (this dissociation rate constant is the constant characterizing the dissociation reaction of the complex formed between the binding site B of the receptor binding reagent and the receptor molecule on the surface of the target cell). The association rate constant ($k_{on}$) for the association reaction between the binding site B of the receptor binding reagent and the receptor molecule on the surface of the target cell may have any value. In order to ensure a sufficiently reversible binding between receptor molecule and receptor binding reagent it is advantageous to select the $k_{off}$ value of the binding equilibrium to have a value of about $3 \times 10$-5 sec$^{-1}$ or greater, of about $5 \times 10^{-5}$ sec$^{-1}$ or greater, such as or as about $1 \times 10^{-4}$ sec$^{-1}$ or greater, $5 \times 10^{-4}$ sec$^{-1}$ or greater, $1 \times 10^{-3}$ sec$^{-1}$ or greater, $5 \times 10^{-3}$ sec$^{-1}$ or greater, a $1 \times 10^{-2}$ sec$^{-1}$ or greater, $1 \times 10^{-1}$ sec$^{-1}$ or greater or $5 \times 10^{-1}$ sec$^{-1}$ or greater. It is noted here that the values of the kinetic and thermodynamic constants as used herein, refer to conditions of atmospheric pressure, i.e. 1.013 bar, and room temperature, i.e. 25 °C.

In some disclosures the receptor binding reagent has a single (monovalent) binding site B capable of specifically binding to the receptor molecule. In some disclosures the receptor binding reagent has at least two (i.e., a plurality of binding sites B including three, four or also five identical binding sites B), capable of binding to the receptor molecule. In any of these disclosures the binding of the receptor molecule via (each of) the binding site(s) B may have a koff value of about $3 \times 10$-5 sec-1 or greater. Thus, the receptor binding reagent can be monovalent (for example a monovalent antibody fragment or a monovalent artificial binding molecule (proteinaceous or other) such as a mutein based on a polypeptide of the lipocalin family (also known as "Anticalin®"), or a bivalent molecule such as an antibody or a fragment in which both binding sites are retained such as an F(ab')2 fragment. In some disclosures the receptor molecule may be a multivalent molecule such as a pentameric IgE molecule, provided the koff rate is $3 \times 10$-5 sec-1 or greater. In some disclosures, the Fab is an anti-CD57 Fab. In particular disclosures, the Fab is an anti-CD4 Fab. In some disclosures, the Fab is an anti-CD8 Fab.

In some disclosures of the invention, it is on a molecular level not the koff rate (of $3 \times 10$-5 sec-1 or greater) of the binding of the receptor binding reagent via the at least binding site B and the receptor molecule on the target cell that provides for the (traceless) isolation of biological material via reversible cell affinity chromatography technology described here. Rather, and as described, for example, in US patent 7,776,562 or International Patent application WO02/054065, a low affinity binding between the receptor molecule and the binding site B of the binding receptor binding reagent together with an avidity effect mediated via the immobilized affinity reagent allows for a reversibly and traceless isolation of a target cell. In these disclosures a complex between the two or more binding sites Z of the affinity reagent and the binding partner C of at least two receptor binding reagents can form, allowing a reversible immobilization and subsequent elution of the target cells from the affinity chromatography matrix (via addition of the competing agent that will disrupt the binding (complex) formed between the binding partner C and the binding sites Z which in turn leads to the dissociation of the receptor binding reagent from the target cell. As mentioned above, such a low binding affinity may be characterized by a dissociation constant ($K_D$) in the range from about $1.0 \times 10^{-3}$ M to about $1.0 \times 10^{-7}$ M for the binding of the receptor binding reagent via the binding site B and the receptor molecule on the target cell surface.

In some disclosures, the selection marker may be CD57 and the receptor-binding agent specifically binds CD57. In some disclosures, the receptor-binding agent that specifically binds CD3 may be selected from the group consisting of an anti-CD57-antibody, a divalent antibody fragment of an anti-CD57 antibody, a monovalent antibody fragment of an anti-CD57-antibody, and a proteinaceous CD57 binding molecule with antibody-like binding properties. In some disclosures, the selection agent comprises an anti-CD57 Fab fragment.

In some disclosures, the selection marker may be CD4 and the receptor-binding agent specifically binds CD4. In some disclosures, the receptor-binding agent that specifically binds CD4 may be selected from the group consisting of an anti-CD4-antibody, a divalent antibody fragment of an anti-CD4 antibody, a monovalent antibody fragment of an anti-CD4-antibody, and a proteinaceous CD4 binding molecule with antibody-like binding properties. In some disclosures, an anti-CD4-antibody, such as a divalent antibody fragment or a monovalent antibody fragment (*e.g.* CD4 Fab fragment) can be derived from antibody 13B8.2 or a functionally active mutant of 13B8.2 that retains specific binding for CD4. For example, exemplary mutants of antibody 13B8.2 or m13B8.2 are described in U.S. Patent Nos. 7,482,000, U.S. Patent Appl. No. US2014/0295458 or International Patent Application No. WO2013/124474; and Bes, C, et al. J Biol Chem 278, 14265-14273 (2003). The mutant Fab fragment termed "ml3B8.2" carries the variable domain of the CD4 binding murine antibody 13B8.2 and a constant domain containing constant human CH1 domain of type gamma for the heavy chain and the constant human light chain domain of type kappa, as described in US Patent 7,482,000. In some disclosures, the anti-CD4 antibody, *e.g.* a mutant of antibody 13B8.2, contains the amino acid replacement H91A in the variable light chain, the amino acid replacement Y92A in the variable light chain, the amino acid replacement H35A in the variable heavy chain and/or the amino acid replacement R53A in the variable heavy chain, each by Kabat numbering. In some disclosures, compared to variable domains of the 13B8.2 Fab fragment in ml3B8.2 the His residue at position 91 of the light chain (position 93 in SEQ ID NO: 96) is mutated to Ala and the Arg residue at position 53 of the heavy chain (position 55 in SEQ ID NO: 95) is mutated to Ala. In some disclosures, the reagent that is reversibly bound to anti-CD4 or a fragment thereof is commercially available or derived from a reagent that is commercially available (e.g. catalog No. 6-8000-206 or 6-8000-205 or 6-8002-100; IBA GmbH, Gottingen, Germany). In some disclosures, the receptor-binding agent comprises an anti-CD4 Fab fragment. In some disclosures, the anti-CD4 Fab fragment comprises a variable heavy chain having the sequence set forth by SEQ ID NO:95 and a variable light chain having the sequence set forth by SEQ ID NO:96. In some disclosures, the anti-CD4 Fab fragment comprises the CDRs of the variable heavy chain having the sequence set forth by SEQ ID NO:95 and the CDRs of the variable light chain having the sequence set forth by SEQ ID NO:96.

In some disclosures, the selection marker may be CD8 and the receptor-binding agent specifically binds CD8. In some disclosures, the receptor-binding agent that specifically binds CD8 may be selected from the group consisting of an anti-CD8-antibody, a divalent antibody fragment of an anti-CD8 antibody, a monovalent antibody fragment of an anti-CD8-antibody, and a proteinaceous CD8 binding molecule with antibody-like binding properties. In some disclosures, an anti-CD8-antibody, such as a divalent antibody fragment or a monovalent antibody fragment (e.g. CD8 Fab fragment) can be derived from antibody OKT8 (e.g. ATCC CRL-8014) or a functionally active mutant thereof that retains specific binding for CD8. In some disclosures, the reagent that is reversibly bound to anti-CD8 or a fragment thereof is commercially available or derived from a reagent that is commercially available (e.g. catalog No. 6-8003 or 6-8000-201; IBA GmbH, Gottingen, Germany). In some disclosures, the receptor-binding agent comprises an anti-CD8 Fab fragment. In some disclosures, the anti-CD8 Fab fragment comprises a variable heavy chain having the sequence set forth by SEQ ID NO:97 and a variable light chain having the sequence set forth by SEQ ID NO:98. In some disclosures, the anti-CD8 Fab fragment comprises the CDRs of the variable heavy chain having the sequence set forth by SEQ ID NO:97 and the CDRs of the variable light chain having the sequence set forth by SEQ ID NO:98.

In some disclosures, the selection marker may be CD3 and the receptor-binding agent specifically binds CD3. In some disclosures, the receptor-binding agent that specifically binds CD3 may be selected from the group consisting of an anti-CD3-antibody, a divalent antibody fragment of an anti-CD3 antibody, a monovalent antibody fragment of an anti-CD3-antibody, and a proteinaceous CD3 binding molecule with antibody-like binding properties. In some disclosures, an anti-CD3-antibody, such as a divalent antibody fragment or a monovalent antibody fragment (e.g. CD3 Fab fragment) can be derived from antibody OKT3 (e.g. ATCC CRL-8001; see e.g., Stemberger et al. pLoS One. 2012; 7(4): e35798) or a functionally active mutant thereof that retains specific binding for CD3. In some disclosures, the reagent that is reversibly bound to anti-CD3 or a fragment thereof is commercially available or derived from a reagent that is commercially available (e.g. catalog No. 6-8000-201, 6-8001-100; IBA GmbH, Gottingen, Germany). In some disclosures, the receptor-binding agent comprises an anti-CD3 Fab fragment. In some disclosures, the anti-CD3 Fab fragment comprises a variable heavy chain having the sequence set forth by SEQ ID NO:93 and a variable light chain having the sequence set forth by SEQ ID NO:94. In some disclosures, the anti-CD3 Fab fragment comprises the CDRs of the variable heavy chain having the sequence set forth by SEQ ID NO:93 and the CDRs of the variable light chain having the sequence set forth by SEQ ID NO:94.

In any of the above disclosures, the divalent antibody fragment may be an (Fab)2'-fragment, or a divalent single-chain Fv fragment while the monovalent antibody fragment may be selected from the group consisting of a Fab fragment, an Fv fragment, and a single-chain Fv fragment (scFv). In any of the above disclosures, the proteinaceous binding molecule with antibody-like binding properties may be an aptamer, a mutein based on a polypeptide of the lipocalin family, a glubody, a protein based on the ankyrin scaffold, a protein based on the crystalline scaffold, an adnectin, and an avimer.

In certain disclosures, the isolation and/or selection by chromatographic isolation results in one or more populations of enriched T cells that includes at least 60%, at least 65%, at least 70%, at least 75%, at least 80%, at least 85%, at least 90%, at least 95%, at least 98%, at least 99%, at least 99.5%, at least 99.9%, or at or at about 100% CD57- CD3+ T cells. In particular disclosures, the population of enriched T cells consists essentially of CD57- CD3+ T cells.

In certain disclosures, the isolation and/or enrichment by chromatographic isolation results in a populations of enriched CD4+ T cells that includes at least 60%, at least 65%, at least 70%, at least 75%, at least 80%, at least 85%, at least 90%, at least 95%, at least 98%, at least 99%, at least 99.5%, at least 99.9%, or at or at about 100% CD57- CD4+ T cells. In certain disclosures, the input composition of CD4+ T cells includes less than 40%, less than 35%, less than 30%, less than 25%, less than 20%, less than 15%, less than 10%, less than 5%, less than 1%, less than 0.1%, or less than 0.01% CD8+ T cells, and/or contains no CD8+ T cells, and/or is free or substantially free of CD8+ T cells. In some disclosures, the population of enriched T cells consists essentially of CD57- CD4+ T cells.

In certain disclosures, the isolation and/or enrichment by chromatographic isolation results in a populations of enriched CD57- CD8+ T cells that includes at least 60%, at least 65%, at least 70%, at least 75%, at least 80%, at least 85%, at least 90%, at least 95%, at least 98%, at least 99%, at least 99.5%, at least 99.9%, or at or at about 100% CD57- CD8+ T cells. In certain disclosures, the population of CD8+ T cells contains less than 40%, less than 35%, less than 30%, less than 25%, less than 20%, less than 15%, less than 10%, less than 5%, less than 1%, less than 0.1%, or less than 0.01% CD4+ T cells, and/or contains no CD4+ T cells, and/or is free of or substantially free of CD4+ T cells. In some disclosures, the population of enriched T cells consists essentially of CD57- CD8+ T cells.

**D. Input Compsitions**

In certain disclosures, the disclosed methods are used in connection with producing or preparing an input population or composition of cells (input composition or input population are used herein interchangeably). In some disclosures, the input composition is cells is generated following any of the disclosed methods as described, e.g. infra, for selecting T cells from a biological sample (e.g. sample containing primary T cells, such as a leukapheresis or apheresis sample). In certain disclosures, the input cell composition includes a population of cells for use in genetic engineering, e.g., cells that will be genetically engineered or that will undergo a process to produce genetically engineered cells. In certain disclosures, the cells will be treated with, contacted with, or incubated with a nucleic acid that encodes a recombinant receptor. In certain disclosures, the input composition contains T cells, viable T cells, CD57- T cells, CD3+ T cells, CD4+ T cells, CD8+ T cells, and/or subpopulations thereof.

In some disclosures, cell viability is assessed with an assay that may include, but is not limited to, dye uptake assays (e.g., calcein AM assays), XTT cell viability assays, and dye exclusion assays (e.g., trypan blue, Eosin, or propidium dye exclusion assays). In particular disclosures, a viable cell has negative expression of one or more apoptotic markers, e.g., Annexin V or active Caspase 3. In some disclosures, the viable cell is negative for the expression of one or more apoptosis marker that may include, but are not limited to, a caspase or an active caspase, e.g., caspase 2, caspase 3, caspase 6, caspase 7, caspase 8, caspase 9, or caspase 10, Bcl-2 family members, e.g., Bax, Bad, and Bid, Annexin V, or TUNEL staining. In particular disclosures, the viable cells are active caspase 3 negative. In certain disclosures, the viable cells are Annexin V negative.

In some disclosures, the input composition comprises a population of enriched CD57-T cells, e.g., viable CD57-T cells. In some disclosures, at least 60%, at least 65%, at least 70%, at least 75%, at least 80%, at least 85%, at least 90%, at least 95%, at least 98%, at least 99%, at least 99.5%, at least 99.9%, or at or at about 100% of the cells of the input population are CD57- T cells, e.g., viable CD57- T cells. In some disclosures, the input population consists essentially of CD57- T cells, e.g., viable CD57- T cells.

In certain disclosures, the input population is a population of cells enriched for enriched CD4+ T cells and CD8+ T cells, e.g., CD4+ T cells and CD8+ T cells. In particular disclosures, the input population is or includes at least 60%, at least 65%, at least 70%, at least 75%, at least 80%, at least 85%, at least 90%, at least 95%, at least 98%, at least 99%, at least 99.5%, at least 99.9%, or at or at about 100% cells that are CD3+T cells. In particular disclosures, the input population is or includes at least 60%, at least 65%, at least 70%, at least 75%, at least 80%, at least 85%, at least 90%, at least 95%, at least 98%, at least 99%, at least 99.5%, at least 99.9%, or at or at about 100% cells that are CD4+ and CD8+T cells. In some disclosures, the input population consists essentially of CD4+ and CD8+ T cells. In particular disclosures, the input population is or includes at least 60%, at least 65%, at least 70%, at least 75%, at least 80%, at least 85%, at least 90%, at least 95%, at least 98%, at least 99%, at least 99.5%, at least 99.9%, or at or at about 100% cells CD3+ T cells (CD4+ and CD8+T cells) that are CD57-, e.g. viable CD57- T cells.

In certain disclosures, the input population is a population of enriched CD4+ T cells. In particular disclosures, at least 60%, at least 65%, at least 70%, at least 75%, at least 80%, at least 85%, at least 90%, at least 95%, at least 98%, at least 99%, at least 99.5%, at least 99.9%, or at or at about 100% of the cells of the input population are CD4+ T cells. In some disclosures, the input population consists essentially of CD4+ T cells. In particular disclosures, the input population is or includes at least 60%, at least 65%, at least 70%, at least 75%, at least 80%, at least 85%, at least 90%, at least 95%, at least 98%, at least 99%, at least 99.5%, at least 99.9%, or at or at about 100% cells CD4+T cells that are CD57-, e.g. viable CD57- T cells.

In certain disclosures, the input population is a population of enriched CD8+ T cells. In particular disclosures, at least 60%, at least 65%, at least 70%, at least 75%, at least 80%, at least 85%, at least 90%, at least 95%, at least 98%, at least 99%, at least 99.5%, at least 99.9%, or at or at about 100% of the cells of the input population are CD8+ T cells. In some disclosures, the input population consists essentially of CD8+ T cells. In particular disclosures, the input population is or includes at least 60%, at least 65%, at least 70%, at least 75%, at least 80%, at least 85%, at least 90%, at least 95%, at least 98%, at least 99%, at least 99.5%, at least 99.9%, or at or at about 100% cells CD8+T cells that are CD57-, e.g. viable CD57- T cells.

In some disclosures, cells from a population of enriched CD57-CD4+ T cells and cells from a population of enriched CD57-CD8+ T cells are mixed, combined, and/or pooled to generate an input population containing CD57-CD4+ T cells and CD57-CD8+ T cells. In certain disclosures, the populations of enriched CD57-CD4+ T cells and CD57-CD8+ T cells are pooled, mixed, and/or combined prior to stimulating cells, e.g., culturing the cells under stimulating conditions. In particular disclosures, the populations of enriched CD57-CD4+ and CD57-CD8+ T cells are pooled, mixed, and/or combined subsequent to freezing, e.g., cryopreserving, and thawing the populations of enriched CD57-CD4+ and CD57-CD8+ T cells.

In certain disclosures, the input population is produced, generated, or made by mixing, pooling, and/or combining cells from a population of enriched CD57-CD4+ cells with cells from a population of enriched CD57-CD8+ cells. In certain disclosures, the population of enriched CD57-CD4+ T cells contains at least 60%, 65%, 70%, 75%, 80%, 85%, 90%, 95%, 98%, 99%, or 99.9% CD57-CD4+ T cells. In particular disclosures, the population of enriched CD57-CD4+ T cells contains 100% CD57-CD4+ T cells or contains about 100% CD57-CD4+ T cells. In certain disclosures, the population of enriched T cells includes or contains less than 20%, less than 10%, less than 5%, less than 1%, less than 0.1%, or less than 0.01% CD57-CD8+ T cells, and/or contains no CD57-CD8+ T cells, and/or is free or substantially free of CD57-CD8+ T cells. In some disclosures, the populations of cells consist essentially of CD57-CD4+ T cells. In certain disclosures, the population of enriched CD57-CD8+ T cells contains at least 75%, 80%, 85%, 90%, 95%, 98%, 99%, or 99.9% CD57-CD8+ T cells, or contains or contains about 100% CD57-CD8+ T cells. In certain disclosures, the population of enriched CD57-CD8+ T cells includes or contains less than 20%, less than 10%, less than 5%, less than 1%, less than 0.1%, or less than 0.01% CD57-CD4+ T cells, and/or contains no CD57-CD4+ T cells, and/or is free or substantially free of CD57-CD4+ T cells. In some disclosures, the populations of cells consist essentially of CD57-CD8+ T cells.

In certain disclosures, CD4+ T cells and CD8+ T cells are pooled, mixed, and/or combined at a ratio of between 1:10 and 10:1, between 1:5 and 5:1, between 4:1 and 1:4, between 1:3 and 3:1, between 2:1 and 1:2, between 1.5:1 and 1:1.5, between 1.25:1 and 1:1.25, between 1.2:1 and 1:1.2, between 1.1:1 and 1:1.1, or about 1:1 or 1:1 CD4+ T cells to CD8+ T cells. In particular disclosures, viable CD4+ T cells and viable CD8+ T cells are pooled, mixed, and/or combined at a ratio of between 1:10 and 10:1, between 1:5 and 5:1, between 4:1 and 1:4, between 1:3 and 3:1, between 2:1 and 1:2, between 1.5:1 and 1:1.5, between 1.25:1 and 1:1.25, between 1.2:1 and 1:1.2, between 1.1:1 and 1:1.1, or about 1:1 or 1:1 CD4+ T cells to CD8+ T cells.

In particular disclosures, the input composition has an amount of, of about, or of at least $50 \times 10^6$, $100 \times 10^6$, $150 \times 10^6$, $200 \times 10^6$, $250 \times 10^6$, $300 \times 10^6$, $350 \times 10^6$, $400 \times 10^6$, $450 \times 10^6$, $500 \times 10^6$, $550 \times 10^6$, $600 \times 10^6$, $700 \times 10^6$, $800 \times 10^6$, $900 \times 10^6$, $1,000 \times 10^6$, $1,100 \times 10^6$, or $1,200 \times 10^6$ T cells, such as viable T cells, viable CD3+ T cells, or viable mixed CD4+ and CD8+ T cells. In particular disclosures, the input composition has an amount of, of about, or of at least $50 \times 10^6$, $100 \times 10^6$, $150 \times 10^6$, $200 \times 10^6$, $250 \times 10^6$, $300 \times 10^6$, $350 \times 10^6$, $400 \times 10^6$, $450 \times 10^6$, $500 \times 10^6$, $550 \times 10^6$, $600 \times 10^6$ CD4+ T cells, e.g., viable CD4+ T cells. In certain disclosures, the input composition has an amount of, of about, or of at least $50 \times 10^6$, $100 \times 10^6$, $150 \times 10^6$, $200 \times 10^6$, $250 \times 10^6$, $300 \times 10^6$, $350 \times 10^6$, $400 \times 10^6$, $450 \times 10^6$, $500 \times 10^6$, $550 \times 10^6$, $600 \times 10^6$ CD8+ T cells, e.g., viable CD8+ T cells. In some disclosures, the amount of cells is an amount of viable CD4+ and CD8+ T cells pooled, mixed and/or combined together in the same composition. In such disclosures, the CD4+ and CD8+ T cell are present at a ratio of between 1:3 and 3:1, between 2:1 and 1:2, between 1.5:1 and 1:1.5, between 1.25:1 and 1:1.25, between 1.2:1 and 1:1.2, between 1.1:1 and 1:1.1, or about 1:1 or 1:1 CD4+ T cells to CD8+ T cells. In some disclosures, the amount of cells is an amount of viable CD4+ and CD8+ T cells pooled, mixed and/or combined together at a ratio of about 1:1 or 1:1 CD4+ T cells to CD8+ T cells.

In particular disclosures, the input composition has an amount of between or between about $300 \times 10^6$ and $600 \times 10^6$ T cells, e.g., viable CD3+ cells, or mixed viable CD4+ and viable CD8+ cells (e.g., mixed at or at about a 1:1 ratio). In some disclosures, the input population has an amount of or of about $300 \times 10^6$, e.g., viable CD3+ cells, or mixed viable CD4+ and viable CD8+ cells (e.g., mixed at or at about a 1:1 ratio). In some disclosures, the input population has an amount of or of about $400 \times 10^6$, e.g., viable CD3+ cells or mixed viable CD4+ and viable CD8+ cells (e.g., mixed at or at about a 1:1 ratio). In some disclosures, the input population has an amount of or of about $500 \times 10^6$, e.g., viable CD3+ cells or mixed viable CD4+ and viable CD8+ cells (e.g., mixed at or at about a 1:1 ratio). In some disclosures, the input population has an amount of or of about $600 \times 10^6$, e.g., viable CD3+ cells or mixed viable CD4+ and viable CD8+ cells (e.g., mixed at or at about a 1:1 ratio). In some disclosures, the input population has an amount of or of about $700 \times 10^6$, e.g., viable CD3+ cells or mixed viable CD4+ and viable CD8+ cells (e.g., mixed at or at about a 1:1 ratio). In some disclosures, the input population has an amount of or of about $800 \times 10^6$, e.g., viable CD3+ cells or mixed viable CD4+ and viable CD8+ cells (e.g., mixed at or at about a 1:1 ratio). In some disclosures, the input population has an amount of or of about $900 \times 10^6$, e.g., viable CD3+ cells or mixed viable CD4+ and viable CD8+ cells (e.g., mixed at or at about a 1:1 ratio). In some disclosures, the input population has an amount of or of about $100 \times 10^7$, e.g., viable CD3+ cells or mixed viable CD4+ and viable CD8+ cells (e.g., mixed at or at about a 1:1 ratio). In some disclosures, the input population has an amount of or of about $110 \times 10^7$, e.g., viable CD3+ cells or mixed viable CD4+ and viable CD8+ cells (e.g., mixed at or at about a 1:1 ratio). In some disclosures, the input population has an amount of or of about $120 \times 10^7$, e.g., viable CD3+ cells or mixed viable CD4+ and viable CD8+ cells (e.g., mixed at or at about a 1:1 ratio).

In certain disclosures, CD4+ T cells and CD8+ T cells are pooled, mixed, and/or combined such that the input composition has up to or up to about a target number (2n) of T cells, such as viable T cells, viable CD3+ T cells, or viable mixed CD4+ and CD8+ T cells. In certain disclosures where a compositon comprising enriched CD4+ T cells contains at least n of CD4+ T cells and a compositon comprising enriched CD8+ T cells (e.g., derived from the same donor, e.g., from the same aphresis or leukaphresis sample from the donor, as the CD4+ T cell composition) contains at least n of CD8+ T cells, n of CD4+ T cells from the CD4+ T cell composition and n of CD8+ T cells from the CD8+ T cell composition are pooled, mixed, and/or combined (i.e. at 1:1 CD4+ to CD8+ ratio) to generate an input composition containing the target number (2n) of T cells. In certain disclosures where a compositon comprising enriched CD4+ T cells contains no more than (e.g., fewer than) n of CD4+ T cells and a compositon comprising enriched CD8+ T cells (e.g., derived from the same donor, e.g., from the same aphresis or leukaphresis sample from the donor, as the CD4+ T cell composition) contains no more than (e.g., fewer than) n of CD8+ T cells, all of the cells of the CD4+ T cell composition and all of the cells of the CD8+ T cell composition are pooled, mixed, and/or combined to generate the input composition. In these disclosures, the input composition may contain fewer than the target number (2n) of T cells. In certain disclosures where a compositon comprising enriched CD4+ T cells contains fewer than n of CD4+ T cells and a compositon comprising enriched CD8+ T cells (e.g., derived from the same donor, e.g., from the same aphresis or leukaphresis sample from the donor, as the CD4+ T cell composition) contains more than n of CD8+ T cells, or vice versa, cells of the CD4+ or CD8+ T cell composition are used to supplement the alternative cell type such that the input composition contains up to the target number (2n) of T cells. In any of the preceding disclosures, the target number 2n can be $300 \times 10^6$, $350 \times 10^6$, $400 \times 10^6$, $450 \times 10^6$, $500 \times 10^6$, $550 \times 10^6$, $600 \times 10^6$, $700 \times 10^6$, $800 \times 10^6$, $900 \times 10^6$, $1,000 \times 10^6$, $1,100 \times 10^6$, or $1,200 \times 10^6$ .

In certain disclosures, $450 \times 106$ CD4+ T cells from a compositon comprising enriched CD4+ T cells and $450 \times 10^6$ CD8+ T cells from a compositon comprising enriched CD8+ T cells (e.g., derived from the same donor, e.g., from the same aphresis or leukaphresis sample from the donor, as the CD4+ T cell composition) are pooled, mixed, and/or combined to generate an input composition containing $900 \times 10^6$ CD4+ and CD8+ T cells. In certain disclosures, when a compositon comprising enriched CD4+ T cells contains fewer than $450 \times 10^6$ CD4+ T cells and a compositon comprising enriched CD8+ T cells (e.g., derived from the same donor, e.g., from the same aphresis or leukaphresis sample from the donor, as the CD4+ T cell composition) contains fewer than $450 \times 10^6$ CD8+ T cells, all of the cells of the CD4+ T cell composition and all of the cells of the CD8+ T cell composition are pooled, mixed, and/or combined to generate the input composition. In certain disclosures, when either of the compositions contains fewer than $450 \times 10^6$ CD4+ or CD8+ cells while the other composition contains more than $450 \times 10^6$ CD8+ cells or CD4+ cells, then up to $900 \times 10^6$ CD4+ T cells and CD8+ T cells are combined to generate an input composition. The total number of CD4+ and CD8+ T cells in the input composition may be lower than $900 \times 10^6$. In other words, cells of the compositon comprising enriched CD4+ T cells may be used to supplement the compositon comprising enriched CD8+ T cells, or vice versa, in order to generate an input composition comprising up to the target number (2n) of T cells, e.g., up to $900 \times 10^6$ T cells to be subjected to stimulation.

Although in the above disclosures, the cell selection, isolation, separation, enrichment, and/or purification processes are discussed in the context of preparing an input composition, it should be understood that the cell selection, isolation, separation, enrichment, and/or purification processes disclosed herein can be used during, prior to, or between any of the subsequent steps (e.g., activation, stimulation, engineering, transduction, transfection, incubation, culturing, harvest, formulation, and/or administering a formulated cell population to a subject), in any suitable combination and/or order. For example, a T cell selection, isolation, separation, enrichment, and/or purification step can be performed between T cell activation/stimulation and T cell transduction. In another example disclosed herein, a T cell selection, isolation, separation, enrichment, and/or purification step can be performed after T cell transduction, but prior to harvesting, prior to collecting, and/or prior to formulating the cells. In a particular example disclosed herein, a T cell selection, isolation, separation, enrichment, and/or purification step can be performed immediately prior to harvesting the cells as a refining or clarification step. In some disclosures, a T cell selection step by chromatography is performed between T cell activation/stimulation and T cell transduction. In some disclosures, a T cell selection step by chromatography is performed after T cell transduction, but prior to harvesting, prior to collecting, and/or prior to formulating the cells. In some disclosures, a T cell selection step by chromatography is performed immediately prior to harvesting the cells.

In some disclosures, the input composition is subjected to one or more dilution and/or wash step, e.g., with a serum-free medium, prior to stimulating the cells, e.g., culturing the cells under stimulating conditions. In some disclosures, the dilution and/or wash step allows media exchange into a serum-free medium, such as one described in PCT/US2018/064627.

In some disclosures, the serum-free medium comprises a basal medium (e.g.OpTmizer™ T-Cell Expansion Basal Medium (ThermoFisher)), supplemented with one or more supplement. In some disclosures, the one or more supplement is serum-free. In some disclosures, the serum-free medium comprises a basal medium supplemented with one or more additional components for the maintenance, expansion, and/or activation of a cell (e.g., a T cell), such as provided by an additional supplement (e.g. OpTmizer™ T-Cell Expansion Supplement (ThermoFisher)). In some disclosures, the serum-free medium further comprises a serum replacement supplement, for example, an immune cell serum replacement, e.g., ThermoFisher, #A2596101, the CTS™ Immune Cell Serum Replacement, or the immune cell serum replacement described in Smith et al. Clin Transl Immunology. 2015 Jan; 4(1): e31. In some disclosures, the serum-free medium further comprises a free form of an amino acid such as L-glutamine. In some disclosures, the serum-free medium further comprises a dipeptide form of L-glutamine (e.g., L-alanyl-L-glutamine), such as the dipeptide in Glutamax™ (ThermoFisher). In some disclosures, the serum-free medium further comprises one or more recombinant cytokines, such as recombinant human IL-2, recombinant human IL-7, and/or recombinant human IL-15.

In some disclosures, the input composition is generated by mixing, combining, and/or pooling a population enriched in CD57-CD8+ T cells generated from a starting sample, such as PBMCs or a leukaphresis sample, with a population enriched in CD57-CD4+ T cells generated from the starting sample. In some disclosures, the population enriched in CD57-CD4+ T cells is generated from the CD8-negative fraction generated during the process of generating the population enriched in CD8+ T cells from the starting sample. In particular disclosures, the input composition has a ratio of or of about 1: 1 CD4+ T cells to CD8+ T cells, and is subjected to one or more wash step, e.g., with a serum-free medium described in PCT/US2018/064627, prior to stimulating the cells, e.g., culturing the cells under stimulating conditions. In some disclosures, the one or more wash step allows media exchange from a PBS/EDTA buffer containing albumin into the serum-free medium, which is also used in cell stimulation.

## III. PROCESS FOR GENETICALLY ENGINEERING POPULATIONS OF ENRICHED CD57- CELLS

Among disclosed methods are methods for genetically engineering T cells with a recombinant receptor, such as a chimeric antigen receptor (CAR) In some disclosures, the disclosed methods can include one or more steps of stimulating, activating, engineering, cultivating, and/or expanding one or more populations of enriched CD57- T cells. In certain disclosures, the one or more populations are or include any population of CD57- T cells described herein, e.g., in Section I. In some disclosures, the one or more populations are isolated, selected, or enriched from a biological sample by any method or process described herein, e.g., in Section II. In some disclosures, the one or more populations of enriched CD57-T cells are stimulated or activated, such as by incubating the cells of the population under stimulating conditions, such as any stimulating condition described herein, e.g., in Section III.A. In certain disclosures, the one or more populations of enriched CD57- T cells are genetically engineered, such as by introducing a heterologous polynucleotide to the cells of the one or more populations. In some disclosures, the introducing is performed by any method for generic engineering disclosed herein, e.g., in Section III.B. In some disclosures, the disclosed methods can include incubating transduced T cells under conditions to permit integration of the viral vector into the genome of the cells.

The disclosed methods can include methods in which the engineered cells are not further cultivated for the purpose of expanding the population of cells. For example, in some disclosures, the cells that are harvested have not undergone any incubation or cultivation where the amount of total viable cells is increased at the end of the incubation or cultivation as compared to the number of total viable cells at the beginning of the incubation or cultivation. In some disclosures, the cells that are harvested have not undergone any incubation or cultivation step explicitly for the purpose of increasing (e.g., expanding) the total number of viable cells at the end of the incubation or cultivation process compared to the beginning of said incubation or cultivation process. In some disclosures, the cells are incubated or cultivated under conditions that may result in expansion, but the incubating or cultivating conditions are not carried out for purposes of expanding the cell population. In some disclosures, the cells that are harvested may have undergone expansion despite having been manufactured in a process that does not include an expansion step. In some disclosures, a manufacturing process that does not include an expansion step is referred to as a non-expanded or minimally expanded process. A "non-expanded" process may also be referred to as a "minimally expanded" process. In some disclosures, a non-expanded or minimally expanded process may result in cells having undergone expansion despite the process not including a step for expansion. In some disclosures, the cells that are harvested may have undergone an incubation or cultivating step that includes a media composition designed to reduce, suppress, minimize, or eliminate expansion of a cell population as a whole. In some disclosures, the collected, harvested, or formulated cells have not previously undergone an incubation or cultivation that was performed in a bioreactor, or under conditions where the cells were rocked, rotated, shaken, or perfused for all or a portion of the incubation or cultivation.

In certain disclosures, the one or more populations of enriched CD57- T cells are cultivated, e.g., cultivated under conditions that promote or allow for T cell division, growth, or expansion, such as for a fixed amount of time or until a threshold limit for expansion is achieved. In some disclosures the cultivation is performed by any method described herein, such as in Section III.C.

In particular cases, disclosed herein are methods for generating genetically engineered T cell composition from one or more initial, e.g., input, populations of CD57- T cells. In some disclosures, a population of enriched CD57- T cells is incubated under stimulating conditions, thereby generating a stimulated population. In certain disclosures, the stimulating, e.g., culturing the cells under stimulating conditions, is performed for a set or fixed amount of time, such as an amount of time under 2 days or for an amount of time between 18 hours and 30 hours. In some disclosures, the stimulating with the stimulatory reagent is carried out for at or about 20 hours ± 4 hours.

In certain disclosures, a heterologous polynucleotide is introduced to cells of the stimulated population, thereby generating a transformed population. In particular disclosures, the cells are incubated either during or after genetically engineering the cells, for example, for an amount of time sufficient to allow for integration of a heterologous or recombinant polynucleotide encoding a recombinant protein or to allow for the expression of the recombinant protein. In certain disclosures, the cells are incubated for a set or fixed amount of time, such as an amount of time greater than 18 hours or less than 4 days, e.g., 72 hours ± 6 hours. In any of the disclosed examples, the introducing can be carried out on cells after they have been stimulated with the stimulatory reagent. In some disclosures, the engineering step is started or initiated within a set amount of time from when the stimulating is started or initiated, such as within 30 hours from when the stimulatory reagent is added, cultured, or contacted to the cells. In particular disclosures, the engineering step is started or initiated between 18 hours and 30 hours, such as 20 hours ± 4 hours, after the stimulatory reagent is added, cultured, or contacted to the cells.

In certain disclosures, the transformed population is then expanded, such as for a set amount of time or until a threshold expansion is achieved, thereby resulting in an expanded population. In particular disclosures, the transformed population or the expanded population is harvested or collected, and optionally formulated, such as for administration to a subject or for cryopreservation. In some disclosures, the population is or contains CD57- CD4+ T cells and CD57- CD8+ T cells. In some disclosures, the population is or contains CD57- CD3+ T cells.

In particular disclosures, the populations of enriched T cells may be collected, formulated for cryoprotection, frozen (e.g.,cryoprotected), and/or stored below 0°C, below - 20°C, or at or below -70C or -80°C prior to, during, or after any stage or step of the process for generating engineered populations of enriched T cells expressing recombinant receptors. In some disclosures, the cells may be stored for an amount of time under 1, 2, 3, 4, 5, 6, 7, 8, 9, or 10 days, or an amount of time under 1, 2, 3, 4, 5, 6, 7, 8 weeks, or for an amount of time at least 1, 2, 3, 4, 5, 6, 7, or 8 weeks, or for more than 8 weeks. After storage, the populations of enriched T cells may be thawed and the processing may be resumed from the same point in the process. In some disclosures, input populations of enriched T cells are cryoprotected and stored prior to further processing, e.g., incubation under stimulating conditions. In particular disclosures, cultivated and/or formulated populations of enriched T cells are cryoprotected and stored prior to being administered to as subject, e.g., as an autologous cell therapy.

In certain disclosures, the methods disclosed herein are used in connection with a process whereby engineered cells are generated by a process that includes steps for stimulating the cells and then introducing a polynucleotide encoding a recombinant receptor, e.g., a CAR, into the cells. In particular disclosures, the stimulating is performed for between 18 and 30 hours, such as for about 24 hours, and the introduction of the polynucleotide is subsequently performed. In certain disclosures, the cells are harvested or collected, such as to be formulated for cryopreservation or administrated to a subject, within 3 days after the introduction of the polynucleotide is initiated. In various disclosures, the cells are harvested or collected, such as to be formulated for cryopreservation or administered to a subject, within 4 days after the incubation under stimulatory conditions is initiated.

In certain disclosures, disclosed herein are methods for generating genetically engineered T cell composition from two initial, e.g., input, populations of CD57- T cells. In some disclosures, the two populations of enriched CD57- T cells are separately incubated under stimulating conditions, thereby generating two separate stimulated populations. In certain disclosures, a heterologous polynucleotide is introduced to cells of the two separate stimulated populations, thereby generating two separate transformed populations. In certain disclosures, the two separate transformed populations are then expanded, such as for a set amount of time or until a threshold expansion is achieved, thereby resulting in two separate expanded populations. In particular disclosures, the two separate transformed populations or the two separate expanded populations are harvested or collected, and optionally formulated, such as for administration to a subject or for cryopreservation. In particular disclosures, the two separate populations originate or are derived from the same biological sample or different biological samples from the same individual subject. In some disclosures, the two separate populations are or contain a population of enriched CD57- CD4+ T cells and a separate population of CD57- CD8+ T cells.

Also disclosed are methods for identifying a population of cells capable of expanding or proliferating, such as during an incubation or cultivation under conditions that promote T cell proliferation or expansion, such as any such conditions described herein, e.g., Section III.C. In some disclosures, such methods are or include measuring the frequency of CD57+ cells in the population, wherein if the frequency of CD57+ cells are below a threshold frequency, the population is capable of expanding. In some disclosures, the threshold frequency is less than 30%, 25%, 20%, 15%, 10%, 5%, or 1%. In some disclosures, the threshold is or is about 20%. In some disclosures, a population that is capable of expanding expands at least 2-fold, 3-fold, 4-fold, or 5-fold within 10, 11, 12, 13, or 14 days during a cultivation under conditions that promote proliferation or expansion. In certain disclosures, a population that is capable of expanding expands at least 4-fold within 11 days during a cultivation, e.g., a cultivation disclosed herein such as in Section III.C.

In some disclosures, the method is or includes measuring a value of a trait associated with CD57 expression of a population of T cells, wherein the population of T cells is capable of expansion cell therapy if the value of the trait is less than a threshold value of the trait. In some disclosures, the trait is a level or amount of a polypeptide encoded by the CD57 present in the total T cells, CD4+ T cells, or CD8+ T cells of the dose. In certain disclosures, the trait is a level or amount of a polypeptide encoded by the CD57 present on the surface of the total T cells, CD4+ T cells, or CD8+ T cells of the dose, in particular disclosures, the trait is a frequency, percentage, or amount of T cells, CD4+ T cells, or CD8+ T cells present positive for expression of the CD57. In some disclosures, the trait is a level or amount of mRNA of the second gene present in the T cells. In particular disclosures a level or amount of accessibility of the CD57.

In certain disclosures, the threshold value is at, at about, or within 25%, within 20%, within 15%, within 10%, or within 5% below a mean or median measurement of the trait associated with CD57 expression, and/or is below one standard deviation less than the mean or median measurement, in a plurality of reference T cell populations. In certain disclosures, the threshold value is below a lowest measurement of the trait associated with CD57 expression, optionally within 50%, within 25%, within 20%, within 15%, within 10%, or within 5% below the lowest measurement, in a population from among a plurality of reference T cell populations. In some disclosures, the threshold is below a mean or median measurement of the trait associated with CD57 expression calculated from among more than 65%, 75%, 80%, 85% of samples from a plurality of reference T cell compositions. In particular disclosures, the plurality of reference T cell populations are a plurality of populations that did not expand when cultivated under conditions that promote proliferation or expansion of T cells, optionally wherein the cells did not expand by at least 3-fold, 4-fold, or 5 fold, within 10, 11, 12, 13, or 14 days of cultivation, e.g., a cultivation as described herein, such as in Section III.C. In some disclosures, the reference T cell populations did not expand by at least 4-fold within 11 days of cultivation.

In some disclosures, the harvesting is performed at or after the time in which the engineered population or the expanded population of T cells include a threshold number of T cells, viable T cells, engineered T cells or viable engineered T cells, or a threshold concentration of T cells, viable T cells, engineered T cells or viable engineered T cells. In some disclosures, the threshold number or concentration of T cells, viable T cells, engineered T cells or viable engineered T cells is reached within at or about 4, 5, 6 or 7 days after the initiation of stimulation.

In some disclosures, among a plurality of populations of engineered T cells or populations of expanded T cells, the threshold number or concentration of T cells, viable T cells, engineered T cells or viable engineered T cells is reached within at or about 5 or 6 days after the initiation of stimulation in at least at or about or at least at or about 70%, 80%, 90% or 95% of the plurality. In some disclosures, the threshold number or concentration of T cells, viable T cells, engineered T cells or viable engineered T cells is reached within at or about 2, 3, 4 or 5 population doublings after the initiation of stimulation.

In some disclosures, the method also includes measuring a value of a trait associated with the expression of a second gene, such that the population is capable of expanding if the value of the trait associated with CD57 expression is less than the threshold value and if a trait associated with expression of the second gene is greater than a second threshold. In some disclosures, the second gene is a marker of a naïve-like cells, such as but not limited to CD25, CD27, CD28, CCR7, or CD45RA. In some disclosures, the second gene encodes CD27.

## A. Stimulation

In some disclosures, the disclosed methods are used in connection with incubating cells, e.g., CD57- T cells, under stimulating conditions. In some disclosures, the stimulating conditions include conditions that activate or stimulate, and/or are capable of activing or stimulating a signal in the cell, e.g., a CD4+ or a CD8+ T cell, such as a signal generated from a TCR and/or a coreceptor. In some disclosures, the stimulating conditions include one or more steps of culturing, cultivating, incubating, activating, propagating the cells with and/or in the presence of a stimulatory reagent, e.g., a reagent that activates or stimulates, and/or is capable of activing or stimulating a signal in the cell. In some disclosures, the stimulatory reagent stimulates and/or activates a TCR and/or a coreceptor. In particular disclosures, the stimulatory reagent is a reagent described in Section III.A. 1.

In certain disclosures, one or more populations of enriched CD57- T cells are incubated under stimulating conditions prior to genetically engineering the cells, e.g., transfecting and/or transducing the cell such as by a technique disclosed in Section III.B. In particular disclosures, one or more populations of enriched CD57- T cells are incubated under stimulating conditions after the one or more compositions have been isolated, selected, enriched, or obtained from a biological sample. In particular disclosures, the one or more populations of enriched CD57- T cells have been previously cryoperserved and stored, and are thawed prior to the incubation.

In certain disclosures, the one or more populations of enriched CD57- T cells are or include two separate populations of enriched CD57- T cells. In particular disclosures, the two separate compositions of enriched T cells, e.g., two separate compositions of enriched T cells selected, isolated, and/or enriched from the same biological sample, are separately incubated under stimulating conditions. In certain disclosures, the two separate compositions include a composition of enriched CD57- CD4+ T cells. In particular disclosures, the two separate compositions include a composition of enriched CD57- CD8+ T cells. In particular disclosures, the two separate compositions include a composition of enriched CD57- CD3+ T cells. In some disclosures, two separate compositions of enriched CD57- CD4+ T cells and enriched CD57-CD8+ T cells are separately incubated under stimulating conditions. In some disclosures, a single composition of enriched T cells is incubated under stimulating conditions. In certain disclosures, the single composition is a composition of enriched CD57- CD4+ T cells. In certain disclosures, the single composition is a composition of enriched CD57- CD8+ T cells. In certain disclosures, the single composition is a composition of enriched CD57- CD3+ T cells. In some disclosures, the single composition is a composition of enriched CD57-CD4+ and CD57- CD8+ T cells that have been combined from separate compositions prior to the incubation.

In some disclosures, the population of enriched CD57- CD4+ T cells that is incubated under stimulating conditions includes at least at or about 60%, at least at or about 65%, at least at or about 70%, at least at or about 75%, at least at or about 80%, at least at or about 85%, at least at or about 90%, at least at or about 95%, at least at or about 98%, at least at or about 99%, at least at or about 99.5%, at least at or about 99.9%, or at or at about 100% CD57-CD4+ T cells. In certain disclosures, the composition of enriched CD57- CD4+ T cells that is incubated under stimulating conditions includes less than at or about 40%, less than at or about 35%, less than at or about 30%, less than at or about 25%, less than at or about 20%, less than at or about 15%, less than at or about 10%, less than at or about 5%, less than at or about 1%, less than at or about 0.1%, or less than at or about 0.01% T cells that positive for CD57 expression or negative for CD4 expression.

In certain disclosures, the population of enriched CD57- CD8+ T cells that is incubated under stimulating conditions includes at least at or about 60%, at least at or about 65%, at least at or about 70%, at least at or about 75%, at least at or about 80%, at least at or about 85%, at least at or about 90%, at least at or about 95%, at least at or about 98%, at least at or about 99%, at least at or about 99.5%, at least at or about 99.9%, or at or at about 100% CD57-CD8+ T cells. In certain disclosures, the composition of enriched CD57- CD4+ T cells that is incubated under stimulating conditions includes less than at or about 40%, less than at or about 35%, less than at or about 30%, less than at or about 25%, less than at or about 20%, less than at or about 15%, less than at or about 10%, less than at or about 5%, less than at or about 1%, less than at or about 0.1%, or less than at or about 0.01% T cells that positive for CD57 expression or negative for CD8 expression. In certain disclosures, the composition of enriched CD57- CD8+ T cells that is incubated under stimulating conditions includes less than at or about 40%, less than at or about 35%, less than at or about 30%, less than at or about 25%, less than at or about 20%, less than at or about 15%, less than at or about 10%, less than at or about 5%, less than at or about 1%, less than at or about 0.1%, or less than at or about 0.01% T cells that positive for CD57 expression or negative for CD8 expression.

In certain disclosures, the population of enriched CD57- CD3+ T cells that is incubated under stimulating conditions includes at least at or about 60%, at least at or about 65%, at least at or about 70%, at least at or about 75%, at least at or about 80%, at least at or about 85%, at least at or about 90%, at least at or about 95%, at least at or about 98%, at least at or about 99%, at least at or about 99.5%, at least at or about 99.9%, or at or at about 100% CD57-CD3+ T cells. In certain disclosures, the composition of enriched CD57- CD3+ T cells that is incubated under stimulating conditions includes less than at or about 40%, less than at or about 35%, less than at or about 30%, less than at or about 25%, less than at or about 20%, less than at or about 15%, less than at or about 10%, less than at or about 5%, less than at or about 1%, less than at or about 0.1%, or less than at or about 0.01% T cells that positive for CD57 expression or negative for CD3 expression.

In certain disclosures, separate compositions of enriched CD57-CD4+ and CD57-CD8+ T cells are combined into a single composition and are incubated under stimulating conditions. In certain disclosures, separate stimulated compositions of enriched CD57-CD4+ and enriched CD57- CD8+ T cells are combined into a single composition after the incubation has been performed and/or completed.

In some disclosures, the incubation under stimulating conditions can include culture, cultivation, stimulation, activation, propagation, including by incubation in the presence of stimulating conditions, for example, conditions designed to induce proliferation, expansion, activation, and/or survival of cells in the population, to mimic antigen exposure, and/or to prime the cells for genetic engineering, such as for the introduction of a recombinant antigen receptor. In particular disclosures, the stimulating conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some disclosures, the stimulation and/or incubation under stimulating conditions is carried out in accordance with techniques such as those described in US Patent No. 6,040,177 to Riddell et al., Klebanoff et al.(2012) J Immunother. 35(9): 651-660, Terakuraet al. (2012) Blood. 1:72-82, and/or Wang et al. (2012) J Immunother. 35(9):689-701.

In some disclosures, the CD57- T cells are expanded by adding to the culture-initiating composition feeder cells, such as non-dividing peripheral blood mononuclear cells (PBMC), (e.g., such that the resulting population of cells contains at least about 5, 10, 20, or 40 or more PBMC feeder cells for each T lymphocyte in the initial population to be expanded); and incubating the culture (e.g. for a time sufficient to expand the numbers of T cells). In some disclosures, the non-dividing feeder cells can comprise gamma- irradiated PBMC feeder cells. In some disclosures, the PBMC are irradiated with gamma rays in the range of about 3000 to 3600 rads to prevent cell division. In some disclosures, the feeder cells are added to culture medium prior to the addition of the populations of T cells.

In some disclosures, the stimulating conditions include temperature suitable for the growth of human T lymphocytes, for example, at least about 25 °C, generally at least about 30 degrees, and generally at or about 37 °C. In some disclosures, a temperature shift is effected during culture, such as from 37 °C to 35 °C. Optionally, the incubation may further comprise adding non-dividing EBV-transformed lymphoblastoid cells (LCL) as feeder cells. LCL can be irradiated with gamma rays in the range of about 6000 to 10,000 rads. The LCL feeder cells in some disclosures is provided in any suitable amount, such as a ratio of LCL feeder cells to initial T lymphocytes of at least about 10:1.

In particular disclosures, the stimulating conditions include incubating, culturing, and/or cultivating the cells with a stimulatory reagent. In particular disclosures, the stimulatory reagent is a reagent described in Section III.A. 1. In certain disclosures, the stimulatory reagent contains or includes a bead. In particular disclosures, the stimulatory reagent contains or includes an oligomeric reagent, e.g., an oligomeric streptavidin mutein reagent. In certain disclosures, the start and or initiation of the incubation, culturing, and/or cultivating cells under stimulating conditions occurs when the cells are come into contact with and/or are incubated with the stimulatory reagent. In particular disclosures, the cells are incubated prior to, during, and/or subsequent to genetically engineering the cells, e.g., introducing a recombinant polynucleotide into the cell such as by transduction or transfection. In some disclosures, the composition of enriched T cells are incubated at a ratio of stimulatory reagent and/or beads to cells at or at about 3:1, 2.5:1, 2:1, 1.5:1, 1.25:1, 1.2:1, 1.1:1, 1:1, 0.9:1, 0.8:1, 0.75:1, 0.67:1, 0.5:1, 0.3:1, or 0.2:1. In particular disclosures, the ratio of stimulatory reagent and/or beads to cells is between 2.5:1 and 0.2:1, between 2:1 and 0.5:1, between 1.5:1 and 0.75:1, between 1.25:1 and 0.8:1, between 1.1:1 and 0.9:1. In particular disclosures, the ratio of stimulatory reagent to cells is about 1:1 or is 1:1.

In some disclosures, the cells are stimulated in the presence of, of about, or of at least 0.01 $\mu$g, 0.02 $\mu$g, 0.03 $\mu$g, 0.04 $\mu$g, 0.05 $\mu$g, 0.1 $\mu$g, 0.2 $\mu$g, 0.3 $\mu$g, 0.4 $\mu$g, 0.5 $\mu$g, 0.75 $\mu$g, 1 $\mu$g, 2 $\mu$g, 3 $\mu$g, 4 $\mu$g, 5 $\mu$g, 6 $\mu$g, 7 $\mu$g, 8 $\mu$g, 9 $\mu$g, or 10 $\mu$g of the stimulatory reagent per $10^6$ cells. In some disclosures, the cells are stimulated in the presence of or of about 4 $\mu$g of the stimulatory reagent per $10^6$ cells. In particular disclosures, the cells are stimulated in the presence of or of about 0.8 $\mu$g of the stimulatory reagent per $10^6$ cells.

In certain disclosures, the cells, e.g., cells of the input population, are stimulated or subjected to stimulation e.g., cultured under stimulating conditions such as in the presence of a stimulatory reagent, at a density of, of about, or at least 0.01 x $10^6$ cells/mL, 0.1 x $10^6$ cells/mL, 0.5 x $10^6$ cells/mL, 1.0 x $10^6$ cells/mL, 1.5 x $10^6$ cells/mL, 2.0 x $10^6$ cells/mL, 2.5 x $10^6$ cells/mL, 3.0 x $10^6$ cells/mL, 4.0 x $10^6$ cells/mL, 5.0 x $10^6$ cells/mL, 10 x $10^6$ cells/mL, or 50 x $10^6$ cells/mL. In certain disclosures, the cells, e.g., cells of the input population, are stimulated or subjected to stimulation e.g., cultured under stimulating conditions such as in the presence of a stimulatory reagent, at a density of, of about, or at least 3.0 x $10^6$ cells/mL. In certain disclosures, the cells of the input are viable cells.

In some disclosures, the compositions or cells are incubated in the presence of stimulating conditions or a stimulatory agent. Such conditions include those designed to induce proliferation, expansion, activation, and/or survival of cells in the population, to mimic antigen exposure, and/or to prime the cells for genetic engineering, such as for the introduction of a recombinant antigen receptor. Exemplary stimulatory reagents are described below.

In some disclosures, at least a portion of the incubation in the presence of one or more stimulating conditions or a stimulatory agents is carried out in the internal cavity of a centrifugal chamber, for example, under centrifugal rotation, such as described in International Publication Number WO2016/073602. In some disclosures, at least a portion of the incubation performed in a centrifugal chamber includes mixing with a reagent or reagents to induce stimulation and/or activation. In some disclosures, cells, such as selected cells, are mixed with a stimulating condition or stimulatory agent in the centrifugal chamber. In some disclosures of such processes, a volume of cells is mixed with an amount of one or more stimulating conditions or agents that is far less than is normally employed when performing similar stimulations in a cell culture plate or other system.

In some disclosures, the stimulating agent is added to cells in the cavity of the chamber in an amount that is substantially less than (e.g. is no more than 5%, 10%, 20%, 30%, 40%, 50%, 60%, 70% or 80% of the amount) as compared to the amount of the stimulating agent that is typically used or would be necessary to achieve about the same or similar efficiency of selection of the same number of cells or the same volume of cells when selection is performed without mixing in a centrifugal chamber, e.g. in a tube or bag with periodic shaking or rotation. In some disclosures, the incubation is performed with the addition of an incubation buffer to the cells and stimulating agent to achieve a target volume with incubation of the reagent of, for example, 10 mL to 200 mL, such as at least or about at least or about or 10 mL, 20 mL, 30 mL, 40 mL, 50 mL, 60 mL, 70 mL, 80 mL, 90 mL, 100 mL, 150 mL or 200 mL. In some disclosures, the incubation buffer and stimulating agent are pre-mixed before addition to the cells. In some disclosures, the incubation buffer and stimulating agent are separately added to the cells. In some disclosures, the stimulating incubation is carried out with periodic gentle mixing condition, which can aid in promoting energetically favored interactions and thereby permit the use of less overall stimulating agent while achieving stimulating and activation of cells.

In some disclosures, the incubation generally is carried out under mixing conditions, such as in the presence of spinning, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm), such as at an RCF at the sample or wall of the chamber or other container of from or from about 80g to 100g (e.g. at or about or at least 80 g, 85 g, 90 g, 95 g, or 100 g). In some disclosures, the spin is carried out using repeated intervals of a spin at such low speed followed by a rest period, such as a spin and/or rest for 1, 2, 3, 4, 5, 6, 7, 8, 9, or 10 seconds, such as a spin at approximately 1 or 2 seconds followed by a rest for approximately 5, 6, 7, or 8 seconds.

In some disclosures, the total duration of the incubation, e.g. with the stimulating agent, is between or between about 1 hour and 96 hours, 1 hour and 72 hours, 1 hour and 48 hours, 4 hours and 36 hours, 8 hours and 30 hours or 12 hours and 24 hours, such as at least or about at least 6 hours, 12 hours, 18 hours, 24 hours, 36 hours or 72 hours. In some disclosures, the further incubation is for a time between or about between 1 hour and 48 hours, 4 hours and 36 hours, 8 hours and 30 hours or 12 hours and 24 hours, inclusive.

In some disclosures, the stimulation, e.g. culturing the cells under stimulating conditions, is performed for, for about, or for less than, 48 hours, 42 hours, 36 hours, 30 hours, 24 hours, 22 hours, 20 hours, 18 hours, 16 hours, or 12 hours. In some disclosures, the stimulation, e.g. culturing the cells under stimulating conditions, is performed for $20 \pm 4$ hours or between or between about 16 hours and 24 hours. In particular disclosures, the stimulation, e.g. culturing the cells under stimulating conditions, is performed for between or between about 36 hours and 12 hours, 30 hours and 18 hours, or for or for about 24 hours, or 22 hours. In some disclosures, the stimulation, e.g. culturing the cells under stimulating conditions, is performed for, for about, or for less than, 2 days or one day.

In particular disclosures, an amount of, of about, or of at least $50 \times 10^6$, $100 \times 10^6$, $150 \times 10^6$, $200 \times 10^6$, $250 \times 10^6$, $300 \times 10^6$, $350 \times 10^6$, $400 \times 10^6$, $450 \times 10^6$, $500 \times 10^6$, $550 \times 10^6$, $600 \times 10^6$, $700 \times 10^6$, $800 \times 10^6$, $900 \times 10^6$, or $1,000 \times 10^6$ cells of the input population are stimulated or subjected to stimulation, e.g., cultured under stimulating conditions. In particular disclosures, the amount of the input population that are stimulated, e.g., cultured under stimulating conditions, is at or about $50 \times 10^6$ cells, at or about $100 \times 10^6$ cells, at or about $150 \times 10^6$ cells, at or about $200 \times 10^6$ cells, at or about $250 \times 10^6$ cells, at or about $300 \times 10^6$ cells, at or about $350 \times 10^6$ cells, at or about $400 \times 10^6$ cells, at or about $450 \times 10^6$ cells, at or about $500 \times 10^6$ cells, at or about $550 \times 10^6$ cells, at or about $600 \times 10^6$ cells, at or about $700 \times 10^6$ cells, at or about $800 \times 10^6$ cells, at or about $900 \times 10^6$ cells, or at or about $1,000 \times 10^6$ cells, or any value between any of the foregoing. In particular disclosures, an amount of or of about $900 \times 10^6$ cells of the input population are stimulated, e.g., cultured under stimulating conditions.

In particular disclosures, the input composition comprises viable CD4+ T cells and viable CD8+ T cells, at a ratio of between 1:10 and 10:1, between 1:5 and 5:1, between 4:1 and 1:4, between 1:3 and 3:1, between 2:1 and 1:2, between 1.5:1 and 1:1.5, between 1.25:1 and 1:1.25, between 1.2:1 and 1:1.2, between 1.1:1 and 1:1.1, or about 1:1, or 1:1 viable CD4+ T cells to viable CD8+ T cells. In particular disclosures, the input composition comprises viable CD4+ T cells and viable CD8+ T cells, at a ratio of about 1:1 or 1:1 viable CD4+ T cells to viable CD8+ T cells. In particular disclosures, an amount of or of about $450 \times 10^6$ CD4+ cells of the input population are stimulated, e.g., cultured under stimulating conditions. In particular disclosures, an amount of or of about $450 \times 10^6$ CD8+ cells of the input population are stimulated, e.g., cultured under stimulating conditions. In particular disclosures, an amount of or of about $450 \times 10^6$ CD4+ T cells and an amount of or of about $450 \times 10^6$ CD8+ T cells of the input population are stimulated, e.g., cultured under stimulating conditions.

In particular disclosures, the stimulating conditions include incubating, culturing, and/or cultivating a composition of enriched T cells with and/or in the presence of one or more cytokines. In particular disclosures, the one or more cytokines are recombinant cytokines. In some disclosures, the one or more cytokines are human recombinant cytokines. In certain disclosures, the one or more cytokines bind to and/or are capable of binding to receptors that are expressed by and/or are endogenous to T cells. In particular disclosures, the one or more cytokines is or includes a member of the 4-alpha-helix bundle family of cytokines. In some disclosures, members of the 4-alpha-helix bundle family of cytokines include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF). In some disclosures, the one or more cytokines is or includes IL-15. In particular disclosures, the one or more cytokines is or includes IL-7. In particular disclosures, the one or more cytokines is or includes IL-2.

In certain disclosures, the amount or concentration of the one or more cytokines are measured and/or quantified with International Units (IU). International units may be used to quantify vitamins, hormones, cytokines, vaccines, blood products, and similar biologically active substances. In some disclosures, IU are or include units of measure of the potency of biological preparations by comparison to an international reference standard of a specific weight and strength e.g., WHO 1st International Standard for Human IL-2, 86/504. International Units are the only recognized and standardized method to report biological activity units that are published and are derived from an international collaborative research effort. In particular disclosures, the IU for population, sample, or source of a cytokine may be obtained through product comparison testing with an analogous WHO standard product. For example, in some disclosures, the IU/mg of a population, sample, or source of human recombinant IL-2, IL-7, or IL-15 is compared to the WHO standard IL-2 product (NIBSC code: 86/500), the WHO standard IL-17 product (NIBSC code: 90/530) and the WHO standard IL-15 product (NIBSC code: 95/554), respectively.

In some disclosures, the biological activity in IU/mg is equivalent to (ED50 in ng/ml)-1 x106. In particular disclosures, the ED50 of recombinant human IL-2 or IL-15 is equivalent to the concentration required for the half-maximal stimulation of cell proliferation (XTT cleavage) with CTLL-2 cells. In certain disclosures, the ED50 of recombinant human IL-7 is equivalent to the concentration required for the half-maximal stimulation for proliferation of PHA-activated human peripheral blood lymphocytes. Details relating to assays and calculations of IU for IL-2 are discussed in Wadhwa et al., Journal of Immunological Methods (2013), 379 (1-2): 1-7; and Gearing and Thorpe, Journal of Immunological Methods (1988), 114 (1-2): 3-9; details relating to assays and calculations of IU for IL-15 are discussed in Soman et al. Journal of Immunological Methods (2009) 348 (1-2): 83-94.

In some disclosures, the cells, e.g., the input cells, are stimulated or subjected to stimulation in the presence of a cytokine, e.g., a recombinant human cytokine, at a concentration of between 1 IU/mL and 1,000 IU/mL, between 10 IU/mL and 50 IU/mL, between 50 IU/mL and 100 IU/mL, between 100 IU/mL and 200 IU/mL, between 100 IU/mL and 500 IU/mL, between 250 IU/mL and 500 IU/mL, or between 500 IU/mL and 1,000 IU/mL.

In some disclosures, the cells, e.g., the input cells, are stimulated or subjected to stimulationin the presence of recombinant IL-2, e.g., human recombinant IL-2, at a concentration between 1 IU/mL and 500 IU/mL, between 10 IU/mL and 250 IU/mL, between 50 IU/mL and 200 IU/mL, between 50 IU/mL and 150 IU/mL, between 75 IU/mL and 125 IU/mL, between 100 IU/mL and 200 IU/mL, or between 10 IU/mL and 100 IU/mL. In particular disclosures, cells, e.g., cells of the input population, are stimulated or subjected to stimulationin the presence of recombinant IL-2 at a concentration at or at about 50 IU/mL, 60 IU/mL, 70 IU/mL, 80 IU/mL, 90 IU/mL, 100 IU/mL, 110 IU/mL, 120 IU/mL, 130 IU/mL, 140 IU/mL, 150 IU/mL, 160 IU/mL, 170 IU/mL, 180 IU/mL, 190 IU/mL, or 100 IU/mL. In some disclosures, the cells, e.g., the input cells, are stimulated or subjected to stimulationin the presence of or of about 100 IU/mL of recombinant IL-2, e.g., human recombinant IL-2.

In some disclosures, the cells, e.g., the input cells, are stimulated or subjected to stimulationin the presence of recombinant IL-7, e.g., human recombinant IL-7, at a concentration between 100 IU/mL and 2,000 IU/mL, between 500 IU/mL and 1,000 IU/mL, between 100 IU/mL and 500 IU/mL, between 500 IU/mL and 750 IU/mL, between 750 IU/mL and 1,000 IU/mL, or between 550 IU/mL and 650 IU/mL. In particular disclosures, the cells, e.g., the input cells, are stimulated or subjected to stimulationin the presence of IL-7 at a concentration at or at about 50 IU/mL, 100 IU/mL, 150 IU/mL, 200 IU/mL, 250 IU/mL, 300 IU/mL, 350 IU/mL, 400 IU/mL, 450 IU/mL, 500 IU/mL, 550 IU/mL, 600 IU/mL, 650 IU/mL, 700 IU/mL, 750 IU/mL, 800 IU/mL, 750 IU/mL, 750 IU/mL, 750 IU/mL, or 1,000 IU/mL. In particular disclosures, the cells, e.g., the input cells, are stimulated or subjected to stimulationin the presence of or of about 600 IU/mL of recombinant IL-7, e.g., human recombinant IL-7.

In some disclosures, the cells, e.g., the input cells, are stimulated or subjected to stimulation in the presence of recombinant IL-15, e.g., human recombinant IL-15, at a concentration between 1 IU/mL and 500 IU/mL, between 10 IU/mL and 250 IU/mL, between 50 IU/mL and 200 IU/mL, between 50 IU/mL and 150 IU/mL, between 75 IU/mL and 125 IU/mL, between 100 IU/mL and 200 IU/mL, or between 10 IU/mL and 100 IU/mL. In particular disclosures, cells, e.g., a cell of the input population, are stimulated or subjected to stimulationin the presence of recombinant IL-15 at a concentration at or at about 50 IU/mL, 60 IU/mL, 70 IU/mL, 80 IU/mL, 90 IU/mL, 100 IU/mL, 110 IU/mL, 120 IU/mL, 130 IU/mL, 140 IU/mL, 150 IU/mL, 160 IU/mL, 170 IU/mL, 180 IU/mL, 190 IU/mL, or 200 IU/mL. In some disclosures, the cells, e.g., the input cells, are stimulated or subjected to stimulationin the presence of or of about 100 IU/mL of recombinant IL-15, e.g., human recombinant IL-15.

In particular disclosures, the cells, e.g., cells from the input population, are stimulated or subjected to stimulationunder stimulating conditions in the presence of IL-2, IL-7, and/or IL-15. In some disclosures, the IL-2, IL-7, and/or IL-15 are recombinant. In certain disclosures, the IL-2, IL-7, and/or IL-15 are human. In particular disclosures, the one or more cytokines are or include human recombinant IL-2, IL-7, and/or IL-15. In certain disclosures, the cells are stimulated or subjected to stimulationunder stimulating conditions in the presence of recombinant IL-2, IL-7, and IL-15. In certain disclosures, the cells are stimulated or subjected to stimulationunder stimulating conditions in the presence of recombinant IL-2 of or of about 100 IU/mL, recombinant IL-7 of or of about 600 IU/mL, and recombinant IL-15 of or of about 100 IU/mL.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some disclosures, stimulation is carried out in accordance with techniques such as those described in US Patent No. 6,040,1 77 to Riddell et al., Klebanoff et al.(2012) J Immunother. 35(9): 651-660, Terakura et al. (2012) Blood. 1:72-82, and/or Wang et al. (2012) J Immunother. 35(9):689-701.

In some disclosures, the stimulation is performed in serum free media. In some disclosures, the serum free media is a defined and/or well-defined cell culture media. In certain disclosures, the serum free media is a controlled culture media that has been processed, e.g., filtered to remove inhibitors and/or growth factors. In some disclosures, the serum free media contains proteins. In certain disclosures, the serum-free media may contain serum albumin, hydrolysates, growth factors, hormones, carrier proteins, and/or attachment factors.

In some disclosures, the stimulation is performed in serum free media described herein or in PCT/US2018/064627. In some disclosures, the serum-free medium comprises a basal medium (e.g.OpTmizer™ T-Cell Expansion Basal Medium (ThermoFisher)), supplemented with one or more supplement. In some disclosures, the one or more supplement is serum-free. In some disclosures, the serum-free medium comprises a basal medium supplemented with one or more additional components for the maintenance, expansion, and/or activation of a cell (e.g., a T cell), such as provided by an additional supplement (e.g. OpTmizer™ T-Cell Expansion Supplement (ThermoFisher)). In some disclosures, the serum-free medium further comprises a serum replacement supplement, for example, an immune cell serum replacement, e.g., ThermoFisher, #A2596101, the CTS™ Immune Cell Serum Replacement, or the immune cell serum replacement described in Smith et al. Clin Transl Immunology. 2015 Jan; 4(1): e31. In some disclosures, the serum-free medium further comprises a free form of an amino acid such as L-glutamine. In some disclosures, the serum-free medium further comprises a dipeptide form of L-glutamine (e.g., L-alanyl-L-glutamine), such as the dipeptide in Glutamax™ (ThermoFisher). In some disclosures, the serum-free medium further comprises one or more recombinant cytokines, such as recombinant human IL-2, recombinant human IL-7, and/or recombinant human IL-15. In some disclosures, at least a portion of the stimulation in the presence of one or more stimulating conditions or a stimulatory reagent is carried out in the internal cavity of a centrifugal chamber, for example, under centrifugal rotation, such as described in International Publication Number WO2016/073602. In some disclosures, at least a portion of the stimulation performed in a centrifugal chamber includes mixing with a reagent or reagents to induce stimulation and/or activation. In some disclosures, cells, such as selected cells, are mixed with a stimulating condition or stimulatory agent in the centrifugal chamber. In some disclosures of such processes, a volume of cells is mixed with an amount of one or more stimulating conditions or agents that is far less than is normally employed when performing similar stimulations in a cell culture plate or other system.

In some disclosures, the stimulation generally is carried out under mixing conditions, such as in the presence of spinning, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm), such as at an RCF at the sample or wall of the chamber or other container of from or from about 80g to 100g (e.g. at or about or at least 80 g, 85 g, 90 g, 95 g, or 100 g). In some disclosures, the spin is carried out using repeated intervals of a spin at such low speed followed by a rest period, such as a spin and/or rest for 1, 2, 3, 4, 5, 6, 7, 8, 9, or 10 seconds, such as a spin at approximately 1 or 2 seconds followed by a rest for approximately 5, 6, 7, or 8 seconds.

In certain disclosures, the stimulation is performed under static conditions, such as conditions that do not involve centrifugation, shaking, rotating, rocking, or perfusion, e.g., continuous or semi-continuous perfusion of the media. In some disclosures, either prior to or shortly after, e.g., within 5, 15, or 30 minutes, the initiation, the cells are transferred (e.g., transferred under sterile conditions) to a container such as a bag or vial, and placed in an incubator. In particular disclosures, incubator is set at, at about, or at least 16°C, 24°C, or 35°C. In some disclosures, the incubator is set at 37°C, at about at 37°C, or at 37°C $\pm2$°C, $\pm1$°C, $\pm0.5$°C, or $\pm0.1$°C. In particular disclosures, the stimulation under static condition is performed in a cell culture bag placed in an incubator. In some disclosures, the culture bag is composed of a single-web polyolefin gas permeable film which enables monocytes, if present, to adhere to the bag surface.

In particular disclosures, the methods employ reversible systems in which at least one reagent (*e.g.,* an affinity reagent or a stimulatory reagent) capable of binding to a molecule on the surface of a cell (cell surface molecule), is reversibly associated with a reagent (e.g., affinity reagent or stimulatory reagent). In some cases disclosed herein, the reagent contains a plurality of binding sites capable of reversibly binding to the reagent (*e.g.,* an affinity reagent or stimulatory reagent). In some cases disclosed herein, the reagent (e.g., affinity reagent or stimulatory reagent) is a multimerization reagent. In some disclosures, the at least one reagent (*e.g.,* an affinity reagent or stimulatory reagent) contains at least one binding site B that can specifically bind an epitope or region of the molecule and also contains a binding partner C that specifically binds to at least one binding site Z of the reagent (e.g., affinity reagent or stimulatory reagent). In some cases disclosed herein, the binding interaction between the binding partner C and the at least one binding site Z is a non-covalent interaction. In some disclosures, the binding interaction, such as non-covalent interaction, between the binding partner C and the at least one binding site Z is reversible.

In some disclosures, the reversible association can be mediated in the presence of a substance, such as a competition reagent, that is or contains a binding site that also is able to bind to the at least one binding site Z. Generally, the substance (*e.g.* competition reagent) can act as a competitor due to a higher binding affinity for the binding site Z present in the reagent and/or due to being present at higher concentrations than the binding partner C, thereby detaching and/or dissociating the binding partner C from the reagent. In some disclosures, the affinity of the substance (*e.g.* competition reagent) for the at least one binding site Z is greater than the affinity of the binding partner C of the agent (*e.g.,* an affinity reagent or stimulatory reagent) for the at least one binding site Z. Thus, in some cases disclosed herein, the bond between the binding site Z of the reagent and the binding partner C of the reagent (*e.g.,* an affinity reagent or stimulatory reagent) can be disrupted by addition of the substance (*e.g.* competition reagent), thereby rendering the association of the reagent (*e.g.,* an affinity reagent or stimulatory reagent) and reagent (e.g., affinity reagent or stimulatory reagent) reversible.

Reagents that can be used in such reversible systems are described and known in the art, see *e.g.,* U.S. Patent Nos. 5,168,049; 5,506,121; 6,103,493; 7,776,562; 7,981,632; 8,298,782; 8,735,540; 9,023,604; and International published PCT Appl. Nos. WO2013/124474 and WO2014/076277. Non-limiting examples disclosed herein of reagents and binding partners capable of forming a reversible interaction, as well as substances (*e.g.* competition agents) capable of reversing such binding, are described below.

In some disclosures, the stimulation results in activation and/or proliferation of the cells, for example, prior to transduction.

### 1. Stimulatory Reagents

In particular disclosures, the stimulating conditions include incubating, culturing, and/or cultivating the cells with a stimulatory reagent. In certain disclosures, the stimulatory reagent contains or includes a bead. In certain disclosures, the initiation of the stimulation occurs when the cells are incubated or contacted with the stimulatory reagent. In particular disclosures, the stimulatory reagent contains or includes an oligomeric reagent, e.g., a streptavidin mutein oligomer. In particular disclosures, the stimulatory reagent activates and/or is capable of activating one or more intracellular signaling domains of one or more components of a TCR complex and/or one or more intracellular signaling domains of one or more costimulatory molecules.

In some disclosures, the stimulating conditions or stimulatory reagents include one or more agent, e.g., ligand, which is capable of activating an intracellular signaling domain of a TCR complex. In some disclosures, an agent as contemplated herein can include, but is not limited to, RNA, DNA, proteins (e.g., enzymes), antigens, polyclonal antibodies, monoclonal antibodies, antibody fragments, carbohydrates, lipids lectins, or any other biomolecule with an affinity for a desired target. In some disclosures, the desired target is a T cell receptor and/or a component of a T cell receptor. In certain disclosures, the desired target is CD3. In certain disclosures, the desired target is a T cell costimulatory molecule, e.g., CD28, CD137 (4-1-BB), OX40, or ICOS. The one or more agents may be attached directly or indirectly to the bead by a variety of known methods. The attachment may be covalent, noncovalent, electrostatic, or hydrophobic and may be accomplished by a variety of attachment means, including for example, a chemical means, a mechanical means, or an enzymatic means. In some disclosures, the agent is an antibody or antigen binding fragment thereof, such as a Fab. In some disclosures, a biomolecule (e.g., a biotinylated anti-CD3 antibody) may be attached indirectly to the bead via another biomolecule (e.g., anti-biotin antibody) that is directly attached to the bead.

In some disclosures, the stimulatory reagent contains one or more agents (e.g. antibody) that is attached to a bead (e.g., a paramagnetic bead) and specifically binds to one or more of the following macromolecules on a cell (e.g., a T cell): CD2, CD3, CD4, CD5, CD8, CD25, CD27, CD28, CD29, CD31, CD44, CD45RA, CD45RO, CD54 (ICAM-1), CD127, MHCI, MHCII, CTLA-4, ICOS, PD-1, OX40, CD27L (CD70), 4-1BB (CD137), 4-1BBL, CD30L, LIGHT, IL-2R, IL-12R, IL-1R, IL-15R; IFN-gammaR, TNF-alphaR, IL-4R, IL- 10R, CD18/CDl la (LFA-1), CD62L (L-selectin), CD29/CD49d (VLA-4), Notch ligand (e.g. Delta-like 1/4, Jagged 1/2, etc.), CCR1, CCR2, CCR3, CCR4, CCR5, CCR7, and CXCR3 or fragment thereof including the corresponding ligands to these macromolecules or fragments thereof. In some disclosures, an agent (e.g. antibody) attached to the bead specifically binds to one or more of the following macromolecules on a cell (e.g. a T cell): CD28, CD62L, CCR7, CD27, CD127, CD3, CD4, CD8, CD45RA, and/or CD45RO.

In some disclosures, one or more of the agents attached to the bead is an antibody. The antibody can include a polyclonal antibody, monoclonal antibody (including full length antibodies which have an immunoglobulin Fc region), antibody compositions with polyepitopic specificity, multispecific antibodies (e.g., bispecific antibodies, diabodies, and single-chain molecules, as well as antibody fragments (e.g., Fab, F(ab')2, and Fv). In some disclosures, the stimulatory reagent is an antibody fragment (including antigen-binding fragment), e.g., a Fab, Fab'-SH, Fv, scFv, or (Fab')2 fragment. It will be appreciated that constant regions of any isotype can be used for the antibodies contemplated herein, including IgG, IgM, IgA, IgD, and IgE constant regions, and that such constant regions can be obtained from any human or animal species (e.g., murine species).

In some disclosures, the agent is an antibody that binds to and/or recognizes one or more components of a T cell receptor. In particular disclosures, the agent is an anti-CD3 antibody. In certain disclosures, the agent is an antibody that binds to and/or recognizes a coreceptor. In some disclosures, the stimulatory reagent comprises an anti-CD28 antibody. In some disclosures, the stimulatory reagent comprises an anti-CD28 antibody and an anti-CD3 antibody. In some disclosures, the stimulatory reagent comprises one or more stimulatory agents. In some disclosures, the stimulatory reagent comprises a primary and a secondary stimulatory agent. In some disclosures, the first stimulatory agent is an anti-CD3 antibody or antigen-binding fragment thereof, for example as described herein, and the second stimulatory agent is an anti-CD28 antibody or antigen-binding fragment thereof, for example as described herein. In some disclosures, the first stimulatory agent is an anti-CD3 Fab, for example as described herein, and the second stimulatory agent is an anti-CD28 Fab, for example as described herein.

In some disclosures, the cells, e.g., cells of the input composition, are stimulated in the presence of a ratio of stimulatory reagent to cells at or at about 3:1, 2.5:1, 2:1, 1.5:1, 1.25:1, 1.2:1, 1.1:1, 1:1, 0.9:1, 0.8:1, 0.75:1, 0.67:1, 0.5:1, 0.3:1, or 0.2:1. In particular disclosures, the ratio of stimulatory reagent to cells is between 2.5:1 and 0.2:1, between 2:1 and 0.5:1, between 1.5:1 and 0.75:1, between 1.25:1 and 0.8:1, between 1.1:1 and 0.9:1. In particular disclosures, the ratio of stimulatory reagent to cells is about 1:1 or is 1:1. In particular disclosures, the ratio of stimulatory reagent to cells is about 0.3:1 or is 0.3:1. In particular disclosures, the ratio of stimulatory reagent to cells is about 0.2:1 or is 0.2:1.

In some disclosures, the cells are stimulated in the presence of, of about, or of at least 0.01 $\mu$g, 0.02 $\mu$g, 0.03 $\mu$g, 0.04 $\mu$g, 0.05 $\mu$g, 0.1 $\mu$g, 0.2 $\mu$g, 0.3 $\mu$g, 0.4 $\mu$g, 0.5 $\mu$g, 0.75 $\mu$g, 1 $\mu$g, 2 $\mu$g, 3 $\mu$g, 4 $\mu$g, 5 $\mu$g, 6 $\mu$g, 7 $\mu$g, 8 $\mu$g, 9 $\mu$g, or 10 $\mu$g of the stimulatory reagent per $10^6$ cells. In some disclosures, the cells are stimulated in the presence of or of about 4 $\mu$g per $10^6$ cells. In particular disclosures, the cells are stimulated or subjected to stimulation in the presence

52

of or of about 3 $\mu$g per $10^6$ cells. In particular disclosures, the cells are stimulated or subjected to stimulation in the presence of or of about 2.5 $\mu$g per $10^6$ cells. In particular disclosures, the cells are stimulated or subjected to stimulation in the presence of or of about 2 $\mu$g per $10^6$ cells. In particular disclosures, the cells are stimulated or subjected to stimulation in the presence of or of about 1.8 $\mu$g per $10^6$ cells. In particular disclosures, the cells are stimulated or subjected to stimulation in the presence of or of about 1.6 $\mu$g per $10^6$ cells. In particular disclosures, the cells are stimulated or subjected to stimulation in the presence of or of about 1.4 $\mu$g per $10^6$ cells. In particular disclosures, the cells are stimulated or subjected to stimulation in the presence of or of about 1.2 $\mu$g per $10^6$ cells. In particular disclosures, the cells are stimulated or subjected to stimulation in the presence of or of about 1 $\mu$g per $10^6$ cells. In particular disclosures, the cells are stimulated in the presence of or of about 0.8 $\mu$g per $10^6$ cells. In various disclosures, the cells are stimulated in the presence of or of about 0.8 $\mu$g per $10^6$ cells.

In some disclosures, the stimulatory reagent binds to a molecule on the surface of a cell, which binding between the stimulatory reagent and the molecule is capable of inducing, delivering, or modulating a stimulatory signal in the cells. In some instances, the cell surface molecule (e.g. receptor) is a signaling molecule. In some such cases disclosed herein, the stimulatory reagent is capable of specifically binding to a signaling molecule expressed by one or more target cells (e.g., T cells). In some instances, the stimulatory reagent is any agent that is capable of inducing or delivering a stimulatory signal in a cell (e.g., a T cell) upon binding to a cell surface molecule, such as a receptor. In some disclosures, the stimulatory signal can be immunostimulatory, in which case the stimulatory agent is capable of inducing, delivering, or modulating a signal that is involved in or that does stimulate an immune response by the cell (e.g. T cell), e.g., increase immune cell proliferation or expansion, immune cell activation, immune cell differentiation, cytokine secretion, cytotoxic activity or one or more other functional activities of an immune cell. In some disclosures, the stimulatory signal can be inhibitory, in which case the stimulatory reagent is capable of inducing, delivering, or modulating a stimulatory signal in the cell (e.g. T cell) that is involved in or that does inhibit an immune response, e.g. inhibits or decreases immune cell proliferation or expansion, immune cell activation, immune cell differentiation, cytokine secretion, cytotoxic activity or one or more other functional activities of an immune cell.

In some disclosures, the stimulatory reagent comprises a primary stimulatory agent. In some disclosures, the primary stimulatory agent binds to a receptor molecule on the surface of the selected cells of the sample. Thus, in some cases disclosed herein, the primary stimulatory agent delivers, induces, or modulates a stimulatory signal. In some disclosures, the delivering, inducing, or modulating of a stimulatory signal by the primary stimulatory agent effects the stimulation of the cells. Thus, in some cases disclosed herein, the primary stimulatory agent delivers a stimulatory signal or provides a primary activation signal to the cells, thereby stimulating and/or activating the cells. In some disclosures, the primary stimulatory agent further induces downregulation of a selection marker. As used herein, downregulation may encompass a reduction in expression, e.g., cell surface expression, of a selection marker compared to an earlier time point.

In some disclosures, the target cells (e.g., T cells) comprise TCR/CD3 complexes and costimulatory molecules, such as CD28. In this case disclosed herein, the primary stimulatory agent binds to a TCR/CD3 complex, thereby delivering a stimulatory signal (e.g., a primary signal, e.g., primary activation signal) in the T cells, and the secondary stimulatory agent binds to a costimulatory CD28 molecule. In particular disclosures, the primary stimulatory agent and/or the secondary stimulatory agent further induce downregulation of a selection marker (e.g., a selection marker used to immobilize the target cells (e.g., T cells)).

In some disclosures, the primary stimulatory agent delivers a TCR/CD3 complex-associated stimulatory signal (e.g., primary signal) in the cells, e.g., T cells. In some disclosures, the primary stimulatory agent specifically binds to a molecule containing an immunoreceptor tyrosine-based activation motif or ITAM. In some disclosures, the primary stimulatory agent specifically binds CD3. In some cases disclosed herein, a primary stimulatory agent that specifically binds CD3 may be selected from the group consisting of an anti-CD3-antibody, a divalent antibody fragment of an anti-CD3 antibody, a monovalent antibody fragment of an anti-CD3-antibody, and a proteinaceous CD3 binding molecule with antibody-like binding properties. The divalent antibody fragment may be a F(ab')2-fragment, or a divalent single-chain Fv fragment while the monovalent antibody fragment may be selected from the group consisting of a Fab fragment, an Fv fragment, and a single-chain Fv fragment (scFv). In some cases disclosed herein, a proteinaceous CD3 binding molecule with antibody-like binding properties may be an aptamer, a mutein based on a polypeptide of the lipocalin family, a glubody, a protein based on the ankyrin scaffold, a protein based on the crystalline scaffold, an adnectin, or an avimer.

In some disclosures, an anti-CD3 Fab fragment can be derived from the CD3 binding monoclonal antibody produced by the hybridoma cell line OKT3 (ATCC® CRL-8001™; see also U.S. Patent No. 4,361,549). The variable domain of the heavy chain and the variable domain of the light chain of the anti-CD3 antibody OKT3 are described in Arakawa et al J. Biochem. 120, 657-662 (1996) and comprise the amino acid sequences set forth in SEQ ID NOs: 93 and 94, respectively. In some disclosures, the anti-CD3 Fab comprises the CDRs of the variable heavy and light chains set forth in SEQ ID NOs: 93 and 94, respectively.

In some disclosures, the stimulatory agent comprises a secondary stimulatory agent. In some disclosures, the secondary stimulatory agent binds to a molecule on the surface of the cells, such as a cell surface molecule, e.g., receptor molecule. In some disclosures, the secondary stimulatory agent is capable of enhancing, dampening, or modifying a stimulatory signal delivered through the molecule bound by the first stimulatory agent. In some disclosures, the secondary stimulatory agent delivers, induces, or modulates a stimulatory signal, e.g., a second or an additional stimulatory signal. In some disclosures, the secondary stimulatory agent enhances or potentiates a stimulatory signal induced by the primary stimulatory agent. In some disclosures, the secondary stimulatory agent binds to an accessory molecule and/or can stimulate or induce an accessory or secondary stimulatory signal in the cell. In some disclosures, the secondary stimulatory agent binds to a costimulatory molecule and/or provides a costimulatory signal.

In some disclosures, the stimulatory agent, which cancomprise the secondary stimulatory agent, binds, e.g. specifically binds, to a second molecule that can be a costimulatory molecule, an accessory molecule, a cytokine receptor, a chemokine receptor, an immune checkpoint molecule, or a member of the TNF family or the TNF receptor family.

In some disclosures, the molecule on the cell, e.g., T cell, may be CD28 and the secondary stimulatory agent) specifically binds CD28. In some disclosures, the secondary stimulatory agent that specifically binds CD28 may be selected from the group consisting of an anti-CD28-antibody, a divalent antibody fragment of an anti-CD28 antibody, a monovalent antibody fragment of an anti-CD28-antibody, and a proteinaceous CD28 binding molecule with antibody-like binding properties. The divalent antibody fragment may be an F(ab')2-fragment, or a divalent single-chain Fv fragment while the monovalent antibody fragment may be selected from the group consisting of a Fab fragment, an Fv fragment, and a single-chain Fv fragment (scFv). A proteinaceous CD28 binding molecule with antibody-like binding properties may be an aptamer, a mutein based on a polypeptide of the lipocalin family, a glubody, a protein based on the ankyrin scaffold, a protein based on the crystalline scaffold, an adnectin, and an avimer.

In some disclosures, an anti-CD28 Fab fragment can be derived from antibody CD28.3 (deposited as a synthetic single chain Fv construct under GenBank Accession No. AF451974.1; see also Vanhove et al, BLOOD, 15 July 2003, Vol. 102, No. 2, pages 564-570) the variable heavy and light chains of which comprise SEQ ID NO: 91 and 92, respectively. In some disclosures, the anti-CD28 Fab comprises the CDRs of the variable heavy and light chains set forth in SEQ ID NOs:91 and 92, respectively.

In some disclosures, the molecule on the cell, *e.g.,* T cell, is CD90 and the secondary stimulatory agent specifically binds CD90. In some disclosures, the the secondary stimulatory agent that specifically binds CD90 may be selected from the group consisting of an anti-CD90-antibody, a divalent antibody fragment of an anti-CD90 antibody, a monovalent antibody fragment of an anti-CD90-antibody, and a proteinaceous CD90 binding molecule with antibody-like binding properties. The antibody or antigen-binding fragment can be derived from any known in the art. See *e.g.* anti-CD90 antibody G7 (Biolegend, cat. no. 105201).

In some disclosures, the molecule on the cell, *e.g.,* T cell, is CD95 and the secondary stimulatory agent specifically binds CD95. In some disclosures, the secondary stimulatory agent that specifically binds CD95 may be selected from the group consisting of an anti-CD95-antibody, a divalent antibody fragment of an anti-CD95 antibody, a monovalent antibody fragment of an anti-CD95-antibody, and a proteinaceous CD95 binding molecule with antibody-like binding properties. The antibody or antigen-binding fragment can be derived from any known in the art. For example, in some disclosures, the anti-CD90 antibody can be monoclonal mouse anti-human CD95 CH11 (Upstate Biotechnology, Lake Placid, NY) or can be anti-CD95 mAb 7C11 or anti-APO-1, such as described in Paulsen et al. Cell Death & Differentiation 18.4 (2011): 619-631.

In some disclosures, the molecule on the cell, *e.g.,* T cell or B cell, may be CD137 and the secondary stimulatory agent specifically binds CD137. In some disclosures, the secondary stimulatory agent that specifically binds CD137 may be selected from the group consisting of an anti-CD137-antibody, a divalent antibody fragment of an anti-CD137 antibody, a monovalent antibody fragment of an anti-CD137-antibody, and a proteinaceous CD137 binding molecule with antibody-like binding properties. The antibody or antigen-binding fragment can be derived from any known in the art. For example, the anti-CD137 antibody can be LOB12, IgG2a or LOB12.3, IgG1 as described in Taraban et al. Eur J Immunol. 2002 Dec;32(12):3617-27. See also *e.g.* US6569997, US6303121, Mittler et al. Immunol Res. 2004;29(1-3):197-208.

In some disclosures, the molecule on the cell, *e.g.* B cell, may be CD40 and the secondary stimulatory agent specifically binds CD40. In some disclosures, the the secondary stimulatory agent that specifically binds CD40 may be selected from the group consisting of an anti-CD40-antibody, a divalent antibody fragment of an anti-CD40 antibody, a monovalent antibody fragment of an anti-CD40-antibody, and a proteinaceous CD40 binding molecule with antibody-like binding properties.

In some disclosures, the molecule on the cell, *e.g.,* T cell, may be CD40L (CD154) and the secondary stimulatory agent specifically binds CD40L. In some disclosures, the secondary stimulatory agent that specifically binds CD40L may be selected from the group consisting of an anti-CD40L-antibody, a divalent antibody fragment of an anti-CD40L antibody, a monovalent antibody fragment of an anti-CD40L-antibody, and a proteinaceous CD40L binding molecule with antibody-like binding properties. The antibody or antigen-binding fragment can be derived from any known in the art. For example, the anti-CD40L antibody can in some disclosures be Hu5C8, as described in Blair et al. JEM vol. 191 no. 4 651-660. See also e.g. WO1999061065, US20010026932, US7547438, WO2001056603.

In some disclosures, the molecule on the cell, *e.g.,* T cell, may be inducible T cell Costimulator (ICOS) and the secondary stimulatory agent specifically binds ICOS. In some disclosures, the secondary stimulatory agent that specifically binds ICOS may be selected from the group consisting of an anti-ICOS-antibody, a divalent antibody fragment of an anti-ICOS antibody, a monovalent antibody fragment of an anti-ICOS-antibody, and a proteinaceous ICOS binding molecule with antibody-like binding properties. The antibody or antigen-binding fragment can be derived from any known in the art. See *e.g.* US20080279851 and Deng et al. Hybrid Hybridomics. 2004 Jun;23(3): 176-82.

In some disclosures, the molecule on the cell, *e.g.,* T cell, may be Linker for Activation of T cells (LAT) and the secondary stimulatory agent) specifically binds LAT. In some disclosures, the secondary stimulatory agent that specifically binds LAT may be selected from the group consisting of an anti-LAT-antibody, a divalent antibody fragment of an anti-LAT antibody, a monovalent antibody fragment of an anti-LAT-antibody, and a proteinaceous LAT binding molecule with antibody-like binding properties. The antibody or antigen-binding fragment can be derived from any known in the art.

In some disclosures, the molecule on the cell, *e.g.,* T cell, may be CD27 and the secondary stimulatory agent specifically binds CD27. In some disclosures, the secondary stimulatory agent that specifically binds CD27 may be selected from the group consisting of an anti-CD27-antibody, a divalent antibody fragment of an anti-CD27 antibody, a monovalent antibody fragment of an anti-CD27-antibody, and a proteinaceous CD27 binding molecule with antibody-like binding properties. The antibody or antigen-binding fragment can be derived from any known in the art. See *e.g.* WO2008051424.

In some disclosures, the molecule on the cell, e.g., T cell, may be OX40 and the secondary stimulatory agent specifically binds OX40. In some disclosures, the secondary stimulatory agen) that specifically binds OX40 may be selected from the group consisting of an anti-OX40-antibody, a divalent antibody fragment of an anti-OX40 antibody, a monovalent antibody fragment of an anti-OX40-antibody, and a proteinaceous OX40 binding molecule with antibody-like binding properties. The antibody or antigen-binding fragment can be derived from any known in the art. See *e.g.* WO2013038191, Melero et al. Clin Cancer Res. 2013 Mar 1;19(5):1044-53.

In some disclosures, the molecule on the cell, *e.g.,* T cell, may be HVEM and the secondary stimulatory agent specifically binds HVEM. In some disclosures, the secondary stimulatory agent that specifically binds HVEM may be selected from the group consisting of an anti-HVEM-antibody, a divalent antibody fragment of an anti-HVEM antibody, a monovalent antibody fragment of an anti-HVEM-antibody, and a proteinaceous HVEM binding molecule with antibody-like binding properties. The antibody or antigen-binding fragment can be derived from any known in the art. See *e.g.* WO2006054961, WO2007001459, Park et al. Cancer Immunol Immunother. 2012 Feb;61(2):203-14.

In any of the above disclosures, the divalent antibody fragment may be a (Fab)2'-fragment, or a divalent single-chain Fv fragment while the monovalent antibody fragment may be selected from the group consisting of a Fab fragment, an Fv fragment, and a single-chain Fv fragment (scFv). In any of the above disclosures, the proteinaceous binding molecule with antibody-like binding properties may be an aptamer, a mutein based on a polypeptide of the lipocalin family, a glubody, a protein based on the ankyrin scaffold, a protein based on the crystalline scaffold, an adnectin, and an avimer.

In some disclosures, the stimulatory agent specifically targets a molecule expressed on the surface of the target cells in which the molecule is a TCR, a chimeric antigen receptor, or a molecule comprising an immunoreceptor tyrosine-based activation motif or ITAM. For example, the molecule expressed on the surface of the target cell is selected from a T cell or B cell antigen receptor complex, a CD3 chain, a CD3 zeta, an antigen-binding portion of a T cell receptor or a B cell receptor, or a chimeric antigen receptor. In some cases disclosed herein, the stimulatory agent targets peptide:MHC class I complexes.

In some disclosures, the stimulatory agent binds to a His-tagged extracellular domain of a molecule expressed on the suface of the target cells. In some cases disclosed herein, the stimulatory agent contains the peptide sequence Trp-Ser-His-Pro-Gln-Phe-Glu-Lys (also called Strep-tag® II, set forth in SEQ ID NO: 69) conjugated with a nickel charged trisNTA (also called His-STREPPER or His/Strep-tag®II Adapter). In some disclosures, the molecule expressed on the surface of the target cells that is His-tagged is CD19.

In some disclosures, the stimulatory agent specifically binds to the antibody portion of the recombinant receptor, *e.g.*, CAR. In some cases disclosed herein, the antibody portion of the recombinant receptor includes at least a portion of an immunoglobulin constant region, such as a hinge region, e.g., an IgG4 hinge region, and/or a CH1/CL and/or Fc region. In some disclosures, the constant region or portion is of a human IgG, such as IgG4 or IgG1. In some cases disclosed herein, the reagent is loaded with αIgG that recognizes the IgG4 spacer.

In some disclosures, the desired target is a T cell receptor and/or a component of a T cell receptor. In certain disclosures, the desired target is CD3. In certain disclosures, the desired target is a T cell costimulatory molecule, e.g., CD28, CD137 (4-1-BB), OX40, or ICOS.

In some disclosures, for example when the stimulatory agent is not bound to a stimulatory reagent or a receptor-binding agent reagent, the stimulatory agent is an antibody, a divalent antibody fragment, a F(ab)$_2$, or a divalent single-chain Fv fragment. In some disclosures, when the stimulatory agent is not bound to the reagent, the stimulatory agent does not include a binding partner C.

*a. Bead Reagents*

In certain disclosures, the stimulatory reagent contains a particle, e.g., a bead, that is conjugated or linked to one or more agents, e.g., biomolecules, that are capable of activating and/or expanding cells, e.g., T cells. In some disclosures, the one or more agents are bound to a bead. In some disclosures, the bead is biocompatible, i.e., composed of a material that is suitable for biological use. In some disclosures, the beads are non-toxic to cultured cells, e.g., cultured T cells. In some disclosures, the beads may be any particles which are capable of attaching agents in a manner that permits an interaction between the agent and a cell.

In some disclosures, the stimulatory reagent contains a bead and one or more agents that directly interact with a macromolecule on the surface of a cell. In certain disclosures, the bead (e.g., a paramagnetic bead) interacts with a cell via one or more agents (e.g., an antibody) specific for one or more macromolecules on the cell (e.g., one or more cell surface proteins). In certain disclosures, the bead (e.g., a paramagnetic bead) is labeled with a first agent described herein, such as a primary antibody (e.g., an anti-biotin antibody) or other biomolecule, and then a second agent, such as a secondary antibody (e.g., a biotinylated anti-CD3 antibody) or other second biomolecule (e.g., streptavidin), is added, whereby the secondary antibody or other second biomolecule specifically binds to such primary antibodies or other biomolecule on the particle.

In some disclosures, the bead has a diameter of greater than about 0.001 $\mu$m, greater than about 0.01 $\mu$m, greater than about 0.1 $\mu$m, greater than about 1.0 $\mu$m, greater than about 10 $\mu$m, greater than about 50 $\mu$m, greater than about 100 $\mu$m or greater than about 1000 $\mu$m and no more than about 1500$\mu$m. In some disclosures, the bead has a diameter of about 1.0 $\mu$m to about 500 $\mu$m, about 1.0 $\mu$m to about 150 $\mu$m, about 1.0 $\mu$m to about 30 $\mu$m, about 1.0 $\mu$m to about 10 $\mu$m, about 1.0 $\mu$m to about 5.0 $\mu$m, about 2.0 $\mu$m to about 5.0 $\mu$m, or about 3.0 $\mu$m to about 5.0 $\mu$m. In some disclosures, the bead has a diameter of about 3 $\mu$m to about 5$\mu$m. In some disclosures, the bead has a diameter of at least or at least about or about 0.001 $\mu$m, 0.01 $\mu$m, 0.1$\mu$m, 0.5$\mu$m, 1.0 $\mu$m, 1.5 $\mu$m, 2.0 $\mu$m, 2.5 $\mu$m, 3.0 $\mu$m, 3.5 $\mu$m, 4.0 $\mu$m, 4.5 $\mu$m, 5.0 $\mu$m, 5.5 $\mu$m, 6.0 $\mu$m, 6.5 $\mu$m, 7.0 $\mu$m, 7.5 $\mu$m, 8.0 $\mu$m, 8.5 $\mu$m, 9.0 $\mu$m, 9.5 $\mu$m, 10 $\mu$m, 12 $\mu$m, 14 $\mu$m, 16 $\mu$m, 18 $\mu$m or 20 $\mu$m. In certain disclosures, the bead has a diameter of or about 4.5 $\mu$m. In certain disclosures, the bead has a diameter of or about 2.8 $\mu$m.

n some disclosures, the beads have a density of greater than 0.001 g/cm$^3$, greater than 0.01 g/cm$^3$, greater than 0.05 g/cm$^3$, greater than 0.1 g/cm$^3$, greater than 0.5 g/cm$^3$, greater than 0.6 g/cm$^3$, greater than 0.7 g/cm$^3$, greater than 0.8 g/cm$^3$, greater than 0.9 g/cm$^3$, greater than 1 g/cm$^3$, greater than 1.1 g/cm$^3$, greater than 1.2 g/cm$^3$, greater than 1.3 g/cm$^3$, greater than 1.4 g/cm$^3$, greater than 1.5 g/cm$^3$, greater than 2 g/cm$^3$, greater than 3 g/cm$^3$, greater than 4 g/cm$^3$, or greater than 5g/cm$^3$. In some disclosures, the beads have a density of between about 0.001 g/cm$^3$ and about 100 g/cm$^3$, about 0.01 g/cm$^3$ and about 50 g/cm$^3$, about 0.1 g/cm$^3$ and about 10 g/cm$^3$, about 0.1 g/cm$^3$ and about .5 g/cm$^3$, about 0.5 g/cm$^3$ and about 1 g/cm$^3$, about 0.5 g/cm$^3$ and about 1.5 g/cm$^3$, about 1 g/cm$^3$ and about 1.5 g/cm$^3$, about 1 g/cm$^3$ and about 2 g/cm$^3$, or about 1 g/cm$^3$ and about 5 g/cm$^3$. In some disclosures, the beads have a density of about 0.5 g/cm$^3$, about 0.5 g/cm$^3$, about 0.6 g/cm$^3$, about 0.7 g/cm$^3$, about 0.8 g/cm$^3$, about 0.9 g/cm$^3$, about 1.0 g/cm$^3$, about 1.1 g/cm$^3$, about 1.2 g/cm$^3$, about 1.3 g/cm$^3$, about 1.4 g/cm$^3$, about 1.5 g/cm$^3$, about 1.6 g/cm$^3$, about 1.7 g/cm$^3$, about 1.8 g/cm$^3$, about 1.9 g/cm$^3$, or about 2.0 g/cm$^3$. In certain disclosures, the beads have a density of about 1.6 g/cm$^3$. In particular disclosures, the beads or particles have a density of about 1.5 g/cm$^3$. In certain disclosures, the particles have a density of about 1.3 g/cm$^3$

In certain disclosures, a plurality of the beads has a uniform density. In certain disclosures, a uniform density comprises a density standard deviation of less than 10%, less than 5%, or less than 1% of the mean bead density.

In some disclosures, the bead contains at least one material at or near the bead surface that can be coupled, linked, or conjugated to an agent. In some disclosures, the bead is surface functionalized, i.e. comprises functional groups that are capable of forming a covalent bond with a binding molecule, e.g., a polynucleotide or a polypeptide. In particular disclosures, the bead comprises surface-exposed carboxyl, amino, hydroxyl, tosyl, epoxy, and/or chloromethyl groups. In particular disclosures, the beads comprise surface exposed agarose and/or sepharose. In certain disclosures, the bead surface comprises attached stimulatory reagents that can bind or attach binding molecules. In particular disclosures, the biomolecules are polypeptides. In some disclosures, the beads comprise surface exposed protein A, protein G, or biotin.

In some disclosures, the bead reacts in a magnetic field. In some disclosures, the bead is a magnetic bead. In some disclosures, the magnetic bead is paramagnetic. In particular disclosures, the magnetic bead is superparamagnetic. In certain disclosures, the beads do not display any magnetic properties unless they are exposed to a magnetic field.

In particular disclosures, the bead comprises a magnetic core, a paramagnetic core, or a superparamagnetic core. In some disclosures, the magnetic core contains a metal. In some disclosures, the metal can be, but is not limited to, iron, nickel, copper, cobalt, gadolinium, manganese, tantalum, zinc, zirconium or any combinations thereof. In certain disclosures, the magnetic core comprises metal oxides (e.g., iron oxides), ferrites (e.g., manganese ferrites, cobalt ferrites, nickel ferrites, etc.), hematite and metal alloys (e.g., CoTaZn). In some disclosures, the magnetic core comprises one or more of a ferrite, a metal, a metal alloy, an iron oxide, or chromium dioxide. In some disclosures, the magnetic core comprises elemental iron or a compound thereof. In some disclosures, the magnetic core comprises one or more of magnetite (Fe3O4), maghemite ($\gamma$Fe2O3), or greigite (Fe3S4). In some disclosures, the inner core comprises an iron oxide (e.g., $Fe_3O_4$).

In certain disclosures, the bead contains a magnetic, paramagnetic, and/or superparamagnetic core that is covered by a surface functionalized coat or coating. In some disclosures, the coat can contain a material that can include, but is not limited to, a polymer, a polysaccharide, a silica, a fatty acid, a protein, a carbon, agarose, sepharose, or a combination thereof. In some disclosures, the polymer can be a polyethylene glycol, poly (lactic-co-glycolic acid), polyglutaraldehyde, polyurethane, polystyrene, or a polyvinyl alcohol. In certain disclosures, the outer coat or coating comprises polystyrene. In particular disclosures, the outer coating is surface functionalized.

In some disclosures, the stimulatory reagent comprises a bead that contains a metal oxide core (e.g., an iron oxide core) and a coat, wherein the metal oxide core comprises at least one polysaccharide (e.g., dextran), and wherein the coat comprises at least one polysaccharide (e.g., amino dextran), at least one polymer (e.g., polyurethane) and silica. In some disclosures the metal oxide core is a colloidal iron oxide core. In certain disclosures, the one or more agents include an antibody or antigen-binding fragment thereof. In particular disclosures, the one or more agents include an anti-CD3 antibody and an anti-CD28 antibody. In some disclosures, the stimulatory reagent comprises an anti-CD3 antibody, anti-CD28 antibody, and an anti-biotin antibody. In some disclosures, the stimulatory reagent comprises an anti-biotin antibody. In some disclosures, the bead has a diameter of about 3 $\mu$m to about 10 $\mu$m. In some disclosures, the bead has a diameter of about 3 $\mu$m to about 5 $\mu$m. In certain disclosures, the bead has a diameter of about 3.5 $\mu$m.

In some disclosures, the stimulatory reagent comprises one or more agents that are attached to a bead comprising a metal oxide core (e.g., an iron oxide inner core) and a coat (e.g., a protective coat), wherein the coat comprises polystyrene. In certain disclosures, the beads are monodisperse, paramagnetic (e.g., superparamagnetic) beads comprising a paramagnetic (e.g., superparamagnetic) iron core, e.g., a core comprising magnetite ($Fe_3O_4$) and/or maghemite (y$Fe_2O_3$) c and a polystyrene coat or coating. In some disclosures, the bead is non-porous. In some disclosures, the beads contain a functionalized surface to which the one or more agents are attached. In certain disclosures, the one or more agents are covalently bound to the beads at the surface. In some disclosures, the one or more agents include an antibody or antigen-binding fragment thereof. In some disclosures, the one or more agents include an anti-CD3 antibody and an anti-CD28 antibody. In some disclosures, the one or more agents include an anti-CD3 antibody and/or an anti-CD28 antibody, and an antibody or antigen fragment thereof capable of binding to a labeled antibody (e.g., biotinylated antibody), such as a labeled anti-CD3 or anti-CD28 antibody. In certain disclosures, the beads have a density of about 1.5 g/cm$^3$ and a surface area of about 1 m$^2$/g to about 4 m$^2$/g. In particular disclosures; the beads are monodisperse superparamagnetic beads that have a diameter of about 4.5 $\mu$m and a density of about 1.5 g/cm$^3$. In some disclosures, the beads the beads are monodisperse superparamagnetic beads that have a mean diameter of about 2.8 $\mu$m and a density of about 1.3 g/cm$^3$.

In some disclosures, the population of enriched T cells is incubated with stimulatory reagent a ratio of beads to cells at or at about 3:1, 2.5:1, 2:1, 1.5:1, 1.25: 1, 1.2:1, 1.1:1, 1:1, 0.9:1, 0.8:1, 0.75:1, 0.67:1, 0.5:1, 0.3:1, or 0.2:1. In particular disclosures, the ratio of beads to cells is between 2.5:1 and 0.2:1, between 2:1 and 0.5:1, between 1.5:1 and 0.75:1, between 1.25:1 and 0.8:1, between 1.1:1 and 0.9:1. In particular disclosures, the ratio of beads to cells is about 1:1 or is 1:1.

*b. Oligomeric Streptavidin Mutein Reagent*

In particular disclosures, the stimulatory reagent contains an oligomeric reagent, e.g., a streptavidin mutein reagent, that is conjugated, linked, or attached to one or more agent, e.g., ligand, which is capable of activating an intracellular signaling domain of a TCR complex. In some disclosures, the one or more agents have an attached binding domain or binding partner (e.g., a binding partner C) that is capable of binding to oligomeric reagent at a particular binding sites (e.g., binding site Z). In some disclosures, a plurality of the agent is reversibly bound to the oligomeric reagent.In various disclosures, the oligomeric reagent has a plurality of the particular binding sites which, in certain disclosures, are reversibly bound to a plurality of agents at the binding domain (e.g., binding partner C). In some disclosures, the amount of bound agents are reduced or decreased in the presence of a competition reagent, e.g., a reagent that is also capable of binding to the particular binding sites (e.g., binding site Z).

In some disclosures, the oligomeric stimulatory reagent is or includes a reversible system in which at least one agent (e.g., an agent that is capable of producing a signal in a cell such as a T cell) is associated, e.g., reversibly associated, with the oligomeric reagent. Non-limiting examples disclosed herein of oligomeric stimulatory reagents may be found, for example, in International published PCT Appl. No. WO 2018/197949. In some disclosures, the reagent contains a plurality of binding sites capable of binding, e.g., reversibly binding, to the agent. In some cases disclosed herein, the reagent is an oligomeric particle reagent having at least one attached agent capable of producing a signal in a cell such as a T cell. In some disclosures, the agent contains at least one binding site, e.g., a binding site B, that can specifically bind an epitope or region of the molecule and also contains a binding partner, also referred to herein as a binding partner C, that specifically binds to at least one binding site of the reagent, e.g., binding site Z of the reagent. In some disclosures, the binding interaction between the binding partner C and the at least one binding site Z is a non-covalent interaction. In some cases disclosed herein, the binding interaction between the binding partner C and the at least one binding site Z is a covalent interaction. In some disclosures, the binding interaction, such as non-covalent interaction, between the binding partner C and the at least one binding site Z is reversible.

Substances that may be used as oligomeric reagents in such reversible systems are known, see *e.g.,* U.S. Patent Nos. 5,168,049; 5,506,121; 6,103,493; 7,776,562; 7,981,632; 8,298,782; 8,735,540; 9,023,604; and International published PCT Appl. Nos. WO2013/124474 and WO2014/076277. Non-limiting examples disclosed herein of reagents and binding partners capable of forming a reversible interaction, as well as substances (e.g. competition reagents) capable of reversing such binding, are described below.

In some disclosures, the oligomeric reagent is an oligomer of streptavidin, streptavidin mutein or analog, avidin, an avidin mutein or analog (such as neutravidin) or a mixture thereof, in which such oligomeric reagent contains one or more binding sites for reversible association with the binding domain of the agent (e.g., a binding partner C). In some disclosures, the binding domain of the agent can be a biotin, a biotin derivative or analog, or a streptavidin-binding peptide or other molecule that is able to specifically bind to streptavidin, a streptavidin mutein or analog, avidin or an avidin mutein or analog.

In certain disclosures, one or more agents (e.g., agents that are capable of producing a signal in a cell such as a T cell) associate with, such as are reversibly bound to, the oligomeric reagent, such as via the plurality of the particular binding sites (e.g., binding sites Z) present on the oligomeric reagent. In some cases disclosed herein, this results in the agents being closely arranged to each other such that an avidity effect can take place if a target cell having (at least two copies of) a cell surface molecule that is bound by or recognized by the agent is brought into contact with the agent.

In some disclosures, the oligomeric reagent is a streptavidin oligomer, a streptavidin mutein oligomer, a streptavidin analog oligomer, an avidin oligomer, an oligomer composed of avidin mutein or avidin analog (such as neutravidin) or a mixture thereof. In particular disclosures, the oligomeric reagents contain particular binding sites that are capable of binding to a binding domain (e.g., the binding partner C) of an agent. In some disclosures, the binding domain can be a biotin, a biotin derivative or analog, or a streptavidin-binding peptide or other molecule that is able to specifically bind to streptavidin, a streptavidin mutein or analog, avidin or an avidin mutein or analog. The methods disclosed herein further contemplate that the oligomeric reagent may comprise a molecule capable of binding to an oligohistidine affinity tag, a glutathione-S-transferase, calmodulin or an analog thereof, calmodulin binding peptide (CBP), a FLAG-peptide, an HA-tag, maltose binding protein (MBP), an HSV epitope, a myc epitope, and/or a biotinylated carrier protein.

In some disclosures, the streptavidin can be wild-type streptavidin, streptavidin muteins or analogs, such as streptavidin-like polypeptides. Likewise, avidin, in some disclosures, includes wild-type avidin or muteins or analogs of avidin such as neutravidin, a deglycosylated avidin with modified arginines that typically exhibits a more neutral pi and is available as an alternative to native avidin. Generally, deglycosylated, neutral forms of avidin include those commercially available forms such as "Extravidin", available through Sigma Aldrich, or "NeutrAvidin" available from Thermo Scientific or Invitrogen, for example.

In some disclosures, the reagent is a streptavidin or a streptavidin mutein or analog. In some disclosures, wild-type streptavidin (wt-streptavidin) has the amino acid sequence disclosed by Argarana et al, Nucleic Acids Res. 14 (1986) 1871-1882 (SEQ ID NO: 66). In general, streptavidin naturally occurs as a tetramer of four identical subunits, i.e. it is a homo-tetramer, where each subunit contains a single binding site for biotin, a biotin derivative or analog or a biotin mimic. An exemplary sequence of a streptavidin subunit is the sequence of amino acids set forth in SEQ ID NO: 66, but such a sequence also can include a sequence present in homologs thereof from other Streptomyces species. In particular, each subunit of streptavidin may exhibit a strong binding affinity for biotin with an equilibrium dissociation constant ($K_D$) on the order of about $10^{-14}$ M. In some cases disclosed herein, streptavidin can exist as a monovalent tetramer in which only one of the four binding sites is functional (Howarth et al. (2006) Nat. Methods, 3:267-73; Zhang et al. (2015) Biochem. Biophys. Res. Commun., 463:1059-63)), a divalent tetramer in which two of the four binding sites are functional (Fairhead et al. (2013) J. Mol. Biol., 426:199-214), or can be present in monomeric or dimeric form (Wu et al. (2005) J. Biol. Chem., 280:23225-31; Lim et al. (2010) Biochemistry, 50:8682-91).

In some disclosures, streptavidin may be in any form, such as wild-type or unmodified streptavidin, such as a streptavidin from a *Streptomyces* species or a functionally active fragment thereof that includes at least one functional subunit containing a binding site for biotin, a biotin derivative or analog or a biotin mimic, such as generally contains at least one functional subunit of a wild-type streptavidin from *Streptomyces avidinii* set forth in SEQ ID NO: 66 or a functionally active fragment thereof. For example, in some disclosures, streptavidin can include a fragment of wild-type streptavidin, which is shortened at the N- and/or C-terminus. Such minimal streptavidins include any that begin N-terminally in the region of amino acid positions 10 to 16 of SEQ ID NO: 66 and terminate C-terminally in the region of amino acid positions 133 to 142 of SEQ ID NO: 66. In some disclosures, a functionally active fragment of streptavidin contains the sequence of amino acids set forth in SEQ ID NO: 67. In some disclosures, streptavidin, such as set forth in SEQ ID NO: 67, can further contain an N-terminal methionine at a position corresponding to Ala13 with numbering set forth in SEQ ID NO: 66. Reference to the position of residues in streptavidin or streptavidin muteins is with reference to numbering of residues in SEQ ID NO: 66.

Examples of streptavidins or streptavidin muteins are mentioned, for example, in WO 86/02077, DE 19641876 A1, US 6,022,951, WO 98/40396 or WO 96/24606. Examples of streptavidin muteins are known, see e.g., U.S. Pat. No. 5,168,049; 5,506,121; 6,022,951; 6,156,493; 6,165,750; 6,103,493; or 6,368,813; or International published PCT App. No. WO2014/076277.

In some disclosures, a streptavidin mutein can contain amino acids that are not part of an unmodified or wild-type streptavidin or can include only a part of a wild-type or unmodified streptavidin. In some disclosures, a streptavidin mutein contains at least one subunit that can have one more amino acid substitutions (replacements) compared to a subunit of an unmodified or wild-type streptavidin, such as compared to the wild-type streptavidin subunit set forth in SEQ ID NO: 66 or a functionally active fragment thereof, e.g. set forth in SEQ ID NO: 67.

In some disclosures, the equilibrium dissociation constant ($K_D$), of streptavidin or a streptavidin mutein for a binding domain is less than $1 \times 10^{-4}$ M, $5 \times 10^{-4}$ M, $1 \times 10^{-5}$ M, $5 \times 10^{-5}$ M, $1 \times 10^{-6}$ M, $5 \times 10^{-6}$ M or $1 \times 10^{-7}$ M, but generally greater than $1 \times 10^{-13}$ M, $1 \times 10^{-12}$ M or $1 \times 10^{-11}$ M. For example, peptide sequences (Strep-tags), such as disclosed in U.S. Pat. No. 5,506,121, can act as biotin mimics and demonstrate a binding affinity for streptavidin, e.g., with a $K_D$ of approximately between $10^{-4}$ and $10^{-5}$ M. In some cases disclosed herein, the binding affinity can be further improved by making a mutation within the streptavidin molecule, see e.g. U.S. Pat. No. 6,103,493 or International published PCT App. No. WO2014/076277. In some disclosures, binding affinity can be determined by known methods, such as any described herein.

In some disclosures, the reagent, such as a streptavidin or streptavidin mutein, exhibits binding affinity for a peptide ligand binding partner, which peptide ligand binding partner can be the binding partner C present in the agent (e.g., receptor-binding agent or selection agent). In some disclosures, the peptide sequence contains a sequence with the general formula His-Pro-Xaa, where Xaa is glutamine, asparagine, or methionine, such as contained in the sequence set forth in SEQ ID NO: 83. In some disclosures, the peptide sequence has the general formula set forth in SEQ ID NO: 83, such as set forth in SEQ ID NO: 74. In one example disclosed herein, the peptide sequence is Trp-Arg-His-Pro-Gln-Phe-Gly-Gly (also called Strep-tag®, set forth in SEQ ID NO: 75). In one example disclosed herein, the peptide sequence is Trp-Ser-His-Pro-Gln-Phe-Glu-Lys (also called Strep-tag® II, set forth in SEQ ID NO: 69). In some disclosures, the peptide ligand contains a sequential arrangement of at least two streptavidin-binding modules, wherein the distance between the two modules is at least 0 and not greater than 50 amino acids, wherein one binding module has 3 to 8 amino acids and contains at least the sequence His-Pro-Xaa, where Xaa is glutamine, asparagine, or methionine, and wherein the other binding module has the same or different streptavidin peptide ligand, such as set forth in SEQ ID NO: 84 (see e.g. International Published PCT Appl. No. WO02/077018; U.S. Patent No. 7,981,632). In some disclosures, the peptide ligand contains a sequence having the formula set forth in any of SEQ ID NO: 76 or 77. In some disclosures, the peptide ligand has the sequence of amino acids set forth in any of SEQ ID NOS: 70-73, 78-79. In most cases disclosed herein, all these streptavidin binding peptides bind to the same binding site, namely the biotin binding site of streptavidin. If one or more of such streptavidin binding peptides is used as binding partners C, e.g. C1 and C2, the multimerization reagent and/or oligomeric particle reagents bound to the one or more agents via the binding partner C is typically composed of one or more streptavidin muteins.

In some disclosures, the streptavidin mutein is a mutant as described in U.S. Pat. No. 6,103,493. In some disclosures, the streptavidin mutein contains at least one mutation within the region of amino acid positions 44 to 53, based on the amino acid sequence of wild-type streptavidin, such as set forth in SEQ ID NO: 66. In some disclosures, the streptavidin mutein contains a mutation at one or more residues 44, 45, 46, and/or 47. In some disclosures, the streptavidin mutein contains a replacement of Glu at position 44 of wild-type streptavidin with a hydrophobic aliphatic amino acid, e.g. Val, Ala, Ile or Leu, any amino acid at position 45, an aliphatic amino acid, such as a hydrophobic aliphatic amino acid at position 46 and/or a replacement of Val at position 47 with a basic amino acid, e.g. Arg or Lys, such as generally Arg. In some disclosures, Ala is at position 46 and/or Arg is at position 47 and/or Val or Ile is at position 44. In some disclosures, the streptavidin mutant contains residues Val44-Thr45-Ala46-Arg47, such as set forth in exemplary streptavidin muteins containing the sequence of amino acids set forth in SEQ ID NO: 80 or SEQ ID NO: 81 or 82 (also known as streptavidin mutant 1, SAM1). In some disclosures, the streptavidin mutein contains residues Ile44-Gly45-Ala46-Arg47, such as set forth in exemplary streptavidin muteins containing the sequence of amino acids set forth in SEQ ID NO: 85, 68, or 73 (also known as SAM2). In some cases disclosed herein, such streptavidin mutein are described, for example, in US patent 6,103,493, and are commercially available under the trademark Strep-Tactin®. In some disclosures, the mutein streptavidin contains the sequence of amino acids set forth in SEQ ID NO: 86 or SEQ ID NO: 87. In particular disclosures, the molecule is a tetramer of streptavidin or a streptavidin mutein comprising a sequence set forth in any of SEQ ID NOS: 67, 81, 68, 86, 88, 82 or 73, which, as a tetramer, is a molecule that contains 20 primary amines, including 1 N-terminal amine and 4 lysines per monomer.

In some disclosures, streptavidin mutein exhibits a binding affinity characterized by an equilibrium dissociation constant ($K_D$) that is or is less than $3.7 \times 10^{-5}$ M for the peptide ligand (Trp-Arg-His-Pro-Gln-Phe-Gly-Gly; also called Strep-tag®, set forth in SEQ ID NO: 75) and/or that is or is less than $7.1 \times 10^{-5}$ M for the peptide ligand (Trp-Ser-His-Pro-Gln-Phe-Glu-Lys; also called Strep-tag® II, set forth in SEQ ID NO: 69) and/or that is or is less than $7.0 \times 10^{-5}$ M, $5.0 \times 10^{-5}$ M, $1.0 \times 10^{-5}$ M, $5.0 \times 10^{-6}$ M, $1.0 \times 10^{-6}$ M, $5.0 \times 10^{-7}$ M, or $1.0 \times 10^{-7}$ M, but generally greater than $1 \times 10^{-13}$ M, $1 \times 10^{-12}$ M or $1 \times 10^{-11}$ M for any of the peptide ligands set forth in any of SEQ ID NOS: 69, 76-78, 70-72, 74, 75, 83, 84.

In some disclosures, the resulting streptavidin mutein exhibits a binding affinity characterized by an equilibrium association constant ($K_A$) that is or is greater than $2.7 \times 10^{4}$ M$^{-1}$ for the peptide ligand (Trp-Arg-His-Pro-Gln-Phe-Gly-Gly; also called Strep-tag®, set forth in SEQ ID NO: 75) and/or that is or is greater than $1.4 \times 10^{4}$ M$^{-1}$ for the peptide ligand (Trp-Ser-His-Pro-Gln-Phe-Glu-Lys; also called Strep-tag® II, set forth in SEQ ID NO: 69) and/or that is or is greater than $1.43 \times 10^{4}$ M$^{-1}$, $1.67 \times 10^{4}$ M$^{-1}$, $2 \times 10^{4}$ M$^{-1}$, $3.33 \times 10^{4}$ M$^{-1}$, $5 \times 10^{4}$ M$^{-1}$, $1 \times 10^{5}$ M$^{-1}$, $1.11 \times 10^{5}$ M$^{-1}$, $1.25 \times 10^{5}$ M$^{-1}$, $1.43 \times 10^{5}$ M$^{-1}$, $1.67 \times 10^{5}$ M$^{-1}$, $2 \times 10^{5}$ M$^{-1}$, $3.33 \times 10^{5}$ M$^{-1}$, $5 \times 10^{5}$ M$^{-1}$, $1 \times 10^{6}$ M$^{-1}$, $1.11 \times 10^{6}$ M$^{-1}$, $1.25 \times 10^{6}$ M$^{-1}$, $1.43 \times 10^{6}$ M$^{-1}$, $1.67 \times 10^{6}$ M$^{-1}$, $2 \times 10^{6}$ M$^{-1}$, $3.33 \times 10^{6}$ M$^{-1}$, $5 \times 10^{6}$ M$^{-1}$, $1 \times 10^{7}$ M$^{-1}$,, but generally less than $1 \times 10^{13}$ M$^{-1}$, $1 \times 10^{12}$ M$^{-1}$ or $1 \times 10^{11}$ M$^{-1}$ for any of the peptide ligands set forth in any of SEQ ID NOS: 69, 76-78, 70-72, 74, 75, 83, 84.

In particular examples, disclosed herein is an oligomeric particle reagent that is composed of and/or contains a plurality of streptavidin or streptavidin mutein tetramers. In certain disclosures, the oligomeric particle reagent disclosed herein contains a plurality of binding sites that reversibly bind or are capable of reversibly binding to one or more agents, e.g., a stimulatory agent and/or a selection agent. In some disclosures, the oligomeric particle has a radius, e.g., an average radius, of between 70 nm and 125 nm, inclusive; a molecular weight of between $1 \times 10^{7}$ g/mol and $1 \times 10^{9}$ g/mol, inclusive; and/or between 1,000 and 5,000 streptavidin or streptavidin mutein tetramers, inclusive. In some disclosures, the oligomeric particle reagent is bound, e.g., reversibly bound, to one or more agents such as an agent that binds to a molecule, e.g. receptor, on the surface of a cell. In certain disclosures, the one or more agents are agents described herein, e.g., in Section II.C.3. In some disclosures, the agent is an anti-CD3 and/or an anti-CD28 antibody or antigen binding fragment thereof, such as an antibody or antigen fragment thereof that contains a binding partner, e.g., a streptavidin binding peptide, e.g. Strep-tag® II. In particular disclosures, the one or more agents is an anti-CD3 and/or an anti CD28 Fab containing a binding partner, e.g., a streptavidin binding peptide, e.g. Strep-tag® II.

In some disclosures, disclosed herein is an oligomeric particle reagent that is composed of and/or contains a plurality of streptavidin or streptavidin mutein tetramers. In certain disclosures, the oligomeric particle reagent disclosed herein contains a plurality of binding sites that reversibly bind or are capable of reversibly binding to one or more agents, e.g., a stimulatory agent and/or a selection agent. In some disclosures, the oligomeric particle has a radius, e.g., an average radius, of between 80 nm and 120 nm, inclusive; a molecular weight, e.g., an average molecular weight of between $7.5 \times 10^{6}$ g/mol and $2 \times 10^{8}$ g/mol, inclusive; and/or an amount, e.g., an average amount, of between 500 and 10,000 streptavidin or streptavidin mutein tetramers, inclusive. In some disclosures, the oligomeric particle reagent is bound, e.g., reversibly bound, to one or more agents, such as an agent that binds to a molecule, e.g. receptor, on the surface of a cell. In certain disclosures, the one or more agents are agents described herein, e.g., in Section II.C.3. In some disclosures, the agent is an anti-CD3 and/or an anti-CD28 Fab, such as a Fab that contains a binding partner, e.g., a streptavidin binding peptide, e.g. Strep-tag® II. In particular disclosures, the one or more agents is an anti-CD3 and/or an anti CD28 Fab containing a binding partner, e.g., a streptavidin binding peptide, e.g. Strep-tag® II.

In some disclosures, the cells are stimulated in the presence of, of about, or of at least 0.01 μg, 0.02 μg, 0.03 μg, 0.04 μg, 0.05 μg, 0.1 μg, 0.2 μg, 0.3 μg, 0.4 μg, 0.5 μg, 0.75 μg, 1 μg, 2 μg, 3 μg, 4 μg, 5 μg, 6 μg, 7 μg, 8 μg, 9 μg, or 10 μg of the oligomeric stimulatory reagent per $10^{6}$ cells. In some disclosures, the cells are stimulated in the presence of or of about 4 μg per $10^{6}$ cells. In some disclosures, the cells are stimulated or subjected to stimulation in the presence of or of about 3 μg per $10^{6}$ cells. In some disclosures, the cells are stimulated or subjected to stimulation in the presence of or of about 2.75 μg per $10^{6}$ cells. In some disclosures, the cells are stimulated or subjected to stimulation in the presence of or of about 2.5 μg per $10^{6}$ cells. In some disclosures, the cells are stimulated or subjected to stimulation in the presence of or of about 2.25 μg per $10^{6}$ cells. In some disclosures, the cells are stimulated or subjected to stimulation in the presence of or of about 2 μg per $10^{6}$ cells. In particular disclosures, the cells are stimulated or subjected to stimulation in the presence of or of about 1.8 μg per $10^{6}$ cells. In particular disclosures, the cells are stimulated or subjected to stimulation in the presence of or of about 1.6 μg per $10^{6}$ cells. In particular disclosures, the cells are stimulated or subjected to stimulation in the presence of or of about 1.4 μg per $10^{6}$ cells. In particular disclosures, the cells are stimulated or subjected to stimulation in the presence of or of about 1.2 μg per $10^{6}$ cells. In particular disclosures, the cells are stimulated or subjected to stimulation in the presence of or of about 1 μg per $10^{6}$ cells. In particular disclosures, the cells are stimulated in the presence of or of about 0.8 μg per $10^{6}$ cells. In certain disclosures, 4 μg of the oligomeric stimulatory reagent is or includes 3 μg of oligomeric particles and 1 μg of attached agents, e.g., 0.5 μg of anti-CD3 Fabs and 0.5 μg of anti-CD28 Fabs. In some disclosures, the cells are stimulated or subjected to stimulation in the presence of or of about $10 \times 10^{8}$, $9 \times 10^{8}$, $8 \times 10^{8}$, $7 \times 10^{8}$, $6 \times 10^{8}$, $5 \times 10^{8}$, $4 \times 10^{8}$, $3 \times 10^{8}$, $2 \times 10^{8}$, $1 \times 10^{8}$ oligomeric reagents. In some disclosures, the cells are stimulated or subjected to stimulation in the presence of or of about $7 \times 10^{8}$, $6 \times 10^{8}$, $5 \times 10^{8}$, $4 \times 10^{8}$, $3 \times 10^{8}$ oligomeric reagents. In some disclosures, the cells are stimulated or subjected to stimulation in the presence of or of about $7 \times 10^{8}$ to $3 \times 10^{8}$ oligomeric reagents. In some disclosures, the cells are stimulated or subjected to stimulation in the presence of or of about $6 \times 10^8$ to $4 \times 10^8$ oligomeric reagents. In some disclosures, the cells are stimulated or subjected to stimulation in the presence of or of about $6 \times 10^8$ to $5 \times 10^8$ oligomeric reagents. In some disclosures, the cells are stimulated or subjected to stimulation in the presence of or of about $5 \times 10^8$ oligomeric reagents.

In some disclosures, the cells, e.g., selected cells of a sample, are stimulated or subjected to stimulation in the presence of a ratio of oligomeric reagent to cells at or at about 3:1, 2.5:1, 2:1, 1.5:1, 1.25:1, 1.2:1, 1.1:1, 1:1, 0.9:1, 0.8:1, 0.75:1, 0.67:1, 0.5:1, 0.3:1, or 0.2:1. In particular disclosures, the ratio of oligomeric reagent to cells is between 2.5:1 and 0.2:1, between 2:1 and 0.5:1, between 1.5:1 and 0.75:1, between 1.25:1 and 0.8:1, between 1.1:1 and 0.9:1. In particular disclosures, the ratio of oligomeric reagent to cells is about 1:1 or is 1:1. In particular disclosures, the ratio of oligomeric reagent to cells is about 0.3:1 or is 0.3:1. In particular disclosures, the ratio of oligomeric reagent to cells is about 0.2:1 or is 0.2:1.

In certain disclosures, within the oligomeric reagent, the mass ratio between the oligomeric particles and the attached agents is about 3:1. In certain disclosures, within the oligomeric reagent, the mass ratio among the oligomeric particles, the attached anti-CD3 Fabs, and the attached anti-CD28 Fabs is about 3:0.5:0.5. In certain disclosures, 4 $\mu$g of the oligomeric reagent is or includes 3 $\mu$g of oligomeric particles and 1 $\mu$g of attached agents, e.g., 0.5 $\mu$g of anti-CD3 Fabs and 0.5 $\mu$g of anti-CD28 Fabs. In other examples disclosed herein, 1.2 $\mu$g of the oligomeric reagent per $10^6$ cells is or includes 0.9 $\mu$g of oligomeric particles and 0.3 $\mu$g of attached agents, e.g., 0.15 $\mu$g of anti-CD3 Fabs and 0.15 $\mu$g of anti-CD28 Fabs, per $10^6$ cells. In some disclosures, the oligomeric reagent is added to a serum-free medium and the stimulation is performed in the serum free medium, e.g., as described in PCT/US2018/064627.

In some disclosures, the serum-free medium comprises a basal medium (e.g.OpTmizer™ T-Cell Expansion Basal Medium (ThermoFisher), supplemented with one or more supplement. In some disclosures, the one or more supplement is serum-free. In some disclosures, the serum-free medium comprises a basal medium supplemented with one or more additional components for the maintenance, expansion, and/or activation of a cell (e.g., a T cell), such as provided by an additional supplement (e.g. OpTmizer™ T-Cell Expansion Supplement (ThermoFisher)). In some disclosures, the serum-free medium further comprises a serum replacement supplement, for example, an immune cell serum replacement, e.g., ThermoFisher, #A2596101, the CTS™ Immune Cell Serum Replacement, or the immune cell serum replacement described in Smith et al. Clin Transl Immunology. 2015 Jan; 4(1): e31. In some disclosures, the serum-free medium further comprises a free form of an amino acid such as L-glutamine. In some disclosures, the serum-free medium further comprises a dipeptide form of L-glutamine (e.g., L-alanyl-L-glutamine), such as the dipeptide in Glutamax™ (ThermoFisher). In some disclosures, the serum-free medium further comprises one or more recombinant cytokines, such as recombinant human IL-2, recombinant human IL-7, and/or recombinant human IL-15.

### 2. Removal of Stimulatory Reagents

In some disclosures, the stimulatory reagent is removed or separated from the cells or cell populations prior to collecting, harvesting, or formulating the cells. In some disclosures, the stimulatory reagents are removed or separated from the cells or cell populations after or during the incubation, e.g., an incubation described herein such as in Section I.D. In certain disclosures, the cells or cell population undergoes a process, procedure, step, or technique to remove the stimulatory reagent after the incubation but prior to steps for collecting, harvesting, or formulating the cells. In particular disclosures, the cells or cell population undergoes a process, procedure, step, or technique to remove the stimulatory reagent after the incubation. In some disclosures, when stimulatory reagent is separated or removed from the cells during the incubation, the cells are returned to the same incubation conditions as prior to the separation or removal for the remaining duration of the incubation.

In certain disclosures, the stimulatory reagent is removed and/or separated from the cells. Without wishing to be bound by theory, particular disclosures contemplate that the binding and/or association between a stimulatory reagent and cells may, in some circumstances, be reduced over time during the incubation. In certain disclosures, one or more agents may be added to reduce the binding and/or association between the stimulatory reagent and the cells. In particular disclosures, a change in cell culture conditions, e.g., the addition of an agent, may reduce the binding and/or association between the stimulatory reagent and the cells. Thus, in some disclosures, the stimulatory reagent may be removed from an incubation, cell culture system, and/or a solution separately from the cells, e.g., without removing the cells from the incubation, cell culture system, and/or a solution as well.

In certain disclosures, the stimulatory reagent is separated and/or removed from the cells after an amount of time. In particular disclosures, the amount of time is an amount of time from the initiation of the stimulation. In particular disclosures the start of the incubation is considered at or at about the time the cells are contacted with the stimulatory reagent and/or a media or solution containing the stimulatory reagent. In particular disclosures, the stimulatory reagent is removed or separated from the cells within or within about 120 hours, 108 hours, 96 hours, 84 hours, 72 hours, 60 hours, 48 hours, 36 hours, 24 hours, or 12 hours, inclusive, of the initiation of the stimulation. In particular disclosures, the stimulatory reagent is removed or separated from the cells at or at about 48 hours after the stimulation is initiated.

In certain disclosures, the stimulatory reagent is removed or separated from the cells at or at about 72 hours after the stimulation is initiated. In some disclosures, the stimulatory reagent is removed or separated from the cells at or at about 96 hours after the stimulation is initiated.

### a. Removal of Bead Reagents

In certain disclosures, the bead stimulatory reagent, e.g., an anti-CD3/anti-CD28 antibody conjugated paramagnetic bead, is separated or removed from the cells or the cell population. Methods for removing stimulatory reagents (e.g. stimulatory reagents that are or contain particles such as bead particles or magnetizable particles) from cells are known. In some disclosures, the use of competing antibodies, such as non-labeled antibodies, can be used, which, for example, bind to a primary antibody of the stimulatory reagent and alter its affinity for its antigen on the cell, thereby permitting for gentle detachment. In some cases disclosed herein, after detachment, the competing antibodies may remain associated with the particle (e.g. bead particle) while the unreacted antibody is or may be washed away and the cell is free of isolating, selecting, enriching and/or activating antibody. Exemplary of such a reagent is DETACaBEAD (Friedl et al. 1995; Entschladen et al. 1997). In some disclosures, particles (e.g. bead particles) can be removed in the presence of a cleavable linker (e.g. DNA linker), whereby the particle-bound antibodies are conjugated to the linker (e.g. CELLection, Dynal). In some cases disclosed herein, the linker region provides a cleavable site to remove the particles (e.g. bead particles) from the cells after isolation, for example, by the addition of DNase or other releasing buffer. In some disclosures, other enzymatic methods can also be employed for release of a particle (e.g. bead particle) from cells. In some disclosures, the particles (e.g. bead particles or magnetizable particles) are biodegradable.

In some disclosures, the stimulatory reagent is magnetic, paramagnetic, and/or superparamagnetic, and/or contains a bead that is magnetic, paramagnetic, or superparamagnetic, and the stimulatory reagent may be removed from the cells by exposing the cells to a magnetic field. Examples disclosed herein of suitable equipment containing magnets for generating the magnetic field include DynaMag CTS (Thermo Fisher), Magnetic Separator (Takara) and EasySep Magnet (Stem Cell Technologies).

In particular disclosures, the stimulatory reagent is removed or separated from the cells prior to the completion of the disclosed methods, e.g., prior to harvesting, collecting, and/or formulating engineered cells produced by the methods disclosed herein. In some disclosures, the stimulatory reagent is removed and/or separated from the cells after engineering, e.g., transducing or transfecting, the cells.

In some disclosures, the stimulatory bead reagent, e.g., the stimulatory magnetic bead reagent, is removed or separated from the cells or cell populations prior to collecting, harvesting, or formulating the cells. In some disclosures, the stimulatory bead reagent, e.g., the stimulatory magnetic bead reagent, are removed or separated from the cells or cell populations by exposure to a magnetic field during or after the incubation, e.g., an incubation described herein such as in Section I-D. In certain disclosures, the cells or cell population are exposed to the magnetic field to remove the stimulatory bead reagent, e.g., the stimulatory magnetic bead reagent, after the incubation but prior to steps for collecting, harvesting, or formulating the cells. In particular disclosures, the cells or cell population undergoes is exposed to the magnetic field to remove the stimulatory bead reagent, e.g., the stimulatory magnetic bead reagent, after the incubation. In some disclosures, when the stimulatory bead reagent is separated or removed from the cells or cell population during the incubation, the cells or cell population are returned to the same incubation conditions as prior to the exposure to the magnetic field for the remaining duration of the incubation.

In particular disclosures, the stimulatory bead reagent, e.g., the stimulatory magnetic bead reagent, is removed or separated from the cells, e.g., by exposure to a magnetic field, within or within about 120 hours, 108 hours, 96 hours, 84 hours, 72 hours, 60 hours, 48 hours, 36 hours, 24 hours, or 12 hours, inclusive, of the incubation. In certain disclosures, the stimulatory bead reagent, e.g., the stimulatory magnetic bead reagent, is removed or separated from the cells, e.g., by exposure to a magnetic field, after or after about 72 hours of incubation. In some disclosures, the stimulatory bead reagent, e.g., the stimulatory magnetic bead reagent, is removed or separated from the cells, e.g., by exposure to a magnetic field, after or after about 96 hours of incubation.

### b. Removal of Oligomeric reagents

In some disclosures, the population of incubated T cells was produced or generated in accord with any of the methods disclosed herein in which a substance, such as a competition agent, was added to T cells to disrupt, such as to lessen and/or terminate, the signaling of the stimulatory agent or agents. In some disclosures, the population of the incubated T cells contains the presence of a substance, such as a competition agent, e.g. biotin or a biotin analog, e.g. D-Biotin. In some disclosures, the substance, such as a competition agent, e.g. biotin or a biotin analog, e.g. D-Biotin, is present in an amount that is at least 1.5-fold greater, at least 2-fold, at least 3-fold, at least 4-fold, at least 5-fold, at least 10-fold, at least 100-fold, at least 1000-fold or more greater than the amount of the substance in a reference population or preparation of cultured T cells in which the substance was not added exogenously during the incubation.

In some disclosures, the amount of the substance, such as a competition agent, e.g. biotin or a biotin analog, e.g. D-Biotin, in the population of cultured T cells is from or from about 10 $\mu$M to 100 $\mu$M, 100 $\mu$M to 1 mM, 100 $\mu$M to 500 $\mu$M or 10 $\mu$M to 100 $\mu$M. In some disclosures, 10 $\mu$M or about 10 $\mu$M of biotin or a biotin analog, e.g., D-biotin, is added to the cells or the cell population to separate or remove the oligomeric stimulatory reagent from the cells or cell population.

In certain disclosures, the one or more agents (e.g., agents that stimulate or activate a TCR and/or a coreceptor) associate with, such as are reversibly bound to, the oligomeric reagent, such as via the plurality of the particular binding sites (e.g., binding sites Z) present on the oligomeric reagent. In some cases disclosed herein, this results in the agents being closely arranged to each other such that an avidity effect can take place if a target cell having (at least two copies of) a cell surface molecule that is bound by or recognized by the agent is brought into contact with the agent. In some disclosures, the receptor binding reagent has a low affinity towards the receptor molecule of the cell at binding site B, such that the receptor binding reagent dissociates from the cell in the presence of the competition reagent. Thus, in some disclosures, the agents are removed from the cells in the presence of the competition reagent.

In some disclosures, the oligomeric stimulatory reagent is a streptavidin mutein oligomer with reversibly attached anti-CD3 and anti-CD28 Fabs. In some disclosures, the Fabs are attached contain streptavidin binding domains, e.g., that allow for the reversible attachment to the streptavidin mutein oligomer. In some cases disclosed herein, anti-CD3 and anti-CD28 Fabs are closely arranged to each other such that an avidity effect can take place if a T cell expressing CD3 and/or CD28 is brought into contact with the oligomeric stimulatory reagent with the reversibly attached Fabs. In some disclosures, the Fabs have a low affinity towards CD3 and CD28, such that the Fabs dissociate from the cell in the presence of the competition reagent, e.g., biotin or a biotin variant or analogue. Thus, in some disclosures, the Fabs are removed or dissociated from the cells in the presence of the competition reagent, e.g., D-biotin.

In some disclosures, the stimulatory oligomeric reagent, e.g., the stimulatory oligomeric streptavidin mutein reagent, is removed or separated from the cells or cell populations prior to collecting, harvesting, or formulating the cells. In some disclosures, stimulatory oligomeric reagent, e.g., the stimulatory oligomeric streptavidin mutein reagent, is removed or separated from the cells or cell populations by contact or exposure to a competition reagent, e.g., biotin or a biotin analog such as D-biotin, after or during the incubation, e.g., an incubation described herein such as in Section I.D. In certain disclosures, the cells or cell population are contacted or exposed to a competition reagent, e.g., biotin or a biotin analog such as D-biotin, to remove stimulatory oligomeric reagent, e.g., the stimulatory oligomeric streptavidin mutein reagent, after the incubation but prior to steps for collecting, harvesting, or formulating the cells. In particular disclosures, the cells or cell population are contacted or exposed to a competition reagent, e.g., biotin or a biotin analog such as D-biotin, to remove the stimulatory oligomeric reagent, e.g., the stimulatory oligomeric streptavidin mutein reagent, after the incubation. In some disclosures, when stimulatory oligomeric reagent, e.g., the stimulatory oligomeric streptavidin mutein reagent, is separated or removed from the cells during the incubation, e.g., by contact or exposure to a competition reagent, e.g., biotin or a biotin analog such as D-biotin, the cells are returned to the same incubation conditions as prior to the separation or removal for the remaining duration of the incubation.

In some disclosures, the cells are contacted with, with about, or with at least 0.01 $\mu$M, 0.05 $\mu$M, 0. 1 $\mu$M, 0.5 $\mu$M, 1 $\mu$M, 2 $\mu$M, 3 $\mu$M, 4 $\mu$M, 5 $\mu$M, 10 $\mu$M, 100 $\mu$M, 500 $\mu$M, 0.01 $\mu$M, 1 mM, or 10 mM of the competition reagent to remove or separate the oligomeric stimulatory reagent from the cells. In various disclosures, the cells are contacted with, with about, or with at least 0.01 $\mu$M, 0.05 $\mu$M, 0. 1 $\mu$M, 0.5 $\mu$M, 1 $\mu$M, 2 $\mu$M, 3 $\mu$M, 4 $\mu$M, 5 $\mu$M, 10 $\mu$M, 100 $\mu$M, 500 $\mu$M, 0.01 $\mu$M, 1 mM, or 10 mM of biotin or a biotin analog such as D-biotin, to remove or separate the stimulatory streptavidin mutein oligomers with reversibly attached anti-CD3 and anti-CD28 Fabs from the cells.

In particular disclosures, the stimulatory oligomeric reagent, e.g., the stimulatory oligomeric streptavidin mutein reagent, is removed or separated from the cells within or within about 120 hours, 108 hours, 96 hours, 84 hours, 72 hours, 60 hours, 48 hours, 36 hours, 24 hours, or 12 hours, inclusive, of incubation, e.g., in the presence of the stimulatory oligomeric streptavidin mutein reagent. In particular disclosures, the stimulatory reagent is removed or separated from the cells after or after about 48 hours of incubation e.g., in the presence of the stimulatory oligomeric reagent. In certain disclosures, the stimulatory oligomeric reagent, e.g., the stimulatory oligomeric streptavidin mutein reagent, is removed or separated from the cells after or after about 72 hours of incubation. In some disclosures, the stimulatory oligomeric reagent, e.g., the stimulatory oligomeric streptavidin mutein reagent is removed or separated from the cells at or at about 96 hours of incubation.

## B. Genetic Engineering

In some disclosures, the disclosed methods include genetically engineering the cells, e.g., cells of or derived from a population of enriched CD57- T cells, such as by introducing a heterologous polynucleotide encoding a recombinant protein. Such recombinant proteins may include recombinant receptors, such as any described herein such as in Section IV. Introduction of the polynucleotides, e.g., heterologous or recombinant polynucleotides, encoding the recombinant protein into the cell may be carried out using any of a number of known vectors. Such vectors include viral, including lentiviral and gammaretroviral, systems. Exemplary methods include those for transfer of heterologous polynucleotides encoding the receptors, including via viral, e.g., retroviral or lentiviral, transduction. In some disclosures, a population of stimulated cells is genetically engineered, such as to introduce a heterologous or recombinant polynucleotide encoding a recombinant receptor, thereby generating a population of transformed cells (also referred to herein as a transformed population of cells).

In particular disclosures, the cells are genetically engineered, transformed, or transduced after the cells have been stimulated, activated, and/or incubated under stimulating conditions, such as by any of the methods disclosed herein, e.g., in Section III.A. In particular disclosures, the one or more stimulated populations have been previously depleted of or separated from CD57+ T cells.

In certain disclosures, methods for genetic engineering are carried out by contacting or introducing one or more cells of a population with a polynucleotide encoding a recombinant protein, e.g. a recombinant receptor. In certain disclosures, the nucleic acid molecule or polynucleotide is heterologous to the cells. In particular disclosures, the heterologous polynucleotide is not native to the cells. In certain disclosures, the heterologous polynucleotide is not native to any vector, e.g., viral vector, from which it is delivered. In certain disclosures, the heterologous heterologous polynucleotide encodes a protein, e.g., a recombinant protein, that is not natively expressed by the cell. In particular disclosures, the heterologous nucleic polynucleotide is or contains a nucleic acid sequence that is not found in the cell prior to the introduction.

In some disclosures, the cells, e.g., stimulated cells, are engineered, e.g., transduced or in the presence of a transduction adjuvant. Exemplary transduction adjuvants include, but are not limited to, polycations, fibronectin or fibronectin-derived fragments or variants, and RetroNectin. In certain disclosures, the cells are engineered in the presence of polycations, fibronectin or fibronectin-derived fragments or variants, and/or RetroNectin. In particular disclosures, the cells are engineered in the presence of a polycation that is polybrene, DEAE-dextran, protamine sulfate, poly-L-lysine, or a cationic liposome. In particular disclosures, the cells are engineered in the presence of protamine sulfate.

In some disclosures, the genetic engineering, e.g., transduction, is carried out in serum free media. In some disclosures, the serum free media is a defined or well-defined cell culture media. In certain disclosures, the serum free media is a controlled culture media that has been processed, e.g., filtered to remove inhibitors and/or growth factors. In some disclosures, the serum free media contains proteins. In certain disclosures, the serum-free media may contain serum albumin, hydrolysates, growth factors, hormones, carrier proteins, and/or attachment factors.

In particular disclosures, the cells are engineered in the presence of one or more cytokines. In certain disclosures, the one or more cytokines are recombinant cytokines. In particular disclosures, the one or more cytokines are human recombinant cytokines. In certain disclosures, the one or more cytokines bind to and/or are capable of binding to receptors that are expressed by and/or are endogenous to T cells. In particular disclosures, the one or more cytokines is or includes a member of the 4-alpha-helix bundle family of cytokines. In some disclosures, members of the 4-alpha-helix bundle family of cytokines include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF). In some disclosures, the one or more cytokines is or includes IL-15. In particular disclosures, the one or more cytokines is or includes IL-7. In particular disclosures, the one or more cytokines is or includes recombinant IL-2.

In some disclosures, the cells are genetically engineered, transformed, or transduced in the presence of the same or similar media as was present during the stimulation. In some disclosures, the cells are genetically engineered, transformed, or transduced in media having the same cytokines as the media present during stimulation. In certain disclosures, the cells are genetically engineered, transformed, or transduced, in media having the same cytokines at the same concentrations as the media present during stimulation.

In some disclosures, the cells are genetically engineered, transformed, or transduced in the presence of the same or similar media as was present during the stimulation. In some disclosures, the cells are genetically engineered, transformed, or transduced in media having the same cytokines as the media present during stimulation. In certain disclosures, the cells are genetically engineered, transformed, or transduced, in media having the same cytokines at the same concentrations as the media present during stimulation.

### 1. Transduction

In some disclosures, genetically engineering the cells is or includes introducing the polynucleotide, e.g., the heterologous polynucleotide, into the cells by transduction. In some disclosures, the cells are transduced with a viral vector. In some disclosures, the virus is a retroviral vector, such as a gammaretroviral vector or a lentiviral vector. Methods of lentiviral transduction are known. Exemplary methods are described in, e.g., Wang et al. (2012) J. Immunother. 35(9): 689-701; Cooper et al. (2003) Blood. 101:1637-1644; Verhoeyen et al. (2009) Methods Mol Biol. 506: 97-114; and Cavalieri et al. (2003) Blood. 102(2): 497-505.

In some disclosures, the transduction is carried out by contacting one or more cells of a population with a nucleic acid molecule encoding the recombinant protein, e.g. recombinant receptor. In some disclosures, the contacting can be effected with centrifugation, such as spinoculation (e.g. centrifugal inoculation). Such methods include any of those as described in International Publication Number WO2016/073602. Exemplary centrifugal chambers include those produced and sold by Biosafe SA, including those for use with the Sepax® and Sepax® 2 system, including an A-200/F and A-200 centrifugal chambers and various kits for use with such systems. Exemplary chambers, systems, and processing instrumentation and cabinets are described, for example, in US Patent No. 6,123,655, US Patent No. 6,733,433 and Published U.S. Patent Application, Publication No.: US 2008/0171951, and published international patent application, publication no. WO 00/38762. Exemplary kits for use with such systems include, but are not limited to, single-use kits sold by BioSafe SA under product names CS-430.1, CS-490.1, CS-600.1 or CS-900.2.

In some disclosures, the disclosed methods are used in connection with transducing a viral vector containing a polynucleotide encoding a recombinant receptor into, into about, or into less than $300 \times 10^6$ cells, e.g., viable T cells of a stimulated cell population. In certain disclosures, at or about $100 \times 10^6$ cells, e.g., viable T cells of a stimulated cell population are transduced.

In some disclosures, the transduction is performed in serum free media. In some disclosures, the transduction is performed in the presence of IL-2, IL-7, and IL-15. In particular disclosures, the cells, e.g., the cells of the stimulated cell population contain at least 80%, at least 85%, at least 90%, or at least 95% cells that are CD4+ T cells or CD8+ T cells. In some disclosures, the transduction is performed for between 24 and 48 hours, between 36 and 12 hours, between 18 and 30 hours, or for or for about 24 hours. In certain disclosures, the transduction step is initiated within two days, within 36 hours, or within 30 hours of the start or initiation of the incubation, e.g., the incubation under stimulating conditions.

In some disclosures, the system is included with and/or placed into association with other instrumentation, including instrumentation to operate, automate, control and/or monitor disclosures of the transduction step and one or more various other processing steps performed in the system, e.g. one or more processing steps that can be carried out with or in connection with the centrifugal chamber system as described herein or in International Publication Number WO2016/073602. This instrumentation in some disclosures is contained within a cabinet. In some disclosures, the instrumentation includes a cabinet, which includes a housing containing control circuitry, a centrifuge, a cover, motors, pumps, sensors, displays, and a user interface. An exemplary device is described in US Patent No. 6,123,655, US Patent No. 6,733,433 and US 2008/0171951.

In some disclosures, the system comprises a series of containers, e.g., bags, tubing, stopcocks, clamps, connectors, and a centrifuge chamber. In some disclosures, the containers, such as bags, include one or more containers, such as bags, containing the cells to be transduced and the viral vector particles, in the same container or separate containers, such as the same bag or separate bags. In some disclosures, the system further includes one or more containers, such as bags, containing medium, such as diluent and/or wash solution, which is pulled into the chamber and/or other components to dilute, resuspend, and/or wash components and/or populations during the methods. The containers can be connected at one or more positions in the system, such as at a position corresponding to an input line, diluent line, wash line, waste line and/or output line.

In some disclosures, the chamber is associated with a centrifuge, which is capable of effecting rotation of the chamber, such as around its axis of rotation. Rotation may occur before, during, and/or after the incubation in connection with transduction of the cells and/or in one or more of the other processing steps. Thus, in some disclosures, one or more of the various processing steps is carried out under rotation, e.g., at a particular force. The chamber is typically capable of vertical or generally vertical rotation, such that the chamber sits vertically during centrifugation and the side wall and axis are vertical or generally vertical, with the end wall(s) horizontal or generally horizontal.

In some disclosures, the population containing cells and population containing viral vector particles, and optionally air, can be combined or mixed prior to providing the populations to the cavity. In some disclosures, the population containing cells and population containing viral vector particles, and optionally air, are provided separately and combined and mixed in the cavity. In some disclosures, a population containing cells, a population containing viral vector particles, and optionally air, can be provided to the internal cavity in any order. In any of such some disclosures, a population containing cells and viral vector particles is the input composition once combined or mixed together, whether such is combined or mixed inside or outside the centrifugal chamber and/or whether cells and viral vector particles are provided to the centrifugal chamber together or separately, such as simultaneously or sequentially.

In some disclosures, intake of the volume of gas, such as air, occurs prior to the incubating the cells and viral vector particles, such as rotation, in the transduction method. In some disclosures, intake of the volume of gas, such as air, occurs during the incubation of the cells and viral vector particles, such as rotation, in the transduction method.

In some disclosures, the liquid volume of the cells or viral vector particles that make up the transduction population, and optionally the volume of air, can be a predetermined volume. The volume can be a volume that is programmed into and/or controlled by circuitry associated with the system.

In some disclosures, intake of the transduction population, and optionally gas, such as air, is controlled manually, semi-automatically and/or automatically until a desired or predetermined volume has been taken into the internal cavity of the chamber. In some disclosures, a sensor associated with the system can detect liquid and/or gas flowing to and from the centrifuge chamber, such as via its color, flow rate and/or density, and can communicate with associated circuitry to stop or continue the intake as necessary until intake of such desired or predetermined volume has been achieved. In some disclosures, a sensor that is programmed or able only to detect liquid in the system, but not gas (e.g. air), can be made able to permit passage of gas, such as air, into the system without stopping intake. In some such disclosures, a non-clear piece of tubing can be placed in the line near the sensor while intake of gas, such as air, is desired. In some disclosures, intake of gas, such as air, can be controlled manually.

In disclosures of the disclosed methods, the internal cavity of the centrifuge chamber is subjected to high speed rotation. In some disclosures, rotation is effected prior to, simultaneously, subsequently or intermittently with intake of the liquid input composition, and optionally air. In some disclosures, rotation is effected subsequent to intake of the liquid input composition, and optionally air. In some disclosures, rotation is by centrifugation of the centrifugal chamber at a relative centrifugal force at the inner surface of side wall of the internal cavity and/or at a surface layer of the cells of at or about or at least at or about 200 g, 300 g, 400 g, 500 g, 600 g, 700 g, 800 g, 1000 g, 1100 g, 1500, 1600 g, 1800 g, 2000 g, 2200 g, 2500 g, 3000 g, 3200 g, 3500 g or 4000 g. In some disclosures, rotation is by centrifugation at a force that is greater than or about 1100 g, such as by greater than or about 1200 g, greater than or about 1400 g, greater than or about 1600 g, greater than or about 1800 g, greater than or about 2000 g, greater than or about 2400 g, greater than or about 2800 g, greater than or about 3000 g or greater than or about 3200 g. In particular disclosures, the rotation by centrifugation is at a force between 600 g and 800 g. In particular disclosures, the rotation by centrifugation is at a force of or of about 693 g. In some disclosures, rotation is by centrifugation at a force that is or is about 1600g.

In some disclosures, the gas, such as air, in the cavity of the chamber is expelled from the chamber. In some disclosures, the gas, such as air, is expelled to a container that is operably linked as part of the closed system with the centrifugal chamber. In some disclosures, the container is a free or empty container. In some disclosures, the air, such as gas, in the cavity of the chamber is expelled through a filter that is operably connected to the internal cavity of the chamber via a sterile tubing line. In some disclosures, the air is expelled using manual, semiautomatic or automatic processes. In some disclosures, air is expelled from the chamber prior to, simultaneously, intermittently or subsequently with expressing the output population containing incubated cells and viral vector particles, such as cells in which transduction has been initiated or cells have been transduced with a viral vector, from the cavity of the chamber.

In some disclosures, the transduction and/or other incubation is performed as or as part of a continuous or semi-continuous process. In some disclosures, a continuous process involves the continuous intake of the cells and viral vector particles, e.g., the transduction composition (either as a single pre-existing composition or by continuously pulling into the same vessel, e.g., cavity, and thereby mixing, its parts), and/or the continuous expression or expulsion of liquid, and optionally expelling of gas (e.g. air), from the vessel, during at least a portion of the incubation, e.g., while centrifuging. In some disclosures, the continuous intake and continuous expression are carried out at least in part simultaneously. In some disclosures, the continuous intake occurs during part of the incubation, e.g., during part of the centrifugation, and the continuous expression occurs during a separate part of the incubation. The two may alternate. Thus, the continuous intake and expression, while carrying out the incubation, can allow for a greater overall volume of sample to be processed, e.g., transduced.

In some disclosures, the incubation is part of a continuous process, the method including, during at least a portion of the incubation, effecting continuous intake of said transduction composition into the cavity during rotation of the chamber and during a portion of the incubation, effecting continuous expression of liquid and, optionally expelling of gas (e.g. air), from the cavity through the at least one opening during rotation of the chamber.

In some disclosures, the semi-continuous incubation is carried out by alternating between effecting intake of the composition into the cavity, incubation, expression of liquid from the cavity and, optionally expelling of gas (e.g. air) from the cavity, such as to an output container, and then intake of a subsequent (e.g., second, third, etc.) composition containing more cells and other reagents for processing, e.g., viral vector particles, and repeating the process. For example, in some disclosures, the incubation is part of a semi-continuous process, the method including, prior to the incubation, effecting intake of the transduction composition into the cavity through said at least one opening, and subsequent to the incubation, effecting expression of fluid from the cavity; effecting intake of another transduction composition comprising cells and the viral vector particles into said internal cavity; and incubating the another transduction composition in said internal cavity under conditions whereby said cells in said another transduction composition are transduced with said vector. The process may be continued in an iterative fashion for a number of additional rounds. In this respect, the semi-continuous or continuous methods may permit production of even greater volume and/or number of cells.

In some disclosures, a portion of the transduction incubation is performed in the centrifugal chamber, which is performed under conditions that include rotation or centrifugation.

In particular disclosures, transduction of the cells with the viral vector is or includes spinoculation, e.g., centrifugation of a mixture containing the cells and the viral particles. In some disclosures, the composition containing cells and viral particles can be rotated, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm). In some disclosures, the rotation is carried at a force, e.g., a relative centrifugal force, of from or from about 100 g to 4000 g (e.g. at or about or at least at or about 100 g, 200 g, 300 g, 400 g, 500 g, 600 g, 700 g, 800 g, 900 g, 1000 g, 1500 g, 2000 g, 2500 g, 3000 g or 3500 g), as measured for example at an internal or external wall of the chamber or cavity.

In some disclosures, the cells are spinoculated with the viral vector at a force, e.g., a relative centrifugal force, of between or between about 100 g and 4000 g, 200 g and 1,000 g, 500 g and 1200 g, 1000 g and 2000 g, 600 g and 800 g, 1200 g and 1800 g, or 1500 g and 1800 g. In certain disclosures, the cells are spinoculated with the viral vector particle for, for at least, or for about 100 g, 200 g, 300 g, 400 g, 500 g, 600 g, 700 g, 800 g, 900 g, 1000 g, 1200g, 1500 g, 1600g, 2000 g, 2500 g, 3000 g, 3200 g, or 3500 g. In some disclosures, the cells are transduced with the viral vector at a force of or of about 692 g. In particular disclosures, the cells are transduced with the viral vector at a force of or of about 1600 g. In some disclosures, the force is the force at the internal surface of the side wall of the internal cavity and/or at a surface layer of the cells.

In certain disclosures, the cells are spinoculated, e.g., the cell composition containing cells and viral vector is rotated, for greater than or about 5 minutes, such as greater than or about 10 minutes, greater than or about 15 minutes, greater than or about 20 minutes, greater than or about 30 minutes, greater than or about 45 minutes, greater than or about 60 minutes, greater than or about 90 minutes or greater than or about 120 minutes; or between or between about 5 minutes and 120 minutes, 30 minutes and 90 minutes, 15 minutes and 60 minutes, 15 minutes and 45 minutes, 30 minutes and 60 minutes or 45 minutes and 60 minutes, each inclusive. In some disclosures, the cells are spinoculated with the viral vector for or for about 30 minutes. In certain disclosures, the cells are spinoculated with the viral vector for or for about 60 minutes.

In some disclosures, the method of transduction includes a spinoculation, e.g., a rotation or centrifugation of the transduction composition, and optionally air, in the centrifugal chamber for greater than or about 5 minutes, such as greater than or about 10 minutes, greater than or about 15 minutes, greater than or about 20 minutes, greater than or about 30 minutes, greater than or about 45 minutes, greater than or about 60 minutes, greater than or about 90 minutes or greater than or about 120 minutes. In some disclosures, the transduction composition, and optionally air, is rotated or centrifuged in the centrifugal chamber for greater than 5 minutes, but for no more than 60 minutes, no more than 45 minutes, no more than 30 minutes or no more than 15 minutes. In particular disclosures, the transduction includes rotation or centrifugation for or for about 60 minutes.

In some disclosures, the method of transduction includes rotation or centrifugation of the transduction composition, and optionally air, in the centrifugal chamber for between or between about 10 minutes and 60 minutes, 15 minutes and 60 minutes, 15 minutes and 45 minutes, 30 minutes and 60 minutes or 45 minutes and 60 minutes, each inclusive, and at a force at the internal surface of the side wall of the internal cavity and/or at a surface layer of the cells of, of about, or at 1000 g, 1100 g, 1200 g, 1400 g, 1500 g, 1600 g, 1800 g, 2000 g, 2200 g, 2400 g, 2800 g, 3200 g or 3600 g. In particular disclosures, the method of transduction includes rotation or centrifugation of the transduction composition, e.g., the cells and the viral vector particles, at or at about 1600 g for or for about 60 minutes.

### 2. Viral Vector Particles

In some disclosures, recombinant nucleic acids are transferred into cells using recombinant infectious virus particles, such as, e.g., vectors derived from simian virus 40 (SV40), adenoviruses, adeno-associated virus (AAV). In some disclosures, recombinant nucleic acids are transferred into T cells using recombinant lentiviral vectors or retroviral vectors, such as gamma-retroviral vectors (see, e.g., Koste et al. (2014) Gene Therapy 2014 Apr 3. doi: 10.1038/gt.2014.25; Carlens et al. (2000) Exp Hematol 28(10): 1137-46; Alonso-Camino et al. (2013) Mol Ther Nucl Acids 2, e93; Park et al., Trends Biotechnol. 2011 November 29(11): 550-557.

In some disclosures, the retroviral vector has a long terminal repeat sequence (LTR), e.g., a retroviral vector derived from the Moloney murine leukemia virus (MoMl,V), myeloproliferative sarcoma virus (MPSV), murine embryonic stem cell virus (MESV), murine stem cell virus (MSCV), or spleen focus forming virus (SFFV). Most retroviral vectors are derived from murine retroviruses. In some disclosures, the retroviruses include those derived from any avian or mammalian cell source. The retroviruses typically are amphotropic, meaning that they are capable of infecting host cells of several species, including humans. In one disclosure, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989) BioTechniques 7:980-990; Miller, A. D. (1990) Human Gene Therapy 1:5-14; Scarpa et al. (1991) Virology 180:849-852; Burns et al. (1993) Proc. Natl. Acad. Sci. USA 90:8033-8037; and Boris-Lawrie and Temin (1993) Cur. Opin. Genet. Develop. 3:102-109.

The viral vector genome is typically constructed in a plasmid form that can be transfected into a packaging or producer cell line. In any of such disclosures, the nucleic acid encoding a recombinant protein, such as a recombinant receptor, is inserted or located in a region of the viral vector, such as generally in a non-essential region of the viral genome. In some disclosures, the nucleic acid is inserted into the viral genome in the place of certain viral sequences to produce a virus that is replication defective.

Any of a variety of known methods can be used to produce retroviral particles whose genome contains an RNA copy of the viral vector genome. In some disclosures, at least two components are involved in making a virus-based gene delivery system: first, packaging plasmids, encompassing the structural proteins as well as the enzymes necessary to generate a viral vector particle, and second, the viral vector itself, i.e., the genetic material to be transferred. Biosafety safeguards can be introduced in the design of one or both of these components.

In some disclosures, the packaging plasmid can contain all retroviral, such as HIV-1, proteins other than envelope proteins (Naldini et al., 1998). In other disclosures, viral vectors can lack additional viral genes, such as those that are associated with virulence, e.g. vpr, vif, vpu and nef, and/or Tat, a primary transactivator of HIV. In some disclosures, lentiviral vectors, such as HIV-based lentiviral vectors, comprise only three genes of the parental virus: gag, pol and rev, which reduces or eliminates the possibility of reconstitution of a wild-type virus through recombination.

In some disclosures, the viral vector genome is introduced into a packaging cell line that contains all the components necessary to package viral genomic RNA, transcribed from the viral vector genome, into viral particles. Alternatively, the viral vector genome may comprise one or more genes encoding viral components in addition to the one or more sequences, e.g., recombinant nucleic acids, of interest. In some disclosures, in order to prevent replication of the genome in the target cell, however, endogenous viral genes required for replication are removed and provided separately in the packaging cell line.

In some disclosures, a packaging cell line is transfected with one or more plasmid vectors containing the components necessary to generate the particles. In some disclosures, a packaging cell line is transfected with a plasmid containing the viral vector genome, including the LTRs, the cis-acting packaging sequence and the sequence of interest, i.e. a nucleic acid encoding an antigen receptor, such as a CAR; and one or more helper plasmids encoding the virus enzymatic and/or structural components, such as Gag, pol and/or rev. In some disclosures, multiple vectors are utilized to separate the various genetic components that generate the retroviral vector particles. In some such disclosures, providing separate vectors to the packaging cell reduces the chance of recombination events that might otherwise generate replication competent viruses. In some disclosures, a single plasmid vector having all of the retroviral components can be used.

In some disclosures, the retroviral vector particle, such as lentiviral vector particle, is pseudotyped to increase the transduction efficiency of host cells. For example, a retroviral vector particle, such as a lentiviral vector particle, in some disclosures is pseudotyped with a VSV-G glycoprotein, which provides a broad cell host range extending the cell types that can be transduced. In some disclosures, a packaging cell line is transfected with a plasmid or polynucleotide encoding a non-native envelope glycoprotein, such as to include xenotropic, polytropic or amphotropic envelopes, such as Sindbis virus envelope, GALV or VSV-G.

In some disclosures, the packaging cell line provides the components, including viral regulatory and structural proteins, that are required in trans for the packaging of the viral genomic RNA into lentiviral vector particles. In some disclosures, the packaging cell line may be any cell line that is capable of expressing lentiviral proteins and producing functional lentiviral vector particles. In some disclosures, suitable packaging cell lines include 293 (ATCC CCL X), 293T, HeLA (ATCC CCL 2), D17 (ATCC CCL 183), MDCK (ATCC CCL 34), BHK (ATCC CCL-10) and Cf2Th (ATCC CRL 1430) cells.

In some disclosures, the packaging cell line stably expresses the viral protein(s). For example, in some disclosures, a packaging cell line containing the gag, pol, rev and/or other structural genes but without the LTR and packaging components can be constructed. In some disclosures, a packaging cell line can be transiently transfected with nucleic acid molecules encoding one or more viral proteins along with the viral vector genome containing a nucleic acid molecule encoding a heterologous protein, and/or a nucleic acid encoding an envelope glycoprotein.

In some disclosures, the viral vectors and the packaging and/or helper plasmids are introduced via transfection or infection into the packaging cell line. The packaging cell line produces viral vector particles that contain the viral vector genome. Methods for transfection or infection are well known. Non-limiting examples disclosed herein include calcium phosphate, DEAE-dextran and lipofection methods, electroporation and microinjection.

When a recombinant plasmid and the retroviral LTR and packaging sequences are introduced into a special cell line (e.g., by calcium phosphate precipitation for example), the packaging sequences may permit the RNA transcript of the recombinant plasmid to be packaged into viral particles, which then may be secreted into the culture media. The media containing the recombinant retroviruses in some disclosures is then collected, optionally concentrated, and used for gene transfer. For example, in some disclosures, after cotransfection of the packaging plasmids and the transfer vector to the packaging cell line, the viral vector particles are recovered from the culture media and titered by standard methods used by those of skill in the art.

In some disclosures, a retroviral vector, such as a lentiviral vector, can be produced in a packaging cell line, such as an exemplary HEK 293T cell line, by introduction of plasmids to allow generation of lentiviral particles. In some disclosures, a packaging cell is transfected and/or contains a polynucleotide encoding gag and pol, and a polynucleotide encoding a recombinant receptor, such as an antigen receptor, for example, a CAR. In some disclosures, the packaging cell line is optionally and/or additionally transfected with and/or contains a polynucleotide encoding a rev protein. In some disclosures, the packaging cell line is optionally and/or additionally transfected with and/or contains a polynucleotide encoding a non-native envelope glycoprotein, such as VSV-G. In some such disclosures, approximately two days after transfection of cells, e.g. HEK 293T cells, the cell supernatant contains recombinant lentiviral vectors, which can be recovered and titered.

Recovered and/or produced retroviral vector particles can be used to transduce target cells using the methods as described. Once in the target cells, the viral RNA is reverse-transcribed, imported into the nucleus and stably integrated into the host genome. One or two days after the integration of the viral RNA, the expression of the recombinant protein, e.g. antigen receptor, such as CAR, can be detected.

### 3. Incubation with Viral Vector

In particular disclosures, transforming or transducing the cells is or includes one or more steps of incubating the cells, e.g., in the presence of the viral vector. In some disclosures, cells, e.g., cells of the transformed cell population, are incubated subsequent to genetically engineering, transforming, transducing, or transfecting the cells.

In certain disclosures, the incubation is performed under static conditions, such as conditions that do not involve centrifugation, shaking, rotating, rocking, or perfusion, e.g., continuous or semi-continuous perfusion of the media. In some disclosures, either prior to or shortly after, e.g., within 5, 15, or 30 minutes, the initiation of the incubation, the cells are transferred (e.g., transferred under sterile conditions) to a container such as a bag or vial, and placed in an incubator.

In some disclosures, the incubation is performed in serum free media. In some disclosures, the serum free media is a defined and/or well-defined cell culture media. In certain disclosures, the serum free media is a controlled culture media that has been processed, e.g., filtered to remove inhibitors and/or growth factors. In some disclosures, the serum free media contains proteins. In certain disclosures, the serum-free media may contain serum albumin, hydrolysates, growth factors, hormones, carrier proteins, and/or attachment factors.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some disclosures, at least a portion of the incubation is carried out in the internal cavity of a centrifugal chamber, for example, under centrifugal rotation, such as described in International Publication Number WO2016/073602.

In some disclosures, the cells, and optionally the heterologous or recombinant polypeptide, e.g., the viral vectors, are transferred into a container for the incubation. In some disclosures, the container is a vial. In particular disclosures, the container is a bag. In some disclosures, the cells, and optionally the heterologous or recombinant polypeptide, are transferred into the container under closed or sterile conditions. In some disclosures, the container, e.g., the vial or bag, is then placed into an incubator for all or a portion of the incubation. In particular disclosures, incubator is set at, at about, or at least 16°C, 24°C, or 35°C. In some disclosures, the incubator is set at 37°C, at about at 37°C, or at 37°C $\pm 2$°C, $\pm 1$°C, $\pm 0.5$°C, or $\pm 0.1$°C.

In particular disclosures, the cells are incubated in the presence of one or more cytokines. In certain disclosures, the one or more cytokines are recombinant cytokines. In particular disclosures, the one or more cytokines are human recombinant cytokines. In certain disclosures, the one or more cytokines bind to and/or are capable of binding to receptors that are expressed by and/or are endogenous to T cells. In particular disclosures, the one or more cytokines is or includes a member of the 4-alpha-helix bundle family of cytokines. In some disclosures, members of the 4-alpha-helix bundle family of cytokines include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF). In some disclosures, the one or more cytokines is or includes IL-15. In particular disclosures, the one or more cytokines is or includes IL-7. In particular disclosures, the one or more cytokines is or includes recombinant IL-2.

In some disclosures, the cells are incubated in the absence of recombinant cytokines.

In some disclosures, all or a portion of the incubation is performed in basal media. In some disclosures, the basal media is a balanced salt solution (e.g., PBS, DPBS, HBSS, EBSS). In some disclosures, the basal media is selected from Dulbecco's Modified Eagle's Medium (DMEM), Minimal Essential Medium (MEM), Basal Medium Eagle (BME), F-10, F-12, RPMI 1640, Glasgow's Minimal Essential Medium (GMEM), alpha Minimal Essential Medium (alpha MEM), Iscove's Modified Dulbecco's Medium, and M199. In some disclosures, the base media is a complex medium (e.g., RPMI-1640, IMDM). In some disclosures, the base medium is OpTmizer™ CTS™ T-Cell Expansion Basal Medium (ThermoFisher).

In some disclosures, the basal medium contains a mixture of inorganic salts, sugars, amino acids, and, optionally, vitamins, organic acids and/or buffers or other well known cell culture nutrients. In addition to nutrients, the medium also helps maintain pH and osmolality. In some disclosures, the reagents of the basal media support cell growth, proliferation and/or expansion. A wide variety of commercially available basal media are well known to those skilled in the art, and include Dulbeccos' Modified Eagles Medium (DMEM), Roswell Park Memorial Institute Medium (RPMI), Iscove modified Dulbeccos' medium and Hams medium. In some disclosures, the basal medium is Iscove's Modified Dulbecco's Medium, RPMI- 1640, or $\alpha$-MEM.

In certain disclosures, the basal media is supplemented with additional additives. In some disclosures, the basal media is not supplemented with any additional additives. Additives to cell culture media may include, but is not limited to nutrients, sugars, e.g., glucose, amino acids, vitamins, or additives such as ATP and NADH.

In some disclosures, cells are incubated with the heterologous polynucleotide, e.g., the viral vector. In certain disclosures, the cells are incubated the cells with the polynucleotide, e.g., viral vector, for, for about, or for at least 18 hours, 24 hours, 30 hours, 36 hours, 40 hours, 48 hours, 54 hours, 60 hours, 72 hours, 84 hours, 96 hours, or more than 96 hours. In certain disclosures, the total duration of the incubation is, is about, or is at least 12 hours, 18 hours, 24 hours, 30 hours, 36 hours, 42 hours, 48 hours, 54 hours, 60 hours, 72 hours, 84 hours, 96 hours, 108 hours, or 120 hours. In particular disclosures, the incubation is completed at, at about, or within 120 hours, 108 hours, 96 hours, 84 hours, 72 hours, 60 hours, 54 hours, 48 hours, 42 hours, 36 hours, 30 hours, 24 hours, 18 hours, or 12 hours. In some disclosures, the total duration of the incubation is between or between about 12 hour and 120 hours, 18 hour and 96 hours, 24 hours and 72 hours, or 24 hours and 48 hours, inclusive. In some disclosures, the total duration of the incubation is between or about between 1 hour and 48 hours, 4 hours and 36 hours, 8 hours and 30 hours or 12 hours and 24 hours, inclusive.

## C. Cultivation

In particular disclosures, processes for generating compositions of engineered T cells disclosed herein are performed in connection with an optional cultivation step or a step where cells undergo expansion or proliferation *in vitro,* such as subsequent to an introduction of a heterologous polynucleotide into the cell. In some disclosures, the disclosed methods include one or more steps for cultivating cells, e.g., cultivating cells under conditions that promote proliferation or expansion. In some disclosures, cells are cultivated under conditions that promote proliferation or expansion subsequent to a step of genetically engineering, e.g., introducing a recombinant polypeptide to the cells by transduction or transfection. In particular disclosures, the cells are cultivated after the cells have been incubated under stimulating conditions and transduced or transfected with a recombinant polynucleotide, e.g., a polynucleotide encoding a recombinant receptor. Thus, in some disclosures, cells of a transformed population of enriched T cells are cultivated. In particular disclosures, the one or more transformed populations have been previously depleted of or separated from CD57+ T cells.

In some disclosures, processes for generating compositions of engineered T cells disclosed herein do not require a cultivation step or a step where cells undergo expansion or proliferation *in vitro* subsequent to an introduction of a heterologous polynucleotide into the cells. In some disclosures, the process or method for generating or manufacturing engineered cell compositions do not include a step for cultivation, e.g., to expand the number of engineered cells in the therapeutic composition.

In certain disclosures, the one or more populations of engineered T cells are or include two separate populations of enriched T cells. In particular disclosures, two separate populations of enriched T cells, e.g., two separate populations of enriched T cells selected, isolated, and/or enriched from the same biological sample, are separately cultivated under stimulating conditions. In certain disclosures, the two separate populations include a population of enriched CD4+ T cells, e.g., enriched CD57- CD4+ T cells. In particular disclosures, the two separate populations include a population of enriched CD8+ T cells, e.g., enriched CD57- CD8+ T cells. In some disclosures, two separate populations of enriched CD4+ T cells and enriched CD8+ T cells, e.g., two separate populations of enriched CD57- CD4+ T cells and enriched CD57- CD8+ T cells, are separately cultivated, e.g., under conditions that promote proliferation and/or expansion. In some disclosures, a single population of enriched T cells is cultivated, e.g., a single population including or containing CD57- CD4+ T cells and CD57- CD8+ T cells. In certain disclosures, the single population is a population of enriched CD4+ T cells. In some disclosures, the single population is a population of enriched CD4+ and CD8+ T cells that have been combined from separate populations prior to the cultivation.

In some disclosures, the population of enriched CD4+ T cells (e.g., CD57- CD4+ T cells) that is cultivated, e.g., under conditions that promote proliferation and/or expansion, includes at least at or about 60%, at least at or about 65%, at least at or about 70%, at least at or about 75%, at least at or about 80%, at least at or about 85%, at least at or about 90%, at least at or about 95%, at least at or about 98%, at least at or about 99%, at least at or about 99.5%, at least at or about 99.9%, or at or at about 100% CD4+ T cells (e.g., CD57- CD4+ T cells). In some disclosures, the population includes at least at or about 30%, at least at or about 40%, at least at or about 50%, at least at or about 60%, at least at or about 70%, at least at or about 80%, at least at or about 90%, at least at or about 95%, at least at or about 98%, at least at or about 99%, at least at or about 99.5%, at least at or about 99.9%, or at or at about 100% CD4+ T (e.g., CD57- CD4+ T cells) cells that express the recombinant receptor and/or have been transduced or transfected with the recombinant polynucleotide. In certain disclosures, the population of enriched CD4+ T cells (e.g., CD57- CD4+ T cells) that is cultivated includes less than at or about 40%, less than at or about 35%, less than at or about 30%, less than at or about 25%, less than at or about 20%, less than at or about 15%, less than at or about 10%, less than at or about 5%, less than at or about 1%, less than at or about 0.1%, or less than at or about 0.01% CD8+ T cells, and/or contains no CD8+ T cells, and/or is free or substantially free of CD8+ T cells.

In some disclosures, the population of enriched CD8+ T cells that is cultivated, e.g., under conditions that promote proliferation and/or expansion, includes at least at or about 60%, at least at or about 65%, at least at or about 70%, at least at or about 75%, at least at or about 80%, at least at or about 85%, at least at or about 90%, at least at or about 95%, at least at or about 98%, at least at or about 99%, at least at or about 99.5%, at least at or about 99.9%, or at or at about 100% CD8+ T cells (e.g., CD57- CD8+ T cells). In particular disclosures, the population includes at least at or about 30%, at least at or about 40%, at least at or about 50%, at least at or about 60%, at least at or about 70%, at least at or about 80%, at least at or about 90%, at least at or about 95%, at least at or about 98%, at least at or about 99%, at least at or about 99.5%, at least at or about 99.9%, or at or at about 100% CD8+ T cells (e.g., CD57- CD8+ T cells) that express the recombinant receptor and/or have been transduced or transfected with the recombinant polynucleotide. In certain disclosures, the population of enriched CD8+ T cells that is incubated under stimulating conditions includes less than at or about 40%, less than at or about 35%, less than at or about 30%, less than at or about 25%, less than at or about 20%, less than at or about 15%, less than at or about 10%, less than at or about 5%, less than at or about 1%, less than at or about 0.1%, or less than at or about 0.01% CD4+ T cells, and/or contains no CD4+ T cells, and/or is free or substantially free of CD4+ T cells.

In some disclosures, the cultivation is performed under conditions that generally include a temperature suitable for the growth of primary immune cells, such as human T lymphocytes, for example, at least at or about 25 degrees Celsius, generally at least at or about 30 degrees, and generally at or about 37 degrees Celsius. In some disclosures, the population of enriched T cells is incubated at a temperature of 25 to 38°C, such as 30 to 37°C, for example at or about 37 °C $\pm$ 2 °C. In some disclosures, the incubation is carried out for a time period until the culture, e.g. cultivation or expansion, results in a desired or threshold density, number or dose of cells. In some disclosures, the cultivation is greater than or greater than about or is for about or 24 hours, 48 hours, 72 hours, 96 hours, 5 days, 6 days, 7 days, 8 days, 9 days or more.

In some disclosures, the cells are cultivated to achieve a threshold expansion that is a an amount, concentration, or density of cells that is least 50%, at least at or about 60%, at least at or about 70%, at least at or about 80%, at least at or about 90%, at least at or about 95%, at least at or about 100%, at least at or about 150%, at least at or about 1-fold, at least at or about 2-fold, at least at or about 3-fold, at least at or about 4-fold, at least at or about 5-fold, at least at or about 10-fold, at least at or about 20-fold, at least at or about 50-fold greater as compared to the amount, concentration, or density of cells at the beginning of the cultivation.

As described in the Examples, the number of population doublings inversely correlated with the probability of progression free survival in patients treated with the therapeutic T cell composition (e.g., output composition). Thus, in some disclosures, the number of population doublings is no greater than 1, 2, 3, 4, 5, 6, 8, 9, or 10 population doublings. In some disclosures, the number of population doublings is no greater than 1, 2, 3, 4, 5, or 6 population doublings. In some disclosures, reduced numbers of population doublings (e.g., less than or equal to 6) are achieved by expanding T cell compositions (e.g., engineered CD4+, CD+ 8 T cells) that include at least or at least about 55%, 60%, 65%, 70%, 75%, 80%, 85%, 90%, 91%, 92%, 93%, 94%, 95%, 96%, 97%, 98%, or 99% naïve-like and/or central memory T cells. In some disclosures, reduced numbers of population doublings (e.g., less than or equal to 6) are achieved by expanding T cell compositions (e.g., engineered CD4+, CD+ 8 T cells) that include no more than 45%, 40%, 35%, 30%, 25%, 20%, 15%, 10%, 9%, 8%, 7%, 6%, 5%, 4%, 3%, 2%, or 1% CD57+ T cells. In some disclosures, reduced numbers of population doublings (e.g., less than or equal to 6) are achieved by using a seed density of greater than $0.05\times10^6$ cells/mL, $0.1\times10^6$ cells/mL, $0.15\times10^6$ cells/mL, $0.2\times10^6$ cells/mL, $0.25\times10^6$ cells/mL, $0.3\times10^6$ cells/mL, $0.35\times10^6$ cells/mL, $0.4\times10^6$ cells/mL, $0.45\times10^6$ cells/mL, or more.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In particular disclosures, a composition of enriched T cells is cultivated in the presence of one or more cytokines. In certain disclosures, the one or more cytokines are recombinant cytokines. In particular disclosures, the one or more cytokines are human recombinant cytokines. In certain disclosures, the one or more cytokines bind to and/or are capable of binding to receptors that are expressed by and/or are endogenous to T cells. In particular disclosures, the one or more cytokines is or includes a member of the 4-alpha-helix bundle family of cytokines. In some disclosures, members of the 4-alpha-helix bundle family of cytokines include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF). In some disclosures, the one or more cytokines is or includes IL-15. In particular disclosures, the one or more cytokines is or includes IL-7. In particular disclosures, the one or more cytokines is or includes recombinant IL-2.

In some disclosures, incubation is carried out in accordance with techniques such as those described in US Patent No. 6,040,1 77 to Riddell et al., Klebanoff et al.(2012) J Immunother. 35(9): 651-660, Terakura et al. (2012) Blood. 1:72-82, and/or Wang et al. (2012) J Immunother. 35(9):689-701.

In some disclosures, at least a portion of the cultivation is carried out in the internal cavity of a centrifugal chamber, for example, under centrifugal rotation, such as described in International Publication Number WO2016/073602. In some disclosures, at least a portion of the incubation performed in a centrifugal chamber includes mixing with a reagent or reagents to induce stimulation and/or activation. In some disclosures, cells, such as selected cells, are mixed with a stimulating condition or stimulatory agent in the centrifugal chamber. In some disclosures of such processes, a volume of cells is mixed with an amount of one or more stimulating conditions or agents that is far less than is normally employed when performing similar stimulations in a cell culture plate or other system.

In some disclosures, the cultivation is performed with the addition of an cultivation buffer to the cells and stimulating agent to achieve a target volume of, for example, 10 mL to 2,000 mL, such as at least or about at least or about or 10 mL, 20 mL, 30 mL, 40 mL, 50 mL, 60 mL, 70 mL, 80 mL, 90 mL, 100 mL, 150 mL, 200 mL, 300 mL, 400 mL, 500 mL, 600 mL, 700 mL, 800 mL, 900 mL, 1,000 mL, 1,200 mL, 1,400 mL, 1,600 mL, 1,800 mL, 2,000 mL, 2,200 mL or 2,400 mL.. In some disclosures, the incubation buffer and stimulating agent are premixed before addition to the cells. In some disclosures, the stimulating incubation is carried out with periodic gentle mixing condition, which can aid in promoting energetically favored interactions and thereby permit the use of less overall stimulating agent while achieving stimulating and activation of cells.

In some disclosures, the incubation generally is carried out under mixing conditions, such as in the presence of spinning, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm), such as at an RCF at the sample or wall of the chamber or other container of from or from about 80g to 100g (e.g. at or about or at least 80 g, 85 g, 90 g, 95 g, or 100 g). In some disclosures, the spin is carried out using repeated intervals of a spin at such low speed followed by a rest period, such as a spin and/or rest for 1, 2, 3, 4, 5, 6, 7, 8, 9, or 10 seconds, such as a spin at approximately 1 or 2 seconds followed by a rest for approximately 5, 6, 7, or 8 seconds.

In particular disclosures, the cultivation is performed in a closed system. In certain disclosures, the cultivation is performed in a closed system under sterile conditions. In particular disclosures, the cultivation is performed in the same closed system as one or more steps of the disclosed systems. In some disclosures the population of enriched T cells is removed from a closed system and placed in and/or connected to a bioreactor for the cultivation. Examples of suitable bioreactors for the cultivation include, but are not limited to, GE Xuri W25, GE Xuri W5, Sartorius BioSTAT RM 20 | 50, Finesse SmartRocker Bioreactor Systems, and Pall XRS Bioreactor Systems. In some disclosures, the bioreactor is used to perfuse and/or mix the cells during at least a portion of the cultivation step.

In some disclosures, the mixing is or includes rocking and/or motioning. In some cases disclosed herein, the bioreactor can be subject to motioning or rocking, which, in some disclosures, can increase oxygen transfer. Motioning the bioreactor may include, but is not limited to rotating along a horizontal axis, rotating along a vertical axis, a rocking motion along a tilted or inclined horizontal axis of the bioreactor or any combination thereof. In some disclosures, at least a portion of the incubation is carried out with rocking. The rocking speed and rocking angle may be adjusted to achieve a desired agitation. In some disclosures the rock angle is 20°, 19°, 18°, 17°, 16°, 15°, 14°, 13°, 12°, 11°, 10°, 9°, 8°, 7°, 6°, 5°, 4°, 3°, 2° or 1°. In certain disclosures, the rock angle is between 6-16°. In other disclosures, the rock angle is between 7-16°. In other disclosures, the rock angle is between 8-12°. In some disclosures, the rock rate is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 rpm. In some disclosures, the rock rate is between 4 and 12 rpm, such as between 4 and 6 rpm, inclusive.

In some disclosures, the bioreactor maintains the temperature at or near 37°C and $CO_2$ levels at or near 5% with a steady air flow at, at about, or at least 0.01 L/min, 0.05 L/min, 0.1 L/min, 0.2 L/min, 0.3 L/min, 0.4 L/min, 0.5 L/min, 1.0 L/min, 1.5 L/min, or 2.0 L/min or greater than 2.0 L/min. In certain disclosures, at least a portion of the cultivation is performed with perfusion, such as with a rate of 290 ml/day, 580 ml/day, and/or 1160 ml/day, e.g., depending on the timing in relation to the start of the cultivation and/or density of the cultivated cells. In some disclosures, at least a portion of the cell culture expansion is performed with a rocking motion, such as at an angle of between 5° and 10°, such as 6°, at a constant rocking speed, such as a speed of between 5 and 15 RPM, such as 6 RMP or 10 RPM.

In some disclosures, the at least a portion of the cultivation step is performed under constant perfusion, e.g., a perfusion at a slow steady rate. In some disclosures, the perfusion is or include an outflow of liquid e.g., used media, and an inflow of fresh media. In certain disclosures, the perfusion replaces used media with fresh media. In some disclosures, at least a portion of the cultivation is performed under perfusion at a steady rate of or of about or of at least 100 ml/day, 200 ml/day, 250 ml/day, 275 ml/day, 290 ml/day, 300 ml/day, 350 ml/day, 400 ml/day, 450 ml/day, 500 ml/day, 550 ml/day, 575 ml/day, 580 ml/day, 600 ml/day, 650 ml/day, 700 ml/day, 750 ml/day, 800 ml/day, 850 ml/day, 900 ml/day, 950 ml/day, 1000 ml/day, 1100 ml/day, 1160 ml/day, 1200 ml/day, 1400 ml/day, 1500 ml day, 1600 ml/day, 1800 ml/day, 2000 ml/day, 2200 ml/day, or 2400 ml/day.

**D. Harvesting, Collecting, and Formulating cells**

In some disclosures, one or more process steps (e.g. carried out in the centrifugal chamber and/or closed system) for manufacturing, generating or producing a cell therapy and/or engineered cells may include formulation of cells, such as formulation of genetically engineered cells resulting from the disclosed transduction processing steps prior to or after the culturing, e.g. cultivation and expansion, and/or one or more other processing steps as described. In some disclosures, the disclosed methods associated with formulation of cells include processing transduced cells, such as cells transduced and/or expanded using the processing steps described above, in a closed system.

In some disclosures, the stimulatory reagent is removed and/or separated from the cells prior to the formulating. In particular disclosures, the stimulatory reagent is removed and/or separated from the cells after the cultivation. In certain disclosures, the stimulatory agent is removed and/or separated from the cells subsequent to the cultivation and prior to formulating the cultivated cells, .e.g., under conditions that promote proliferation and/or expansion. In certain disclosures, the stimulatory reagent is a stimulatory reagent that is described in herein, e.g., in Section III.A. 1. In particular disclosures, the stimulatory reagent is removed and/or separated from the cells as described herein, e.g., in Section III.A.2.

In certain disclosures, cells of a cultivated population of cells are In some disclosures, the cells are formulated between 0 days and 10 days, between 0 and 5 days, between 2 days and 7 days, between 0.5 days, and 4 days, or between 1 day and 3 days after the cells after the threshold cell count, density, and/or expansion has been achieved during the cultivation. In certain disclosures, the cells are formulated at or at or about or within 12 hours, 18 hours, 24 hours, 1 day, 2 days, or 3 days after the threshold cell count, density, and/or expansion has been achieved during the cultivation. In some disclosures, the cells are formulated within or within about 1 day after the threshold cell count, density, and/or expansion has been achieved during the cultivation.

In certain disclosures, the amount of time from the initiation of the stimulation to collecting, harvesting, or formulating the cells is, is about, or is less than 36 hours, 42 hours, 48 hours, 54 hours, 60 hours, 72 hours, 84 hours, 96 hours, 108 hours, or 120 hours. In certain disclosures, the amount of time from the initiation of the stimulation to collecting, harvesting, or formulating the cells is, is about, or is less than 1.5 days, 2 days, 3 days, 4 days, or 5 days. In some disclosures, the amount of time from the initiation of the stimulation to collecting, harvesting, or formulating the cells for generating engineered cells, from the initiation of the stimulation to collecting, harvesting, or formulating the cells is between or between about 36 hours and 120 hours, 48 hours and 96 hours, or 48 hours and 72 hours, inclusive, or between or between about 1.5 days and 5 days, 2 days and 4 days, or 2 day and 3 days, inclusive,. In particular disclosures, the amount of time from the initiation of incubation to harvesting, collecting, or formulating the cells is, is about, or is less than 48 hours, 72 hours, or 96 hours. In particular disclosures, the amount of time from the initiation of incubation to harvesting, collecting, or formulating the cells is, is about, or is less than 2 days, 3 days, or 4 days. In particular disclosures, the amount of time from the initiation of incubation to harvesting, collecting, or formulating the cells is 48 hours ± 6 hours, 72 hours ± 6 hours, or 96 hours ± 6 hours. In particular disclosures, the amount of time from the initiation of incubation to harvesting, collecting, or formulating the cells is or is about 96 hours or four days.

In certain disclosures, the cells are harvested or collected at least when the integrated vector is detected in the genome. In some disclosures, the cells are harvested or collected prior to stable integrated vector copy number (iVCN) per diploid genome. In particular disclosures, the cells are harvested or collected after the integrated vector is detected in the genome but prior to when a stable iVCN per diploid genome is achieved.

In some disclosures, the cells are harvested or collected before the iVCN of reaches, reaches about, or reaches at least 5.0, 4.0, 3.0, 2.5, 2.0, 1.75, 1.5, 1.25, 1.2, 1.1, 1.0, 0.9, 0.8, 0.75, 0.7, 0.6, 0.5, 0.4, 0.3, or 0.25 copies per diploid genome. In particular disclosures, the cells are harvested or collected before the iVCN reaches or about 1.0 copy per diploid genome. In some disclosures, the cells are collected or harvested before the iVCN reaches or about 0.5 copies per diploid genome.

In certain disclosures, the cells are havested prior to, prior to about, or prior to at least one, two, three, four, five, six, eight, ten, twenty, or more cell doublings of the cell population, e.g., doublings that occur during the incubating.

In particular disclosures, the cells are harvested or collected at a time before the total number cells, e.g., total number of incubated cells or cells undergoing the incubation, is greater than or than about one, two, three, four, five, six, eight, ten, twenty, or more than twenty times the number of cells of the input population, e.g., the total number of cells that were contacted with the stimulatory reagent. In some disclosures, the cells are harvested or collected at a time before the total number of incubated cells is greater than or than about one, two, three, four, five, six, eight, ten, twenty, or more than twenty times the total number of cells that were transformed, transduced, or spinoculated, e.g., the total number of cells that were contacted with a viral vector. In certain disclosures, the cells are T cells, viable T cells, CD3+ T cells, CD4+ T cells, CD8+ T cells, CAR expressing T cells, or a combination of any of the foregoing. In particular disclosures, the cells are harvested or collected at a time before the total number of cells is greater than the total number of cells of the input population. In various disclosures, the cells are harvested or collected at a time before the total number of viable CD3+ T cells is greater than the total number of viable CD3+ cells of the input population. In particular disclosures, the cells are harvested or collected at a time before the total number of cells is greater than the total number of cells of the transformed, transduced, or spinoculated cells. In various disclosures, the cells are harvested or collected at a time before the total number of viable CD3+ T cells is greater than the total number of viable CD3+ cells of the transformed, transduced, or spinoculated cells. In various disclosures, the cells are harvested or collected at a time before the total number of viable CD4+ cells and CD8+ cells is greater than the total number of viable CD4+ cells and CD8+ cells of the input population. In particular disclosures, the cells are harvested or collected at a time before the total number of cells is greater than the total number of cells of the transformed, transduced, or spinoculated cells. In various disclosures, the cells are harvested or collected at a time before the total number of viable CD4+ cells and CD8+ cells is greater than the total number of viable CD4+ cells and CD8+ cells of the transformed, transduced, or spinoculated cells.

In some disclosures, the disclosed methods for manufacturing, generating or producing a cell therapy and/or engineered cells may include formulation of cells, such as formulation of genetically engineered cells resulting from the disclosed processing steps prior to or after the incubating, engineering, and cultivating, and/or one or more other processing steps as described. In some disclosures, the disclosed methods associated with formulation of cells include processing transduced cells, such as cells transduced and/or expanded using the processing steps described above, in a closed system. In some disclosures, the dose of cells comprising cells engineered with a recombinant antigen receptor, e.g. CAR or TCR, is disclosed as a composition or formulation, such as a pharmaceutical composition or formulation. Such compositions can be used in accord with the disclosed methods, such as in the prevention or treatment of diseases, conditions, and disorders, or in detection, diagnostic, and prognostic methods.

In some cases disclosed herein, the cells are processed in one or more steps (e.g. carried out in the centrifugal chamber and/or closed system) for manufacturing, generating or producing a cell therapy and/or engineered cells may include formulation of cells, such as formulation of genetically engineered cells resulting from the disclosed transduction processing steps prior to or after the culturing, e.g. cultivation and expansion, and/or one or more other processing steps as described. In some cases disclosed herein, the cells can be formulated in an amount for dosage administration, such as for a single unit dosage administration or multiple dosage administration. In some disclosures, the disclosed methods associated with formulation of cells include processing transduced cells, such as cells transduced and/or expanded using the processing steps described above, in a closed system.

In certain disclosures, one or more compositions of enriched T cells are formulated. In particular disclosures, one or more compositions of enriched T cells are formulated after the one or more compositions have been engineered and/or cultivated. In particular disclosures, the one or more compositions are input compositions. In some disclosures, the one or more input compositions have been previously cryopreserved and stored, and are thawed prior to the incubation.

In certain disclosures, the formulated cells are output cells. In some disclosures, a formulated composition of enriched T cells is an output composition of enriched T cells. In particular disclosures, the formulated CD4+ T cells and formulated CD8+ T cells are the output CD4+ and CD8+ T cells. In particular disclosures, a formulated cell composition, e.g., a formulated composition of enriched CD4+ and CD8+ cells, is an output cell composition, e.g., an output composition of enriched CD4+ and CD8+ cells.

In some disclosures, cells can be formulated into a container, such as a bag or vial. In some disclosures, the cells are formulated between 0 days and 10 days, between 0 and 5 days, between 2 days and 7 days, between 0.5 days, and 4 days, or between 1 day and 3 days after the cells after the threshold cell count, density, and/or expansion has been achieved during the cultivation. In certain disclosures, the cells are formulated at or at or about or within 12 hours, 18 hours, 24 hours, 1 day, 2 days, or 3 days after the threshold cell count, density, and/or expansion has been achieved during the cultivation. In some disclosures, the cells are formulated within or within about 1 day after the threshold cell count, density, and/or expansion has been achieved during the cultivation.

In certain disclosures, the cells are cultivated for a minimum duration or amount of time, for example, so that cells are harvested in a less activated state than if they were formulated at an earlier time point during the cultivation, regardless of when the threshold is achieved. In some disclosures, the cells are cultivated between 0 day and 3 days, e.g., between 0 and 3 days, between 1 and 2 days, at or at about 1 day, at or at about 2 days, or at or at about 3 days, after the threshold cell count, density, and/or expansion has been achieved during the cultivation. In certain disclosures, the cells active the threshold cell count, density, and/or expansion and remain cultivated for a minimum time or duration prior to the formulation. In some disclosures, cells that have achieved the threshold are not formulated until they have been cultivated for a minimum duration and/or amount of time, such as a minimum time or duration of between 1 day and 14 days, 2 days and 7 days, or 3 days and 6 days, or a minimum time or duration of the cultivation of or of about 2 days, 3 days, 4 days, 5 days, 6 days, 7 days, or more than 7 days. In some disclosures, the minimum time or duration of the cultivation is between 3 days and 6 days.

In some disclosures, the cells are formulated in a pharmaceutically acceptable buffer, which may, in some disclosures, include a pharmaceutically acceptable carrier or excipient. In some disclosures, the processing includes exchange of a medium into a medium or formulation buffer that is pharmaceutically acceptable or desired for administration to a subject. In some disclosures, the processing steps can involve washing the transduced and/or expanded cells to replace the cells in a pharmaceutically acceptable buffer that can include one or more optional pharmaceutically acceptable carriers or excipients. Exemplary of such pharmaceutical forms, including pharmaceutically acceptable carriers or excipients, can be any described below in conjunction with forms acceptable for administering the cells and compositions to a subject. The pharmaceutical composition in some disclosures contains the cells in amounts effective to treat or prevent the disease or condition, such as a therapeutically effective or prophylactically effective amount.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In some disclosures, the choice of carrier is determined in part by the particular cell and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some disclosures, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffering agents in some disclosures are included in the compositions. Suitable buffering agents include, for example, citric acid, sodium citrate, phosphoric acid, potassium phosphate, and various other acids and salts. In some disclosures, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulations can include aqueous solutions. The formulation or composition may also contain more than one active ingredient useful for the particular indication, disease, or condition being treated with the cells, preferably those with activities complementary to the cells, where the respective activities do not adversely affect one another. Such active ingredients are suitably present in combination in amounts that are effective for the purpose intended. Thus, in some disclosures, the pharmaceutical composition further includes other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, and/or vincristine.

Compositions in some disclosures are disclosed as sterile liquid preparations, e.g., isotonic aqueous solutions, suspensions, emulsions, dispersions, or viscous compositions, which may in some disclosures be buffered to a selected pH. Liquid compositions can comprise carriers, which can be a solvent or dispersing medium containing, for example, water, saline, phosphate buffered saline, polyol (for example, glycerol, propylene glycol, liquid polyethylene glycol) and suitable mixtures thereof. Sterile injectable solutions can be prepared by incorporating the cells in a solvent, such as in admixture with a suitable carrier, diluent, or excipient such as sterile water, physiological saline, glucose, dextrose, or the like. The compositions can contain auxiliary substances such as wetting, dispersing, or emulsifying agents (e.g., methylcellulose), pH buffering agents, gelling or viscosity enhancing additives, preservatives, flavoring agents, and/or colors, depending upon the route of administration and the preparation desired. Standard texts may in some disclosures be consulted to prepare suitable preparations.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, and sorbic acid. Prolonged absorption of the injectable pharmaceutical form can be brought about by the use of agents delaying absorption, for example, aluminum monostearate and gelatin.

In some disclosures, the formulation buffer contains a cryopreservative. In some disclosures, the cell are formulated with a cyropreservative solution that contains 1.0% to 30% DMSO solution, such as a 5% to 20% DMSO solution or a 5% to 10% DMSO solution. In some disclosures, the cryopreservation solution is or contains, for example, PBS containing 20% DMSO and 8% human serum albumin (HSA), or other suitable cell freezing media. In some disclosures, the cryopreservative solution is or contains, for example, at least or about 7.5% DMSO. In some disclosures, the processing steps can involve washing the transduced and/or expanded cells to replace the cells in a cryopreservative solution. In some disclosures, the cells are frozen, e.g., cryopreserved or cryoprotected, in media and/or solution with a final concentration of or of about 12.5%, 12.0%, 11.5%, 11.0%, 10.5%, 10.0%, 9.5%, 9. 0%, 8.5%, 8.0%, 7.5%, 7.0%, 6.5%, 6.0%, 5.5%, or 5.0% DMSO, or between 1% and 15%, between 6% and 12%, between 5% and 10%, or between 6% and 8% DMSO. In particular disclosures, the cells are frozen, e.g., cryopreserved or cryoprotected, in media and/or solution with a final concentration of or of about 5.0%, 4.5%, 4.0%, 3.5%, 3.0%, 2.5%, 2.0%, 1.5%, 1.25%, 1.0%, 0.75%, 0.5%, or 0.25% HSA, or between 0.1% and -5%, between 0.25% and 4%, between 0.5% and 2%, or between 1% and 2% HSA.

In particular disclosures, the composition of enriched T cells, e.g., T cells that have been stimulated, engineered, and/or cultivated, are formulated, cryopreserved, and then stored for an amount of time. In certain disclosures, the formulated, cryopreserved cells are stored until the cells are released for infusion. In particular disclosures, the formulated cryopreserved cells are stored for between 1 day and 6 months, between 1 month and 3 months, between 1 day and 14 days, between 1 day and 7 days, between 3 days and 6 days, between 6 months and 12 months, or longer than 12 months. In some disclosures, the cells are cryopreserved and stored for, for about, or for less than 1 days, 2 days, 3 days, 4 days, 5 days, 6 days, or 7 days. In certain disclosures, the cells are thawed and administered to a subject after the storage. In certain disclosures, the cells are stored for or for about 5 days.

In some disclosures, the formulation is carried out using one or more processing step including washing, diluting or concentrating the cells, such as the cultured or expanded cells. In some disclosures, the processing can include dilution or concentration of the cells to a desired concentration or number, such as unit dose form compositions including the number of cells for administration in a given dose or fraction thereof. In some disclosures, the processing steps can include a volume-reduction to thereby increase the concentration of cells as desired. In some disclosures, the processing steps can include a volume-addition to thereby decrease the concentration of cells as desired. In some disclosures, the processing includes adding a volume of a formulation buffer to transduced and/or expanded cells. In some disclosures, the volume of formulation buffer is from or from about 10 mL to 1000 mL, such as at least or about at least or about or 50 mL, 100 mL, 200 mL, 300 mL, 400 mL, 500 mL, 600 mL, 700 mL, 800 mL, 900 mL or 1000 mL.

In some disclosures, such processing steps for formulating a cell composition is carried out in a closed system. Exemplary of such processing steps can be performed using a centrifugal chamber in conjunction with one or more systems or kits associated with a cell processing system, such as a centrifugal chamber produced and sold by Biosafe SA, including those for use with the Sepax® or Sepax 2® cell processing systems. An exemplary system and process is described in International Publication Number WO2016/073602. In some disclosures, the method includes effecting expression from the internal cavity of the centrifugal chamber a formulated composition, which is the resulting composition of cells formulated in a formulation buffer, such as pharmaceutically acceptable buffer, in any of the above disclosures as described. In some disclosures, the expression of the formulated composition is to a container, such as a bag that is operably linked as part of a closed system with the centrifugal chamber. In some disclosures, the container, such as bag, is connected to a system at an output line or output position.

In some disclosures, the closed system, such as associated with a centrifugal chamber or cell processing system, includes a multi-port output kit containing a multi-way tubing manifold associated at each end of a tubing line with a port to which one or a plurality of containers can be connected for expression of the formulated composition. In some disclosures, a desired number or plurality of output containers, e.g., bags, can be sterilely connected to one or more, generally two or more, such as at least 3, 4, 5, 6, 7, 8 or more of the ports of the multi-port output. For example, in some disclosures, one or more containers, e.g., bags can be attached to the ports, or to fewer than all of the ports. Thus, in some disclosures, the system can effect expression of the output composition into a plurality of output bags.

In some disclosures, cells can be expressed to the one or more of the plurality of output bags in an amount for dosage administration, such as for a single unit dosage administration or multiple dosage administration. For example, in some disclosures, the output bags may each contain the number of cells for administration in a given dose or fraction thereof. Thus, each bag, in some disclosures, may contain a single unit dose for administration or may contain a fraction of a desired dose such that more than one of the plurality of output bags, such as two of the output bags, or 3 of the output bags, together constitute a dose for administration.

Thus, the containers, e.g., output bags, generally contain the cells to be administered, e.g., one or more unit doses thereof. The unit dose may be an amount or number of the cells to be administered to the subject or twice the number (or more) of the cells to be administered. It may be the lowest dose or lowest possible dose of the cells that would be administered to the subj ect.

In some disclosures, each of the containers, e.g., bags, individually comprises a unit dose of the cells. Thus in some disclosures, each of the containers comprises the same or approximately or substantially the same number of cells. In some disclosures, each unit dose contains at least or about at least $1 \times 10^6$, $2 \times 10^6$, $5 \times 10^6$, $1 \times 10^7$, $5 \times 10^7$, or $1 \times 10^8$ engineered cells, total cells, T cells, or PBMCs. In some disclosures, the volume of the formulated cell composition in each bag is 10 mL to 100 mL, such as at least or about at least 20 mL, 30 mL, 40 mL, 50 mL, 60 mL, 70 mL, 80 mL, 90 mL or 100 mL.

In some disclosures, such cells produced by the method, or a composition comprising such cells, are administered to a subject for treating a disease or condition.

**E. Sequential Selection and Parallel Selection**

The methods disclosed herein allow for multiple selection steps, for example by column chromatography, to isolate and/or enrich a target cell population (e.g., T cells, CD3+, CD4+, CD8+, CD57- T cells). In some disclosures, one or more selection steps are carried out at one or more time points or following certain steps of the process for creating an output therapeutic cell composition. In some disclosures, a selection step includes multiple selection steps for, for example, further purifying the cell composition, selection of specific cell subtypes, selection of viable cells, selection of engineered cells, and/or adjusting the ratio, total number, or concentration of cells. In some disclosures, a selection step is performed prior to incubation. In some disclosures, a selection step is performed prior to harvesting and collection.

In some disclosures, such methods (e.g., selection steps) are achieved by a single process stream, such as in a closed system, by employing sequential selections in which a plurality of different cell populations from a sample (e.g., output composition of stimulated and/or engineered cells), as disclosed herein, are enriched and/or isolated. In some disclosures, carrying out the separation or isolation in the same vessel or set of vessels, e.g., tubing set, is achieved by carrying out sequential positive and negative selection steps, the subsequent step subjecting the negative and/or positive fraction from the previous step to further selection, where the entire process is carried out in the same tube or tubing set. In one disclosure, a sample (e.g., output composition of stimulated and/or engineered cells) containing target cells is subjected to a sequential selection in which a first selection is effected to enrich for one of the CD4+ or CD8+ populations, and the non-selected cells from the first selection are used as the source of cells for a second selection to enrich for the other of the CD4+ or CD8+ populations. In some disclosures, a further selection or selections can be effected to enrich for sub-populations of one or both of the CD4+ or CD8+ population, for example, CD57- cells. In some disclosures, a cell population (e.g., output composition of stimulated and/or engineered cells) containing target cells is subjected to a sequential selection for viable cells. In some disclosures, the ratio or total number of cells in the cell population (e.g., output composition of stimulated and/or engineered cells) containing target cells is controlled or adjusted.

In some disclosures, such methods (e.g., selection steps) are achieved by a single process stream, such as in a closed system, by employing sequential selections in which a plurality of different cell populations from a sample (e.g., output composition of stimulated and/or engineered cells), as disclosed herein, are enriched and/or isolated. In some disclosures, carrying out the separation or isolation in the same vessel or set of vessels, e.g., tubing set, is achieved by carrying out sequential negative and postive selection steps, the subsequent step subjecting the negative and/or positive fraction from the previous step to further selection, where the entire process is carried out in the same tube or tubing set. In one disclosure, a sample (e.g., output composition of stimulated and/or engineered cells) containing target cells is subjected to a sequential selection in which a first selection is effected to remove CD57+ populations. In some disclosures, a further selection or selections can be effected to enrich for one or both of CD4+ or CD8+ population, for example, CD57-CD4+ or CD57-CD8+ cells. In some disclosures, a cell population (e.g., output composition of stimulated and/or engineered cells) containing target cells is subjected to a sequential selection for viable cells. In some disclosures, the ratio or total number of cells in the cell population (e.g., output composition of stimulated and/or engineered cells) containing target cells is controlled or adjusted.

In one disclosure, a sample (e.g., output composition of stimulated and/or engineered cells) containing target cells is subjected to a sequential selection in which a first selection is effected to enrich for a CD3+ population. In some disclosures, a further selection or selections can be effected to enrich for sub-populations of the CD3+ population, for example, CD57- cells. In some disclosures, the further selection or selections can be effected to enrich for viable cells. In some disclosures, the further selection or selections can be effected to enrich subpopulations of CD57-CD3+ cells, for example CD3+CD57-CD4+ or CD57-CD3+CD8+ cells that are viable. In some disclosures, selecting viable cells includes or consists of removing dead cells from the cell population (e.g., output composition of stimulated and/or engineered cells or subpopulations thereof).

In one disclosure, a sample (e.g., output composition of stimulated and/or engineered cells) containing target cells is subjected to a sequential selection in which a first selection is effected to remove CD57+ cells. In some disclosures, a further selection or selections can be effected to enrich for sub-populations of the CD57- population, for example, CD4+ and/or CD8+ cells. In some disclosures, the further selection or selections can be effected to enrich for viable cells. In some disclosures, selecting viable cells includes or consists of removing dead cells from the cell population (e.g., output composition of stimulated and/or engineered cells or subpopulations thereof).

In some disclosures, the methods (e.g., selection steps) disclosed in this Section do not need to be carried out using sequential selection techniques. In some disclosures, the methods (e.g., selection steps) disclosed in this Section can be carried out using sequential selection techniques in combination with parallel selection techniques. In some disclosures, the selection step does not employ sequential selection or may employ sequential selection that does not occur in a closed system or in a set of vessels using the same tubing. In some disclosures, the selection step is accomplished in a single step, for example using a single chromatography column. In some disclosures, the selection step is accomplished using a parallel selection technique. For example, the selection step is achieved by carrying out positive and/or negative selection steps simultaneously, for example in a closed system where the entire process is carried out in the same tube or tubing set. In some disclosures, a sample (e.g., output composition of stimulated and/or engineered cells) containing target cells is subjected to a parallel selection in which the sample (e.g., output composition of stimulated and/or engineered cells) is load onto two or more chromatapgraphy columns, where each column effects selection of a cell population. In some disclosures, the two or more chromatograpy columns effect selection of CD57-, CD3+, CD4+, or CD8+ populations individually. In some disclosures, the two or more chromatorgraphy columns effect selection of the same cell population. For example, the two or more chromatography columns may effect selection of CD57- cells. In some disclosures, the two or more chromatography columns, including affinity chromatography or gel permeation chromatography, independently effect selection of the same cell population. In some disclosures, the two or more chromatography columns, including affinity chromatography or gel permeation chromatography, independently effect selection of different cell populations. In some disclosures, a further selection or selections can be effected to enrich for subpopulations of one or all cell populations selected via parallel selection. In some disclosures, a sample (e.g., output composition of stimulated and/or engineered cells) containing target cells (e.g., CD57-cells) is subjected to a parallel selection in which parallel selection is effected to enrich for a CD4+ population and a CD8+ population or a CD3+ population.

In some disclosures, a selection step can be carried out using beads labeled with selection agents as described herein, and the positive and negative fractions from the first selection step can be retained, followed by further positive selection of the positive fraction to enrich for a second selection marker, such as by using beads labeled with a second selection agent or by subjecting the positive fraction to column chromatography as described above. In some disclosures, one or more selection steps are carried out using column chromatography as described herein. In some disclosures, selection steps are accomplished using one or more methods including bead separation and column chromatography. In some disclosures, the selection are accomplished using column chromatography.

In some disclosures, isolating the plurality of populations in a single or in the same isolation or separation vessel or set of vessels, such as a single column or set of columns, and/or same tube, or tubing set or using the same separation matrix or media or reagents, such as the same magnetic matrix, affinity-labeled solid support, or antibodies or other binding partners, include features that streamline the isolation, for example, resulting in reduced cost, time, complexity, need for handling of samples, use of resources, reagents, or equipment. In some disclosures, such features are advantageous in that they minimize cost, efficiency, time, and/or complexity associated with the methods, and/or avoid potential harm to the cell product, such as harm caused by infection, contamination, and/or changes in temperature. The methods disclosed herein allow for multiple selection steps to enrich target populations both prior to or following cell selection combined with on-column stimulation.

The methods disclosed herein further allow for the selection and enrichment of successfully stimulated and engineered cells. For example, in some disclosures, the sequential selection, parallel selection, or single selection procedures described above may be used to identify stimulated cells expressing recombinant receptors (e.g., CARs, TCRs). In some disclosures, cells expressing the recombinant receptor (e.g., CAR) can be further enriched for sub-population cells, e.g., CD4+ CAR+ T cells, CD8+ CAR+ T cells, and/or viable cells. In some disclosures, the selection step allows control or adjustment of the ratio, concentration, or total number of cells expressing a recombinant receptor (e.g., CAR, TCR) and/or subpopulations thereof. In some disclosures, enriched populations can be formulated for use (e.g., administration) for cell therapy.

## F. Exemplary Features of the Process

In some disclosures, the disclosed methods and compositions relate to populations of T cells enriched for CD57- T cells. In particular disclosures are drawn to methods of generating populations of enriched CD57- T cells, such as by negative selection of CD57+ cells and, in some disclosures, positive selection of T cells, such as CD4+ T cells and/or CD8+ T cells. In particular disclosures are drawn to methods of generating populations of enriched CD57- T cells, such as by negative selection of CD57+ cells and, in some disclosures, positive selection of T cells, such as CD3+ T cells. Certain disclosures are drawn to methods of incubating, stimulating, activating, engineering, transducing, cultivating, and/or expanding populations of enriched CD57- T cells. In certain disclosures, incubating, stimulating, activating, engineering, transducing, cultivating, and/or expanding populations of enriched CD57- T cells provide advantages over such steps or processes with alternative populations of T cells, such as populations containing amounts or high amounts of CD57+ T cells. Such advantages include, but are not limited to, improved proliferation or expansion and less differentiation, e.g., terminal differentiation.

In some disclosures, the disclosed methods are or include steps of enriching T cells, such as by selecting CD57+ T cells from a biological sample containing primary human T cells to generate a population of T cells depleted of CD57+ T cells, e.g., a population of enriched CD57- T cells. In some disclosures, the population contains less than or less than about 10%, 5%, 1%, or 0.1% CD57+ T cells. In particular disclosures, the population contains less than or less than about 25%, 20%, 15%, 10%, or 5% of the frequency of CD57+ T cells that were present the biological sample. In certain disclosures, at least 85%, 90%, 95%, or 99% of the CD4+ T cells of the population are CD57-CD4+ T cells.

In particular disclosures, at least 85%, 90%, 95%, or 99% of the CD8+ T cells of the population are CD57-CD8+ T cells. In particular disclosures, at least 85%, 90%, 95%, or 99% of the CD3+ T cells of the population are CD57-CD3+ T cells.

In certain disclosures, enriching T cells includes selecting or removing CD57+ cells from a biological sample, and then separately selecting for CD4+ T cells and CD8+ T cells from the population negatively selected for CD57, such as to generated a population of enriched CD57-CD4+ T cells and a population of enriched CD57-CD8+ T cells. In some disclosures, these populations remain separate, such as are subsequently separately cryoprotected and stored and/or are separately engineered to express a recombinant receptor. In particular disclosures, the separate populations are combined, such as at a ratio of 1:1 CD57-CD4+ T cells to CD57-CD8+ T cells.

In some disclosures, the disclosed methods are or include stimulating populations of enriched CD57- T cells. In certain disclosures, the disclosed methods include one or more steps for stimulating populations of enriched CD57-CD4+ T cells. In particular disclosures, the disclosed methods include one or more steps for stimulating populations of enriched CD57-CD8+ T cells. In certain disclosures, the populations of enriched CD57- CD4+ T cells and populations of enriched CD57- CD8+ T cells are stimulated such as by incubating the cells under stimulating conditions, e.g., any stimulating conditions described herein such as in Section III.A. In particular disclosures, the stimulating conditions are or include the presence of a stimulatory reagent. In certain disclosures, separate populations of enriched CD57- CD4+ T cells and enriched CD57- CD8+ T cells are separately stimulated. In particular disclosures, separate populations of enriched CD57- CD4+ T cells and enriched CD57- CD8+ T cells are combined or mixed prior to being stimulated, such that a combined composition of enriched CD57- CD4+ T cells and CD57- CD8+ T cells is stimulated.

In certain disclosures, a method or process for stimulating T cells includes selecting or removing CD57+ cells from a biological sample, and then separately selecting for CD4+ T cells and CD8+ T cells from the population negatively selected for CD57, such as to generated a population of enriched CD57-CD4+ T cells and a population of enriched CD57-CD8+ T cells. In certain disclosures, the separate populations of enriched CD57-CD4+ T cells and enriched CD57-CD8+ T cells are separately incubated under stimulating conditions. In particular disclosures, the separate populations of enriched CD57-CD4+ T cells and enriched CD57-CD8+ T cells are combined, such as a separately incubated under stimulating conditions. In some disclosures, the stimulating includes the presence of a stimulatory reagent. In certain disclosures, the stimulatory reagent is or includes an anti-CD3 and anti-CD28 antibody conjugated paramagnetic bead. In particular disclosures, the stimulatory reagent is or includes a streptavidin mutein oligomeric particle with reversibly bound anti-CD3 and anti-CD28 Fabs.

In particular disclosures, the disclosed methods are or include genetically engineering populations of enriched CD57- T cells, such as by any methods disclosed herein, e.g., in Section III.B. In some disclosures, a heterologous polynucleotide is introduced to the cells of the populations, e.g., by transduction. In certain disclosures, the disclosed methods include one or more steps for engineering populations of enriched CD57-CD4+ T cells or populations of cells that originate or are derived from such cells. In particular disclosures, the disclosed methods include one or more steps for genetically engineering populations of enriched CD57-CD8+ T cells. In certain disclosures, the populations of enriched CD57- CD4+ T cells and populations of enriched CD57- CD8+ T cells are genetically engineered, such as by transduction with a viral vector. In certain disclosures, separate populations of enriched CD57- CD4+ T cells and enriched CD57- CD8+ T cells, e.g., stimulated enriched CD57- CD4+ T cells and stimulated enriched CD57- CD8+ T cells are genetically engineered. In particular disclosures, a single population of enriched CD57- T cells, e.g., stimulated CD57- T cells, including both CD57-CD4+ T cells and CD57- CD8+ T cells, e.g., stimulated enriched CD57- CD4+ T cells and stimulated enriched CD57- CD8+ T cells are genetically engineered.

In particular disclosures, a method for generically engineering T cells is or includes steps selecting or removing CD57+ cells from a biological sample, and then separately selecting for CD4+ T cells and CD8+ T cells from the population negatively selected for CD57, such as to generated a population of enriched CD57-CD4+ T cells and a population of enriched CD57-CD8+ T cells. In particular disclosures, the separate populations of enriched CD57-CD4+ T cells and enriched CD57-CD8+ T cells are separately genetically engineered. In particular disclosures, the separate populations of enriched CD57-CD4+ T cells and enriched CD57-CD8+ T cells are combined prior to steps for genetically engineering the cells. In some disclosures the genetic engineering is or includes introducing a heterologous polynucleotide, e.g., encoding a recombinant receptor. In some disclosures, the introducing is or includes steps for transduction, such as with spinoculation and optionally subsequent incubation in the presence of the viral vector. In some disclosures, the heterologous polynucleotide is introduced by any methods disclosed herein, e.g., in Section III.B. In particular disclosures, the CD57-CD4+ T cells and CD57-CD8+ T cells are stimulated, e.g., by any method disclosed herein, e.g., in Section III.A, prior to being genetically engineered.

In some disclosures, the genetic engineering is performed by (a) incubating populations of enriched CD57- T cells in the presence of a stimulatory reagent capable of activating one or more intracellular signaling domains of one or more components of a TCR complex and one or more intracellular signaling domains of one or more costimulatory molecules thereby generating stimulated T cells; (b) introducing a heterologous polynucleotide into the stimulated T cells of the first and second enriched populations by transducing the stimulated T cells with a viral vector continaing a heterologous polynucleotide to generate transformed T cells; (c) cultivating the transformed T cells under conditions to promote proliferation or expansion of the transformed T cells; and (d) harvesting or collecting the expanded T cells.

In some disclosures, a method for generically engineering T cells is or includes steps selecting or removing CD57+ cells from a biological sample, and then separately selecting for CD4+ T cells and CD8+ T cells from the population negatively selected for CD57, such as to generated a population of enriched CD57-CD4+ T cells and a population of enriched CD57-CD8+ T cells. In particular disclosures, the separate populations of enriched CD57-CD4+ T cells and enriched CD57-CD8+ T cells are separately genetically engineered or are combined prior to one or more steps for genetic engineering. In certain disclosures, the CD57- CD4+ T cells and CD57-CD8+ T cells are incubated in the presence of a stimulatory reagent, e.g., an anti-CD3 and anti-CD28 antibody conjugated paramagnetic bead. In particular disclosures, the stimulatory reagent is or includes a streptavidin mutein oligomeric particle with reversibly bound anti-CD3 and anti-CD28 Fabs, to stimulate the T cells prior steps for introducing a heterologous polynucleotide encoding a recombinant receptor. In some disclosures, the stimulated CD57- CD4+ T cells and CD57-CD8+ T cells are transduced with a virus carrying the heterologous polynucleotide, such as by steps including spinoculation and/or incubation in the presence of the virus, such as to generate transformed CD57- CD4+ T cells and CD57-CD8+ T cells.

In some disclosures, a method for generically engineering T cells is or includes steps selecting or removing CD57+ cells from a biological sample, and then separately selecting for CD3+ T cells from the population negatively selected for CD57, such as to generated a population of enriched CD57-CD3+ T cells. In certain disclosures, the CD57-CD3+ T cells are incubated in the presence of a stimulatory reagent, e.g., an anti-CD3 and anti-CD28 antibody conjugated paramagnetic bead. In particular disclosures, the stimulatory reagent is or includes a streptavidin mutein oligomeric particle with reversibly bound anti-CD3 and anti-CD28 Fabs, to stimulate the T cells prior steps for introducing a heterologous polynucleotide encoding a recombinant receptor. In some disclosures, the stimulated CD57-CD3+ T cells are transduced with a virus carrying the heterologous polynucleotide, such as by steps including spinoculation and/or incubation in the presence of the virus, such as to generate transformed CD57-CD3+ T cells.

In some disclosures, the transformed T cells are cultivated under conditions to promote proliferation or expansion of the transformed T cells, e.g., in the presence of cytokines such as IL-2, IL-7, or IL-15. In some disclosures, the cells are cultivated until the cells achieve an expansion of at least 3-fold, 4-fold, or 5-fold.

In certain disclosures, the expanded cells are collected or harvested, such as to be formulated for cryoprotection and storage, or for administration to a subject as a cell therapy. In certain disclosures, the transformed cells are collected or harvested.

In certain disclosures, the disclosed methods are used in connection with successfully generating or producing output compositions of engineered T cells that are suitable for use in cell therapy. In some disclosures, an output composition is successfully generated if the cells of the composition achieve the threshold cell count, density, and/or expansion during cultivation. In particular disclosures, the disclosed methods have an at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% probability or likelihood of successfully generating or producing an population of T cells suitable for a cell therapy, e.g., from an initial population of enriched T cells or from a biological sample.

In certain disclosures, the population of enriched T cells generated from the disclosed methods for use in a cell therapy are active and expand, and/or are capable of activation and expansion, in vivo, when administered to a subject. In particular disclosures, the cells display features and/or characteristics that indicate or are associated with *in vivo* efficacy, activity, and/or expansion. For example, in some disclosures, such features or characters may include the expression of a protein, such as a surface protein, that is associated with activation, proliferation, and/or expansion after administration to a subject in vivo.

In certain disclosures, the populations of enriched T cells that are generated or produced by the disclosed methods, e.g., for use in a cell therapy, have greater expression of CD25 than the cells that are generated or produced by the alternative and/or exemplary process (e.g., a process of stimulating, engineering and/or cultivating populations of T cells containing higher frequencies of CD57+ T cells). In some disclosures, the T cells of a population that is generated or produced by the disclosed methods have a greater expression of CD25 than T cells that are generated or produced by an alternative and/or exemplary process. In some disclosures, at least 20%, at least 30%, at least 40%, at least 50%, at least 60%, at least 70%, at least 75%, at least 80%, at least 85%, at least 90%, at least 100%, of the T cells of a population generated by the disclosed process are positive for CD25 staining, e.g., express a detectable amount of CD25. In particular disclosures, the output composition contains a greater frequency of cells that are positive for CD25 than a composition of cells produced or generated by the alternative and/or exemplary process. In some disclosures, the cells of the population express at least 20%, at least 30%, at least 40%, at least 50%, at least 60%, at least 70%, at least 75%, at least 80%, at least 85%, at least 90%, at least 100%, at least 150%, at least 1-fold, at least 2-fold, at least 3-fold, at least 4-fold, or at least 5-fold, more CD25, e.g., as compared to cells produced or generated by the alternative and/or exemplary process.

In certain disclosures, the populations of enriched T cells that are generated or produced by the disclosed methods, e.g., for use in a cell therapy, have greater expression of CD27 than the cells that are generated or produced by the alternative and/or exemplary process (e.g., a process of stimulating, engineering and/or cultivating populations of T cells containing higher frequencies CD57+ T cells). In particular disclosures, the T cells of a population that is generated or produced by the disclosed methods have a greater expression of CD27 than T cells that are generated or produced by an alternative and/or exemplary process. In certain disclosures, at least 20%, at least 30%, at least 40%, at least 50%, at least 60%, at least 70%, at least 75%, at least 80%, at least 85%, at least 90%,at least 100%, of the T cells of a population generated by the disclosed process are positive for CD27 staining, e.g., express a detectable amount of CD27. In particular disclosures, the output composition contains a greater frequency of cells that are positive for CD27 than a composition of cells produced or generated by the alternative and/or exemplary process. In some disclosures, the cells of the population express at least 20%, at least 30%, at least 40%, at least 50%, at least 60%, at least 70%, at least 75%, at least 80%, at least 85%, at least 90%,at least 100%, at least 150%, at least 1-fold, at least 2-fold, at least 3-fold, at least 4-fold, or at least 5-fold, more CD25, e.g., as compared to cells produced or generated by the alternative and/or exemplary process.

In particular disclosures, the populations of enriched T cells that are generated or produced by the disclosed methods, e.g., for use in a cell therapy, have greater expression of CD28 than the cells that are generated or produced by the alternative and/or exemplary process (e.g., a process of stimulating, engineering and/or cultivating populations of T cells containing higher frequencies of CD57+ T cells). In some disclosures, the T cells of a population that is generated or produced by the disclosed methods have a greater expression of CD28 than T cells that are generated or produced by an alternative and/or exemplary process. In certain disclosures, at least 20%, at least 30%, at least 40%, at least 50%, at least 60%, at least 70%, at least 75%, at least 80%, at least 85%, at least 90%,at least 100%, of the T cells of a population generated by the disclosed process are positive for CD28 staining, e.g., express a detectable amount of CD28. In particular disclosures, the output composition contains a greater frequency of cells that are positive for CD28 than a composition of cells produced or generated by the alternative and/or exemplary process. In some disclosures, the cells of the population express at least 20%, at least 30%, at least 40%, at least 50%, at least 60%, at least 70%, at least 75%, at least 80%, at least 85%, at least 90%,at least 100%, at least 150%, at least 1-fold, at least 2-fold, at least 3-fold, at least 4-fold, or at least 5-fold, more CD28, e.g., as compared to cells produced or generated by the alternative and/or exemplary process.

In certain disclosures, the disclosed methods are used in connection with successfully generating or producing compositions of engineered T cells that are suitable for use in cell therapy. In some disclosures, a composition is successfully generated if the cells of the composition achieve a target cell count, density, and/or expansion during cultivation.

In particular disclosures, the disclosed methods have an at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% probability or likelihood of successfully generating or producing a population of enriched T cells suitable for a cell therapy. In certain disclosures, the probability or likelihood is between 85% and 100%, between 90% and 95%, or between 92% and 94%. In certain disclosures, the disclosed methods successfully generate or produce an a population of enriched T cells suitable for a cell therapy from at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% of the samples or populations of enriched CD57- T cells.

In certain disclosures, the total duration of the disclosed process for generating engineered cells, from the initiation of the stimulation to collecting, harvesting, or formulating the cells is, is about, or is less than 36 hours, 42 hours, 48 hours, 54 hours, 60 hours, 72 hours, 84 hours, 96 hours, 108 hours, or 120 hours. In certain disclosures, the total duration of the disclosed process for generating engineered cells, from the initiation of the stimulation to collecting, harvesting, or formulating the cells is, is about, or is less than 1.5 days, 2 days, 3 days, 4 days, or 5 days. In some disclosures, the total duration of the disclosed process for generating engineered cells, from the initiation of the stimulation to collecting, harvesting, or formulating the cells is between or between about 36 hours and 120 hours, 48 hours and 96 hours, or 48 hours and 72 hours, inclusive, or between or between about 1.5 days and 5 days, 2 days and 4 days, or 2 days and 3 days, inclusive. In particular disclosures, the amount of time to complete the disclosed process as measured from the initiation of incubation to harvesting, collecting, or formulating the cells is, is about, or is less than 48 hours, 72 hours, or 96 hours, or is, is about, or is less than 2 days, 3 days, or 4 days . In particular disclosures, the amount of time to complete the disclosed process as measured from the initiation of incubation to harvesting, collecting, or formulating the cells is 48 hours ± 6 hours, 72 hours ± 6 hours, or 96 hours ± 6 hours.

In some disclosures, the incubation, is completed between or between about 24 hour and 120 hours, 36 hour and 108 hours, 48 hours and 96 hours, or 48 hours and 72 hours, inclusive, after the initiation of the stimulation. In some disclosures, the incubation is completed at, about, or within 120 hours, 108 hours, 96 hours, 72 hours, 48 hours, or 36 hours from the initiation of the stimulation. In particular disclosures, the incubation are completed after 24 hours ± 6 hours, 48 hours ± 6 hours, or 72 hours ± 6 hours. In some disclosures, the incubation is completed between or between about one day and 5 days, 1.5 days and 4.5 days, 2 days and 4 days, or 2 day and 3 days, inclusive, after the initiation of the stimulation. In some disclosures, the incubation is completed at, about, or within 5 days, 4 days, 3 days, 2 days, or 1.5 days from the initiation of the stimulation.

In some disclosures, the entire process is performed with a single population of enriched T cells, e.g., CD4+ and CD8+ T cells or CD3+ cells. In certain disclosures, the process is performed with two or more input populations of enriched T cells (e.g., CD4 and CD8 cells) that are combined prior to and/or during the process to generate or produce a single output population of enriched T cells. In some disclosures, the enriched T cells are or include engineered T cells, e.g., T cells transduced to express a recombinant receptor.

In some disclosures, an output population, e.g., a population of engineered T cells, is generated by (i) incubating an input population of or containing T cells under stimulating conditions for between or between about 18 and 30 hours, inclusive, (ii) introducing a heterologous or recombinant polynucleotide encoding a recombinant receptor into T cells of the stimulated population, (iii) incubating the cells, and then (iv) collecting or harvesting the incubated cells.

In some disclosures, the cells are collected or harvested within between 36 and 108 hours or between 1.5 days and 4.5 days after the incubation under stimulatory conditions is initiated. In particular disclosures, the cells are collected or harvested within 48 hours or two days after the transformed (e.g., genetically engineered, transduced, or transfected) T cells achieve a stable integrated vector copy number (iVCN) per genome that does not increase or decrease by more than 20% within a span of 24-48 hours or one to two days. In some disclosures, the integration is considered stable when the measured iVCN of a cell population is within or within about 20%, 15%, 10%, or 5% of the total vector copy number (VCN) measured in the population. Particular disclosures contemplate that to achieve a stable integration, the cells must be incubated for, for about, or for at least 48 hours, 60 hours, or 72 hours, or one day, 2 days, or 3 days, after the viral vector is contacted or introduced to the cells. In some disclosures, the stable integration occurs within or with about 72 hours of the incubation. In some disclosures, the cells are collected or harvested at a time when the total number of transformed T cells is at or less than the total number of cells of the input population. In various disclosures, the cells are collected or harvested at a time before the cells of the input population have doubled more than three, two, or one time(s).

In certain disclosures, an output population, e.g., a population of engineered T cells, is generated by (i) incubating an input population comprising T cells under stimulating conditions for between 18 and 30 hours, inclusive, in the presence of a stimulatory reagent, e.g., a stimulatory reagent described herein, (ii) transducing the stimulated T cells with a viral vector encoding a recombinant receptor, such as by spinoculating the stimulated T cells in the presence of the viral vector, (iii) incubating the transduced T cells under static conditions for between or between 18 hours and 96 hours, inclusive, and (iv) harvesting T cells of the transformed population within between or between about 36 and 108 hours after the incubation under stimulatory conditions is initiated.

In some disclosures, the process associated with the disclosed methods is compared to an alternative process. For example, in some disclosures, the disclosed methods herein are compared an alternative process that contains a step for expanding the cells. In particular disclosures, the alternative process may differ in one or more specific aspects, but otherwise contains similar or the same features, aspects, steps, stages, reagents, and/or conditions of the process associated with the disclosed methods. In some disclosures, the alternative process is similar as the process associated with the disclosed methods, e.g., lacks or does not include expansion, but differs in a manner that includes, but is not limited to, one or more of; different reagents and/or media formulations; presence of serum during the incubation, transduction, transfection, and/or cultivation; different cellular makeup of the input population, e.g., ratio of CD4+ to CD8+ T cells; different stimulating conditions and/or a different stimulatory reagent; different ratio of stimulatory reagent to cells; different vector and/or method of transduction; different timing or order for incubating, transducing, and/or transfecting the cells; absence or difference of one or more recombinant cytokines present during the incubation or transduction (e.g., different cytokines or different concentrations), or different timing for harvesting or collecting the cells.

In some disclosures, the duration or amount of time required to complete the disclosed process, as measured from the isolation, enrichment, and/or selection input cells (e.g., CD4+ or CD8+ T cells) from a biological sample to the time at which a the output cells are collected, formulated, and/or cryoprotected is, is about, or is less than 48 hours, 72 hours, 96 hours, 120 hours, 2 days, 3 days, 4 days, 5 days, 7 days, or 10 days. In some disclosures, isolated, selected, or enriched cells are not cryoprotected prior to the stimulation, and the duration or amount of time required to complete the disclosed process, as measured from the isolation, enrichment, and/or selection input cells (to the time at which a the output cells are collected, formulated, and/or cryoprotected is, is about, or is less than 48 hours, 72 hours, 96 hours, or 120 hours, or 2 days, 3 days, 4 days, or 5 days.

In certain disclosures, the disclosed processes are performed on a population of cells, e.g., CD4+ and CD8+ T cells or CD3+ cells, that were isolated, enriched, or selected from a biological sample. In some disclosures, the disclosed methods can produce or generate a composition of engineered T cells from when a biological sample is collected from a subject within a shortened amount of time as compared to other methods or processes. In some disclosures, the disclosed methods can produce or generate engineered T cells, including any or all times where biological samples, or enriched, isolated, or selected cells are cryopreserved and stored prior to steps for stimulation or transduction, within or within about 10 days, 9 days, 8 days, 7 days, 6 days, 5 days, 4 days, 3 days, 2 days, or within or within about 120 hours, 96 hours, 72 hours, or 48 hours, from when a biological sample is collected from a subject to when the engineered T cells are collected, harvested, or formulated (e.g., for cryopreservation or administration). In particular disclosures, the disclosed methods can produce or generate engineered T cells, including any or all times where biological samples, or enriched, isolated, or selected cells are cryopreserved and stored prior to steps for stimulation or transduction, within between or between about 6 days and 8 days, inclusive, from when the biological sample is collected from a subject to when the engineered T cells are collected, harvested, or formulated.

## IV. HETEROLOGOUS POLYNUCLEOTIDES ENCODING RECOMBINANT PROTEINS

In some disclosures, the disclosed methods are or include introducing a heterologous polynucleotide into cells of a population enriched for CD57- cells. In some disclosures, the heterologous polynucleotide encodes a recombinant protein. Such recombinant proteins may include recombinant receptors, such as any described in Section III.A. Introduction of the polynucleotides, e.g., heterologous or recombinant polynucleotides, encoding the recombinant protein into the cell may be carried out using any of a number of known vectors. Such vectors include viral, including lentiviral and gammaretroviral, systems. Exemplary methods include those for transfer of heterologous polynucleotides encoding the receptors, including via viral, e.g., retroviral or lentiviral, transduction. In some disclosures, a population of stimulated cells is genetically engineered, such as to introduce a heterologous or recombinant polynucleotide encoding a recombinant receptor, thereby generating a population of transformed cells (also referred to herein as a transformed population of cells).

In certain disclosures, a polynucleotide encoding the recombinant protein, e.g. a recombinant receptor, is introduced to the cells. In certain disclosures, the polynucleotide or nucleic acid molecule is heterologous to the cells. In particular disclosures, the heterologous polynucleotide is not native to the cells. In certain disclosures, the heterologous nucleic acid molecule or heterologous polynucleotide encodes a protein, e.g., a recombinant protein that is not natively expressed by the cell. In particular disclosures, the heterologous nucleic acid molecule or polynucleotide is or contains a nucleic acid sequence that is not found in the cell prior to the contact or introduction.

In particular disclosures, the heterologous polynucleotide encodes a recombinant protein. In certain disclosures, the recombinant protein is a recombinant receptor. In some disclosures, the recombinant protein is a recombinant antigen receptor, such as a recombinant TCR or a chimeric antigen receptor (CAR).

## A. Recombinant Receptors

In some disclosures, disclosed are engineered cells, such as CD57- T cells, that express or are engineered to express one or more recombinant receptor(s). Among the receptors are antigen receptors and receptors containing one or more components thereof. The recombinant receptors may include chimeric receptors, such as those containing ligand-binding domains or binding fragments thereof and intracellular signaling domains or regions, functional non-TCR antigen receptors, chimeric antigen receptors (CARs), T cell receptors (TCRs), such as recombinant or transgenic TCRs, chimeric autoantibody receptor (CAAR) and components of any of the foregoing. The recombinant receptor, such as a CAR, generally includes the extracellular antigen (or ligand) binding domain linked to one or more intracellular signaling components, in some disclosures via linkers and/or transmembrane domain(s). In some disclosures, the engineered cells express two or more receptors that contain different components, domains or regions. In some disclosures, two or more receptors allows spatial or temporal regulation or control of specificity, activity, antigen (or ligand) binding, function and/or expression of the recombinant receptors.

## 1. Chimeric Antigen Receptors (CARs)

In some disclosures of the disclosed methods and uses, chimeric receptors, such as a chimeric antigen receptors, contain one or more domains that combine a ligand-binding domain (e.g. antibody or antibody fragment) that provides specificity for a desired antigen (e.g., tumor antigen) with intracellular signaling domains. In some disclosures, the intracellular signaling domain is a stimulating or an activating intracellular domain portion, such as a T cell stimulating or activating domain, providing a primary activation signal or a primary signal. In some disclosures, the intracellular signaling domain contains or additionally contains a costimulatory signaling domain to facilitate effector functions. In some disclosures, chimeric receptors when genetically engineered into immune cells can modulate T cell activity, and, in some cases disclosed herein, can modulate T cell differentiation or homeostasis, thereby resulting in genetically engineered cells with improved longevity, survival and/or persistence *in vivo,* such as for use in adoptive cell therapy methods.

Exemplary antigen receptors, including CARs, and methods for engineering and introducing such receptors into cells, include those described, for example, in international patent application publication numbers WO200014257, WO2013126726, WO2012/129514, WO2014031687, WO2013/166321, WO2013/071154, WO2013/123061 U.S. patent application publication numbers US2002131960, US2013287748, US20130149337, U.S. Patent Nos.: 6,451,995, 7,446,190, 8,252,592, 8,339,645, 8,398,282, 7,446,179, 6,410,319, 7,070,995, 7,265,209, 7,354,762, 7,446,191, 8,324,353, and 8,479,118, and European patent application number EP2537416,and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75. In some disclosures, the antigen receptors include a CAR as described in U.S. Patent No.: 7,446,190, and those described in International Patent Application Publication No.: WO/2014055668 A1. Examples of the CARs include CARs as disclosed in any of the aforementioned publications, such as WO2014031687, US 8,339,645, US 7,446,179, US 2013/0149337, U.S. Patent No.: 7,446,190, US Patent No.: 8,389,282, Kochenderfer et al., 2013, Nature Reviews Clinical Oncology, 10, 267-276 (2013); Wang et al. (2012) J. Immunother. 35(9): 689-701; and Brentjens et al., Sci Transl Med. 2013 5(177). See also WO2014031687, US 8,339,645, US 7,446,179, US 2013/0149337, U.S. Patent No.: 7,446,190, and US Patent No.: 8,389,282.

The chimeric receptors, such as CARs, generally include an extracellular antigen binding domain, such as a portion of an antibody molecule, generally a variable heavy ($V_H$) chain region and/or variable light ($V_L$) chain region of the antibody, e.g., an scFv antibody fragment.

In some disclosures, the antigen targeted by the receptor is a polypeptide. In some disclosures, it is a carbohydrate or other molecule. In some disclosures, the antigen is selectively expressed or overexpressed on cells of the disease or condition, e.g., the tumor or pathogenic cells, as compared to normal or non-targeted cells or tissues. In other disclosures, the antigen is expressed on normal cells and/or is expressed on the engineered cells.

In some disclosures, the antigen targeted by the receptor is or includes $\alpha v\beta 6$ integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22R$\alpha$), IL-13 receptor alpha 2 (IL-13R$\alpha$2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some disclosures include antigens associated with a B cell malignancy, such as any of a number of known B cell marker. In some disclosures, the antigen is or includes CD20, CD19, CD22, ROR1, CD45, CD21, CD5, CD33, Igkappa, Iglambda, CD79a, CD79b or CD30.

In some disclosures, the antigen is or includes a pathogen-specific or pathogen-expressed antigen. In some disclosures, the antigen is a viral antigen (such as a viral antigen from HIV, HCV, HBV, etc.), bacterial antigens, and/or parasitic antigens.

In some disclosures, the antigen or antigen binding domain is CD19. In some disclosures, the scFv contains a VH and a VL derived from an antibody or an antibody fragment specific to CD19. In some disclosures, the antibody or antibody fragment that binds CD19 is a mouse derived antibody such as FMC63 and SJ25C1. In some disclosures, the antibody or antibody fragment is a human antibody, e.g., as described in U.S. Patent Publication No. US 2016/0152723.

In some disclosures, the scFv is derived from FMC63. FMC63 generally refers to a mouse monoclonal IgG1 antibody raised against Nalm-1 and -16 cells expressing CD19 of human origin (Ling, N. R., et al. (1987). Leucocyte typing III. 302). In some disclosures, the FMC63 antibody comprises CDRH1 and H2 set forth in SEQ ID NOS: 38 and 39, respectively, and CDRH3 set forth in SEQ ID NO: 40 or 54 and CDRL1 set forth in SEQ ID NO: 35 and CDR L2 set forth in SEQ ID NO: 36 or 55 and CDR L3 set forth in SEQ ID NO: 37 or 56. In some disclosures, the FMC63 antibody comprises the heavy chain variable region (V$_H$) comprising the amino acid sequence of SEQ ID NO: 41 and the light chain variable region (V$_L$) comprising the amino acid sequence of SEQ ID NO: 42.

In some disclosures, the scFv comprises a variable light chain containing the CDRL1 sequence of SEQ ID NO:35, a CDRL2 sequence of SEQ ID NO:36, and a CDRL3 sequence of SEQ ID NO:37 and/or a variable heavy chain containing a CDRH1 sequence of SEQ ID NO:38, a CDRH2 sequence of SEQ ID NO:39, and a CDRH3 sequence of SEQ ID NO:40. In some disclosures, the scFv comprises a variable heavy chain region set forth in SEQ ID NO:41 and a variable light chain region set forth in SEQ ID NO:42. In some disclosures, the variable heavy and variable light chains are connected by a linker. In some disclosures, the linker is set forth in SEQ ID NO:58. In some disclosures, the scFv comprises, in order, a V$_H$, a linker, and a V$_L$. In some disclosures, the scFv comprises, in order, a V$_L$, a linker, and a V$_H$. In some disclosures, the scFv is encoded by a sequence of nucleotides set forth in SEQ ID NO:43 or a sequence that exhibits at least 85%, 86%, 87%, 88%, 89%, 90%, 91%, 92%, 93%, 94%, 95%, 96%, 97%, 98%, or 99% sequence identity to SEQ ID NO:43. In some disclosures, the scFv comprises the sequence of amino acids set forth in SEQ ID NO:43 or a sequence that exhibits at least 85%, 86%, 87%, 88%, 89%, 90%, 91%, 92%, 93%, 94%, 95%, 96%, 97%, 98%, or 99% sequence identity to SEQ ID NO:43.

In some disclosures the scFv is derived from SJ25C1. SJ25C1 is a mouse monoclonal IgG1 antibody raised against Nalm-1 and -16 cells expressing CD19 of human origin (Ling, N. R., et al. (1987). Leucocyte typing III. 302). In some disclosures, the SJ25C1 antibody comprises CDRH1, H2 and H3 set forth in SEQ ID NOS: 47-49, respectively, and CDRL1, L2 and L3 sequences set forth in SEQ ID NOS: 44-46, respectively. In some disclosures, the SJ25C1 antibody comprises the heavy chain variable region (V$_H$) comprising the amino acid sequence of SEQ ID NO: 50 and the light chain variable region (V$_L$) comprising the amino acid sequence of SEQ ID NO: 51.

In some disclosures, the scFv comprises a variable light chain containing the CDRL1 sequence of SEQ ID NO:44, a CDRL2 sequence of SEQ ID NO: 45, and a CDRL3 sequence of SEQ ID NO:46 and/or a variable heavy chain containing a CDRH1 sequence of SEQ ID NO:47, a CDRH2 sequence of SEQ ID NO:48, and a CDRH3 sequence of SEQ ID NO:49. In some disclosures, the scFv comprises a variable heavy chain region set forth in SEQ ID NO:50 and a variable light chain region set forth in SEQ ID NO:51. In some disclosures, the variable heavy and variable light chain are connected by a linker. In some disclosures, the linker is set forth in SEQ ID NO:52. In some disclosures, the scFv comprises, in order, a V$_H$, a linker, and a V$_L$. In some disclosures, the scFv comprises, in order, a V$_L$, a linker, and a V$_H$. In some disclosures, the scFv comprises the sequence of amino acids set forth in SEQ ID NO:53 or a sequence that exhibits at least 85%, 86%, 87%, 88%, 89%, 90%, 91%, 92%, 93%, 94%, 95%, 96%, 97%, 98%, or 99% sequence identity to SEQ ID NO:53.

In some disclosures, the chimeric antigen receptor includes an extracellular portion containing an antibody or antibody fragment. In some disclosures, the chimeric antigen receptor includes an extracellular portion containing the antibody or fragment and an intracellular signaling domain. In some disclosures, the antibody or fragment includes an scFv.

In some disclosures, the antibody portion of the recombinant receptor, e.g., CAR, further includes at least a portion of an immunoglobulin constant region, such as a hinge region, e.g., an IgG4 hinge region, and/or a C$_H$1/C$_L$ and/or Fc region. In some disclosures, the constant region or portion is of a human IgG, such as IgG4 or IgG1. In some disclosures, the portion of the constant region serves as a spacer region between the antigen-recognition component, e.g., scFv, and transmembrane domain. The spacer can be of a length that provides for increased responsiveness of the cell following antigen binding, as compared to in the absence of the spacer. Exemplary spacers include, but are not limited to, those described in Hudecek et al. (2013) Clin. Cancer Res., 19:3153, international patent application publication number WO2014031687, U.S. Patent No. 8,822,647 or published app. No. US2014/0271635.

In some disclosures, the constant region or portion is of a human IgG, such as IgG4 or IgG1. In some disclosures, the spacer has the sequence ESKYGPPCPPCP (set forth in SEQ ID NO: 1), and is encoded by the sequence set forth in SEQ ID NO: 2. In some disclosures, the spacer has the sequence set forth in SEQ ID NO: 3. In some disclosures, the spacer has the sequence set forth in SEQ ID NO: 4. In some disclosures, the constant region or portion is of IgD. In some disclosures, the spacer has the sequence set forth in SEQ ID NO: 5. In some disclosures, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 1, 3, 4 or 5. In some disclosures, the spacer has the sequence set forth in SEQ ID NOS: 27-34. In some disclosures, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 27-34.

In some disclosures, the antigen receptor comprises an intracellular domain linked directly or indirectly to the extracellular domain. In some disclosures, the chimeric antigen receptor includes a transmembrane domain linking the extracellular domain and the intracellular signaling domain. In some disclosures, the intracellular signaling domain comprises an ITAM. For example, in some disclosures, the antigen recognition domain (e.g. extracellular domain) generally is linked to one or more intracellular signaling components, such as signaling components that mimic activation through an antigen receptor complex, such as a TCR complex, in the case of a CAR, and/or signal via another cell surface receptor. In some disclosures, the chimeric receptor comprises a transmembrane domain linked or fused between the extracellular domain (e.g. scFv) and intracellular signaling domain. Thus, in some disclosures, the antigen-binding component (e.g., antibody) is linked to one or more transmembrane and intracellular signaling domains.

In one disclosure, a transmembrane domain that naturally is associated with one of the domains in the receptor, e.g., CAR, is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some disclosures is derived either from a natural or from a synthetic source. Where the source is natural, the domain in some disclosures is derived from any membrane-bound or transmembrane protein. Transmembrane regions include those derived from (i.e. comprise at least the transmembrane region(s) of) the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154. Alternatively the transmembrane domain in some disclosures is synthetic. In some disclosures, the synthetic transmembrane domain comprises predominantly hydrophobic residues such as leucine and valine. In some disclosures, a triplet of phenylalanine, tryptophan and valine will be found at each end of a synthetic transmembrane domain. In some disclosures, the linkage is by linkers, spacers, and/or transmembrane domain(s). In some disclosures, the transmembrane domain contains a transmembrane portion of CD28.

In some disclosures, the extracellular domain and transmembrane domain can be linked directly or indirectly. In some disclosures, the extracellular domain and transmembrane are linked by a spacer, such as any described herein. In some disclosures, the receptor contains extracellular portion of the molecule from which the transmembrane domain is derived, such as a CD28 extracellular portion.

Among the intracellular signaling domains are those that mimic or approximate a signal through a natural antigen receptor, a signal through such a receptor in combination with a costimulatory receptor, and/or a signal through a costimulatory receptor alone. In some disclosures, a short oligo- or polypeptide linker, for example, a linker of between 2 and 10 amino acids in length, such as one containing glycines and serines, e.g., glycine-serine doublet, is present and forms a linkage between the transmembrane domain and the cytoplasmic signaling domain of the CAR.

T cell activation is in some disclosures described as being mediated by two classes of cytoplasmic signaling sequences: those that initiate antigen-dependent primary activation through the TCR (primary cytoplasmic signaling sequences), and those that act in an antigen-independent manner to provide a secondary or co-stimulatory signal (secondary cytoplasmic signaling sequences). In some disclosures, the CAR includes one or both of such signaling components.

The receptor, e.g., the CAR, generally includes at least one intracellular signaling component or components. In some disclosures, the CAR includes a primary cytoplasmic signaling sequence that regulates primary activation of the TCR complex. Primary cytoplasmic signaling sequences that act in a stimulatory manner may contain signaling motifs which are known as immunoreceptor tyrosine-based activation motifs or ITAMs. Examples of ITAM containing primary cytoplasmic signaling sequences include those derived from CD3 zeta chain, FcR gamma, CD3 gamma, CD3 delta and CD3 epsilon. In some disclosures, cytoplasmic signaling molecule(s) in the CAR contain(s) a cytoplasmic signaling domain, portion thereof, or sequence derived from CD3 zeta.

In some disclosures, the receptor includes an intracellular component of a TCR complex, such as a TCR CD3 chain that mediates T-cell activation and cytotoxicity, e.g., CD3 zeta chain. Thus, in some disclosures, the antigen-binding portion is linked to one or more cell signaling modules. In some disclosures, cell signaling modules include CD3 transmembrane domain, CD3 intracellular signaling domains, and/or other CDtransmembrane domains. In some disclosures, the receptor, e.g., CAR, further includes a portion of one or more additional molecules such as Fc receptor $\gamma$, CD8, CD4, CD25, or CD 16. For example, in some disclosures, the CAR or other chimeric receptor includes a chimeric molecule between CD3-zeta (CD3-$\zeta$) or Fc receptor $\gamma$ and CD8, CD4, CD25 or CD16.

In some disclosures, upon ligation of the CAR or other chimeric receptor, the cytoplasmic domain or intracellular signaling domain of the receptor activates at least one of the normal effector functions or responses of the immune cell, e.g., T cell engineered to express the CAR. For example, in some contexts, the CAR induces a function of a T cell such as cytolytic activity or T-helper activity, such as secretion of cytokines or other factors. In some disclosures, a truncated portion of an intracellular signaling domain of an antigen receptor component or costimulatory molecule is used in place of an intact immunostimulatory chain, for example, if it transduces the effector function signal. In some disclosures, the intracellular signaling domain or domains include the cytoplasmic sequences of the T cell receptor (TCR), and in some disclosures also those of co-receptors that in the natural context act in concert with such receptors to initiate signal transduction following antigen receptor engagement.

In the context of a natural TCR, full activation generally requires not only signaling through the TCR, but also a costimulatory signal. Thus, in some disclosures, to promote full activation, a component for generating secondary or co-stimulatory signal is also included in the CAR. In other disclosures, the CAR does not include a component for generating a costimulatory signal. In some disclosures, an additional CAR is expressed in the same cell and provides the component for generating the secondary or costimulatory signal.

In some disclosures, the chimeric antigen receptor contains an intracellular domain of a T cell costimulatory molecule. In some disclosures, the CAR includes a signaling domain and/or transmembrane portion of a costimulatory receptor, such as CD28, 4-1BB, OX40, DAP10, and ICOS. In some disclosures, the same CAR includes both the activating and costimulatory components. In some disclosures, the chimeric antigen receptor contains an intracellular domain derived from a T cell costimulatory molecule or a functional variant thereof, such as between the transmembrane domain and intracellular signaling domain. In some disclosures, the T cell costimulatory molecule is CD28 or 41BB.

In some disclosures, the activating domain is included within one CAR, whereas the costimulatory component is disclosed by another CAR recognizing another antigen. In some disclosures, the CARs include activating or stimulatory CARs, costimulatory CARs, both expressed on the same cell (see WO2014/055668). In some disclosures, the cells include one or more stimulatory or activating CAR and/or a costimulatory CAR. In some disclosures, the cells further include inhibitory CARs (iCARs, see Fedorov et al., Sci. Transl. Medicine, 5(215) (December, 2013), such as a CAR recognizing an antigen other than the one associated with and/or specific for the disease or condition whereby an activating signal delivered through the disease-targeting CAR is diminished or inhibited by binding of the inhibitory CAR to its ligand, e.g., to reduce off-target effects.

In certain disclosures, the intracellular signaling domain comprises a CD28 transmembrane and signaling domain linked to a CD3 (e.g., CD3-zeta) intracellular domain. In some disclosures, the intracellular signaling domain comprises a chimeric CD28 and CD137 (4-1BB, TNFRSF9) co-stimulatory domains, linked to a CD3 zeta intracellular domain.

In some disclosures, the CAR encompasses one or more, e.g., two or more, costimulatory domains and an activation domain, e.g., primary activation domain, in the cytoplasmic portion. Exemplary CARs include intracellular components of CD3-zeta, CD28, and 4-1BB.

In some disclosures, the antigen receptor further includes a marker and/or cells expressing the CAR or other antigen receptor further includes a surrogate marker, such as a cell surface marker, which may be used to confirm transduction or engineering of the cell to express the receptor. In some disclosures, the marker includes all or part (e.g., truncated form) of CD34, a NGFR, or epidermal growth factor receptor, such as truncated version of such a cell surface receptor (e.g., tEGFR). In some disclosures, the nucleic acid encoding the marker is operably linked to a polynucleotide encoding for a linker sequence, such as a cleavable linker sequence, e.g., T2A. For example, a marker, and optionally a linker sequence, can be any as disclosed in published patent application No. WO2014031687. For example, the marker can be a truncated EGFR (tEGFR) that is, optionally, linked to a linker sequence, such as a T2A cleavable linker sequence.

An exemplary polypeptide for a truncated EGFR (e.g. tEGFR) comprises the sequence of amino acids set forth in SEQ ID NO: 7 or 16 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 7 or 16. An exemplary T2A linker sequence comprises the sequence of amino acids set forth in SEQ ID NO: 6 or 17 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 6 or 17.

In some disclosures, the marker is a molecule, e.g., cell surface protein, not naturally found on T cells or not naturally found on the surface of T cells, or a portion thereof. In some disclosures, the molecule is a non-self molecule, e.g., non-self protein, i.e., one that is not recognized as "self" by the immune system of the host into which the cells will be adoptively transferred.

In some disclosures, the marker serves no therapeutic function and/or produces no effect other than to be used as a marker for genetic engineering, e.g., for selecting cells successfully engineered. In other disclosures, the marker may be a therapeutic molecule or molecule otherwise exerting some desired effect, such as a ligand for a cell to be encountered in vivo, such as a costimulatory or immune checkpoint molecule to enhance and/or dampen responses of the cells upon adoptive transfer and encounter with ligand.

In some cases disclosed herein, CARs are referred to as first, second, and/or third generation CARs. In some disclosures, a first generation CAR is one that solely provides a CD3-chain induced signal upon antigen binding; in some disclosures, a second-generation CARs is one that provides such a signal and costimulatory signal, such as one including an intracellular signaling domain from a costimulatory receptor such as CD28 or CD137; in some disclosures, a third generation CAR is one that includes multiple costimulatory domains of different costimulatory receptors.

For example, in some disclosures, the CAR contains an antibody, e.g., an antibody fragment, a transmembrane domain that is or contains a transmembrane portion of CD28 or a functional variant thereof, and an intracellular signaling domain containing a signaling portion of CD28 or functional variant thereof and a signaling portion of CD3 zeta or functional variant thereof. In some disclosures, the CAR contains an antibody, e.g., antibody fragment, a transmembrane domain that is or contains a transmembrane portion of CD28 or a functional variant thereof, and an intracellular signaling domain containing a signaling portion of a 4-1BB or functional variant thereof and a signaling portion of CD3 zeta or functional variant thereof. In some such disclosures, the receptor further includes a spacer containing a portion of an Ig molecule, such as a human Ig molecule, such as an Ig hinge, e.g. an IgG4 hinge, such as a hinge-only spacer.

In some disclosures, the transmembrane domain of the recombinant receptor, e.g., the CAR, is or includes a transmembrane domain of human CD28 (e.g. Accession No. P01747.1) or variant thereof, such as a transmembrane domain that comprises the sequence of amino acids set forth in SEQ ID NO: 8 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 8; in some disclosures, the transmembrane-domain containing portion of the recombinant receptor comprises the sequence of amino acids set forth in SEQ ID NO: 9 or a sequence of amino acids having at least at or about 85%, 86%, 87%, 88%, 89%, 90%, 91%, 92%, 93%, 94%, 95%, 96%, 97%, 98%, 99% or more sequence identity thereto.

In some disclosures, the intracellular signaling component(s) of the recombinant receptor, e.g. the CAR, contains an intracellular costimulatory signaling domain of human CD28 or a functional variant or portion thereof, such as a domain with an LL to GG substitution at positions 186-187 of a native CD28 protein. For example, the intracellular signaling domain can comprise the sequence of amino acids set forth in SEQ ID NO: 10 or 11 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 10 or 11. In some disclosures, the intracellular domain comprises an intracellular costimulatory signaling domain of 4-1BB (e.g. (Accession No. Q07011.1) or functional variant or portion thereof, such as the sequence of amino acids set forth in SEQ ID NO: 12 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 12.

In some disclosures, the intracellular signaling domain of the recombinant receptor, e.g. the CAR, comprises a human CD3 zeta stimulatory signaling domain or functional variant thereof, such as an 112 AA cytoplasmic domain of isoform 3 of human CD3ζ (Accession No.: P20963.2) or a CD3 zeta signaling domain as described in U.S. Patent No.: 7,446,190 or U.S. Patent No. 8,911,993. For example, in some disclosures, the intracellular signaling domain comprises the sequence of amino acids as set forth in SEQ ID NO: 13, 14 or 15 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 13, 14 or 15.

In some disclosures, the spacer contains only a hinge region of an IgG, such as only a hinge of IgG4 or IgG1, such as the hinge only spacer set forth in SEQ ID NO: 1. In other disclosures, the spacer is or contains an Ig hinge, e.g., an IgG4-derived hinge, optionally linked to a $C_H2$ and/or $C_H3$ domains. In some disclosures, the spacer is an Ig hinge, e.g., an IgG4 hinge, linked to $C_H2$ and $C_H3$ domains, such as set forth in SEQ ID NO: 4. In some disclosures, the spacer is an Ig hinge, e.g., an IgG4 hinge, linked to a $C_H3$ domain only, such as set forth in SEQ ID NO: 3. In some disclosures, the spacer is or comprises a glycine-serine rich sequence or other flexible linker such as known flexible linkers.

For example, in some disclosures, the CAR includes an antibody such as an antibody fragment, including scFvs, a spacer, such as a spacer containing a portion of an immunoglobulin molecule, such as a hinge region and/or one or more constant regions of a heavy chain molecule, such as an Ig-hinge containing spacer, a transmembrane domain containing all or a portion of a CD28-derived transmembrane domain, a CD28-derived intracellular signaling domain, and a CD3 zeta signaling domain. In some disclosures, the CAR includes an antibody or fragment, such as scFv, a spacer such as any of the Ig-hinge containing spacers, a CD28-derived transmembrane domain, a 4-1BB-derived intracellular signaling domain, and a CD3 zeta-derived signaling domain.

In some disclosures, nucleic acid molecules encoding such CAR constructs further includes a sequence encoding a T2A ribosomal skip element and/or a tEGFR sequence, e.g., downstream of the sequence encoding the CAR. In some disclosures, the sequence encodes a T2A ribosomal skip element set forth in SEQ ID NO: 6 or 17, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 6 or 17. In some disclosures, T cells expressing an antigen receptor (e.g. CAR) can also be generated to express a truncated EGFR (EGFRt) as a non-immunogenic selection epitope (e.g. by introduction of a construct encoding the CAR and EGFRt separated by a T2A ribosome switch to express two proteins from the same construct), which then can be used as a marker to detect such cells (see e.g. U.S. Patent No. 8,802,374). In some disclosures, the sequence encodes an tEGFR sequence set forth in SEQ ID NO: 7 or 16, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 7 or 16. In some cases disclosed herein, the peptide, such as T2A, can cause the ribosome to skip (ribosome skipping) synthesis of a peptide bond at the C-terminus of a 2A element, leading to separation between the end of the 2A sequence and the next peptide downstream (see, for example, de Felipe. Genetic Vaccines and Ther. 2:13 (2004) and deFelipe et al. Traffic 5:616-626 (2004)). Many 2A elements are known. Examples of 2A sequences that can be used in the methods and nucleic acids disclosed herein, without limitation, 2A sequences from the foot-and-mouth disease virus (F2A, e.g., SEQ ID NO: 21), equine rhinitis A virus (E2A, e.g., SEQ ID NO: 20), Thosea asigna virus (T2A, e.g., SEQ ID NO: 6 or 17), and porcine teschovirus-1 (P2A, e.g., SEQ ID NO: 18 or 19) as described in U.S. Patent Publication No. 20070116690.

The recombinant receptors, such as CARs, expressed by the cells administered to the subject generally recognize or specifically bind to a molecule that is expressed in, associated with, and/or specific for the disease or condition or cells thereof being treated. Upon specific binding to the molecule, e.g., antigen, the receptor generally delivers an immunostimulatory signal, such as an ITAM-transduced signal, into the cell, thereby promoting an immune response targeted to the disease or condition. For example, in some disclosures, the cells express a CAR that specifically binds to an antigen expressed by a cell or tissue of the disease or condition or associated with the disease or condition.

### 2. T Cell Receptors (TCRs)

In some disclosures, engineered cells, such as T cells, used in connection with the disclosed methods, uses, articles of manufacture or compositions are cells that express a T cell receptor (TCR) or antigen-binding portion thereof that recognizes a peptide epitope or T cell epitope of a target polypeptide, such as an antigen of a tumor, viral or autoimmune protein.

In some disclosures, a "T cell receptor" or "TCR" is a molecule that contains a variable $\alpha$ and $\beta$ chains (also known as TCR$\alpha$ and TCR$\beta$, respectively) or a variable $\gamma$ and $\delta$ chains (also known as TCR$\alpha$ and TCR$\beta$, respectively), or antigen-binding portions thereof, and which is capable of specifically binding to a peptide bound to an MHC molecule. In some disclosures, the TCR is in the $\alpha\beta$ form. Typically, TCRs that exist in $\alpha\beta$ and $\gamma\delta$ forms are generally structurally similar, but T cells expressing them may have distinct anatomical locations or functions. A TCR can be found on the surface of a cell or in soluble form. Generally, a TCR is found on the surface of T cells (or T lymphocytes) where it is generally responsible for recognizing antigens bound to major histocompatibility complex (MHC) molecules.

Unless otherwise stated, the term "TCR" should be understood to encompass full TCRs as well as antigen-binding portions or antigen-binding fragments thereof. In some disclosures, the TCR is an intact or full-length TCR, including TCRs in the $\alpha\beta$ form or $\gamma\delta$ form. In some disclosures, the TCR is an antigen-binding portion that is less than a full-length TCR but that binds to a specific peptide bound in an MHC molecule, such as binds to an MHC-peptide complex. In some cases disclosed herein, an antigen-binding portion or fragment of a TCR can contain only a portion of the structural domains of a full-length or intact TCR, but yet is able to bind the peptide epitope, such as MHC-peptide complex, to which the full TCR binds. In some cases disclosed herein, an antigen-binding portion contains the variable domains of a TCR, such as variable $\alpha$ chain and variable $\beta$ chain of a TCR, sufficient to form a binding site for binding to a specific MHC-peptide complex. Generally, the variable chains of a TCR contain complementarity determining regions involved in recognition of the peptide, MHC and/or MHC-peptide complex.

In some disclosures, the variable domains of the TCR contain hypervariable loops, or complementarity determining regions (CDRs), which generally are the primary contributors to antigen recognition and binding capabilities and specificity. In some disclosures, a CDR of a TCR or combination thereof forms all or substantially all of the antigen-binding site of a given TCR molecule. The various CDRs within a variable region of a TCR chain generally are separated by framework regions (FRs), which generally display less variability among TCR molecules as compared to the CDRs (see, e.g., Jores et al., Proc. Nat'l Acad. Sci. U.S.A. 87:9138, 1990; Chothia et al., EMBO J. 7:3745, 1988; see also Lefranc et al., Dev. Comp. Immunol. 27:55, 2003). In some disclosures, CDR3 is the main CDR responsible for antigen binding or specificity, or is the most important among the three CDRs on a given TCR variable region for antigen recognition, and/or for interaction with the processed peptide portion of the peptide-MHC complex. In some contexts, the CDR1 of the alpha chain can interact with the N-terminal part of certain antigenic peptides. In some contexts, CDR1 of the beta chain can interact with the C-terminal part of the peptide. In some contexts, CDR2 contributes most strongly to or is the primary CDR responsible for the interaction with or recognition of the MHC portion of the MHC-peptide complex. In some disclosures, the variable region of the $\beta$-chain can contain a further hypervariable region (CDR4 or HVR4), which generally is involved in superantigen binding and not antigen recognition (Kotb (1995) Clinical Microbiology Reviews, 8:411-426).

In some disclosures, a TCR also can contain a constant domain, a transmembrane domain and/or a short cytoplasmic tail (see, e.g., Janeway et al., Immunobiology: The Immune System in Health and Disease, 3rd Ed., Current Biology Publications, p. 4:33, 1997). In some disclosures, each chain of the TCR can possess one N-terminal immunoglobulin variable domain, one immunoglobulin constant domain, a transmembrane region, and a short cytoplasmic tail at the C-terminal end. In some disclosures, a TCR is associated with invariant proteins of the CD3 complex involved in mediating signal transduction.

In some disclosures, a TCR chain contains one or more constant domain. For example, the extracellular portion of a given TCR chain (e.g., $\alpha$-chain or $\beta$-chain) can contain two immunoglobulin-like domains, such as a variable domain (e.g., V$\alpha$ or V$\beta$; typically amino acids 1 to 116 based on Kabat numbering Kabat et al., "Sequences of Proteins of Immunological Interest, US Dept. Health and Human Services, Public Health Service National Institutes of Health, 1991, 5th ed.) and a constant domain (e.g., $\alpha$-chain constant domain or C$\alpha$, typically positions 117 to 259 of the chain based on Kabat numbering or $\beta$ chain constant domain or C$_\beta$, typically positions 117 to 295 of the chain based on Kabat) adjacent to the cell membrane. For example, in some cases disclosed herein, the extracellular portion of the TCR formed by the two chains contains two membrane-proximal constant domains, and two membrane-distal variable domains, which variable domains each contain CDRs. The constant domain of the TCR may contain short connecting sequences in which a cysteine residue forms a disulfide bond, thereby linking the two chains of the TCR. In some disclosures, a TCR may have an additional cysteine residue in each of the α and β chains, such that the TCR contains two disulfide bonds in the constant domains.

In some disclosures, the TCR chains contain a transmembrane domain. In some disclosures, the transmembrane domain is positively charged. In some cases disclosed herein, the TCR chain contains a cytoplasmic tail. In some cases disclosed herein, the structure allows the TCR to associate with other molecules like CD3 and subunits thereof. For example, a TCR containing constant domains with a transmembrane region may anchor the protein in the cell membrane and associate with invariant subunits of the CD3 signaling apparatus or complex. The intracellular tails of CD3 signaling subunits (e.g. CD3γ, CD36, CD3ε and CD3ζ chains) contain one or more immunoreceptor tyrosine-based activation motif or ITAM that are involved in the signaling capacity of the TCR complex.

In some disclosures, the TCR may be a heterodimer of two chains α and β (or optionally γ and δ) or it may be a single chain TCR construct. In some disclosures, the TCR is a heterodimer containing two separate chains (α and β chains or γ and δ chains) that are linked, such as by a disulfide bond or disulfide bonds.

In some disclosures, the TCR can be generated from a known TCR sequence(s), such as sequences of Vα,β chains, for which a substantially full-length coding sequence is readily available. Methods for obtaining full-length TCR sequences, including V chain sequences, from cell sources are well known. In some disclosures, nucleic acids encoding the TCR can be obtained from a variety of sources, such as by polymerase chain reaction (PCR) amplification of TCR-encoding nucleic acids within or isolated from a given cell or cells, or synthesis of publicly available TCR DNA sequences.

In some disclosures, the TCR is obtained from a biological source, such as from cells such as from a T cell (e.g. cytotoxic T cell), T-cell hybridomas or other publicly available source. In some disclosures, the T-cells can be obtained from *in vivo* isolated cells. In some disclosures, the TCR is a thymically selected TCR. In some disclosures, the TCR is a neoepitope-restricted TCR. In some disclosures, the T- cells can be a cultured T-cell hybridoma or clone. In some disclosures, the TCR or antigen-binding portion thereof or antigen-binding fragment thereof can be synthetically generated from knowledge of the sequence of the TCR.

In some disclosures, the TCR is generated from a TCR identified or selected from screening a library of candidate TCRs against a target polypeptide antigen, or target T cell epitope thereof. TCR libraries can be generated by amplification of the repertoire of Vα and Vβ from T cells isolated from a subject, including cells present in PBMCs, spleen or other lymphoid organ. In some cases disclosed herein, T cells can be amplified from tumor-infiltrating lymphocytes (TILs). In some disclosures, TCR libraries can be generated from CD4+ or CD8+ cells. In some disclosures, the TCRs can be amplified from a T cell source of a normal of healthy subject, i.e. normal TCR libraries. In some disclosures, the TCRs can be amplified from a T cell source of a diseased subject, i.e. diseased TCR libraries. In some disclosures, degenerate primers are used to amplify the gene repertoire of Vα and Vβ, such as by RT-PCR in samples, such as T cells, obtained from humans. In some disclosures, scTv libraries can be assembled from naive Vα and Vβ libraries in which the amplified products are cloned or assembled to be separated by a linker. Depending on the source of the subject and cells, the libraries can be HLA allele-specific. Alternatively, in some disclosures, TCR libraries can be generated by mutagenesis or diversification of a parent or scaffold TCR molecule. In some disclosures, the TCRs are subjected to directed evolution, such as by mutagenesis, e.g., of the α or β chain. In some disclosures, particular residues within CDRs of the TCR are altered. In some disclosures, selected TCRs can be modified by affinity maturation. In some disclosures, antigen-specific T cells may be selected, such as by screening to assess CTL activity against the peptide. In some disclosures, TCRs, e.g. present on the antigen-specific T cells, may be selected, such as by binding activity, e.g., particular affinity or avidity for the antigen.

In some disclosures, the TCR or antigen-binding portion thereof is one that has been modified or engineered. In some disclosures, directed evolution methods are used to generate TCRs with altered properties, such as with higher affinity for a specific MHC-peptide complex. In some disclosures, directed evolution is achieved by display methods including, but not limited to, yeast display (Holler et al. (2003) Nat Immunol, 4, 55-62; Holler et al. (2000) Proc Natl Acad Sci USA, 97, 5387-92), phage display (Li et al. (2005) Nat Biotechnol, 23, 349-54), or T cell display (Chervin et al. (2008) J Immunol Methods, 339, 175-84). In some disclosures, display approaches involve engineering, or modifying, a known, parent or reference TCR. For example, in some cases disclosed herein, a wild-type TCR can be used as a template for producing mutagenized TCRs in which in one or more residues of the CDRs are mutated, and mutants with an desired altered property, such as higher affinity for a desired target antigen, are selected.

In some disclosures, peptides of a target polypeptide for use in producing or generating a TCR of interest are known or can be readily identified. In some disclosures, peptides suitable for use in generating TCRs or antigen-binding portions can be determined based on the presence of an HLA-restricted motif in a target polypeptide of interest, such as a target polypeptide described below. In some disclosures, peptides are identified using available computer prediction models. In some disclosures, for predicting MHC class I binding sites, such models include, but are not limited to, ProPred1 (Singh and Raghava (2001) Bioinformatics 17(12): 1236-1237, and SYFPEITHI (see Schuler et al. (2007) Immunoinformatics Methods in Molecular Biology, 409(1): 75-93 2007). In some disclosures, the MHC-restricted epitope is HLA-A0201, which is expressed in approximately 39-46% of all Caucasians and therefore, represents a suitable choice of MHC antigen for use preparing a TCR or other MHC-peptide binding molecule.

HLA-A0201-binding motifs and the cleavage sites for proteasomes and immune-proteasomes using computer prediction models are known. For predicting MHC class I binding sites, such models include, but are not limited to, ProPred1 (described in more detail in Singh and Raghava, ProPred: prediction of HLA-DR binding sites. BIOINFORMATICS 17(12):1236-1237 2001), and SYFPEITHI (see Schuler et al. SYFPEITHI, Database for Searching and T-Cell Epitope Prediction. in Immunoinformatics Methods in Molecular Biology, vol 409(1): 75-93 2007)

In some disclosures, the TCR or antigen binding portion thereof may be a recombinantly produced natural protein or mutated form thereof in which one or more property, such as binding characteristic, has been altered. In some disclosures, a TCR may be derived from one of various animal species, such as human, mouse, rat, or other mammal. A TCR may be cell-bound or in soluble form. In some disclosures, for purposes of the disclosed methods, the TCR is in cell-bound form expressed on the surface of a cell.

In some disclosures, the TCR is a full-length TCR. In some disclosures, the TCR is an antigen-binding portion. In some disclosures, the TCR is a dimeric TCR (dTCR). In some disclosures, the TCR is a single-chain TCR (sc-TCR). In some disclosures, a dTCR or scTCR have the structures as described in WO 03/020763, WO 04/033685, WO2011/044186.

In some disclosures, the TCR contains a sequence corresponding to the transmembrane sequence. In some disclosures, the TCR does contain a sequence corresponding to cytoplasmic sequences. In some disclosures, the TCR is capable of forming a TCR complex with CD3. In some disclosures, any of the TCRs, including a dTCR or scTCR, can be linked to signaling domains that yield an active TCR on the surface of a T cell. In some disclosures, the TCR is expressed on the surface of cells.

In some disclosures a dTCR contains a first polypeptide wherein a sequence corresponding to a TCR $\alpha$ chain variable region sequence is fused to the N terminus of a sequence corresponding to a TCR $\alpha$ chain constant region extracellular sequence, and a second polypeptide wherein a sequence corresponding to a TCR $\beta$ chain variable region sequence is fused to the N terminus a sequence corresponding to a TCR $\beta$ chain constant region extracellular sequence, the first and second polypeptides being linked by a disulfide bond. In some disclosures, the bond can correspond to the native inter-chain disulfide bond present in native dimeric $\alpha\beta$ TCRs. In some disclosures, the interchain disulfide bonds are not present in a native TCR. For example, in some disclosures, one or more cysteines can be incorporated into the constant region extracellular sequences of dTCR polypeptide pair. In some cases disclosed herein, both a native and a non-native disulfide bond may be desirable. In some disclosures, the TCR contains a transmembrane sequence to anchor to the membrane.

In some disclosures, a dTCR contains a TCR $\alpha$ chain containing a variable $\alpha$ domain, a constant $\alpha$ domain and a first dimerization motif attached to the C-terminus of the constant $\alpha$ domain, and a TCR $\beta$ chain comprising a variable $\beta$ domain, a constant $\beta$ domain and a first dimerization motif attached to the C-terminus of the constant $\beta$ domain, wherein the first and second dimerization motifs easily interact to form a covalent bond between an amino acid in the first dimerization motif and an amino acid in the second dimerization motif linking the TCR $\alpha$ chain and TCR $\beta$ chain together.

In some disclosures, the TCR is a scTCR. Typically, a scTCR can be generated using methods known, See e.g., Soo Hoo, W. F. et al. PNAS (USA) 89, 4759 (1992); Wülfing, C. and Plückthun, A., J. Mol. Biol. 242, 655 (1994); Kurucz, I. et al. PNAS (USA) 90 3830 (1993); International published PCT Nos. WO 96/13593, WO 96/18105, WO99/60120, WO99/18129, WO 03/020763, WO2011/044186; and Schlueter, C. J. et al. J. Mol. Biol. 256, 859 (1996). In some disclosures, a scTCR contains an introduced non-native disulfide interchain bond to facilitate the association of the TCR chains (see e.g. International published PCT No. WO 03/020763). In some disclosures, a scTCR is a non-disulfide linked truncated TCR in which heterologous leucine zippers fused to the C-termini thereof facilitate chain association (see e.g. International published PCT No. WO99/60120). In some disclosures, a scTCR contain a TCR$\alpha$ variable domain covalently linked to a TCR$\beta$ variable domain via a peptide linker (see e.g., International published PCT No. WO99/18129).

In some disclosures, a scTCR contains a first segment constituted by an amino acid sequence corresponding to a TCR $\alpha$ chain variable region, a second segment constituted by an amino acid sequence corresponding to a TCR $\beta$ chain variable region sequence fused to the N terminus of an amino acid sequence corresponding to a TCR $\beta$ chain constant domain extracellular sequence, and a linker sequence linking the C terminus of the first segment to the N terminus of the second segment.

In some disclosures, a scTCR contains a first segment constituted by an $\alpha$ chain variable region sequence fused to the N terminus of an $\alpha$ chain extracellular constant domain sequence, and a second segment constituted by a $\beta$ chain variable region sequence fused to the N terminus of a sequence $\beta$ chain extracellular constant and transmembrane sequence, and, optionally, a linker sequence linking the C terminus of the first segment to the N terminus of the second segment.

In some disclosures, a scTCR contains a first segment constituted by a TCR $\beta$ chain variable region sequence fused to the N terminus of a β chain extracellular constant domain sequence, and a second segment constituted by an α chain variable region sequence fused to the N terminus of a sequence α chain extracellular constant and transmembrane sequence, and, optionally, a linker sequence linking the C terminus of the first segment to the N terminus of the second segment.

In some disclosures, the linker of a scTCRs that links the first and second TCR segments can be any linker capable of forming a single polypeptide strand, while retaining TCR binding specificity. In some disclosures, the linker sequence may, for example, have the formula -P-AA-P- wherein P is proline and AA represents an amino acid sequence wherein the amino acids are glycine and serine. In some disclosures, the first and second segments are paired so that the variable region sequences thereof are orientated for such binding. Hence, in some cases disclosed herein, the linker has a sufficient length to span the distance between the C terminus of the first segment and the N terminus of the second segment, or vice versa, but is not too long to block or reduces bonding of the scTCR to the target ligand. In some disclosures, the linker can contain from or from about 10 to 45 amino acids, such as 10 to 30 amino acids or 26 to 41 amino acids residues, for example 29, 30, 31 or 32 amino acids. In some disclosures, the linker has the formula -PG-GG-(SGGGG)$_5$-P- wherein P is proline, G is glycine and S is serine (SEQ ID NO:22). In some disclosures, the linker has the sequence GSADDAKKDAAKKDGKS (SEQ ID NO:23)

In some disclosures, the scTCR contains a covalent disulfide bond linking a residue of the immunoglobulin region of the constant domain of the α chain to a residue of the immunoglobulin region of the constant domain of the β chain. In some disclosures, the interchain disulfide bond in a native TCR is not present. For example, in some disclosures, one or more cysteines can be incorporated into the constant region extracellular sequences of the first and second segments of the scTCR polypeptide. In some cases disclosed herein, both a native and a non-native disulfide bond may be desirable.

In some disclosures of a dTCR or scTCR containing introduced interchain disulfide bonds, the native disulfide bonds are not present. In some disclosures, the one or more of the native cysteines forming a native interchain disulfide bonds are substituted to another residue, such as to a serine or alanine. In some disclosures, an introduced disulfide bond can be formed by mutating non-cysteine residues on the first and second segments to cysteine. Exemplary non-native disulfide bonds of a TCR are described in published International PCT No. WO2006/000830.

In some disclosures, the TCR or antigen-binding fragment thereof exhibits an affinity with an equilibrium binding constant for a target antigen of between or between about 10-5 and 10-12 M and all individual values and ranges therein. In some disclosures, the target antigen is an MHC-peptide complex or ligand.

In some disclosures, nucleic acid or nucleic acids encoding a TCR, such as α and β chains, can be amplified by PCR, cloning or other suitable means and cloned into a suitable expression vector or vectors. The expression vector can be any suitable recombinant expression vector, and can be used to transform or transfect any suitable host. Suitable vectors include those designed for propagation and expansion or for expression or both, such as plasmids and viruses.

In some disclosures, the vector can a vector of the pUC series (Fermentas Life Sciences), the pBluescript series (Stratagene, LaJolla, Calif.), the pET series (Novagen, Madison, Wis.), the pGEX series (Pharmacia Biotech, Uppsala, Sweden), or the pEX series (Clontech, Palo Alto, Calif.). In some cases disclosed herein, bacteriophage vectors, such as λG10, λGT11, λZapII (Stratagene), λEMBL4, and λNM1149, also can be used. In some disclosures, plant expression vectors can be used and include pBI01, pBI101.2, pBI101.3, pBI121 and pBIN19 (Clontech). In some disclosures, animal expression vectors include pEUK-Cl, pMAM and pMAMneo (Clontech). In some disclosures, a viral vector is used, such as a retroviral vector.

In some disclosures, the recombinant expression vectors can be prepared using standard recombinant DNA techniques. In some disclosures, vectors can contain regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA-based. In some disclosures, the vector can contain a nonnative promoter operably linked to the nucleotide sequence encoding the TCR or antigen-binding portion (or other MHC-peptide binding molecule). In some disclosures, the promoter can be a non-viral promoter or a viral promoter, such as a cytomegalovirus (CMV) promoter, an SV40 promoter, an RSV promoter, and a promoter found in the long-terminal repeat of the murine stem cell virus. Other known promoters also are contemplated.

In some disclosures, to generate a vector encoding a TCR, the α and β chains are PCR amplified from total cDNA isolated from a T cell clone expressing the TCR of interest and cloned into an expression vector. In some disclosures, the α and β chains are cloned into the same vector. In some disclosures, the α and β chains are cloned into different vectors. In some disclosures, the generated α and β chains are incorporated into a retroviral, e.g. lentiviral, vector.

Genetically Engineered Cells and Methods of Producing Cells

In some disclosures, the disclosed methods involve administering to a subject having a disease or condition cells expressing a recombinant antigen receptor. Various methods for the introduction of genetically engineered components, e.g., recombinant receptors, e.g., CARs or TCRs, are well known and may be used with the disclosed methods and compositions. Exemplary methods include those for transfer of nucleic acids encoding the receptors, including via viral, e.g., retroviral or lentiviral, transduction, transposons, and electroporation.

Among the cells expressing the receptors and administered by the disclosed methods are engineered cells. The genetic engineering generally involves introduction of a nucleic acid encoding the recombinant or engineered component into a composition containing the cells, such as by retroviral transduction, transfection, or transformation.

### 3. Chimeric Auto-Antibody Receptors (CAARs)

In some disclosures, among the recombinant receptor expressed by the engineered cells used in connection with the disclosed methods, uses, articles of manufacture and compositions is a chimeric autoantibody receptor (CAAR). In some disclosures, the CAAR is specific for an autoantibody. In some disclosures, a cell expressing the CAAR, such as a T cell engineered to express a CAAR, can be used to specifically bind to and kill autoantibody-expressing cells, but not normal antibody expressing cells. In some disclosures, CAAR-expressing cells can be used to treat an autoimmune disease associated with expression of self-antigens, such as autoimmune diseases. In some disclosures, CAAR-expressing cells can target B cells that ultimately produce the autoantibodies and display the autoantibodies on their cell surfaces, mark these B cells as disease-specific targets for therapeutic intervention. In some disclosures, CAAR-expressing cells can be used to efficiently targeting and killing the pathogenic B cells in autoimmune diseases by targeting the disease-causing B cells using an antigen-specific chimeric autoantibody receptor. In some disclosures, the recombinant receptor is a CAAR, such as any described in U.S. Patent Application Pub. No. US 2017/0051035.

In some disclosures, the CAAR comprises an autoantibody binding domain, a transmembrane domain, and an intracellular signaling region. In some disclosures, the intracellular signaling region comprises an intracellular signaling domain. In some disclosures, the intracellular signaling domain is or comprises a primary signaling domain, a signaling domain that is capable of inducing a primary activation signal in a T cell, a signaling domain of a T cell receptor (TCR) component, and/or a signaling domain comprising an immunoreceptor tyrosine-based activation motif (ITAM). In some disclosures, the intracellular signaling region comprises a secondary or costimulatory signaling region (secondary intracellular signaling regions).

In some disclosures, the autoantibody binding domain comprises an autoantigen or a fragment thereof. The choice of autoantigen can depend upon the type of autoantibody being targeted. For example, the autoantigen may be chosen because it recognizes an autoantibody on a target cell, such as a B cell, associated with a particular disease state, e.g. an autoimmune disease, such as an autoantibody-mediated autoimmune disease. In some disclosures, the autoimmune disease includes pemphigus vulgaris (PV). Exemplary autoantigens include desmoglein 1 (Dsg1) and Dsg3.

### 4. Multi-targeting

In some disclosures, the cells used in connection with the disclosed methods, uses, articles of manufacture and compositions include cells employing multi-targeting strategies, such as expression of two or more genetically engineered receptors on the cell, each recognizing the same of a different antigen and typically each including a different intracellular signaling component. Such multi-targeting strategies are described, for example, in International Patent Application, Publication No.: WO 2014055668 A1 (describing combinations of activating and costimulatory CARs, e.g., targeting two different antigens present individually on off-target, e.g., normal cells, but present together only on cells of the disease or condition to be treated) and Fedorov et al., Sci. Transl. Medicine, 5(215) (2013) (describing cells expressing an activating and an inhibitory CAR, such as those in which the activating CAR binds to one antigen expressed on both normal or non-diseased cells and cells of the disease or condition to be treated, and the inhibitory CAR binds to another antigen expressed only on the normal cells or cells which it is not desired to treat).

For example, in some disclosures, the cells include a receptor expressing a first genetically engineered antigen receptor (e.g., CAR or TCR) which is capable of inducing an activating or stimulatory signal to the cell, generally upon specific binding to the antigen recognized by the first receptor, e.g., the first antigen. In some disclosures, the cell further includes a second genetically engineered antigen receptor (e.g., CAR or TCR), e.g., a chimeric costimulatory receptor, which is capable of inducing a costimulatory signal to the immune cell, generally upon specific binding to a second antigen recognized by the second receptor. In some disclosures, the first antigen and second antigen are the same. In some disclosures, the first antigen and second antigen are different.

In some disclosures, the first and/or second genetically engineered antigen receptor (e.g. CAR or TCR) is capable of inducing an activating signal to the cell. In some disclosures, the receptor includes an intracellular signaling component containing ITAM or ITAM-like motifs. In some disclosures, the activation induced by the first receptor involves a signal transduction or change in protein expression in the cell resulting in initiation of an immune response, such as ITAM phosphorylation and/or initiation of ITAM-mediated signal transduction cascade, formation of an immunological synapse and/or clustering of molecules near the bound receptor (e.g. CD4 or CD8, etc.), activation of one or more transcription factors, such as NF-κB and/or AP-1, and/or induction of gene expression of factors such as cytokines, proliferation, and/or survival.

In some disclosures, the first and/or second receptor includes intracellular signaling domains or regions of costimulatory receptors such as CD28, CD 137 (4-1BB), OX40, and/or ICOS. In some disclosures, the first and second receptor include an intracellular signaling domain of a costimulatory receptor that are different. In one disclosure, the first receptor contains a CD28 costimulatory signaling region and the second receptor contain a 4-1BB costimulatory signaling region or vice versa.

In some disclosures, the first and/or second receptor includes both an intracellular signaling domain containing ITAM or ITAM-like motifs and an intracellular signaling domain of a costimulatory receptor.

In some disclosures, the first receptor contains an intracellular signaling domain containing ITAM or ITAM-like motifs and the second receptor contains an intracellular signaling domain of a costimulatory receptor. The costimulatory signal in combination with the activating signal induced in the same cell is one that results in an immune response, such as a robust and sustained immune response, such as increased gene expression, secretion of cytokines and other factors, and T cell mediated effector functions such as cell killing.

In some disclosures, neither ligation of the first receptor alone nor ligation of the second receptor alone induces a robust immune response. In some disclosures, if only one receptor is ligated, the cell becomes tolerized or unresponsive to antigen, or inhibited, and/or is not induced to proliferate or secrete factors or carry out effector functions. In some such disclosures, however, when the plurality of receptors are ligated, such as upon encounter of a cell expressing the first and second antigens, a desired response is achieved, such as full immune activation or stimulation, e.g., as indicated by secretion of one or more cytokine, proliferation, persistence, and/or carrying out an immune effector function such as cytotoxic killing of a target cell.

In some disclosures, the two receptors induce, respectively, an activating and an inhibitory signal to the cell, such that binding by one of the receptor to its antigen activates the cell or induces a response, but binding by the second inhibitory receptor to its antigen induces a signal that suppresses or dampens that response. Examples disclosed herein are combinations of activating CARs and inhibitory CARs or iCARs. Such a strategy may be used, for example, in which the activating CAR binds an antigen expressed in a disease or condition but which is also expressed on normal cells, and the inhibitory receptor binds to a separate antigen which is expressed on the normal cells but not cells of the disease or condition.

In some disclosures, the multi-targeting strategy is employed in a case disclosed herein where an antigen associated with a particular disease or condition is expressed on a non-diseased cell and/or is expressed on the engineered cell itself, either transiently (e.g., upon stimulation in association with genetic engineering) or permanently. In such cases disclosed herein, by requiring ligation of two separate and individually specific antigen receptors, specificity, selectivity, and/or efficacy may be improved.

In some disclosures, the plurality of antigens, e.g., the first and second antigens, are expressed on the cell, tissue, or disease or condition being targeted, such as on the cancer cell. In some disclosures, the cell, tissue, disease or condition is multiple myeloma or a multiple myeloma cell. In some disclosures, one or more of the plurality of antigens generally also is expressed on a cell which it is not desired to target with the cell therapy, such as a normal or non-diseased cell or tissue, and/or the engineered cells themselves. In such disclosures, by requiring ligation of multiple receptors to achieve a response of the cell, specificity and/or efficacy is achieved.

## B. Nucleic Acids, Vectors and Methods for Genetic Engineering

In some disclosures, the cells, e.g., cells of a population of enriched CD57- T cells, are genetically engineered to express a recombinant receptor. In some disclosures, the engineering is carried out by introducing one or more polynucleotide(s) that encode the recombinant receptor or portions or components thereof. Also disclosed are polynucleotides encoding a recombinant receptor, and vectors or constructs containing such nucleic acids and/or polynucleotides.

In some disclosures, the polynucleotide encoding the recombinant receptor contains at least one promoter that is operatively linked to control expression of the recombinant receptor. In some examples disclosed herein, the polynucleotide contains two, three, or more promoters operatively linked to control expression of the recombinant receptor. In some disclosures, polynucleotide can contain regulatory sequences, such as transcription and translation initiation and termination codons, which are specific to the type of host (e.g., bacterium, fungus, plant, or animal) into which the polynucleotide is to be introduced, as appropriate and taking into consideration whether the polynucleotide is DNA- or RNA-based. In some disclosures, the polynucleotide can contain regulatory/control elements, such as a promoter, an enhancer, an intron, a polyadenylation signal, a Kozak consensus sequence, internal ribosome entry sites (IRES), a 2A sequence, and splice acceptor or donor. In some disclosures, the polynucleotide can contain a nonnative promoter operably linked to the nucleotide sequence encoding the recombinant receptor and/or one or more additional polypeptide(s). In some disclosures, the promoter is selected from among an RNA pol I, pol II or pol III promoter. In some disclosures, the promoter is recognized by RNA polymerase II (e.g., a CMV, SV40 early region or adenovirus major late promoter). In another disclosure, the promoter is recognized by RNA polymerase III (e.g., a U6 or H1 promoter). In some disclosures, the promoter can be a non-viral promoter or a viral promoter, such as a cytomegalovirus (CMV) promoter, an SV40 promoter, an RSV promoter, and a promoter found in the long-terminal repeat of the murine stem cell virus. Other known promoters also are contemplated.

In some disclosures, the promoter is or comprises a constitutive promoter. Exemplary constitutive promoters include, e.g., simian virus 40 early promoter (SV40), cytomegalovirus immediate-early promoter (CMV), human Ubiquitin C promoter (UBC), human elongation factor 1α promoter (EF1α), mouse phosphoglycerate kinase 1 promoter (PGK), and chicken β-Actin promoter coupled with CMV early enhancer (CAGG). In some disclosures, the constitutive promoter is a synthetic or modified promoter. In some disclosures, the promoter is or comprises an MND promoter, a synthetic promoter that contains the U3 region of a modified MoMuLV LTR with myeloproliferative sarcoma virus enhancer (see Challita et al. (1995) J. Virol. 69(2):748-755). In some disclosures, the promoter is a tissue-specific promoter. In another disclosure, the promoter is a viral promoter. In another disclosure, the promoter is a non-viral promoter. In some disclosures, exemplary promoters can include, but are not limited to, human elongation factor 1 alpha (EF1α) promoter or a modified form thereof or the MND promoter.

In another disclosure, the promoter is a regulated promoter (e.g., inducible promoter). In some disclosures, the promoter is an inducible promoter or a repressible promoter. In some disclosures, the promoter comprises a Lac operator sequence, a tetracycline operator sequence, a galactose operator sequence or a doxycycline operator sequence, or is an analog thereof or is capable of being bound by or recognized by a Lac repressor or a tetracycline repressor, or an analog thereof. In some disclosures, the polynucleotide does not include a regulatory element, e.g. promoter.

In some cases disclosed herein, the nucleic acid sequence encoding the recombinant receptor contains a signal sequence that encodes a signal peptide. In some disclosures, the signal sequence may encode a signal peptide derived from a native polypeptide. In other disclosures, the signal sequence may encode a heterologous or non-native signal peptide, such as the exemplary signal peptide of the GMCSFR alpha chain set forth in SEQ ID NO:25 and encoded by the nucleotide sequence set forth in SEQ ID NO:24. In some cases disclosed herein, the nucleic acid sequence encoding the recombinant receptor, e.g., chimeric antigen receptor (CAR) contains a signal sequence that encodes a signal peptide. Non-limiting exemplary signal peptides include, for example, the GMCSFR alpha chain signal peptide set forth in SEQ ID NO: 25 and encoded by the nucleotide sequence set forth in SEQ ID NO:24, or the CD8 alpha signal peptide set forth in SEQ ID NO:26.

In some disclosures, the polynucleotide contains a nucleic acid sequence encoding one or more additional polypeptides, e.g., one or more marker(s) and/or one or more effector molecules. In some disclosures, the one or more marker(s) includes a transduction marker, a surrogate marker and/or a selection marker. Among additional nucleic acid sequences introduced, e.g., encoding for one or more additional polypeptide(s), include nucleic acid sequences that can improve the efficacy of therapy, such as by promoting viability and/or function of transferred cells; ucleic acid sequences to provide a genetic marker for selection and/or evaluation of the cells, such as to assess in vivo survival or localization; ucleic acid sequences to improve safety, for example, by making the cell susceptible to negative selection in vivo as described by Lupton S. D. et al., Mol. and Cell Biol., 11:6 (1991); and Riddell et al., Human Gene Therapy 3:319-338 (1992); see also WO 1992008796 and WO 1994028143 describing the use of bifunctional selectable fusion genes derived from fusing a dominant positive selectable marker with a negative selectable marker, and US Patent No. 6,040,177.

In some disclosures, the marker is a transduction marker or a surrogate marker. A transduction marker or a surrogate marker can be used to detect cells that have been introduced with the polynucleotide, e.g., a polynucleotide encoding a recombinant receptor. In some disclosures, the transduction marker can indicate or confirm modification of a cell. In some disclosures, the surrogate marker is a protein that is made to be co-expressed on the cell surface with the recombinant receptor, e.g. CAR. In particular disclosures, such a surrogate marker is a surface protein that has been modified to have little or no activity. In certain disclosures, the surrogate marker is encoded on the same polynucleotide that encodes the recombinant receptor. In some disclosures, the nucleic acid sequence encoding the recombinant receptor is operably linked to a nucleic acid sequence encoding a marker, optionally separated by an internal ribosome entry site (IRES), or a nucleic acid encoding a self-cleaving peptide or a peptide that causes ribosome skipping, such as a 2A sequence. Extrinsic marker genes may in some cases disclosed herein be utilized in connection with engineered cell to permit detection or selection of cells and, in some cases disclosed herein, also to promote cell elimination and/or cell suicide.

Exemplary surrogate markers can include truncated forms of cell surface polypeptides, such as truncated forms that are non-functional and to not transduce or are not capable of transducing a signal or a signal ordinarily transduced by the full-length form of the cell surface polypeptide, and/or do not or are not capable of internalizing. Exemplary truncated cell surface polypeptides including truncated forms of growth factors or other receptors such as a truncated human epidermal growth factor receptor 2 (tHER2), a truncated epidermal growth factor receptor (tEGFR, exemplary tEGFR sequence set forth in SEQ ID NO: 7 or 16) or a prostate-specific membrane antigen (PSMA) or modified form thereof, such as a truncated PSMA (tPSMA). In some disclosures, tEGFR may contain an epitope recognized by the antibody cetuximab (Erbitux®) or other therapeutic anti-EGFR antibody or binding molecule, which can be used to identify or select cells that have been engineered with the tEGFR construct and an encoded exogenous protein, and/or to eliminate or separate cells expressing the encoded exogenous protein. See U.S. Patent No. 8,802,374 and Liu et al., Nature Biotech. 2016 April; 34(4): 430-434). In some disclosures, the marker, e.g. surrogate marker, includes all or part (e.g., truncated form) of CD34, a NGFR, a CD19 or a truncated CD19, e.g., a truncated non-human CD19. An exemplary polypeptide for a truncated EGFR (*e.g.* tEGFR) comprises the sequence of amino acids set forth in SEQ ID NO: 7 or 16 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 7 or 16.

In some disclosures, the marker is or comprises a detectable protein, such as a fluorescent protein, such as green fluorescent protein (GFP), enhanced green fluorescent protein (EGFP), such as super-fold GFP (sfGFP), red fluorescent protein (RFP), such as tdTomato, mCherry, mStrawberry, AsRed2, DsRed or DsRed2, cyan fluorescent protein (CFP), blue green fluorescent protein (BFP), enhanced blue fluorescent protein (EBFP), and yellow fluorescent protein (YFP), and variants thereof, including species variants, monomeric variants, codon-optimized, stabilized and/or enhanced variants of the fluorescent proteins. In some disclosures, the marker is or comprises an enzyme, such as a luciferase, the lacZ gene from *E. coli,* alkaline phosphatase, secreted embryonic alkaline phosphatase (SEAP), chloramphenicol acetyl transferase (CAT). Exemplary light-emitting reporter genes include luciferase (luc), β-galactosidase, chloramphenicol acetyltransferase (CAT), β-glucuronidase (GUS) or variants thereof. In some disclosures, expression of the enzyme can be detected by addition of a substrate that can be detected upon the expression and functional activity of the enzyme.

In some disclosures, the marker is a selection marker. In some disclosures, the selection marker is or comprises a polypeptide that confers resistance to exogenous agents or drugs. In some disclosures, the selection marker is an antibiotic resistance gene. In some disclosures, the selection marker is an antibiotic resistance gene confers antibiotic resistance to a mammalian cell. In some disclosures, the selection marker is or comprises a Puromycin resistance gene, a Hygromycin resistance gene, a Blasticidin resistance gene, a Neomycin resistance gene, a Geneticin resistance gene or a Zeocin resistance gene or a modified form thereof.

Any of the recombinant receptors and/or the additional polypeptide(s) described herein can be encoded by one or more polynucleotides containing one or more nucleic acid sequences encoding recombinant receptors, in any combinations, orientation or arrangements. For example, one, two, three or more polynucleotides can encode one, two, three or more different polypeptides, e.g., recombinant receptors or portions or components thereof, and/or one or more additional polypeptide(s), e.g., a marker and/or an effector molecule. In some disclosures, one polynucleotide contains a nucleic acid sequence encoding a recombinant receptor, e.g., CAR, or portion or components thereof, and a nucleic acid sequence encoding one or more additional polypeptide(s). In some disclosures, one vector or construct contains a nucleic acid sequence encoding a recombinant receptor, e.g., CAR, or portion or components thereof, and a separate vector or construct contains a nucleic acid sequence encoding one or more additional polypeptide(s). In some disclosures, the nucleic acid sequence encoding the recombinant receptor and the nucleic acid sequence encoding the one or more additional polypeptide(s) are operably linked to two different promoters. In some disclosures, the nucleic acid encoding the recombinant receptor is present upstream of the nucleic acid encoding the one or more additional polypeptide(s). In some disclosures, the nucleic acid encoding the recombinant receptor is present downstream of the nucleic acid encoding one or more additional polypeptide(s).

In certain cases disclosed herein, one polynucleotide contains nucleic acid sequences encode two or more different polypeptide chains, e.g., a recombinant receptor and one or more additional polypeptide(s), e.g., a marker and/or an effector molecule. In some disclosures, the nucleic acid sequences encoding two or more different polypeptide chains, e.g., a recombinant receptor and one or more additional polypeptide(s), are present in two separate polynucleotides. For example, two separate polynucleotides are disclosed, and each can be individually transferred or introduced into the cell for expression in the cell. In some disclosures, the nucleic acid sequences encoding the marker and the nucleic acid sequences encoding the recombinant receptor are present or inserted at different locations within the genome of the cell. In some disclosures, the nucleic acid sequences encoding the marker and the nucleic acid sequences encoding the recombinant receptor are operably linked to two different promoters.

In some disclosures, such as those where the polynucleotide contains a first and second nucleic acid sequence, the coding sequences encoding each of the different polypeptide chains can be operatively linked to a promoter, which can be the same or different. In some disclosures, the nucleic acid molecule can contain a promoter that drives the expression of two or more different polypeptide chains. In some disclosures, such nucleic acid molecules can be multicistronic (bicistronic or tricistronic, see *e.g.,* U.S. Patent No. 6,060,273). In some disclosures, the nucleic acid sequences encoding the recombinant receptor and the nucleic acid sequences encoding the one or more additional polypeptide(s) are operably linked to the same promoter and are optionally separated by an internal ribosome entry site (IRES), or a nucleic acid encoding a self-cleaving peptide or a peptide that causes ribosome skipping, such as a 2A element. For example, an exemplary marker, and optionally a ribosome skipping sequence sequence, can be any as disclosed in PCT Pub. No. WO2014031687.

In some disclosures, transcription units can be engineered as a bicistronic unit containing an IRES, which allows coexpression of gene products (*e.g.* encoding the recombinant receptor and the additional polypeptide) by a message from a single promoter. Alternatively, in some cases disclosed herein, a single promoter may direct expression of an RNA that contains, in a single open reading frame (ORF), two or three genes (e.g. encoding the marker and encoding the recombinant receptor) separated from one another by sequences encoding a self-cleavage peptide (e.g., 2A sequences) or a protease recognition site (e.g., furin). The ORF thus encodes a single polypeptide, which, either during (in the case of 2A) or after translation, is processed into the individual proteins. In some cases disclosed herein, the peptide, such as a T2A, can cause the ribosome to skip (ribosome skipping) synthesis of a peptide bond at the C-terminus of a 2A element, leading to separation between the end of the 2A sequence and the next peptide downstream (see, e.g., de Felipe, Genetic Vaccines and Ther. 2:13 (2004) and de Felipe et al. Traffic 5:616-626 (2004)). Various 2A elements are known. Examples of 2A sequences that can be used in the methods and system disclosed herein, without limitation, 2A sequences from the foot-and-mouth disease virus (F2A, *e.g.,* SEQ ID NO: 21), equine rhinitis A virus (E2A, *e.g.,* SEQ ID NO: 20), Thosea asigna virus (T2A, *e.g.,* SEQ ID NO: 6 or 17), and porcine teschovirus-1 (P2A, *e.g.,* SEQ ID NO: 18 or 19) as described in U.S. Patent Pub. No. 20070116690.

In some disclosures, the polynucleotide encoding the recombinant receptor and/or additional polypeptide is contained in a vector or can be cloned into one or more vector(s). In some disclosures, the one or more vector(s) can be used to transform or transfect a host cell, e.g., a cell for engineering. Exemplary vectors include vectors designed for introduction, propagation and expansion or for expression or both, such as plasmids and viral vectors. In some disclosures, the vector is an expression vector, e.g., a recombinant expression vector. In some disclosures, the recombinant expression vectors can be prepared using standard recombinant DNA techniques.

In some disclosures, the vector can be a vector of the pUC series (Fermentas Life Sciences), the pBluescript series (Stratagene, LaJolla, Calif.), the pET series (Novagen, Madison, Wis.), the pGEX series (Pharmacia Biotech, Uppsala, Sweden), or the pEX series (Clontech, Palo Alto, Calif.). In some cases disclosed herein, bacteriophage vectors, such as λG10, λGT11, λZapII (Stratagene), λEMBL4, and λNM1149, also can be used. In some disclosures, plant expression vectors can be used and include pBI01, pBI101.2, pBI101.3, pBI121 and pBIN19 (Clontech). In some disclosures, animal expression vectors include pEUK-Cl, pMAM and pMAMneo (Clontech).

In some disclosures, the vector is a viral vector, such as a retroviral vector. In some disclosures, the polynucleotide encoding the recombinant receptor and/or additional polypeptide(s) are introduced into the cell via retroviral or lentiviral vectors, or via transposons (see, e.g., Baum et al. (2006) Molecular Therapy: The Journal of the American Society of Gene Therapy. 13:1050-1063; Frecha et al. (2010) Molecular Therapy 18:1748-1757; and Hackett et al. (2010) Molecular Therapy 18:674-683).

In some disclosures, one or more polynucleotide(s) are introduced into cells using recombinant infectious virus particles, such as, e.g., vectors derived from simian virus 40 (SV40), adenoviruses, adeno-associated virus (AAV). In some disclosures, one or more polynucleotide(s) are introduced into T cells using recombinant lentiviral vectors or retroviral vectors, such as gamma-retroviral vectors (see, e.g., Koste et al. (2014) Gene Therapy 2014 Apr 3. doi: 10.1038/gt.2014.25; Carlens et al. (2000) Exp Hematol 28(10): 1137-46; Alonso-Camino et al. (2013) Mol Ther Nucl Acids 2, e93; Park et al., Trends Biotechnol. 2011 November 29(11): 550-557.

In some disclosures, the vector is a retroviral vector. In some disclosures, a retroviral vector has a long terminal repeat sequence (LTR), e.g., a retroviral vector derived from the Moloney murine leukemia virus (MoMI,V), myeloproliferative sarcoma virus (MPSV), murine embryonic stem cell virus (MESV), murine stem cell virus (MSCV), spleen focus forming virus (SFFV), or adeno-associated virus (AAV). Most retroviral vectors are derived from murine retroviruses. In some disclosures, the retroviruses include those derived from any avian or mammalian cell source. The retroviruses typically are amphotropic, meaning that they are capable of infecting host cells of several species, including humans. In one disclosure, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989) BioTechniques 7:980-990; Miller, A. D. (1990) Human Gene Therapy 1:5-14; Scarpa et al. (1991) Virology 180:849-852; Burns et al. (1993) Proc. Natl. Acad. Sci. USA 90:8033-8037; and Boris-Lawrie and Temin (1993) Cur. Opin. Genet. Develop. 3: 102-109.

Methods of lentiviral transduction are known. Exemplary methods are described in, e.g., Wang et al. (2012) J. Immunother. 35(9): 689-701; Cooper et al. (2003) Blood. 101:1637-1644; Verhoeyen et al. (2009) Methods Mol Biol. 506: 97-114; and Cavalieri et al. (2003) Blood. 102(2): 497-505. In some disclosures, the polynucleotide encoding the recombinant receptor and/or one or more additional polypeptide(s), is introduced into a population containing cultured cells, such as by retroviral transduction, transfection, or transformation.

In some disclosures, one or more polynucleotide(s) are introduced into a T cell using electroporation (see, e.g., Chicaybam et al, (2013) PLoS ONE 8(3): e60298 and Van Tedeloo et al. (2000) Gene Therapy 7(16): 1431-1437). In some disclosures, recombinant nucleic acids are transferred into T cells via transposition (see, e.g., Manuri et al. (2010) Hum Gene Ther 21(4): 427-437; Sharma et al. (2013) Molec Ther Nucl Acids 2, e74; and Huang et al. (2009) Methods Mol Biol 506: 115-126). Other methods of introducing and expressing genetic material, e.g., polynucleotides and/or vectors, into immune cells include calcium phosphate transfection (e.g., as described in Current Protocols in Molecular Biology, John Wiley & Sons, New York. N.Y.), protoplast fusion, cationic liposome-mediated transfection; tungsten particle-facilitated microparticle bombardment (Johnston, Nature, 346: 776-777 (1990)); and strontium phosphate DNA co-precipitation (Brash et al., Mol. Cell Biol., 7: 2031-2034 (1987) and other approaches described in, e.g., International Pat. App. Pub. No. WO 2014055668, and U.S. Patent No. 7,446,190.

In some disclosures, the one or more polynucleotide(s) or vector(s) encoding a recombinant receptor and/or additional polypeptide(s) may be introduced into cells, e.g., T cells, either during or after expansion. This introduction of the polynucleotide(s) or vector(s) can be carried out with any suitable retroviral vector, for example. Resulting genetically engineered cells can then be liberated from the initial stimulus (e.g., anti-CD3/anti-CD28 stimulus) and subsequently be stimulated with a second type of stimulus (e.g., via a de novo introduced recombinant receptor). This second type of stimulus may include an antigenic stimulus in form of a peptide/MHC molecule, the cognate (cross-linking) ligand of the genetically introduced receptor (e.g. natural antigen and/or ligand of a CAR) or any ligand (such as an antibody) that directly binds within the framework of the new receptor (e.g. by recognizing constant regions within the receptor). See, for example, Cheadle et al, "Chimeric antigen receptors for T-cell based therapy" Methods Mol Biol. 2012; 907:645-66 or Barrett et al., Chimeric Antigen Receptor Therapy for Cancer Annual Review of Medicine Vol. 65: 333-347 (2014).

In some cases disclosed herein, a vector may be used that does not require that the cells, e.g., T cells, are activated. In some such instances, the cells may be selected and/or transduced prior to activation. Thus, the cells may be engineered prior to, or subsequent to culturing of the cells, and in some cases disclosed herein, at the same time as or during at least a portion of the culturing.

## C. Cells and Preparation of Cells for Genetic Engineering

In some disclosures, disclosed are engineered cells, e.g., genetically engineered or modified cells, and methods of engineering cells. In some disclosures, one or more polynucleotides, e.g., encoding a recombinant receptor and/or additional polypeptide(s), such as any described herein, are introduced into one a cell for engineering. In some disclosures, the polynucleotides and/or portions thereof are heterologous, i.e., normally not present in a cell or sample obtained from the cell, such as one obtained from another organism or cell, which for example, is not ordinarily found in the cell being engineered and/or an organism from which such cell is derived. In some disclosures, the nucleic acid sequences are not naturally occurring, such as a nucleic acid sequences not found in nature or is modified from a nucleic acid sequence found in nature, including one comprising chimeric combinations of nucleic acids encoding various domains from multiple different cell types.

The cells generally are eukaryotic cells, such as mammalian cells, and typically are human cells. In some disclosures, the cells are derived from the blood, bone marrow, lymph, or lymphoid organs, are cells of the immune system, such as cells of the innate or adaptive immunity, *e.g.,* myeloid or lymphoid cells, including lymphocytes, typically T cells and/or NK cells. Other exemplary cells include stem cells, such as multipotent and pluripotent stem cells, including induced pluripotent stem cells (iPSCs). The cells typically are primary cells, such as those isolated directly from a subject and/or isolated from a subject and frozen. In some disclosures, the cells include one or more subsets of T cells or other cell types, such as whole T cell populations, CD4+ cells, CD8+ cells, and subpopulations thereof, such as those defined by function, activation state, maturity, potential for differentiation, expansion, recirculation, localization, and/or persistence capacities, antigen-specificity, type of antigen receptor, presence in a particular organ or compartment, marker or cytokine secretion profile, and/or degree of differentiation. With reference to the subject to be treated, the cells may be allogeneic and/or autologous. Among the methods include off-the-shelf methods. In some disclosures, such as for off-the-shelf technologies, the cells are pluripotent and/or multipotent, such as stem cells, such as iPSCs. In some disclosures, the methods include isolating cells from the subject, preparing, processing, culturing, and/or engineering them, and re-introducing them into the same subject, before or after cryopreservation.

Among the sub-types and subpopulations of T cells and/or of CD4+ and/or of CD8+ T cells are naive T ($T_N$) cells, effector T cells ($T_{EFF}$), memory T cells and sub-types thereof, such as stem cell memory T ($T_{SCM}$), central memory T ($T_{CM}$), effector memory T ($T_{EM}$), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, alpha/beta T cells, and delta/gamma T cells.

In some disclosures, the cells are natural killer (NK) cells. In some disclosures, the cells are monocytes or granulocytes, e.g., myeloid cells, macrophages, neutrophils, dendritic cells, mast cells, eosinophils, and/or basophils.

In some disclosures, the cells include one or more nucleic acids introduced via genetic engineering, and thereby express recombinant or genetically engineered products of such nucleic acids. In some disclosures, the nucleic acids are heterologous, i.e., normally not present in a cell or sample obtained from the cell, such as one obtained from another organism or cell, which for example, is not ordinarily found in the cell being engineered and/or an organism from which such cell is derived. In some disclosures, the nucleic acids are not naturally occurring, such as a nucleic acid not found in nature, including one comprising chimeric combinations of nucleic acids encoding various domains from multiple different cell types.

In some disclosures, preparation of the engineered cells includes one or more culture and/or preparation steps. The cells for introduction of the nucleic acid encoding the transgenic receptor such as the CAR, may be isolated from a sample, such as a biological sample, e.g., one obtained from or derived from a subject. In some disclosures, the subject from which the cell is isolated is one having the disease or condition or in need of a cell therapy or to which cell therapy will be administered. The subject in some disclosures is a human in need of a particular therapeutic intervention, such as the adoptive cell therapy for which cells are being isolated, processed, and/or engineered.

Accordingly, the cells in some disclosures are primary cells, e.g., primary human cells. The samples include tissue, fluid, and other samples taken directly from the subject, as well as samples resulting from one or more processing steps, such as separation, centrifugation, genetic engineering (e.g. transduction with viral vector), washing, and/or incubation. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some disclosures, the sample from which the cells are derived or isolated is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In some disclosures, the cells are derived from cell lines, e.g., T cell lines. The cells in some disclosures are obtained from a xenogeneic source, for example, from mouse, rat, non-human primate, and pig.

In some disclosures, isolation of the cells includes one or more preparation and/or non-affinity based cell separation steps. In some examples disclosed herein, cells are washed, centrifuged, and/or incubated in the presence of one or more reagents, for example, to remove unwanted components, enrich for desired components, lyse or remove cells sensitive to particular reagents. In some examples disclosed herein, cells are separated based on one or more property, such as density, adherent properties, size, sensitivity and/or resistance to particular components.

In some examples disclosed herein, cells from the circulating blood of a subject are obtained, e.g., by apheresis or leukapheresis. The samples, in some disclosures, contain lymphocytes, including T cells, monocytes, granulocytes, B cells, other nucleated white blood cells, red blood cells, and/or platelets, and in some disclosures contains cells other than red blood cells and platelets.

In some disclosures, the blood cells collected from the subject are washed, e.g., to remove the plasma fraction and to place the cells in an appropriate buffer or media for subsequent processing steps. In some disclosures, the cells are washed with phosphate buffered saline (PBS). In some disclosures, the wash solution lacks calcium and/or magnesium and/or many or all divalent cations. In some disclosures, a washing step is accomplished a semi-automated "flow-through" centrifuge (for example, the Cobe 2991 cell processor, Baxter) according to the manufacturer's instructions. In some disclosures, a washing step is accomplished by tangential flow filtration (TFF) according to the manufacturer's instructions. In some disclosures, the cells are resuspended in a variety of biocompatible buffers after washing, such as, for example, Ca$^{++}$/Mg$^{++}$ free PBS. In certain disclosures, components of a blood cell sample are removed and the cells directly resuspended in culture media.

In some disclosures, the methods include density-based cell separation methods, such as the preparation of white blood cells from peripheral blood by lysing the red blood cells and centrifugation through a Percoll or Ficoll gradient.

In some disclosures, the isolation methods include the separation of different cell types based on the expression or presence in the cell of one or more specific molecules, such as surface markers, e.g., surface proteins, intracellular markers, or nucleic acid. In some disclosures, any known method for separation based on such markers may be used. In some disclosures, the separation is affinity- or immunoaffinity-based separation. For example, the isolation in some disclosures includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

Such separation steps can be based on positive selection, in which the cells having bound the reagents are retained for further use, and/or negative selection, in which the cells having not bound to the antibody or binding partner are retained. In some examples disclosed herein, both fractions are retained for further use. In some disclosures, negative selection can be particularly useful where no antibody is available that specifically identifies a cell type in a heterogeneous population, such that separation is best carried out based on markers expressed by cells other than the desired population.

The separation need not result in 100% enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells.

In some examples disclosed herein, multiple rounds of separation steps are carried out, where the positively or negatively selected fraction from one step is subjected to another separation step, such as a subsequent positive or negative selection. In some examples disclosed herein, a single separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types.

For example, in some disclosures, specific subpopulations of T cells, such as cells positive or expressing high levels of one or more surface markers, e.g., CD28$^+$, CD62L$^+$, CCR7$^+$, CD27$^+$, CD127$^+$, CD4$^+$, CD8$^+$, CD45RA$^+$, and/or CD45RO$^+$ T cells, are isolated by positive or negative selection techniques.

For example, CD3$^+$, CD28$^+$ T cells can be positively selected using anti-CD3/anti-CD28 conjugated magnetic beads (e.g., DYNABEADS$^®$ M-450 CD3/CD28 T Cell Expander).

In some disclosures, isolation is carried out by enrichment for a particular cell population by positive selection, or depletion of a particular cell population, by negative selection. In some disclosures, positive or negative selection is accomplished by incubating cells with one or more antibodies or other binding agent that specifically bind to one or more surface markers expressed or expressed (marker$^+$) at a relatively higher level (marker$^{high}$) on the positively or negatively selected cells, respectively.

In some disclosures, T cells are separated from a PBMC sample by negative selection of markers expressed on non-T cells, such as B cells, monocytes, or other white blood cells, such as CD14. In some disclosures, a CD4$^+$ or CD8$^+$ selection step is used to separate CD4$^+$ helper and CD8$^+$ cytotoxic T cells. Such CD4$^+$ and CD8$^+$ populations can be further sorted into sub-populations by positive or negative selection for markers expressed or expressed to a relatively higher degree on one or more naive, memory, and/or effector T cell subpopulations.

In some disclosures, CD8$^+$ cells are further enriched for or depleted of naive, central memory, effector memory, and/or central memory stem cells, such as by positive or negative selection based on surface antigens associated with the respective subpopulation. In some disclosures, enrichment for central memory T (T$_{CM}$) cells is carried out to increase efficacy, such as to improve long-term survival, expansion, and/or engraftment following administration, which in some disclosures is particularly robust in such sub-populations. *See* Terakura et al. (2012) Blood. 1:72-82; Wang et al. (2012) J Immunother. 35(9):689-701. In some disclosures, combining T$_{CM}$-enriched CD8$^+$ T cells and CD4$^+$T cells further enhances efficacy.

In disclosures, memory T cells are present in both CD62L$^+$ and CD62L$^-$ subsets of CD8$^+$ peripheral blood lymphocytes. PBMC can be enriched for or depleted of CD62L$^-$CD8$^+$ and/or CD62L$^+$CD8$^+$ fractions, such as using anti-CD8 and anti-CD62L antibodies.

In some disclosures, the enrichment for central memory T (T$_{CM}$) cells is based on positive or high surface expression of CD45RO, CD62L, CCR7, CD28, CD3, and/or CD127; in some disclosures, it is based on negative selection for cells expressing or highly expressing CD45RA and/or granzyme B. In some disclosures, the enrichment for central memory T (T$_{CM}$) cells is based on positive or high surface expression of CD45RO, CD62L, CCR7, CD28, CD3, CD27, and/or CD127. In some disclosures, the enrichment for central memory T (T$_{CM}$) cells is based on positive or high surface expression of CD62L, CCR7, CD28, and/or CD27. In some disclosures, the enrichment for central memory T (T$_{CM}$) cells is based on positive or high surface expression of CCR7, CD28, and/or CD27. In some disclosures, the enrichment for central memory T (T$_{CM}$) cells is based on positive or high surface expression of CD28 and CD27. In some disclosures, isolation of a CD8$^+$ population enriched for T$_{CM}$ cells is carried out by depletion of cells expressing CD4, CD14, CD45RA, and positive selection or enrichment for cells expressing CD62L. In some disclosures, isolation of a CD8$^+$ population enriched for T$_{CM}$ cells is carried out by depletion of cells expressing CD4, CD14, CD45RA, and positive selection or enrichment for cells expressing CD27 and CD28. In one disclosure, enrichment for central memory T (T$_{CM}$) cells is carried out starting with a negative fraction of cells selected based on CD4 expression, which is subjected to a negative selection based on expression of CD14 and CD45RA, and a positive selection based on CD62L. Such selections in some disclosures are carried out simultaneously and in other disclosures are carried out sequentially, in either order. In some disclosures, the same CD4 expression-based selection step used in preparing the CD8$^+$ cell population or sub-population, also is used to generate the CD4$^+$ cell population or subpopulation, such that both the positive and negative fractions from the CD4-based separation are retained and used in subsequent steps of the methods, optionally following one or more further positive or negative selection steps.

In a particular example disclosed herein, a sample of PBMCs or other white blood cell sample is subjected to selection of CD4$^+$ cells, where both the negative and positive fractions are retained. The negative fraction then is subjected to negative selection based on expression of CD14 and CD45RA or CD19, and positive selection based on a marker characteristic of central memory T cells, such as CD62L or CCR7, where the positive and negative selections are carried out in either order.

CD4$^+$ T helper cells are sorted into naïve, central memory, and effector cells by identifying cell populations that have cell surface antigens. CD4$^+$ lymphocytes can be obtained by standard methods. In some disclosures, naive CD4$^+$ T lymphocytes are CD45RO$^-$, CD45RA$^+$, CD62L$^+$, CD4$^+$ T cells. In some disclosures, central memory CD4$^+$ cells are CD62L$^+$ and CD45RO$^+$. In some disclosures, effector CD4$^+$ cells are CD62L$^-$ and CD45RO$^-$.

In one example disclosed herein, to enrich for CD4$^+$ cells by negative selection, a monoclonal antibody cocktail typically includes antibodies to CD14, CD20, CD11b, CD16, HLA-DR, and CD8. In some disclosures, the antibody or binding partner is bound to a solid support or matrix, such as a magnetic bead or paramagnetic bead, to allow for separation of cells for positive and/or negative selection. For example, in some disclosures, the cells and cell populations are separated or isolated using immunomagnetic (or affinitymagnetic) separation techniques (reviewed in Methods in Molecular Medicine, vol. 58: Metastasis Research Protocols, Vol. 2: Cell Behavior In Vitro and In Vivo, p 17-25 Edited by: S. A. Brooks and U. Schumacher © Humana Press Inc., Totowa, NJ).

In some disclosures, the sample or population of cells to be separated is incubated with small, magnetizable or magnetically responsive material, such as magnetically responsive particles or microparticles, such as paramagnetic beads (e.g., such as Dynabeads or MACS beads). The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select.

In some disclosures, the magnetic particle or bead comprises a magnetically responsive material bound to a specific binding member, such as an antibody or other binding partner. There are many well-known magnetically responsive materials used in magnetic separation methods. Suitable magnetic particles include those described in Molday, U.S. Pat. No. 4,452,773, and in European Patent Specification EP 452342 B. Colloidal sized particles, such as those described in Owen U.S. Pat. No. 4,795,698, and Liberti et al., U.S. Pat. No. 5,200,084 are other examples.

The incubation generally is carried out under conditions whereby the antibodies or binding partners, or molecules, such as secondary antibodies or other reagents, which specifically bind to such antibodies or binding partners, which are attached to the magnetic particle or bead, specifically bind to cell surface molecules if present on cells within the sample.

In some disclosures, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some disclosures, a combination of positive and negative selection is performed during the same selection step, where the positive and negative fractions are retained and further processed or subject to further separation steps.

In certain disclosures, the magnetically responsive particles are coated in primary antibodies or other binding partners, secondary antibodies, lectins, enzymes, or streptavidin. In certain disclosures, the magnetic particles are attached to cells via a coating of primary antibodies specific for one or more markers. In certain disclosures, the cells, rather than the beads, are labeled with a primary antibody or binding partner, and then cell-type specific secondary antibody- or other binding partner (e.g., streptavidin)-coated magnetic particles, are added. In certain disclosures, streptavidin-coated magnetic particles are used in conjunction with biotinylated primary or secondary antibodies.

In some disclosures, the magnetically responsive particles are left attached to the cells that are to be subsequently incubated, cultured and/or engineered; in some disclosures, the particles are left attached to the cells for administration to a patient. In some disclosures, the magnetizable or magnetically responsive particles are removed from the cells. Methods for removing magnetizable particles from cells are known and include, e.g., the use of competing non-labeled antibodies, and magnetizable particles or antibodies conjugated to cleavable linkers. In some disclosures, the magnetizable particles are biodegradable.

In some disclosures, the affinity-based selection is via magnetic-activated cell sorting (MACS) (Miltenyi Biotec, Auburn, CA). Magnetic Activated Cell Sorting (MACS) systems are capable of high-purity selection of cells having magnetized particles attached thereto. In certain disclosures, MACS operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain disclosures, the non-target cells are labelled and depleted from the heterogeneous population of cells.

In certain disclosures, the isolation or separation is carried out using a system, device, or apparatus that carries out one or more of the isolation, cell preparation, separation, processing, incubation, culture, and/or formulation steps of the methods. In some disclosures, the system is used to carry out each of these steps in a closed or sterile environment, for example, to minimize error, user handling and/or contamination. In one example disclosed herein, the system is a system as described in International Pat. App. Pub. No. WO2009/072003 or US 20110003380.

In some disclosures, the system or apparatus carries out one or more, e.g., all, of the isolation, processing, engineering, and formulation steps in an integrated or self-contained system, and/or in an automated or programmable fashion. In some disclosures, the system or apparatus includes a computer and/or computer program in communication with the system or apparatus, which allows a user to program, control, assess the outcome of, and/or adjust various disclosures of the processing, isolation, engineering, and formulation steps.

In some disclosures, the separation and/or other steps is carried out using CliniMACS system (Miltenyi Biotec), for example, for automated separation of cells on a clinical-scale level in a closed and sterile system. Components can include an integrated microcomputer, magnetic separation unit, peristaltic pump, and various pinch valves. The integrated computer in some disclosures controls all components of the instrument and directs the system to perform repeated procedures in a standardized sequence. The magnetic separation unit in some disclosures includes a movable permanent magnet and a holder for the selection column. The peristaltic pump controls the flow rate throughout the tubing set and, together with the pinch valves, ensures the controlled flow of buffer through the system and continual suspension of cells.

The CliniMACS system in some disclosures uses antibody-coupled magnetizable particles that are supplied in a sterile, non-pyrogenic solution. In some disclosures, after labelling of cells with magnetic particles the cells are washed to remove excess particles. A cell preparation bag is then connected to the tubing set, which in turn is connected to a bag containing buffer and a cell collection bag. The tubing set consists of pre-assembled sterile tubing, including a pre-column and a separation column, and are for single use only. After initiation of the separation program, the system automatically applies the cell sample onto the separation column. Labelled cells are retained within the column, while unlabeled cells are removed by a series of washing steps. In some disclosures, the cell populations for use with the methods described herein are unlabeled and are not retained in the column. In some disclosures, the cell populations for use with the methods described herein are labeled and are retained in the column. In some disclosures, the cell populations for use with the methods described herein are eluted from the column after removal of the magnetic field, and are collected within the cell collection bag.

In certain disclosures, separation and/or other steps are carried out using the CliniMACS Prodigy system (Miltenyi Biotec). The CliniMACS Prodigy system in some disclosures is equipped with a cell processing unity that permits automated washing and fractionation of cells by centrifugation. The CliniMACS Prodigy system can also include an onboard camera and image recognition software that determines the optimal cell fractionation endpoint by discerning the macroscopic layers of the source cell product. For example, peripheral blood is automatically separated into erythrocytes, white blood cells and plasma layers. The CliniMACS Prodigy system can also include an integrated cell cultivation chamber which accomplishes cell culture protocols such as, e.g., cell differentiation and expansion, antigen loading, and long-term cell culture. Input ports can allow for the sterile removal and replenishment of media and cells can be monitored using an integrated microscope. See, e.g., Klebanoff et al. (2012) J Immunother. 35(9): 651-660, Terakura et al. (2012) Blood.1:72-82, and Wang et al. (2012) J Immunother. 35(9):689-701.

In some disclosures, a cell population described herein is collected and enriched (or depleted) via flow cytometry, in which cells stained for multiple cell surface markers are carried in a fluidic stream. In some disclosures, a cell population described herein is collected and enriched (or depleted) via preparative scale (FACS)-sorting. In certain disclosures, a cell population described herein is collected and enriched (or depleted) by use of microelectromechanical systems (MEMS) chips in combination with a FACS-based detection system (see, e.g., WO 2010/033140, Cho et al. (2010) Lab Chip 10, 1567-1573; and Godin et al. (2008) J Biophoton. 1(5):355-376. In both cases, cells can be labeled with multiple markers, allowing for the isolation of well-defined T cell subsets at high purity.

In some disclosures, the antibodies or binding partners are labeled with one or more detectable marker, to facilitate separation for positive and/or negative selection. For example, separation may be based on binding to fluorescently labeled antibodies. In some examples disclosed herein, separation of cells based on binding of antibodies or other binding partners specific for one or more cell surface markers are carried in a fluidic stream, such as by fluorescence-activated cell sorting (FACS), including preparative scale (FACS) and/or microelectromechanical systems (MEMS) chips, e.g., in combination with a flow-cytometric detection system. Such methods allow for positive and negative selection based on multiple markers simultaneously.

In some disclosures, the preparation methods include steps for freezing, e.g., cryopreserving, the cells, either before or after isolation, incubation, and/or engineering. In some disclosures, the freeze and subsequent thaw step removes granulocytes and, to some extent, monocytes in the cell population. In some disclosures, the cells are suspended in a freezing solution, e.g., following a washing step to remove plasma and platelets. Any of a variety of known freezing solutions and parameters in some disclosures may be used. One example disclosed herein involves using PBS containing 20% DMSO and 8% human serum albumin (HSA), or other suitable cell freezing media. This is then diluted 1:1 with media so that the final concentration of DMSO and HSA are 10% and 4%, respectively. The cells are generally then frozen to -80° C. at a rate of 1° per minute and stored in the vapor phase of a liquid nitrogen storage tank.

In some disclosures, the cells are incubated and/or cultured prior to or in connection with genetic engineering. The incubation steps can include culture, cultivation, stimulation, activation, and/or propagation. The incubation and/or engineering may be carried out in a culture vessel, such as a unit, chamber, well, column, tube, tubing set, valve, vial, culture dish, bag, or other container for culture or cultivating cells. In some disclosures, the populations or cells are incubated in the presence of stimulating conditions or a stimulatory agent. Such conditions include those designed to induce proliferation, expansion, activation, and/or survival of cells in the population, to mimic antigen exposure, and/or to prime the cells for genetic engineering, such as for the introduction of a recombinant antigen receptor.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some disclosures, the stimulating conditions or agents include one or more agent, e.g., ligand, which is capable of activating an intracellular signaling domain of a TCR complex. In some disclosures, the agent turns on or initiates TCR/CD3 intracellular signaling cascade in a T cell. Such agents can include antibodies, such as those specific for a TCR, e.g. anti-CD3. In some disclosures, the stimulating conditions include one or more agent, e.g. ligand, which is capable of stimulating a costimulatory receptor, e.g., anti-CD28. In some disclosures, such agents and/or ligands may be, bound to solid support such as a bead, and/or one or more cytokines. Optionally, the expansion method may further comprise the step of adding anti-CD3 and/or anti CD28 antibody to the culture medium (e.g., at a concentration of at least about 0.5 ng/ml). In some disclosures, the stimulating agents include IL-2, IL-15 and/or IL-7. In some disclosures, the IL-2 concentration is at least about 10 units/mL.

In some disclosures, incubation is carried out in accordance with techniques such as those described in US Patent No. 6,040,177, Klebanoff et al. (2012) J Immunother. 35(9): 651-660, Terakura et al. (2012) Blood.1:72-82, and/or Wang et al. (2012) J Immunother. 35(9):689-701.

In some disclosures, the T cells are expanded by adding to a culture-initiating population feeder cells, such as non-dividing peripheral blood mononuclear cells (PBMC), (e.g., such that the resulting population of cells contains at least about 5, 10, 20, or 40 or more PBMC feeder cells for each T lymphocyte in the initial population to be expanded); and incubating the culture (e.g. for a time sufficient to expand the numbers of T cells). In some disclosures, the non-dividing feeder cells can comprise gamma-irradiated PBMC feeder cells. In some disclosures, the PBMC are irradiated with gamma rays in the range of about 3000 to 3600 rads to prevent cell division. In some disclosures, the feeder cells are added to culture medium prior to the addition of the populations of T cells.

In some disclosures, the stimulating conditions include temperature suitable for the growth of human T lymphocytes, for example, at least about 25 degrees Celsius, generally at least about 30 degrees, and generally at or about 37 degrees Celsius. Optionally, the incubation may further comprise adding non-dividing EBV-transformed lymphoblastoid cells (LCL) as feeder cells. LCL can be irradiated with gamma rays in the range of about 6000 to 10,000 rads. The LCL feeder cells in some disclosures is provided in any suitable amount, such as a ratio of LCL feeder cells to initial T lymphocytes of at least about 10:1.

In disclosures, antigen-specific T cells, such as antigen-specific CD4+ and/or CD8+ T cells, are obtained by stimulating naive or antigen specific T lymphocytes with antigen. For example, antigen-specific T cell lines or clones can be generated to cytomegalovirus antigens by isolating T cells from infected subjects and stimulating the cells in vitro with the same antigen.


## V. COMPOSITIONS AND FORMULATIONS

In some examples, disclosed herein are therapeutic compositions (e.g., therapeutic T cell compositions) generated by any of the manufacturing processes disclosed herein, e.g., an output composition containing engineered (recombinant receptor-expressing) T cells. In some examples, disclosed herein are therapeutic compositions (e.g., therapeutic T cell compositions) having any one or more of the features disclosed herein, e.g., in Section III-F. In some disclosures, the dose of cells comprising cells engineered with a recombinant antigen receptor, e.g. CAR or TCR, is disclosed as a composition or formulation, such as a pharmaceutical composition or formulation. Such compositions can be used in accord with the disclosed methods, and/or with the disclosed articles of manufacture or compositions, such as in the prevention or treatment of diseases, conditions, and disorders, or in detection, diagnostic, and prognostic methods.

In come embodimetns, the therapeutic T cell composition contains CD4+ T cells expressing a recombinant receptor and CD8+ T cells expressing a recombinant receptor, wherein at least 80% or of the total receptor+/CD8+ cells in the composition are CD57- and at least 80% of the total receptor+/CD4+ cells in the composition are CD57-. In some disclosures, the therapeutic T cell composition is one in which at least or at least about 80%, at least or at least about 85%, at least or at least about 90%, at least or at least about 95%, at least or at least about 96%, at least or at least about 97%, at least or at least about 98%, at least or at least about 99%, about 100%, or 100% of the cells in the composition are CD4+ T cells and CD8+ T cells.

In some disclosures, the therapeutic T cell composition includes CD3+ T cells expressing a recombinant receptor, wherein at least 80% or of the total receptor+/CD3+ cells in the composition are CD57-. In some disclosures, the therapeutic T cell compositionis one in which at least or at least about 80%, at least or at least about 85%, at least or at least about 90%, at least or at least about 95%, at least or at least about 96%, at least or at least about 97%, at least or at least about 98%, at least or at least about 99%, about 100%, or 100% of the cells in the composition are CD3+ T cells.

In some disclosures, the therapeutic T cell composition contains a defined ratio or of CD4 and CD8 T cells. In some disclosures, the ratio of receptor+/CD4+ T cells to receptor+/CD8+ T cells in the composition is between about 1:3 and about 3: 1, such as is at or about 1:1.

In some disclosures, the recombinant receptor is any as described in Section IVA. In some disclosures, the recombinant receptor is capable of binding to a target protein that is associated with, specific to, and/or expressed on a cell or tissue of a disease, disorder or condition. In some disclosures, the recombinant receptor is a chimeric antigen receptor (CAR).

In some disclosures, the number of viable T cells in a disclosed therapeutic composition is between at or about $10 \times 106$ cells and at or about $200 \times 106$ cells. In some disclosures, number of viable T cells in a disclosed therapeutic composition is between at or about $10 \times 106$ cells and at or about $100 \times 106$ cells. In some disclosures, number of viable T cells in a disclosed therapeutic composition is between at or about $10 \times 106$ cells and at or about $70 \times 106$ cells. In some disclosures, number of viable T cells in a disclosed therapeutic composition is between at or about $10 \times 106$ cells and at or about $50 \times 106$ cells. In some disclosures, the number of viable T cells in a disclosed therapeutic composition is between at or about $50 \times 106$ cells and at or about $200 \times 106$ cells. In some disclosures, the number of viable T cells in a disclosed therapeutic composition is between at or about $50 \times 106$ cells and at or about $100 \times 106$ cells. In some disclosures, the number of viable T cells in a disclosed therapeutic composition is between at or about $50 \times 106$ cells and at or about $70 \times 106$ cells. In some disclosures, the number of viable T cells in a disclosed therapeutic composition is between at or about $70 \times 106$ cells and at or about $200 \times 106$ cells. In some disclosures, the number of viable T cells in a disclosed therapeutic composition is between at or about $70 \times 106$ cells and at or about $100 \times 106$ cells. In some disclosures, the number of viable T cells in a disclosed therapeutic composition is between at or about $100 \times 106$ cells and at or about $200 \times 106$ cells. In some disclosures, the volume of the composition is between 1.0 mL and 10 mL. In some disclosures, the volume is at or about 2 mL, at or about 3 mL, at or about 4 mL, at or about 5 mL, at or about 6 mL, at or about 7 mL, at or about 8 mL, at or about 9 mL, or at or about 10 mL, or any value between any of the foregoing.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In some disclosures, the choice of carrier is determined in part by the particular cell or agent and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some disclosures, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffering agents in some disclosures are included in the compositions. Suitable buffering agents include, for example, citric acid, sodium citrate, phosphoric acid, potassium phosphate, and various other acids and salts. In some disclosures, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulation or composition may also contain more than one active ingredient useful for the particular indication, disease, or condition being prevented or treated with the cells or agents, where the respective activities do not adversely affect one another. Such active ingredients are suitably present in combination in amounts that are effective for the purpose intended. Thus, in some disclosures, the pharmaceutical composition further includes other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc. In some disclosures, the agents or cells are administered in the form of a salt, e.g., a pharmaceutically acceptable salt. Suitable pharmaceutically acceptable acid addition salts include those derived from mineral acids, such as hydrochloric, hydrobromic, phosphoric, metaphosphoric, nitric, and sulphuric acids, and organic acids, such as tartaric, acetic, citric, malic, lactic, fumaric, benzoic, glycolic, gluconic, succinic, and arylsulphonic acids, for example, p-toluenesulphonic acid.

The pharmaceutical composition in some disclosures contains agents or cells in amounts effective to treat or prevent the disease or condition, such as a therapeutically effective or prophylactically effective amount. Therapeutic or prophylactic efficacy in some disclosures is monitored by periodic assessment of treated subjects. For repeated administrations over several days or longer, depending on the condition, the treatment is repeated until a desired suppression of disease symptoms occurs. However, other dosage regimens may be useful and can be determined. The desired dosage can be delivered by a single bolus administration of the composition, by multiple bolus administrations of the composition, or by continuous infusion administration of the composition.

The agents or cells can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some disclosures, they are administered by parenteral, intrapulmonary, and intranasal, and, if desired for local treatment, intralesional administration. Parenteral infusions include intramuscular, intravenous, intraarterial, intraperitoneal, or subcutaneous administration. In some disclosures, a given dose is administered by a single bolus administration of the cells or agent. In some disclosures, it is administered by multiple bolus administrations of the cells or agent, for example, over a period of no more than 3 days, or by continuous infusion administration of the cells or agent.

For the prevention or treatment of disease, the appropriate dosage may depend on the type of disease to be treated, the type of agent or agents, the type of cells or recombinant receptors, the severity and course of the disease, whether the agent or cells are administered for preventive or therapeutic purposes, previous therapy, the subject's clinical history and response to the agent or the cells, and the discretion of the attending physician. The compositions are in some disclosures suitably administered to the subject at one time or over a series of treatments.

The cells or agents may be administered using standard administration techniques, formulations, and/or devices. Disclosed are formulations and devices, such as syringes and vials, for storage and administration of the compositions. With respect to cells, administration can be autologous or heterologous. For example, immunoresponsive cells or progenitors can be obtained from one subject, and administered to the same subject or a different, compatible subject. Peripheral blood derived immunoresponsive cells or their progeny (e.g., in vivo, ex vivo or in vitro derived) can be administered via localized injection, including catheter administration, systemic injection, localized injection, intravenous injection, or parenteral administration. When administering a therapeutic composition (e.g., a pharmaceutical composition containing a genetically modified immunoresponsive cell or an agent that treats or ameliorates symptoms of neurotoxicity), it will generally be formulated in a unit dosage injectable form (solution, suspension, emulsion).

Formulations include those for oral, intravenous, intraperitoneal, subcutaneous, pulmonary, transdermal, intramuscular, intranasal, buccal, sublingual, or suppository administration. In some disclosures, the agent or cell populations are administered parenterally. The term "parenteral," as used herein, includes intravenous, intramuscular, subcutaneous, rectal, vaginal, and intraperitoneal administration. In some disclosures, the agent or cell populations are administered to a subject using peripheral systemic delivery by intravenous, intraperitoneal, or subcutaneous injection.

Compositions in some disclosures are disclosed as sterile liquid preparations, e.g., isotonic aqueous solutions, suspensions, emulsions, dispersions, or viscous compositions, which may in some disclosures be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol) and suitable mixtures thereof.

Sterile injectable solutions can be prepared by incorporating the agent or cells in a solvent, such as in admixture with a suitable carrier, diluent, or excipient such as sterile water, physiological saline, glucose, dextrose, or the like.

The formulations to be used for in vivo administration are generally sterile. Sterility may be readily accomplished, e.g., by filtration through sterile filtration membranes.

## VI. METHODS OF TREATMENT

Disclosed herein are methods of treatment, e.g., including administering any of the engineered cells or compositions containing engineered cells described herein. In some disclosures, also disclosed are methods of administering any of the engineered cells or compositions containing engineered cells described herein to a subject, such as a subject that has a disease or disorder. In some disclosures, also disclosed are uses of any of the engineered cells or compositions containing engineered cells described herein for treatment of a disease or disorder. In some disclosures, also disclosed are uses of any of the engineered cells or compositions containing engineered cells described herein for the manufacture of a medicament for the treatment of a disease or disorder. In some disclosures, also disclosed are any of the engineered cells or compositions containing engineered cells described herein, for use in treatment of a disease or disorder, or for administration to a subject having a disease or disorder.

Methods for administration of cells for adoptive cell therapy are known and may be used in connection with the disclosed methods and compositions. For example, adoptive T cell therapy methods are described, e.g., in US Pat. App. Pub. No. 2003/0170238 to Gruenberg et al; US Patent No. 4,690,915 to Rosenberg; Rosenberg (2011) Nat Rev Clin Oncol. 8(10):577-85). See, e.g., Themeli et al. (2013) Nat Biotechnol. 31(10): 928-933; Tsukahara et al. (2013) Biochem Biophys Res Commun 438(1): 84-9; Davila et al. (2013) PLoS ONE 8(4): e61338.

In some disclosures, the subject, e.g., a subject having or suspected of having a disease or disorder, is administered a cell therapy having a low or reduced amount of CD57+ T cells. In particular disclosures, the amount or frequency of CD57+ cells in the cell therapy is measured prior to administration. In certain disclosures the cell therapy has less than or less than about 50%, 40%, 30%, 25%, 20%, 15%, 10%, 5%, or 1% CD57+ T cells. In certain disclosures, the amount or frequency of CD57+ cells in the cell therapy is measured, and the cell therapy is administered to the subject if the cell therapy has less than or less than about 50%, 40%, 30%, 25%, 20%, 15%, 10%, 5%, or 1% CD57+ T cells. In some disclosures, the cell therapy has less than or less than about 25% CD57 T cells. In particular disclosures, the cell therapy has less than or less than about 10% CD57 T cells. In certain disclosures, the cell therapy has less than or less than about a threshold amount of CD57+ T cells.

In particular disclosures, the subject, e.g., a subject having or suspected of having a disease or disorder, is administered a cell therapy having a low or reduced amount of T cells positive for CD57 expression or a low or reduced amount of a trait associated CD57 expression. In some disclosures, the trait associated with CD57 expression is measured in cells of a cell therapy prior to administering the cell therapy to a subject. In particular, disclosures, the trait associated with CD57 expression is measured in the cells therapy, and the cell therapy is administered to the subject if the cell therapy has less than a threshold value of the trait associated with CD57 expression.

In particular disclosures, the threshold value is a predetermined value. In some disclosures, the threshold value is experimentally derived. In some disclosures, the threshold value is an average, median, or mean value of the trait measured in a plurality of cell therapies. In some disclosures, the threshold value is experimentally derived from measurements of a plurality cell therapies, e.g., reference cell therapies. In some disclosures, the reference cell therapies are reference compositions of T cells, e.g., T cell compositions including T cells expressing a recombinant receptor. In particular disclosures, the reference compositions of T cells, e.g., T cells expressing a recombinant receptor, are measured prior to administration to subjects.

In particular disclosures, the reference cell therapies or reference T cell compositions are compositions of the cell therapy comprising T cells, e.g., including T cells expressing a recombinant receptor, from among a group of subjects that went on to receive the cell therapy for treating the same a disease, disorder, or condition. In some disclosures, reference cell therapy or reference T cell compositions are T cell compositions that were administered to subjects that went on to develop a partial response or progressive disease.

In some disclosures, the trait associated with the CD57 expression is a level or amount of CD57 polypeptide present in the total T cells, total CD4+ T cells, or total CD8+ T cells. In particular disclosures, the trait is a level or amount of CD57 polypeptide present in the total T cells, CD4+ T cells, or CD8+ T cells of the dose. In some disclosures, the trait is a level or amount of CD57 polypeptide present on the surface of the total T cells, CD4+ T cells, or CD8+ T cells. In various disclosures, the trait is a frequency, percentage, or amount of CD57+ T cells, CD57+CD4+ T cells, or CD57+CD8+ T cells. In certain disclosures, the trait is a level or amount of CD57 mRNA present in the T cells of the dose. In some disclosures, the trait is a level or amount of accessibility of the gene encoding CD57 (B3GAT1).

In some disclosures, the trait associated with the CD57 expression is a level or amount of CD57 polypeptide present in the total CD3+ T cells. In particular disclosures, the trait is a level or amount of CD57 polypeptide present in the total CD3+ T cells of the dose. In some disclosures, the trait is a level or amount of CD57 polypeptide present on the surface of the total CD3+ T cells. In various disclosures, the trait is a frequency, percentage, or amount of CD57+CD3+ T cells. In certain disclosures, the trait is a level or amount of CD57 mRNA present in the T cells of the dose. In some disclosures, the trait is a level or amount of accessibility of the gene encoding CD57 (B3GAT1).

In particular disclosures, the threshold value is, is about, or is within 75%, 60%, 50%, 40%, 30%, 25%, 20%, 15%, 10%, 5%, 1%, or within less than 1% below an average, mean, or median measurement of the trait associated with CD57 expression in a plurality of reference cell therapies or reference T cell compositions. The amount is below one, one half, one third, one forth, one fifth, one sixth, one eighth, or one tenth of a standard deviation less than the average, mean, or median measurement of the trait. In particular disclosures, the threshold value is below a lowest measurement of the trait associated with CD57 expression in a composition from among a plurality of reference T cell compositions or reference cell therapies. In some disclosures, the threshold is within or within about 50%, 40%, 35%, 30%, 25%, 20%, 15%, 10%, 5%, 1%, or within less than 1% below the lowest measurement of the trait measured among the plurality of reference cell therapies or reference T cell compositions. In certain disclosures, threshold value is below an average, median, or mean measurement of the trait associated with CD57 expression calculated from among a frequency in the reference T cell compositions or reference cell therapies. In some disclosures, the threshold value is the average, median, or mean measurement of, of about, or of at least 25%, 33%, 50%, 60%, 65%, 70%, 75%, 80%, 85%, 90%, or 95% of the measurements taken from a plurality of reference T cell compositions.

In particular disclosures, the trait associated with CD57 expression is measured in the cell therapy prior to administering the cell therapy to a subject. In some disclosures, the measurement is used to assess the risk, probability or likelihood that the subject will not experience a complete response. In certain disclosures, the measurement is used to assess the risk, probability or likelihood that the subject will experience a partial response or a progressive disease outcome following administration of a cell therapy. In particular disclosures, the subject is determined to have an increased risk, likelihood, or probability of failing to experiencing a complete response following the administration of the cell therapy if the value of the trait is greater than a threshold value of the trait. In some disclosures, the subject is determined to have an increased risk, likelihood, or probability of experiencing a partial response or a progressive disease following the administration of the cell therapy if the value of the trait is greater than a threshold value of the trait. In some disclosures, the threshold value is any threshold value of the trait associated with CD57 described herein.

In some disclosures, subjects considered to have an increased risk of failing to achieve a complete response following administration of a cell therapy go on to achieve a complete response with a frequency of less than or less than about 50%, 40%, 30%, 25%, 20%, 15% 10%, or less than 10% following administration of a dose of the cell therapy. In some disclosures, the subjects considered to have an increased risk of failing to achieve a complete response go on to achieve a complete response with a frequencies of less than 20% following administration of a dose of the cell therapy. In various disclosures, the subjects considered to have an increased risk of failing to achieve a complete response go on to achieve a complete response with a frequencies of or of about 0% following administration of a dose of the cell therapy.

In certain disclosures, subjects considered to have an increased risk of achieving a partial response or a progressive disease outcome following administration of a cell therapy go on to achieve a complete response with a frequency of less than or less than about 50%, 40%, 30%, 25%, 20%, 15% 10%, or less than 10% following administration of a dose of the cell therapy. In various disclosures, the subjects considered to have an increased risk of achieving a partial response or a progressive disease outcome go on to achieve a complete response with a frequencies of less than 20% following administration of a dose of the cell therapy. In various disclosures, the subjects considered to have an increased risk of achieving a partial response or a progressive disease outcome response go on to achieve a complete response with a frequencies of or of about 0% following administration of a dose of the cell therapy.

In particular disclosures, subjects considered to have an increased risk of failing to achieve a complete response receive an increased dose of the cell therapy, for example to improve the likelihood or probability that the subjects will go on to achieve a complete response. In some disclosures, subjects considered to have an increased risk of achieving a partial response or a progressive disease outcome receive an increased dose of the cell therapy, for example to improve the likelihood or probability that the subject will go on to achieve a complete response.

The disease or condition that is treated can be any in which expression of an antigen is associated with and/or involved in the etiology of a disease condition or disorder, e.g. causes, exacerbates or otherwise is involved in such disease, condition, or disorder. Exemplary diseases and conditions can include diseases or conditions associated with malignancy or transformation of cells (e.g. cancer), autoimmune or inflammatory disease, or an infectious disease, e.g. caused by a bacterial, viral or other pathogen. Exemplary antigens, which include antigens associated with various diseases and conditions that can be treated, are described above. In particular disclosures, the chimeric antigen receptor or transgenic TCR specifically binds to an antigen associated with the disease or condition.

Among the diseases, conditions, and disorders are tumors, including solid tumors, hematologic malignancies, and melanomas, and including localized and metastatic tumors, infectious diseases, such as infection with a virus or other pathogen, e.g., HIV, HCV, HBV, CMV, HPV, and parasitic disease, and autoimmune and inflammatory diseases. In some disclosures, the disease, disorder or condition is a tumor, cancer, malignancy, neoplasm, or other proliferative disease or disorder. Such diseases include but are not limited to leukemia, lymphoma, e.g., acute myeloid (or myelogenous) leukemia (AML), chronic myeloid (or myelogenous) leukemia (CML), acute lymphocytic (or lymphoblastic) leukemia (ALL), chronic lymphocytic leukemia (CLL), hairy cell leukemia (HCL), small lymphocytic lymphoma (SLL), Mantle cell lymphoma (MCL), Marginal zone lymphoma, Burkitt lymphoma, Hodgkin lymphoma (HL), non-Hodgkin lymphoma (NHL), Anaplastic large cell lymphoma (ALCL), follicular lymphoma, refractory follicular lymphoma, diffuse large B-cell lymphoma (DLBCL) and multiple myeloma (MM). In some disclosures, disease or condition is a B cell malignancy selected from among acute lymphoblastic leukemia (ALL), adult ALL, chronic lymphoblastic leukemia (CLL), non-Hodgkin lymphoma (NHL), and Diffuse Large B-Cell Lymphoma (DLBCL). In some disclosures, the disease or condition is NHL and the NHL is selected from the group consisting of aggressive NHL, diffuse large B cell lymphoma (DLBCL), NOS (de novo and transformed from indolent), primary mediastinal large B cell lymphoma (PMBCL), T cell/histocyte-rich large B cell lymphoma (TCHRBCL), Burkitt's lymphoma, mantle cell lymphoma (MCL), and/or follicular lymphoma (FL), optionally, follicular lymphoma Grade 3B (FL3B).

In some disclosures, the disease or condition is an infectious disease or condition, such as, but not limited to, viral, retroviral, bacterial, and protozoal infections, immunodeficiency, Cytomegalovirus (CMV), Epstein-Barr virus (EBV), adenovirus, BK polyomavirus. In some disclosures, the disease or condition is an autoimmune or inflammatory disease or condition, such as arthritis, e.g., rheumatoid arthritis (RA), Type I diabetes, systemic lupus erythematosus (SLE), inflammatory bowel disease, psoriasis, scleroderma, autoimmune thyroid disease, Grave's disease, Crohn's disease, multiple sclerosis, asthma, and/or a disease or condition associated with transplant.

In some disclosures, the antigen associated with the disease or disorder is or includes $\alpha v\beta 6$ integrin (avb6 integrin), B cell maturation antigen (BCMA), B7-H3, B7-H6, carbonic anhydrase 9 (CA9, also known as CAIX or G250), a cancer-testis antigen, cancer/testis antigen $_{1B}$ (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, epidermal growth factor protein (EGFR), truncated epidermal growth factor protein (tEGFR), type III epidermal growth factor receptor mutation (EGFR vIII), epithelial glycoprotein 2 (EPG-2), epithelial glycoprotein 40 (EPG-40), ephrinB2, ephrine receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPCR5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha (IL-22R$\alpha$), IL-13 receptor alpha 2 (IL-13R$\alpha$2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT) vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some disclosures include antigens associated with a B cell malignancy, such as any of a number of known B cell marker. In some disclosures, the antigen is or includes CD20, CD19, CD22, ROR1, CD45, CD21, CD5, CD33, Igkappa, Iglambda, CD79a, CD79b or CD30. In some disclosures, the antigen is or includes a pathogen-specific or pathogen-expressed antigen, such as a viral antigen (e.g., a viral antigen from HIV, HCV, HBV), bacterial antigens, and/or parasitic antigens.

In some disclosures, the antibody or an antigen-binding fragment (e.g. scFv or $V_H$ domain) specifically recognizes an antigen, such as CD19. In some disclosures, the antibody or antigen-binding fragment is derived from, or is a variant of, antibodies or antigen-binding fragment that specifically binds to CD19. In some disclosures, the cell therapy, e.g., adoptive T cell therapy, is carried out by autologous transfer, in which the cells are isolated and/or otherwise prepared from the subject who is to receive the cell therapy, or from a sample derived from such a subject. Thus, in some disclosures, the cells are derived from a subject, e.g., patient, in need of a treatment and the cells, following isolation and processing are administered to the same subject.

In some disclosures, the cell therapy, e.g., adoptive T cell therapy, is carried out by allogeneic transfer, in which the cells are isolated and/or otherwise prepared from a subject other than a subject who is to receive or who ultimately receives the cell therapy, e.g., a first subject. In such disclosures, the cells then are administered to a different subject, e.g., a second subject, of the same species. In some disclosures, the first and second subjects are genetically identical. In some disclosures, the first and second subjects are genetically similar. In some disclosures, the second subject expresses the same HLA class or supertype as the first subject.

The cells can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some disclosures, they are administered by parenteral, intrapulmonary, and intranasal, and, if desired for local treatment, intralesional administration. Parenteral infusions include intramuscular, intravenous, intraarterial, intraperitoneal, or subcutaneous administration. In some disclosures, a given dose is administered by a single bolus administration of the cells. In some disclosures, it is administered by multiple bolus administrations of the cells, for example, over a period of no more than 3 days, or by continuous infusion administration of the cells. In some disclosures, administration of the cell dose or any additional therapies, e.g., the lymphodepleting therapy, intervention therapy and/or combination therapy, is carried out via outpatient delivery.

For the prevention or treatment of disease, the appropriate dosage may depend on the type of disease to be treated, the type of cells or recombinant receptors, the severity and course of the disease, whether the cells are administered for preventive or therapeutic purposes, previous therapy, the subject's clinical history and response to the cells, and the discretion of the attending physician. The compositions and cells are in some disclosures suitably administered to the subject at one time or over a series of treatments.

In some disclosures, the cells are administered as part of a combination treatment, such as simultaneously with or sequentially with, in any order, another therapeutic intervention, such as an antibody or engineered cell or receptor or agent, such as a cytotoxic or therapeutic agent. The cells in some disclosures are co-administered with one or more additional therapeutic agents or in connection with another therapeutic intervention, either simultaneously or sequentially in any order. In some contexts, the cells are co-administered with another therapy sufficiently close in time such that the cell populations enhance the effect of one or more additional therapeutic agents, or vice versa. In some disclosures, the cells are administered prior to the one or more additional therapeutic agents. In some disclosures, the cells are administered after the one or more additional therapeutic agents. In some disclosures, the one or more additional agents include a cytokine, such as IL-2, for example, to enhance persistence. In some disclosures, the methods comprise administration of a chemotherapeutic agent.

In some disclosures, the methods comprise administration of a chemotherapeutic agent, e.g., a conditioning chemotherapeutic agent, for example, to reduce tumor burden prior to the administration.

Preconditioning subjects with immunodepleting (e.g., lymphodepleting) therapies in some disclosures can improve the effects of adoptive cell therapy (ACT).

Thus, in some disclosures, the methods include administering a preconditioning agent, such as a lymphodepleting or chemotherapeutic agent, such as cyclophosphamide, fludarabine, or combinations thereof, to a subject prior to the initiation of the cell therapy. For example, the subject may be administered a preconditioning agent at least 2 days prior, such as at least 3, 4, 5, 6, or 7 days prior, to the initiation of the cell therapy. In some disclosures, the subject is administered a preconditioning agent no more than 7 days prior, such as no more than 6, 5, 4, 3, or 2 days prior, to the initiation of the cell therapy.

In some disclosures, the subject is preconditioned with cyclophosphamide at a dose between or between about 20 mg/kg and 100 mg/kg, such as between or between about 40 mg/kg and 80 mg/kg. In some disclosures, the subject is preconditioned with or with about 60 mg/kg of cyclophosphamide. In some disclosures, the cyclophosphamide can be administered in a single dose or can be administered in a plurality of doses, such as given daily, every other day or every three days. In some disclosures, the cyclophosphamide is administered once daily for one or two days. In some disclosures, where the lymphodepleting agent comprises cyclophosphamide, the subject is administered cyclophosphamide at a dose between or between about 100 mg/m$^2$ and 500 mg/m$^2$, such as between or between about 200 mg/m$^2$ and 400 mg/m$^2$, or 250 mg/m$^2$ and 350 mg/m$^2$, inclusive. In some instances, the subject is administered about 300 mg/m$^2$ of cyclophosphamide. In some disclosures, the cyclophosphamide can be administered in a single dose or can be administered in a plurality of doses, such as given daily, every other day or every three days. In some disclosures, cyclophosphamide is administered daily, such as for 1-5 days, for example, for 3 to 5 days. In some instances, the subject is administered about 300 mg/m$^2$ of cyclophosphamide, daily for 3 days, prior to initiation of the cell therapy.

In some disclosures, where the lymphodepleting agent comprises fludarabine, the subject is administered fludarabine at a dose between or between about 1 mg/m$^2$ and 100 mg/m$^2$, such as between or between about 10 mg/m$^2$ and 75 mg/m$^2$, 15 mg/m$^2$ and 50 mg/m$^2$, 20 mg/m$^2$ and 40 mg/m$^2$, or 24 mg/m$^2$ and 35 mg/m$^2$, inclusive. In some instances, the subject is administered about 30 mg/m$^2$ of fludarabine. In some disclosures, the fludarabine can be administered in a single dose or can be administered in a plurality of doses, such as given daily, every other day or every three days. In some disclosures, fludarabine is administered daily, such as for 1-5 days, for example, for 3 to 5 days. In some instances, the subject is administered about 30 mg/m$^2$ of fludarabine, daily for 3 days, prior to initiation of the cell therapy.

In some disclosures, the lymphodepleting agent comprises a combination of agents, such as a combination of cyclophosphamide and fludarabine. Thus, the combination of agents may include cyclophosphamide at any dose or administration schedule, such as those described above, and fludarabine at any dose or administration schedule, such as those described above. For example, in some disclosures, the subject is administered 60 mg/kg (~2 g/m$^2$) of cyclophosphamide and 3 to 5 doses of 25 mg/m$^2$ fludarabine prior to the first or subsequent dose.

Following administration of the cells, the biological activity of the engineered cell populations in some disclosures is measured, e.g., by any of a number of known methods. Parameters to assess include specific binding of an engineered or natural T cell or other immune cell to antigen, in vivo, e.g., by imaging, or ex vivo, e.g., by ELISA or flow cytometry. In certain disclosures, the ability of the engineered cells to destroy target cells can be measured using any suitable known methods, such as cytotoxicity assays described in, for example, Kochenderfer et al., J. Immunotherapy, 32(7): 689-702 (2009), and Herman et al. J. Immunological Methods, 285(1): 25-40 (2004). In certain disclosures, the biological activity of the cells is measured by assaying expression and/or secretion of one or more cytokines, such as CD107a, IFN$\gamma$, IL-2, and TNF. In some disclosures the biological activity is measured by assessing clinical outcome, such as reduction in tumor burden or load.

In certain disclosures, the engineered cells are further modified in any number of ways, such that their therapeutic or prophylactic efficacy is increased. For example, the engineered CAR or TCR expressed by the population can be conjugated either directly or indirectly through a linker to a targeting moiety. The practice of conjugating compounds, e.g., the CAR or TCR, to targeting moieties is known. See, for instance, Wadwa et al., J. Drug Targeting 3: 1 1 1 (1995), and U.S. Patent 5,087,616.

In some disclosures, the cells are administered as part of a combination treatment, such as simultaneously with or sequentially with, in any order, another therapeutic intervention, such as an antibody or engineered cell or receptor or agent, such as a cytotoxic or therapeutic agent. The cells in some disclosures are co-administered with one or more additional therapeutic agents or in connection with another therapeutic intervention, either simultaneously or sequentially in any order. In some contexts, the cells are co-administered with another therapy sufficiently close in time such that the cell populations enhance the effect of one or more additional therapeutic agents, or vice versa. In some disclosures, the cells are administered prior to the one or more additional therapeutic agents. In some disclosures, the cells are administered after the one or more additional therapeutic agents. In some disclosures, the one or more additional agent includes a cytokine, such as IL-2, for example, to enhance persistence.

## A. Dosing

In some disclosures, a dose of cells is administered to subjects in accord with the disclosed methods, and/or with the disclosed articles of manufacture or compositions. In some disclosures, the size or timing of the doses is determined as a function of the particular disease or condition in the subject. In some cases disclosed herein, the size or timing of the doses for a particular disease in view of the disclosed description may be empirically determined.

In some disclosures, the dose of cells comprises between at or about $2 \times 10^5$ of the cells/kg and at or about $2 \times 10^6$ of the cells/kg, such as between at or about $4 \times 10^5$ of the cells/kg and at or about $1 \times 10^6$ of the cells/kg or between at or about $6 \times 10^5$ of the cells/kg and at or about $8 \times 10^5$ of the cells/kg. In some disclosures, the dose of cells comprises no more than $2 \times 10^5$ of the cells (e.g. antigen-expressing, such as CAR-expressing cells) per kilogram body weight of the subject (cells/kg), such as no more than at or about $3 \times 10^5$ cells/kg, no more than at or about $4 \times 10^5$ cells/kg, no more than at or about $5 \times 10^5$ cells/kg, no more than at or about $6 \times 10^5$ cells/kg, no more than at or about $7 \times 10^5$ cells/kg, no more than at or about $8 \times 10^5$ cells/kg, no more than at or about $9 \times 10^5$ cells/kg, no more than at or about $1 \times 10^6$ cells/kg, or no more than at or about $2 \times 10^6$ cells/kg. In some disclosures, the dose of cells comprises at least or at least about or at or about $2 \times 10^5$ of the cells (e.g. antigen-expressing, such as CAR-expressing cells) per kilogram body weight of the subject (cells/kg), such as at least or at least about or at or about $3 \times 10^5$ cells/kg, at least or at least about or at or about $4 \times 10^5$ cells/kg, at least or at least about or at or about $5 \times 10^5$ cells/kg, at least or at least about or at or about $6 \times 10^5$ cells/kg, at least or at least about or at or about $7 \times 10^5$ cells/kg, at least or at least about or at or about $8 \times 10^5$ cells/kg, at least or at least about or at or about $9 \times 10^5$ cells/kg, at least or at least about or at or about $1 \times 10^6$ cells/kg, or at least or at least about or at or about $2 \times 10^6$ cells/kg.

In certain disclosures, the cells, or individual populations of sub-types of cells, are administered to the subject at a range of about one million to about 100 billion cells and/or that amount of cells per kilogram of body weight, such as, e.g., 1 million to about 50 billion cells (e.g., about 5 million cells, 10 million cells, about 15 million cells, about 20 million cells, about 25 million cells, about 500 million cells, about 1 billion cells, about 5 billion cells, about 20 billion cells, about 30 billion cells, about 40 billion cells, or a range defined by any two of the foregoing values), such as about 10 million to about 100 billion cells (e.g., about 20 million cells, about 30 million cells, about 40 million cells, about 60 million cells, about 70 million cells, about 80 million cells, about 90 million cells, about 10 billion cells, about 25 billion cells, about 50 billion cells, about 75 billion cells, about 90 billion cells, or a range defined by any two of the foregoing values), and in some cases disclosed herein about 100 million cells to about 50 billion cells (e.g., about 120 million cells, about 250 million cells, about 350 million cells, about 450 million cells, about 650 million cells, about 800 million cells, about 900 million cells, about 3 billion cells, about 30 billion cells, about 45 billion cells) or any value in between these ranges and/or per kilogram of body weight. Dosages may vary depending on attributes particular to the disease or disorder and/or patient and/or other treatments.

In some disclosures, the dose of cells is a flat dose of cells or fixed dose of cells such that the dose of cells is not tied to or based on the body surface area or weight of a subject.

In some disclosures, for example, where the subject is a human, the dose includes fewer than about $5 \times 10^8$ total recombinant receptor (e.g., CAR)-expressing cells, T cells, or peripheral blood mononuclear cells (PBMCs), e.g., in the range of about $1 \times 10^6$ to $5 \times 10^8$ such cells, such as $2 \times 10^6$, $5 \times 10^6$, $1 \times 10^7$, $5 \times 10^7$, $1 \times 10^8$, or $5 \times 10^8$ total such cells, or the range between any two of the foregoing values.

In some disclosures, the dose of genetically engineered cells comprises from or from about $1 \times 10^5$ to $5 \times 10^8$ total CAR-expressing T cells, $1 \times 10^5$ to $2.5 \times 10^8$ total CAR-expressing T cells, $1 \times 10^5$ to $1 \times 10^8$ total CAR-expressing T cells, $1 \times 10^5$ to $5 \times 10^7$ total CAR-expressing T cells, $1 \times 10^5$ to $2.5 \times 10^7$ total CAR-expressing T cells, $1 \times 10^5$ to $1 \times 10^7$ total CAR-expressing T cells, $1 \times 10^5$ to $5 \times 10^6$ total CAR-expressing T cells, $1 \times 10^5$ to $2.5 \times 10^6$ total CAR-expressing T cells, $1 \times 10^5$ to $1 \times 10^6$ total CAR-expressing T cells, $1 \times 10^6$ to $5 \times 10^8$ total CAR-expressing T cells, $1 \times 10^6$ to $2.5 \times 10^8$ total CAR-expressing T cells, $1 \times 10^6$ to $1 \times 10^8$ total CAR-expressing T cells, $1 \times 10^6$ to $5 \times 10^7$ total CAR-expressing T cells, $1 \times 10^6$ to $2.5 \times 10^7$ total CAR-expressing T cells, $1 \times 10^6$ to $1 \times 10^7$ total CAR-expressing T cells, $1 \times 10^6$ to $5 \times 10^6$ total CAR-expressing T cells, $1 \times 10^6$ to $2.5 \times 10^6$ total CAR-expressing T cells, $2.5 \times 10^6$ to $5 \times 10^8$ total CAR-expressing T cells, $2.5 \times 10^6$ to $2.5 \times 10^8$ total CAR-expressing T cells, $2.5 \times 10^6$ to $1 \times 10^8$ total CAR-expressing T cells, $2.5 \times 10^6$ to $5 \times 10^7$ total CAR-expressing T cells, $2.5 \times 10^6$ to $2.5 \times 10^7$ total CAR-expressing T cells, $2.5 \times 10^6$ to $1 \times 10^7$ total CAR-expressing T cells, $2.5 \times 10^6$ to $5 \times 10^6$ total CAR-expressing T cells, $5 \times 10^6$ to $5 \times 10^8$ total CAR-expressing T cells, $5 \times 10^6$ to $2.5 \times 10^8$ total CAR-expressing T cells, $5 \times 10^6$ to $1 \times 10^8$ total CAR-expressing T cells, $5 \times 10^6$ to $5 \times 10^7$ total CAR-expressing T cells, $5 \times 10^6$ to $2.5 \times 10^7$ total CAR-expressing T cells, $5 \times 10^6$ to $1 \times 10^7$ total CAR-expressing T cells, $1 \times 10^7$ to $5 \times 10^8$ total CAR-expressing T cells, $1 \times 10^7$ to $2.5 \times 10^8$ total CAR-expressing T cells, $1 \times 10^7$ to $1 \times 10^8$ total CAR-expressing T cells, $1 \times 10^7$ to $5 \times 10^7$ total CAR-expressing T cells, $1 \times 10^7$ to $2.5 \times 10^7$ total CAR-expressing T cells, $2.5 \times 10^7$ to $5 \times 10^8$ total CAR-expressing T cells, $2.5 \times 10^7$ to $2.5 \times 10^8$ total CAR-expressing T cells, $2.5 \times 10^7$ to $1 \times 10^8$ total CAR-expressing T cells, $2.5 \times 10^7$ to $5 \times 10^7$ total CAR-expressing T cells, $5 \times 10^7$ to $5 \times 10^8$ total CAR-expressing T cells, $5 \times 10^7$ to $2.5 \times 10^8$ total CAR-expressing T cells, $5 \times 10^7$ to $1 \times 10^8$ total CAR-expressing T cells, $1 \times 10^8$ to $5 \times 10^8$ total CAR-expressing T cells, $1 \times 10^8$ to $2.5 \times 10^8$ total CAR-expressing T cells, or $2.5 \times 10^8$ to $5 \times 10^8$ total CAR-expressing T cells.

In some disclosures, the dose of genetically engineered cells comprises at least or at least about $1 \times 10^5$ CAR-expressing cells, at least or at least about $2.5 \times 10^5$ CAR-expressing cells, at least or at least about $5 \times 10^5$ CAR-expressing cells, at least or at least about $1 \times 10^6$ CAR-expressing cells, at least or at least about $2.5 \times 10^6$ CAR-expressing cells, at least or at least about $5 \times 10^6$ CAR-expressing cells, at least or at least about $1 \times 10^7$ CAR-expressing cells, at least or at least about $2.5 \times 10^7$ CAR-expressing cells, at least or at least about $5 \times 10^7$ CAR-expressing cells, at least or at least about $1 \times 10^8$ CAR-expressing cells, at least or at least about $2.5 \times 10^8$ CAR-expressing cells, or at least or at least about $5 \times 10^8$ CAR-expressing cells.

In some disclosures, the cell therapy comprises administration of a dose comprising a number of cell from or from about $1 \times 10^5$ to $5 \times 10^8$ total recombinant receptor-expressing cells, total T cells, or total peripheral blood mononuclear cells (PBMCs), from or from about $5 \times 10^5$ to $1 \times 10^7$ total recombinant receptor-expressing cells, total T cells, or total peripheral blood mononuclear cells (PBMCs) or from or from about $1 \times 10^6$ to $1 \times 10^7$ total recombinant receptor-expressing cells, total T cells, or total peripheral blood mononuclear cells (PBMCs), each inclusive. In some disclosures, the cell therapy comprises administration of a dose of cells comprising a number of cells at least or at least about $1 \times 10^5$ total recombinant receptor-expressing cells, total T cells, or total peripheral blood mononuclear cells (PBMCs), such at least or at least $1 \times 10^6$, at least or at least about $1 \times 10^7$, at least or at least about $1 \times 10^8$ of such cells. In some disclosures, the number is with reference to the total number of CD3+ or CD8+, in some cases disclosed herein also recombinant receptor-expressing (e.g. CAR+) cells. In some disclosures, the cell therapy comprises administration of a dose comprising a number of cell from or from about $1 \times 10^5$ to $5 \times 10^8$ CD3+ or CD8+ total T cells or CD3+ or CD8+ recombinant receptor-expressing cells, from or from about $5 \times 10^5$ to $1 \times 10^7$ CD3+ or CD8+ total T cells or CD3+ or CD8+ recombinant receptor-expressing cells, or from or from about $1 \times 10^6$ to $1 \times 10^7$ CD3+ or CD8+ total T cells or CD3+ or CD8+recombinant receptor-expressing cells, each inclusive. In some disclosures, the cell therapy comprises administration of a dose comprising a number of cell from or from about $1 \times 10^5$ to $5 \times 10^8$ total CD3+/CAR+ or CD8+/CAR+ cells, from or from about $5 \times 10^5$ to $1 \times 10^7$ total CD3+/CAR+ or CD8+/CAR+ cells, or from or from about $1 \times 10^6$ to $1 \times 10^7$ total CD3+/CAR+ or CD8+/CAR+ cells, each inclusive.

In some disclosures, the T cells of the dose include CD4+ T cells, CD8+ T cells or CD4+ and CD8+ T cells.

In some disclosures, for example, where the subject is human, the CD8+ T cells of the dose, including in a dose including CD4+ and CD8+ T cells, includes between about $1 \times 10^6$ and $5 \times 10^8$ total recombinant receptor (e.g., CAR)-expressing CD8+cells, e.g., in the range of about $5 \times 10^6$ to $1 \times 10^8$ such cells, such cells $1 \times 10'$, $2.5 \times 10^7$, $5 \times 10^7$, $7.5 \times 10^7$, $1 \times 10^8$, or $5 \times 10^8$ total such cells, or the range between any two of the foregoing values. In some disclosures, the patient is administered multiple doses, and each of the doses or the total dose can be within any of the foregoing values. In some disclosures, the dose of cells comprises the administration of from or from about $1 \times 10^7$ to $0.75 \times 10^8$ total recombinant receptor-expressing CD8+ T cells, $1 \times 10^7$ to $2.5 \times 10^7$ total recombinant receptor-expressing CD8+ T cells, from or from about $1 \times 10^7$ to $0.75 \times 10^8$ total recombinant receptor-expressing CD8+ T cells, each inclusive. In some disclosures, the dose of cells comprises the administration of or about $1 \times 10'$, $2.5 \times 10^7$, $5 \times 10^7$ $7.5 \times 10^7$, $1 \times 10^8$, or $5 \times 10^8$ total recombinant receptor-expressing CD8+ T cells.

In some disclosures, the dose of cells, e.g., recombinant receptor-expressing T cells, is administered to the subject as a single dose or is administered only one time within a period of two weeks, one month, three months, six months, 1 year or more.

In the context of adoptive cell therapy, administration of a given "dose" encompasses administration of the given amount or number of cells as a single composition and/or single uninterrupted administration, e.g., as a single injection or continuous infusion, and also encompasses administration of the given amount or number of cells as a split dose or as a plurality of compositions, provided in multiple individual compositions or infusions, over a specified period of time, such as over no more than 3 days. Thus, in some contexts, the dose is a single or continuous administration of the specified number of cells, given or initiated at a single point in time. In some contexts, however, the dose is administered in multiple injections or infusions over a period of no more than three days, such as once a day for three days or for two days or by multiple infusions over a single day period.

Thus, in some disclosures, the cells of the dose are administered in a single pharmaceutical composition. In some disclosures, the cells of the dose are administered in a plurality of compositions, collectively containing the cells of the dose.

In some disclosures, the term "split dose" refers to a dose that is split so that it is administered over more than one day. This type of dosing is encompassed by the present methods and is considered to be a single dose.

Thus, the dose of cells may be administered as a split dose, e.g., a split dose administered over time. For example, in some disclosures, the dose may be administered to the subject over 2 days or over 3 days. Exemplary methods for split dosing include administering 25% of the dose on the first day and administering the remaining 75% of the dose on the second day. In other disclosures, 33% of the dose may be administered on the first day and the remaining 67% administered on the second day. In some disclosures, 10% of the dose is administered on the first day, 30% of the dose is administered on the second day, and 60% of the dose is administered on the third day. In some disclosures, the split dose is not spread over more than 3 days.

In some disclosures, cells of the dose may be administered by administration of a plurality of compositions or solutions, such as a first and a second, optionally more, each containing some cells of the dose. In some disclosures, the plurality of compositions, each containing a different population and/or sub-types of cells, are administered separately or independently, optionally within a certain period of time. For example, the populations or sub-types of cells can include CD8+ and CD4+ T cells, respectively, and/or CD8+- and CD4+-enriched populations, respectively, e.g., CD4+ and/or CD8+ T cells each individually including cells genetically engineered to express the recombinant receptor. In some disclosures, the administration of the dose comprises administration of a first composition comprising a dose of CD8+ T cells or a dose of CD4+ T cells and administration of a second composition comprising the other of the dose of CD4+ T cells and the CD8+ T cells.

In some disclosures, the administration of the composition or dose, e.g., administration of the plurality of cell compositions, involves administration of the cell compositions separately. In some disclosures, the separate administrations are carried out simultaneously, or sequentially, in any order. In some disclosures, the dose comprises a first composition and a second composition, and the first composition and second composition are administered 0 to 12 hours apart, 0 to 6 hours apart or 0 to 2 hours apart. In some disclosures, the initiation of administration of the first composition and the initiation of administration of the second composition are carried out no more than 2 hours, no more than 1 hour, or no more than 30 minutes apart, no more than 15 minutes, no more than 10 minutes or no more than 5 minutes apart. In some disclosures, the initiation and/or completion of administration of the first composition and the completion and/or initiation of administration of the second composition are carried out no more than 2 hours, no more than 1 hour, or no more than 30 minutes apart, no more than 15 minutes, no more than 10 minutes or no more than 5 minutes apart.

In some composition, the first composition, e.g., first composition of the dose, comprises CD4+ T cells. In some composition, the first composition, e.g., first composition of the dose, comprises CD8+ T cells. In some disclosures, the first composition is administered prior to the second composition.

In some disclosures, the dose or composition of cells includes a defined or target ratio of CD4+ cells expressing a recombinant receptor to CD8+ cells expressing a recombinant receptor and/or of CD4+ cells to CD8+ cells, which ratio optionally is approximately 1:1 or is between approximately 1:3 and approximately 3:1, such as approximately 1:1. In some disclosures, the administration of a composition or dose with the target or desired ratio of different cell populations (such as CD4+:CD8+ ratio or CAR+CD4+:CAR+CD8+ ratio, e.g., 1:1) involves the administration of a cell composition containing one of the populations and then administration of a separate cell composition comprising the other of the populations, where the administration is at or approximately at the target or desired ratio. In some disclosures, administration of a dose or composition of cells at a defined ratio leads to improved expansion, persistence and/or antitumor activity of the T cell therapy.

In some disclosures, the subject receives multiple doses, e.g., two or more doses or multiple consecutive doses, of the cells. In some disclosures, two doses are administered to a subject. In some disclosures, the subject receives the consecutive dose, e.g., second dose, is administered approximately 4, 5, 6, 7, 8, 9, 10, 11, 12, 13, 14, 15, 16, 17, 18, 19, 20 or 21 days after the first dose. In some disclosures, multiple consecutive doses are administered following the first dose, such that an additional dose or doses are administered following administration of the consecutive dose. In some disclosures, the number of cells administered to the subject in the additional dose is the same as or similar to the first dose and/or consecutive dose. In some disclosures, the additional dose or doses are larger than prior doses.

In some disclosures, the size of the first and/or consecutive dose is determined based on one or more criteria such as response of the subject to prior treatment, e.g. chemotherapy, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

In some disclosures, the time between the administration of the first dose and the administration of the consecutive dose is about 9 to about 35 days, about 14 to about 28 days, or 15 to 27 days. In some disclosures, the administration of the consecutive dose is at a time point more than about 14 days after and less than about 28 days after the administration of the first dose. In some disclosures, the time between the first and consecutive dose is about 21 days. In some disclosures, an additional dose or doses, e.g. consecutive doses, are administered following administration of the consecutive dose. In some disclosures, the additional consecutive dose or doses are administered at least about 14 and less than about 28 days following administration of a prior dose. In some disclosures, the additional dose is administered less than about 14 days following the prior dose, for example, 4, 5, 6, 7, 8, 9, 10, 11, 12, or 13 days after the prior dose. In some disclosures, no dose is administered less than about 14 days following the prior dose and/or no dose is administered more than about 28 days after the prior dose.

In some disclosures, the dose of cells, e.g., recombinant receptor-expressing cells, comprises two doses (e.g., a double dose), comprising a first dose of the T cells and a consecutive dose of the T cells, wherein one or both of the first dose and the second dose comprises administration of the split dose of T cells.

In some disclosures, the dose of cells is generally large enough to be effective in reducing disease burden.

In some disclosures, the cells are administered at a desired dosage, which in some disclosures includes a desired dose or number of cells or cell type(s) and/or a desired ratio of cell types. Thus, the dosage of cells in some disclosures is based on a total number of cells (or number per kg body weight) and a desired ratio of the individual populations or sub-types, such as the CD4+ to CD8+ ratio. In some disclosures, the dosage of cells is based on a desired total number (or number per kg of body weight) of cells in the individual populations or of individual cell types. In some disclosures, the dosage is based on a combination of such features, such as a desired number of total cells, desired ratio, and desired total number of cells in the individual populations.

In some disclosures, the populations or sub-types of cells, such as CD8$^+$ and CD4$^+$ T cells, are administered at or within a tolerated difference of a desired dose of total cells, such as a desired dose of T cells. In some disclosures, the desired dose is a desired number of cells or a desired number of cells per unit of body weight of the subject to whom the cells are administered, e.g., cells/kg. In some disclosures, the desired dose is at or above a minimum number of cells or minimum number of cells per unit of body weight. In some disclosures, among the total cells, administered at the desired dose, the individual populations or sub-types are present at or near a desired output ratio (such as CD4$^+$ to CD8$^+$ ratio), e.g., within a certain tolerated difference or error of such a ratio.

In some disclosures, the cells are administered at or within a tolerated difference of a desired dose of one or more of the individual populations or sub-types of cells, such as a desired dose of CD4+ cells and/or a desired dose of CD8+ cells. In some disclosures, the desired dose is a desired number of cells of the sub-type or population, or a desired number of such cells per unit of body weight of the subject to whom the cells are administered, e.g., cells/kg. In some disclosures, the desired dose is at or above a minimum number of cells of the population or sub-type, or minimum number of cells of the population or sub-type per unit of body weight.

Thus, in some disclosures, the dosage is based on a desired fixed dose of total cells and a desired ratio, and/or based on a desired fixed dose of one or more, e.g., each, of the individual sub-types or sub-populations. Thus, in some disclosures, the dosage is based on a desired fixed or minimum dose of T cells and a desired ratio of CD4$^+$ to CD8$^+$ cells, and/or is based on a desired fixed or minimum dose of CD4$^+$ and/or CD8$^+$ cells.

In some disclosures, the cells are administered at or within a tolerated range of a desired output ratio of multiple cell populations or sub-types, such as CD4+ and CD8+ cells or sub-types. In some disclosures, the desired ratio can be a specific ratio or can be a range of ratios. for example, in some disclosures, the desired ratio (e.g., ratio of CD4$^+$ to CD8$^+$ cells) is between at or about 5:1 and at or about 5:1 (or greater than about 1:5 and less than about 5:1), or between at or about 1:3 and at or about 3:1 (or greater than about 1:3 and less than about 3:1), such as between at or about 2:1 and at or about 1:5 (or greater than about 1:5 and less than about 2:1, such as at or about 5:1, 4.5:1, 4:1, 3.5:1, 3:1, 2.5:1, 2:1, 1.9:1, 1.8:1, 1.7:1, 1.6:1, 1.5:1, 1.4:1, 1.3:1, 1.2:1, 1.1:1, 1:1, 1:1.1, 1:1.2, 1:1.3, 1:1.4, 1:1.5, 1:1.6, 1:1.7, 1:1.8, 1:1.9: 1:2, 1:2.5, 1:3, 1:3.5, 1:4, 1:4.5, or 1:5. In some disclosures, the tolerated difference is within about 1%, about 2%, about 3%, about 4% about 5%, about 10%, about 15%, about 20%, about 25%, about 30%, about 35%, about 40%, about 45%, about 50% of the desired ratio, including any value in between these ranges.

In particular disclosures, the numbers and/or concentrations of cells refer to the number of recombinant receptor (e.g., CAR)-expressing cells. In other disclosures, the numbers and/or concentrations of cells refer to the number or concentration of all cells, T cells, or peripheral blood mononuclear cells (PBMCs) administered.

In some disclosures, the size of the dose is determined based on one or more criteria such as response of the subject to prior treatment, e.g. chemotherapy, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

In some disclosures, the methods also include administering one or more additional doses of cells expressing a chimeric antigen receptor (CAR) and/or lymphodepleting therapy, and/or one or more steps of the methods are repeated. In some disclosures, the one or more additional dose is the same as the initial dose. In some disclosures, the one or more additional dose is different from the initial dose, e.g., higher, such as 2-fold, 3-fold, 4-fold, 5-fold, 6-fold, 7-fold, 8-fold, 9-fold or 10-fold or more higher than the initial dose, or lower, such as e.g., higher, such as 2-fold, 3-fold, 4-fold, 5-fold, 6-fold, 7-fold, 8-fold, 9-fold or 10-fold or more lower than the initial dose. In some disclosures, administration of one or more additional doses is determined based on response of the subject to the initial treatment or any prior treatment, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

## VII. COMPOSITIONS AND FORMULATIONS

In some disclosures, the dose of cells comprising cells engineered with a recombinant antigen receptor, e.g. CAR or TCR, is disclosed as a composition or formulation, such as a pharmaceutical composition or formulation. Such compositions can be used in accord with the disclosed methods, and/or with the disclosed articles of manufacture or compositions, such as in the prevention or treatment of diseases, conditions, and disorders, or in detection, diagnostic, and prognostic methods.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In some disclosures, the choice of carrier is determined in part by the particular cell or agent and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some disclosures, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffering agents in some disclosures are included in the compositions. Suitable buffering agents include, for example, citric acid, sodium citrate, phosphoric acid, potassium phosphate, and various other acids and salts. In some disclosures, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulation or composition may also contain more than one active ingredient useful for the particular indication, disease, or condition being prevented or treated with the cells or agents, where the respective activities do not adversely affect one another. Such active ingredients are suitably present in combination in amounts that are effective for the purpose intended. Thus, in some disclosures, the pharmaceutical composition further includes other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc. In some disclosures, the agents or cells are administered in the form of a salt, e.g., a pharmaceutically acceptable salt. Suitable pharmaceutically acceptable acid addition salts include those derived from mineral acids, such as hydrochloric, hydrobromic, phosphoric, metaphosphoric, nitric, and sulphuric acids, and organic acids, such as tartaric, acetic, citric, malic, lactic, fumaric, benzoic, glycolic, gluconic, succinic, and arylsulphonic acids, for example, p-toluenesulphonic acid.

The pharmaceutical composition in some disclosures contains agents or cells in amounts effective to treat or prevent the disease or condition, such as a therapeutically effective or prophylactically effective amount. Therapeutic or prophylactic efficacy in some disclosures is monitored by periodic assessment of treated subjects. For repeated administrations over several days or longer, depending on the condition, the treatment is repeated until a desired suppression of disease symptoms occurs. However, other dosage regimens may be useful and can be determined. The desired dosage can be delivered by a single bolus administration of the composition, by multiple bolus administrations of the composition, or by continuous infusion administration of the composition.

The agents or cells can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some disclosures, they are administered by parenteral, intrapulmonary, and intranasal, and, if desired for local treatment, intralesional administration. Parenteral infusions include intramuscular, intravenous, intraarterial, intraperitoneal, or subcutaneous administration. In some disclosures, a given dose is administered by a single bolus administration of the cells or agent. In some disclosures, it is administered by multiple bolus administrations of the cells or agent, for example, over a period of no more than 3 days, or by continuous infusion administration of the cells or agent.

For the prevention or treatment of disease, the appropriate dosage may depend on the type of disease to be treated, the type of agent or agents, the type of cells or recombinant receptors, the severity and course of the disease, whether the agent or cells are administered for preventive or therapeutic purposes, previous therapy, the subject's clinical history and response to the agent or the cells, and the discretion of the attending physician. The compositions are in some disclosures suitably administered to the subject at one time or over a series of treatments.

The cells or agents may be administered using standard administration techniques, formulations, and/or devices. Disclosed are formulations and devices, such as syringes and vials, for storage and administration of the compositions. With respect to cells, administration can be autologous or heterologous. For example, immunoresponsive cells or progenitors can be obtained from one subject, and administered to the same subject or a different, compatible subject. Peripheral blood derived immunoresponsive cells or their progeny (e.g., in vivo, ex vivo or in vitro derived) can be administered via localized injection, including catheter administration, systemic injection, localized injection, intravenous injection, or parenteral administration. When administering a therapeutic composition (e.g., a pharmaceutical composition containing a genetically modified immunoresponsive cell or an agent that treats or ameliorates symptoms of neurotoxicity), it will generally be formulated in a unit dosage injectable form (solution, suspension, emulsion).

Formulations include those for oral, intravenous, intraperitoneal, subcutaneous, pulmonary, transdermal, intramuscular, intranasal, buccal, sublingual, or suppository administration. In some disclosures, the agent or cell populations are administered parenterally. The term "parenteral," as used herein, includes intravenous, intramuscular, subcutaneous, rectal, vaginal, and intraperitoneal administration. In some disclosures, the agent or cell populations are administered to a subject using peripheral systemic delivery by intravenous, intraperitoneal, or subcutaneous injection.

Compositions in some disclosures are disclosed as sterile liquid preparations, e.g., isotonic aqueous solutions, suspensions, emulsions, dispersions, or viscous compositions, which may in some disclosures be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol) and suitable mixtures thereof.

Sterile injectable solutions can be prepared by incorporating the agent or cells in a solvent, such as in admixture with a suitable carrier, diluent, or excipient such as sterile water, physiological saline, glucose, dextrose, or the like.

The formulations to be used for in vivo administration are generally sterile. Sterility may be readily accomplished, e.g., by filtration through sterile filtration membranes.

## VIII. ARTICLES OF MANUFACTURE AND KITS

Also disclosed are articles of manufacture, systems, apparatuses, and kits useful in performing the disclosed methods. Also disclosed are articles of manufacture, including: (i) one or more reagents for immunoaffinity-based selection of cells specific for CD57, CD4 and/or CD8; and (ii) instructions for use of the one or more reagents for performing any methods described herein.

Also disclosed are articles of manufacture, including: (i) one or more reagents for immunoaffinity-based selection of cells specific for CD57, CD4 and/or CD8; (ii) one or more stimulatory reagents capable of activating one or more intracellular signaling domains of one or more components of a TCR complex and one or more intracellular signaling domains of one or more costimulatory molecules; and (iii) instructions for use of the one or more reagents for performing any methods described herein.

In some of any such disclosures, the reagent for immunoaffinity-based selection is or includes an antibody capable of specifically binding to CD57, CD4 or CD8. In some of any such disclosures, the reagent for immunoaffinity-based selection is or includes an antibody capable of specifically binding to CD57. In some of any such disclosures, the antibody is immobilized on a magnetic particle or is immobilized on or attached to an affinity chromatography matrix.

In some of any such disclosures, the stimulatory reagent includes (i) a primary agent that specifically binds to a member of a TCR complex, optionally that specifically binds to CD3 and (ii) a secondary agent that specifically binds to a T cell costimulatory molecule, optionally wherein the costimulatory molecule is selected from CD28, CD137 (4-1-BB), OX40, or ICOS. In some of any such disclosures, the one or both of the primary and secondary agents include an antibody or an antigen-binding fragment thereof. In some of any such disclosures, the primary and secondary agents include an antibody, optionally wherein the stimulatory reagent includes incubation with an anti-CD3 antibody and an anti-CD28 antibody, or an antigen-binding fragment thereof. In some of any such disclosures, the primary agent and secondary agent are present or attached on the surface of a solid support. In some of any such disclosures, the solid support is or includes a bead, optionally a paramagnetic bead. In some of any such disclosures, the primary agent and secondary agent are reversibly bound on the surface of an oligomeric particle reagent including a plurality of streptavidin or streptavidin mutein molecules.

Also disclosed are articles of manufacture, including (i) any composition described herein; and (ii) instructions for administering the composition to a subject.

In some disclosures, the articles of manufacture or kits include one or more containers, typically a plurality of containers, packaging material, and a label or package insert on or associated with the container or containers and/or packaging, generally including instructions for use, e.g., instructions for reagents for immunoaffinity-based selection of particular cells, e.g., positive or negative selection of cells expressing CD57, CD4 and/or CD8, and instructions to carry out any of the methods disclosed herein, such as for engineering T cells to generate a composition, such as a therapeutic composition, for cell therapy. In some disclosures, the disclosed articles of manufacture contain reagents for stimulation and/or cultivation of cells, for example, at one or more steps of the manufacturing process, such as any reagents described in any steps of Section II and Section III.

Also disclosed are articles of manufacture and kits containing engineered cells expressing a recombinant receptor or compositions thereof, such as those generated using the methods disclosed herein, and optionally instructions for use, for example, instructions for administering. In some disclosures, the instructions provide directions or specify methods for assessing if a subject, prior to receiving a cell therapy, is likely or suspected of being likely to respond and/or the degree or level of response following administration of engineered cells expressing a recombinant receptor for treating a disease or disorder. In some disclosures, the articles of manufacture can contain a dose or a composition of engineered cells.

The articles of manufacture disclosed herein contain packaging materials. Packaging materials for use in packaging the disclosed materials are well known to those of skill in the art. See, for example, U.S. Patent Nos. 5,323,907, 5,052,558 and 5,033,252, each of which is incorporated herein in its entirety. Examples of packaging materials include, but are not limited to, blister packs, bottles, tubes, inhalers, pumps, bags, vials, containers, syringes, disposable laboratory supplies, e.g., pipette tips and/or plastic plates, or bottles. The articles of manufacture or kits can include a device so as to facilitate dispensing of the materials or to facilitate use in a high-throughput or large-scale manner, e.g., to facilitate use in robotic equipment. Typically, the packaging is non-reactive with the compositions contained therein.

In some disclosures, the reagents and/or cell compositions are packaged separately. In some disclosures, each container can have a single compartment. In some disclosures, other components of the articles of manufacture or kits are packaged separately, or together in a single compartment.

## IX. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

As used herein, the singular forms "a," "an," and "the" include plural referents unless the context clearly dictates otherwise. For example, "a" or "an" means "at least one" or "one or more." It is understood that aspects and variations described herein include "consisting" and/or "consisting essentially of" aspects and variations.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

The term "about" as used herein refers to the usual error range for the respective value readily known. Reference to "about" a value or parameter herein includes (and describes) embodiments that are directed to that value or parameter per se. For example, description referring to "about X" includes description of "X". In certain embodiments, "about X" refers to a value of $\pm 25\%$, $\pm 10\%$, $\pm 5\%$, $\pm 2\%$, $\pm 1\%$, $\pm 0.1\%$, or $\pm 0.01\%$ of X.

As used herein, recitation that nucleotides or amino acid positions "correspond to" nucleotides or amino acid positions in a disclosed sequence, such as set forth in the Sequence listing, refers to nucleotides or amino acid positions identified upon alignment with the disclosed sequence to maximize identity using a standard alignment algorithm, such as the GAP algorithm. By aligning the sequences, corresponding residues can be identified, for example, using conserved and identical amino acid residues as guides. In general, to identify corresponding positions, the sequences of amino acids are aligned so that the highest order match is obtained (see, e.g. : Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New.Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; Carrillo et al. (1988) SIAM J Applied Math 48: 1073).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors." Among the vectors are viral vectors, such as retroviral, e.g., gammaretroviral and lentiviral vectors.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control under otherwise identical conditions, and/or at a level substantially lower than that for cell known to be positive for the marker, and/or at a level substantially similar as compared to that for a cell known to be negative for the marker.

As used herein, "percent (%) amino acid sequence identity" and "percent identity" when used with respect to an amino acid sequence (reference polypeptide sequence) is defined as the percentage of amino acid residues in a candidate sequence (e.g., the subject antibody or fragment) that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various known ways, for instance, using publicly available computer software such as BLAST, BLAST-2, ALIGN or Megalign (DNASTAR) software. Appropriate parameters for aligning sequences can be determined, including any algorithms needed to achieve maximal alignment over the full length of the sequences being compared.

An amino acid substitution may include replacement of one amino acid in a polypeptide with another amino acid. The substitution may be a conservative amino acid substitution or a non-conservative amino acid substitution. Amino acid substitutions may be introduced into a binding molecule, e.g., antibody, of interest and the products screened for a desired activity, *e.g.*, retained/improved antigen binding, decreased immunogenicity, or improved ADCC or CDC.

Amino acids generally can be grouped according to the following common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

In some embodiments, conservative substitutions can involve the exchange of a member of one of these classes for another member of the same class. In some embodiments, non-conservative amino acid substitutions can involve exchanging a member of one of these classes for another class.

As used herein, a composition refers to any mixture of two or more products, substances, or compounds, including cells. It may be a solution, a suspension, liquid, powder, a paste, aqueous, non-aqueous or any combination thereof.

As used herein, a "subject" is a mammal, such as a human or other animal, and typically is human.

## X. EXAMPLES

The following examples are included for illustrative purposes.

### Example 1: Assessment of Variability in T Cell Expansion, Cell Viability and Cell Cycle Entry During a Process for Engineering T Cells

CD8+ T cells were obtained from seven exemplary donors (Donors A-G) and were engineered to express a chimeric antigen receptor (CAR) by a manufacturing process involving stimulation, transduction with a lentiviral vector encoding the CAR construct and cultivation for expansion, or were subjected to a similar process for stimulation and cultivation. Cell expansion, cell viability, and cell cycle entry in the stimulated and cultivated cells were assessed.

To generate the engineered cell compositions, CD8+ cells were isolated from human donor leukapheresis samples by immunoaffinity-based enrichment and cryopreserved. The cryopreserved cell compositions were subsequently thawed and stimulated by incubating the cells under stimulatory conditions in the presence of anti-CD3/anti-CD28 antibody conjugated paramagnetic beads and recombinant cytokines (e.g. IL-2, IL-7 and IL-15) for approximately 24 hours. Cells from four donors (Donors D-G) were then transduced with a viral preparation containing nucleic acid encoding a chimeric antigen receptor (CAR). After transduction, the cells were cultivated in the presence of recombinant cytokines (e.g. IL-2, IL-7 and IL-15) in an incubator at 37 degrees Celsius and media was replenished daily. Cells from three donors (Donors A-C) were not subjected to transduction and were cultivated in the presence of recombinant cytokines after stimulation.

Cells were monitored for total cell number for up to approximately 240 hours after the start of the stimulation, and fold expansion over time was determined. Viability was assessed and cells were stained for a marker indicative of cell division (Ki-67) and analyzed by flow cytometry at 72 hours after the start of stimulation.

As shown in **FIGS. 1A-1D,** variability in T cell expansion **(FIGS. 1A** and **1B),** cell viability **(FIG. 1C)** and cell cycle entry **(FIG. 1D)** were observed among CD8+ T cells from different donors.

**Example 2: Characterization of CD57+ and CD57- Populations Following Stimulation of Donor-Derived Cells during Cell Manufacturing**

The phenotypes of CD57+ and CD57- cells were characterized during an exemplary cell manufacturing process to engineer the cells to express a chimeric antigen receptor (CAR).

CD8+ and CD4+ T cells from three exemplary donors (Donors A-C) were subjected to an exemplary process similar to as described in Example 1, but without transduction of the cells. Samples of the cells were collected immediately prior to the start of, and at various time points during stimulation, for up to approximately 216 hours after the start of the stimulation. The cells were stained for various markers, including markers associated with activation (CD25 and CD69), proliferative capacity (CD57), cell division (Ki-67), and various surface markers associated with T cell differentiation phenotypes (e.g., naïve-like T cells, effector T ($T_{EFF}$) cells, memory T cells, central memory T cells ($T_{CM}$), effector memory T ($T_{EM}$) cells, effector memory RA T ($T_{EMRA}$) cells), for analysis by flow cytometry. Hierarchical clustering analysis was performed to assess the association between CD57 expression, Ki-67 expression, and T cell differentiation phenotypes.

As shown in **FIGS. 2A-2C,** CD57+ T cells were present in the population at the start of stimulation, and the percentage of CD57+ cells was reduced in the population during stimulation and cultivation (see **FIG. 2A** and **2B**). The frequency of CD57+ T cells decreased approximately 48 hours after the start of stimulation, which coincided generally with T cell expansion and increased viability. Following stimulation, only a subset of CD8+ T cells entered the cell cycle, based on the expression of Ki-67. Expression of Ki-67 did not increase substantially among CD57+ cells following stimulation. In comparison, the percentage of Ki-67+ cells increased substantially among CD57- cells (see **FIG. 2A** and **2C**). In contrast with the CD8+ cell compartment, among CD4+ cells, CD57+ cells were present at low levels at the start of stimulation, and the percentage of CD57+ cells increased toward the end of the stimulation and cultivation process **(FIG. 2D).** This results are consistent with an observation that the CD8+ cell compartment may be more homogenous than the CD4+ cell compartment.

Notably, the donor-derived compositions exhibited variability in the time required to reach an exemplary threshold cell number for harvest (harvest criterion), which corresponded with variability in expression of various immunophenotype markers. One donor-derived composition required only 7 days of cultivation time to reach harvest criterion. By contrast, another donor-derived composition required 8 days of cultivation time to reach harvest criterion. Analysis of CD57, Ki-67, CD45RA, and CD27 expression (as determined by flow cytometry) between the two donor compositions is shown in **FIGS. 2E** and **2F.** Expression was analyzed beginning at time point t=0, which represented the time just prior to stimulation. As shown in **FIG. 2E,** at all assessed time points, substantially higher CD57 expression was observed in the donor cell composition that required 8 days to reach harvest criterion ("8d to harvest"), compared to the donor cell composition that required 7 days to reach harvest criterion ("7d to harvest"). As shown in **FIG. 2F,** at all time points assessed, cells from the composition requiring 7 days exhibited a higher frequency of CD27+CD45RA+, compared to cells from the composition requiring 8 days. Cells from the composition requiring 8 days exhibited a substantially lower frequency of CD27+ cells at all assessed time points. These observations are consistent with a finding that cell compositions exhibiting a higher frequency of CD27+ cells in the starting material exhibit better expansion and a higher frequency of such cells at the end of the cultivation process.

As shown in **FIG. 3A,** CD57+ T cells were observed to express markers indicative of activation (CD69 and CD25) following stimulation. CD57+ cells exhibited phenotypes associated with activation, and persisted throughout early process stages. Hierarchical clustering analysis showed that CD57+ and Ki67- T cells exhibited phenotypic features associated with more differentiated effector cell populations **(FIG. 3B),** compared to CD57- and Ki67+ cells **(FIG. 3C).** Ki-67+ populations primarily included cells that were CD27+CD28+, and Ki67-populations were enriched for CD57+ cells and corresponded with CD27-CD28- phenotypes.

The results were consistent with an observation that CD57+ T cells, while capable of being stimulated, exhibited phenotypes associated with more terminally differentiated cells and a reduced proliferative capacity. In some aspects, CD27+ T cells were observed to contribute to the majority of expanded cells during the CAR T cell manufacturing process. In comparison, CD57+ T cells did not expand, and contributed minimally to the cells in the final manufactured CAR T cell compositions. In some aspects, the presence of CD57+ cells in a cell population, which may exhibit reduced proliferative capacity, can contribute to variations and heterogeneity in cell populations undergoing the T cell manufacturing process.

**Example 3: Frequency of CD57+ Cells in Input Composition and Effect on Cell Expansion, Viability, Stimulation Reagents and Cytokines**

Input compositions containing donor-matched CD57+ and CD57- cells were mixed at specific ratios and subjected to an exemplary manufacturing process. Cell expansion and viability of the cell compositions were assessed.

CD57+CD8+ T cells and CD57-CD8+ T cells were isolated from a composition of CD8+ T cells obtained from a donor subject by positive selection of CD57+ cells by immunoaffinity-based enrichment. Purity of the isolated populations was determined by flow cytometry. To assess the impact of different frequencies of CD57+ T cells on the manufacturing process, input compositions containing the following frequency of CD57+ cells were generated by mixing the isolated CD57+ and CD57- populations, prior to stimulation: (1) 100% CD57+ cells; (2) 75% CD57+ cells; (3) 25% CD57+ cells; and (4) 0% CD57+ cells. The different titrated input compositions were subjected to an exemplary manufacturing process for CAR-expressing cells, similar to as described in Example 1, including stimulation by incubation with anti-CD3/anti-CD28 conjugated beads and recombinant cytokines, and transduction with a viral preparation containing nucleic acid encoding a CAR. Cells were monitored for total cell number and viability over time for up to approximately 288 hours after the start of the stimulation. Images of the wells of the cell culture plates were obtained at 48 hours after the start of stimulation, and assessed for cell clustering in the presence of the stimulation reagent. The concentration of IL-2 in the culture medium was assessed at 24 and 48 hours after the start of stimulation.

As shown in **FIG. 4A,** input compositions containing higher frequencies of CD57+ cells exhibited slower cell expansion, required longer cultivation time to reach an exemplary threshold cell number for harvest (harvest criterion), and exhibited lower cell viability, compared to input compositions containing lower frequencies of CD57+ cells. Very low or no expansion was observed in the composition containing 100% CD57+ cells. In general, the frequency of CD57+ cells in the input composition was observed to be associated with the duration of culture required to achieve the harvest criterion. As shown in **FIG. 4B,** input compositions containing higher frequencies of CD57+ cells also formed cell clusters in the presence of the stimulation reagent (anti-CD3/anti-CD28 antibody conjugated paramagnetic beads) at 48 hours, demonstrating the cells were responsive to the stimulation. It was observed that, 24 and 48 hours after the start of stimulation, the concentration of IL-2 in the culture media was lower in cultures of input compositions containing higher frequencies of CD57+ cells, compared to cultures of input compositions containing lower frequencies of CD57+ cells **(FIG. 4C).**

The results were consistent with an observation that the frequency of CD57+ cells in the input composition can affect total cell expansion and cell viability. Input compositions with higher frequencies of CD57+ cells required longer cultivation times to reach harvest criterion. In some aspects, CD57+ cells, which were capable of being stimulated and occupying or using stimulation reagents, were observed to consume the IL-2 present in the cultivation conditions and contribute to cell count numbers in the culture. This indicated that the presence of CD57+ cells may affect other cells in the population during the manufacturing process.

## Example 4: Depletion of CD57+ Cells in the Input Composition and Effect on Culture Duration

Input compositions that were depleted of CD57+ cells were subjected to an exemplary manufacturing process, and cell phenotypes and duration of cultivation before reaching harvest criterion were assessed.

CD8+ cells were obtained from four different subjects and each separated into two arms. In one arm, CD57+ cells were depleted from the cell composition to generate an input composition (depleted), and purity was assessed by flow cytometry. In the other arm, the CD8+ cells were used as input composition without depletion of CD57+ cells (undepleted). The depleted and undepleted input compositions were subjected to an exemplary manufacturing process for CAR-expressing cells by viral transduction, similar to as described in Example 1, including stimulation by incubation with anti-CD3/anti-CD28 conjugated beads and recombinant cytokines, transduction with a viral preparation containing nucleic acid encoding a CAR, and cultivation under conditions for expansion. Immediately prior to the start of the stimulation, samples of the cell compositions were stained for Ki67, CD3, CD57, CD27 and CD28. The duration for the depleted and undepleted cultures to reach an exemplary harvest criterion was assessed. The harvest criterion represented an approximately 10-fold expansion of cells.

As shown in **FIG. 5A,** the undepleted input compositions exhibited a varying frequency of CD57 cells. As shown in **FIG. 5B,** the frequency of CD27+ CD28+ cells was higher and more consistent among the depleted input composition, compared to the undepleted input compositions. Flow plots for CD27 expression among the four donor undepleted and depleted input compositions is shown in **FIG. 5C.** The top panel of **FIG. 5C** shows that CD8+ T cells from the undepleted samples from the four donors exhibited varying degrees of CD27 expression. By contrast, as shown in the bottom panel of **FIG. 5C,** depletion of CD8+CD57+ cells from the input compositions improved the starting material consistency across the donors, as evidenced by the similarly consistent CD27 expression. Together, these results demonstrate variability in naïve-like or central memory cells among donor cells in starting donor leukapheresis samples that can be improved by depleting CD57+ cells.

Expression of Ki67, a marker of T cell proliferation and growth, was assessed among total CD3+ T cells in the donor input compositions 72 hours after the start of stimulation. As shown in **FIG. 5D,** Ki67 expression was higher among depleted input compositions, as compared to undepleted compositions.

As shown in **FIG. 5E,** the duration of cultivation to harvest (from the start of stimulation to the time at which harvest criterion was reached) was generally shorter and more consistent among the depleted input compositions, compared to the undepleted input compositions. Expansion of cells from one exemplary donor, as assessed by total T cells at various days following stimulation, is shown in **FIG. 5F.** The results show that the total number of cells expanded from depleted input compositions was observed to be higher than the total number of cells expanded from undepleted input compositions, at multiple time points following stimulation **(FIG. 5F).** These findings are consistent with an observation that depleted populations may exhibit shorter duration to harvest criterion due to a greater number of dividing and total cells.

Following transduction of the depleted ad undepleted cells with an exemplary chimeric antigen receptor (CAR), the percentage of cells expressing the CAR was assessed. As shown in **FIG. 5G,** the percentage of cells expressing the CAR among the depleted donor compositions was more consistent than the percentage of cells expressing the anti-CD19 CAR among the undepleted donor compositions. This result is consistent with an observation that depletion of CD57+ cells may improve the consistency of CAR expression by T cells.

Together, the results were consistent with an observation that selected depletion of CD57+ cells improved expansion and reduced the duration of cultivation required to reach the harvest criterion. Further, the findings indicate that depletion of CD57+ cells may reduce variability in the phenotype of cells in starting material (e.g. CD27+ cells) among various input compositions. In some aspects, the variability in the presence and frequency of CD57+ T cells among various input compositions can impact the CAR T cell manufacturing process, and can result in variability in duration of cultivation and cell composition attributes. In some aspects, depletion of CD57+ T cells in the input composition can improve process control and consistency of the manufactured CAR T cell compositions.

### Example 5: CD57+CD8+ T Cell Frequency in Peripheral Blood of Non-Hodgkin Lymphoma (NHL) Patients

Cells from the peripheral blood of non-Hodgkin lymphoma (NHL) patients were assessed for CD57+ expression and various surface markers associated with T cell differentiation phenotypes.

Separate compositions of CD4+ and CD8+ cells from leukapheresis samples from NHL patients in a clinical study were isolated by immunoaffinity-based enrichment and cryopreserved. The cells from each isolated cell composition were subsequently thawed and stimulated by separately incubating the cells under stimulatory conditions in the presence of anti-CD3/anti-CD28 antibody-conjugated paramagnetic beads and recombinant cytokines (e.g. IL-2, IL-7 and IL-15) for 48 hours. 48 hours after the start of the stimulation, samples from the cell compositions were stained for various markers for analysis by flow cytometry, including markers associated with lineage (CD4 and CD8), proliferative capacity (CD57), cell division (Ki-67), and T cell differentiation phenotypes (e.g., naïve-like T cells, effector T ($T_{EFF}$) cells, memory T cells, central memory T ($T_{CM}$) cells). Hierarchical clustering was performed to assess association between CD57+ expression and a variety of the markers associated with T cell differentiation phenotypes.

As shown in **FIG. 6A,** the frequency of CD57+ cells in the input composition varied among the CD8+ cells from different NHL patients. As shown in **FIG. 6B,** a general inverse relationship between the percentage of CD57+CD8+ cells and the percentage of Ki67+ cells was observed at 48 hours after the start of stimulation. As shown in **FIG. 6C,** hierarchical clustering showed clusters that were distinguished by memory and effector T cell differentiation phenotypes, and these phenotype clusters further clustered samples with either high or low frequencies of CD57+ T cells.

Analysis of CD57+ cells from donor input compositions revealed that Ki67 expression varied among cells with different T cell differentiation immunophenotypes. As shown in **FIG. 6D,** the percentage of live, CD57+ cells expressing Ki67 was lowest in CD27-CD45RA+ cells, and was higher in CD27-CD45RA- cells, CD27+CD45RA+ and CD27+CD45RA- cells.

The separate CD4+ and CD8+ donor cell compositions were analyzed for expression of Ki67, CD27, CD45RA, and CD57. As shown in **FIG. 6E,** a large percentage of CD8+CD57+ cells were CD27-CD45RA+, indicating that these T cells were terminally differentiated effector memory RA (TEMRA) T cells. A high percentage of CD4+CD57+ cells were CD27-CD45RA-, consistent with these cells being effector memory (EM) T cells. These findings demonstrate that CD57+ T cells are differentiated effector T cells of various immunophenotypes.

The results were consistent with an observation of variable frequencies of CD57+ T cells in peripheral circulation in NHL subjects. Similar to the findings with cell compositions generated from donor cells (as described in previous examples), high CD57 expression was observed to be associated with phenotypes indicative of more differentiated cells with reduced proliferative capacity.

### Example 6: Relationship of Number of Doublings and Cell Differentiation to Patient Response

Exemplary therapeutic T cell compositions containing autologous T cells expressing a chimeric antigen-receptor (CAR) specific for CD19 were generated. The anti-CD19 CAR contained an anti-CD19 scFv derived from a murine antibody (variable region derived from FMC63), an immunoglobulin-derived spacer, a transmembrane domain derived from CD28, a costimulatory region derived from 4-1BB, and a CD3-zeta intracellular signaling domain.

For generation of cell compositions for administration to subjects with Relapsed and Refractory Non-Hodgkin's Lymphoma (NHL), autologous cells were isolated from the subjects via leukapheresis. Leukapheresis samples were subjected to a process for generation of CAR-expressing cells. The process involved washing of cells using an automated wash and immunoaffinity based selection for purification of CD4$^+$ and CD8$^+$ T cells, resulting in two compositions, enriched for CD8$^+$ (in which a median of 99%, Inter Quartile Range (IQR) 98-100%, of cells were CD8$^+$) and CD4$^+$ (in which a median of 99%, IQR 99-100%, cells were CD4$^+$) cells, respectively.

Cells of the enriched CD4$^+$ and CD8$^+$ compositions were activated with anti-CD3/anti-CD28 paramagnetic beads and then were separately subjected to lentiviral transduction with a vector encoding an anti-CD19 CAR with a 4-1BB costimulatory domain. The CAR contained an anti-CD19 scFv derived from a murine antibody, an immunoglobulin spacer, a transmembrane domain derived from CD28, a costimulatory region derived from 4-1BB, and a CD3-zeta intracellular signaling domain. Transduced populations then were separately incubated in the presence of recombinant IL-2 and IL-15 cytokines (and additionally recombinant IL-7 for the CD4+ T cell composition) for cell expansion. The incubation under conditions for expansion was carried out in a rocking motion bioreactor until reaching a threshold of about 4-fold expansion. Expanded CD8$^+$ and CD4$^+$ cells were formulated and cryopreserved separately and stored prior to administration. To minimize variations between lots and/or cell compositions derived from different patients, such as those having different patient attributes, in parameters indicative of cell health, cells were held at constant volumes across lots. Cell products exhibited a tight range of viable cell concentrations (based on an assessment of cell compositions for one group of subjects, CD8$^+$: median 31 $\times$ 10$^6$ cells/mL, IQR 28-40 $\times$ 10$^6$ cells/mL, N=38; CD4$^+$: median 35 $\times$ 10$^6$ cells/mL, IQR 31-40 $\times$ 10$^6$, N=36).

The generated T cell compositions were used for administration in subjects with Relapsed and Refractory Non-Hodgkin's Lymphoma (NHL) as described below.

## A. Exemplary Attributes of Therapeutic T Cell Compositions

The generated therapeutic T cell compositions, used for administration in subjects with Relapsed and Refractory Non-Hodgkin's Lymphoma (NHL) described below, were assessed for CCR7 and CD27 using flow cytometry. Surface expression levels of CD4 and truncated receptor used as a surrogate marker, also were assessed.

For each generated therapeutic composition, the number of doublings of the generated cell compositions also was determined based on total nuclear count (TNC) of cells, by dual-fluorescence staining with acridine orange (AO) and either propridum iodide (PI) or DAPI, using the following formula:

$$1)\ Cell\ doublings = \frac{ln\left(\frac{TNC\ at\ harvest}{TNC\ 3\ days\ post-activation}\right)}{ln\ 2}$$

**FIG. 7** shows the correlation between the number of doublings in the process for producing the therapeutic composition and the percentage of CD27+ cells of CD4+CAR+ cells. Similar results were observed for CD8+ T cells. The results show that, in general, a lower number of doublings during the process to generate therapeutic T cell compositions is associated with increased levels of CD27+ cells. Without wishing to be bound by theory, the results are consistent with an observation that an increased percentage of CD27+ cells in a process for producing engineered T cells may be influenced by the limited doublings in the processes.

Studies modeling in-process seed density during the expansion step in a process substantially the same as described above demonstrated that a relatively higher seed density (*e.g.,* 0.35×10^6 cells/mL or greater) was expected to reduce the number of population doublings to achieve harvest criterion compared to a lower seed density (*e.g.,* 0.05×10^6 cells/mL or **lower(FIG. 8A).** Modeling also revealed that a relatively higher seed density **(FIG. 8B)** or a shorter process duration **(FIG. 8C)** also are expected to result in an increased percentage of CD8+CAR+ T cells positive for CD27 in the output therapeutic T cell composition (e.g., drug product) compared to a lower seed density or longer process duration, respectively. These data are consistent with a finding that a higher seed density of cells at the initiation of the expansion step or a shorter process duration can result in an increase in the proportion of central memory cells in the engineered therapeutic T cell composition.

## B. Administration of Anti-CD19 CAR+ T Cell Composition

The therapeutic CAR$^+$ T cell compositions described above were administered to subjects with relapsed or refractory (R/R) aggressive non-Hodgkin's lymphoma (NHL) in a clinical study. Specifically, a cohort of adult human subjects with R/R NHL, including diffuse large B-cell lymphoma (DLBCL), de novo or transformed from indolent lymphoma (NOS), high-grade B-cell lymphoma (including double/triple hit), DLBCL transformed from chronic lymphocytic leukemia (CLL) or marginal zone lymphomas (MZL), primary mediastinal large b-cell lymphoma (PMBCL), and follicular lymphoma grade 3b (FLG3B), were administered with anti-CD19 CAR-expressing T cell compositions. Outcomes were separately assessed for a core subset of subjects within the full cohort (excluding those subjects with a poor performance status (ECOG 2), DLBCL transformed from marginal zone lymphomas (MZL) and/or chronic lymphocytic leukemia (CLL, Richter's), and excluding those subjects with primary mediastinal large b-cell lymphoma (PMBCL), and follicular lymphoma grade 3b (FLG3B) (core cohort)). The core cohort included subjects with DLBCL, NOS and transformed follicular lymphoma (tFL), or high grade B-cell lymphoma (double/triple hit) or high-grade B-cell lymphoma with MYC and BCL2 and/or BCL6 rearrangements with DLBCL histology (double/triple hit) and with Eastern Cooperative Oncology Group performance status (ECOG PS) of 0 or 1. The analysis at the time point presented in this example was based on assessment of a total of 91 subjects in the full cohort (88 (65 from the CORE cohort) assessed for response and 91 (67 from the CORE cohort) assessed for safety) that had been administered the anti-CD19 CAR-expressing cells.

The cryopreserved cell compositions containing anti-CD19 CAR-expressing cells were thawed prior to intravenous administration. The therapeutic T cell dose was administered as a defined cell composition by administering the formulated $CD4^+$ $CAR^+$ cell population and the formulated $CD8^+$ $CAR^+$ population separately administered at a target ratio of approximately 1:1. Subjects were administered a single or double dose of CAR-expressing T cells (each single dose via separate infusions of $CD4^+$ CAR-expressing T cells and $CD8^+$ CAR-expressing T cells, respectively) as follows: a single dose of dose level 1 (DL1) containing $5 \times 10^7$ total CAR-expressing T cells, or a single dose of dose level 2 (DL2) containing $1 \times 10^8$ total CAR-expressing T cells. In some cases, the subjects were administered a double dose of DL1 in which each dose was administered approximately fourteen (14) days apart, administered on day 1 and day 14, including one subject that inadvertently received two DL2 doses via the two-dose schedule, due to a dosing error. The dose level and the target numbers of T cell subsets for the administered composition at DL1 and DL2 are set forth in **Table E1.** In the core cohort, 34 subjects were administered DL1, and 27 subjects were administered DL2.

| Table E1. Target dose level and number of T cell subsets for cell compositions containing anti-CD19 CAR T cells | | | |
|---|---|---|---|
| Dose level | Helper T cell ($T_H$) Dose ($CD4^+CAR^+$) | Cytotoxic T Cell ($T_C$) Dose ($CD8^+CAR^+$) | Total T Cell Dose ($CD3^+$ $CAR^+$) |
| 1 | 25 x $10^6$ | 25 x $10^6$ | 50 x $10^6$ |
| 2 | 50 x $10^6$ | 50 x $10^6$ | 100 x $10^6$ |

**Table E2** shows the overall response and safety outcomes for the full cohort and the core cohort at the two dose levels. The objective response rate (ORR) was 74%, including 52% subjects who showed a complete response (CR). The incidence of any grade of cytokine release syndrome (CRS) was 35%, with 1% severe CRS; and the incidence of any grade of neurotoxicity (NT) was 19%, with 1% severe NT.

| Table E2. Response and Safety After $CAR^+$ Cell Administration | | | | |
|---|---|---|---|---|
| | **FULL** | **CORE** | | |
| | **All Dose Levels** | **All Dose Levels[a]** | **DL1** | **DL2** |
| **Best Overall Response (BOR), n[b]** | 88 | 65 | 34 | 27 |
| ORR, % (95% CI) | 74 (63,83) | 80(68, 89) | 77 (59,89) | 82 (62, 94) |
| CR, % (95% CI) | 52 (41,63) | 55(43, 68) | 47 (30, 65) | 63 (42, 81) |
| **Safety, n[c]** | 91 | 67 | 34 | 29 |
| Any CRS, % (95% CI) | 35 (25, 46) | 36 (24, 48) | 41 (25, 59) | 24 (10, 44) |
| sCRS(grade 3-4), % (95% CI) | 1 (0, 6) | 1 (0, 8) | 38 (0, 15) | 0 |
| Any NT, % (95% CI) | 19 (11,28) | 21 (12, 33) | 24 (11,41) | 17 (6, 36) |
| sNT(grade 3-4), % (95% CI) | 12 (6, 21) | 15 (7, 26) | 21 (9, 38) | 7 (1, 23) |
| [a] Four patients treated on DL1D (dose level 1, two-dose schedule) with similar outcomes. | | | | |
| [b] Includes patients with event of PD, death, or 28-day restaging scans. One patient did not have restaging scans available. | | | | |
| [c] Includes all subjects who have received at least one dose of conforming CAR-expressing cell product 28 days prior to data snapshot date or died. | | | | |

**C. Association between Cell Attributes of Anti-CD19 CAR-Expressing T Cells and Response**

The relationship between certain phenotypic attributes of the CAR+ T cells in the therapeutic compositions and parameters associated with clinical response outcomes were assessed. The correlations between memory phenotype in the composition and function translated to a positive correlation between central memory subset composition and peak *in vivo* expansion of CAR+ cells ($\rho$=0.42, P=0.002), and progression-free survival (PFS) (Kaplan-Meier survival estimate, P=0.0164) that were observed. **FIGS. 9A-9D** show the Kaplan-Meier survival curves for subjects who were administered CAR+ T cell compositions, divided into groups that were administered compositions containing a frequency of CCR7+CD27+ CAR+ T cells among CD4+ CAR+ T cells **(FIG. 9A** for progression free survival, **FIG. 9C** for duration of response) and among CD8+ CAR+ T cells **(FIG. 9B** for progression free survival, **FIG. 9D** for duration of response) that is above or below a certain threshold level. Higher frequency of CCR7+CD27+ memory cells in the composition was observed to be correlated with longer progression free survival.

Univariate analysis revealed an inverse correlation between T cell population doublings (PDL) during the process for producing the therapeutic T cell composition and the probability of progression free survival (PFS) in Non-Hodgkin Lymphoma (NHL) patients. **FIG. 10** shows a PFS curve based on an optimal-split log-rank test for patients with "high" (>6 PDL) or "low" (<6 PDL) numbers of PDL in CD8+/CAR+ T cells. This result is consistent with a finding that factors that may limit the number of population doublings of T cells in a process for producing a therapeutic T cell composition may improve the likelihood of subjects exhibiting progression free survival that is durable.

**Example 7: Assessment of Features of Selected (Input) Cell Compositions in a Process for Producing a Therapeutic Cell Composition.**

Phenotypic attributes of T cells selected from subjects with Relapsed and Refractory Non-Hodgkin's Lymphoma (NHL), prior to engineering with an anti-CD19 CAR by the process described in Example 6, were assessed. CD4+ and CD8+ T cells were selected by immunoaffinity-based enrichment from leukapheresis of human peripheral blood mononuclear cells (PBMC) from a plurality of subjects. A composition of such selected T cells (before genetic engineering, designated "pre-engineering composition") were assessed for expression of cell surface markers indicative of certain T cell subtypes, such as effector memory or central memory cell subtypes, including C-C chemokine receptor type 7 (CCR7), CD27 and CD45RA. Surface expression of CD4 or CD8 was also assessed.

Assessment of the enriched CD4+ and CD8+ compositions (e.g., input compositions) revealed that the percentage of T cells positive for naïve-like markers (e.g. CD27+CD28+ T cells), such as present on central memory T cell subsets, was highly variable between NHL patients **(FIG. 11)**. These data indicate that interpatient T cell heterogeneity of central memory T cell subsets exists in selected (input) T cell compositions used to generate CAR+ T cell therapeutic T cell compositions.

The selected (input) T cell compositions were used to generate CD4+ and CD8+ therapeutic T cell compositions substantially as described in Example 6. The number of doublings of the generated cell compositions also was determined based on total nuclear count (TNC) of cells as described in Example 6. The correlation between phenotypic attributes of cells in the selected (input) T cell compositions and the number of doublings in the process for producing the therapeutic composition was determined. As shown in **FIG. 12,** a higher percentage of effector memory CD4+ T cells identified by negative staining for CD45RA and CCR7 (CD45RA-CCR7-) in the enriched CD4+ (input) compositions correlated positively with the number of population doublings during production of the therapeutic T cell composition. A similar result was observed for CD8+ T cells.

The above results are supportive of an approach that includes reducing the percentage of effector memory T cells and/or enriching for T cells positive for markers of naïve-like or central memory cell subsets in an input composition used in a process for producing an engineered T cell composition. Consistent with the results above, such an approach may improve one or more feature of an engineered therapeutic T cell composition, such as reducing patient-to-patient variability, lowering the number of population doublings in a process for producing an engineered therapeutic T cell composition and/or increasing the likelihood a subject may exhibit PFS that is durable.

**Example 8: Kinetics of non-expanded and expanded engineering processes**

Human T cells (CD4+ and CD8+) were engineered with a chimeric antigen receptor (CAR) by a variety of manufacturing process, including processes that did not include a cultivation step for expansion (non-expansion cohort) and processes that did include a cultivation step for expansion (expansion cohort). A composite analysis of cells produced during and after manufacturing runs by the various processes was carried out. The manufacturing runs for producing engineered T cell compositions included at-scale runs as described in Example 8 as well as processes as described below. The manufacturing runs also included scale-down models (SDMs) that were carried out substantially the same as the manufacturing runs but in which a lower number of T cells were used in the process for engineering cells. In general, the scale-down manufacturing runs shared the process activities described in Table E3.

In the analyzed processes, the T cells were engineered with either an anti-CD19 CAR or an anti-BCMA CAR. The exemplary anti-CD19 CAR contained an anti-CD19 scFv derived from a murine antibody FMC63, an immunoglobulin spacer, a transmembrane domain derived from CD28, a costimulatory region derived from 4-1BB, and a CD3-zeta intracellular signaling domain. The vector also encoded a truncated receptor molecule that served as a surrogate marker for CAR expression that was separated from the CAR construct by a T2A sequence. The exemplary anit-BCMA CAR contained an scFv antigen-binding domain specific for BCMA, a CD28 transmembrane region, a 4-1BB costimulatory signaling region, and a CD3-zeta derived intracellular signaling domain. The vector also encoded a truncated receptor molecule that served as a surrogate marker for CAR expression that was separated from the CAR construct by a T2A sequence.

The processes included stimulation of the T cells either with anti-CD3/anti-CD28 paramagnetic beads or with anti-CD3/anti-CD28 Fab conjugated oligomeric streptavidin mutein reagents. The oligomeric reagent contained a polymer of a streptavidin mutein designated STREP-TACTIN® M2 (a streptavidin homo-tetramer containing the mutein sequence of amino acids set forth in SEQ ID NO:73 (International published app. No. WO2018/197949). The streptavidin mutein is also described in U.S. Patent No. 6,103,493 and Voss and Skerra (1997) Protein Eng., 1:975-982, and Argarana et al. (1986) Nucleic Acids Research, 1871-1882). Stimulatory agents (anti-CD3 and anti-CD28 Fab fragments) were multimerized by reversible binding to oligomeric streptavidin mutein reagent. Anti-CD3 and anti-CD28 Fab fragments were reversibly bound to the streptavidin mutein oligomer via a streptavidin peptide-binding partner fused to each Fab fragment. The anti-CD3 Fab fragment was derived from the CD3 binding monoclonal antibody produced by the hybridoma cell line OKT3 (ATCC® CRL-8001™; see also U.S. Patent No. 4,361,549), and contained the heavy chain variable domain and light chain variable domain of the anti-CD3 antibody OKT3 described in Arakawa et al J. Biochem. 120, 657-662 (1996). These sequences are set forth in SEQ ID NOs: 93 and 94, respectively. The anti-CD28 Fab fragment was derived from antibody CD28.3 (deposited as a synthetic single chain Fv construct under GenBank Accession No. AF451974.1; see also Vanhove et al., BLOOD, 15 July 2003, Vol. 102, No. 2, pages 564-570) and contained the heavy and light chain variable domains of the anti-CD28 antibody CD28.3 set forth in SEQ ID NOS: 91 and 92, respectively. The Fab fragments were individually fused at the carboxy-terminus of their heavy chain to a streptavidin peptide-binding sequence containing a sequential arrangement of two streptavidin binding modules having the sequence of amino acids SAWSHPQFEK(GGGS)2GGSAWSHPQFEK (SEQ ID NO: 79). The peptide-tagged Fab fragments were recombinantly produced (see International Patent App. Pub. Nos. WO 2013/011011 and WO 2013/124474). Binding of the peptide-tagged anti-CD3 and anti-CD28 to the oligomeric strepaviding mutein reagent can be disrupted, or reversed, by addition of D-biotin. D-biotin competes with the strep-tag on the agents for binding to the binding partner on the streptavidin mutein, thereby disrupting binding.

Cells were collected at various times during and at the end of the manufacturing runs, and were counted, and assessed for viability and by flow cytometry following staining with antibodies recognizing surface markers including CD4, CD8, CCR7, CD27, and CD45RA.

| Table E3 | |
| --- | --- |
| **Day of Process** | **Summary of Activity** |
| Day 1 | Cells are stimulated with stimulatory reagent (e.g. anti-CD3/anti-CD28 paramagnetic beads or anti-CD3/anti-CD28 Fab conjugated oligomeric streptavidin mutein reagents) |
| Day 2 | Cells are transduced by spinoculation with lentivirus encoding a CAR (e.g. anti-CD19 CAR or anti-BCMA CAR) |
| Day 3 | For non-expansion cohort, biotin is added for cells stimulated with anti-CD3/anti-CD28 Fab conjugated oligomeric streptavidin mutein reagents |
| Day 4 | Cells are in recovery |
| Day 5 | For non-expansion cohort, cells activated with anti-CD3/anti-CD28 paramagnetic beads, cells are debeaded<br>Cells are harvested as a non-expanded cohort; cells are washed in formulation buffer and cryopreserved |
| Day 5 | For expansion cohort, cells are transferred to a shaking incubator |
| Day 7 | For cells in expansion cohort activated with anti-CD3/anti-CD28 Fab conjugated oligomeric streptavidin mutein reagents, biotin is added |

(continued)

| Table E3 | |
|---|---|
| **Day of Process** | **Summary of Activity** |
| Day 9 | For expansion cohort, cells activated with anti-CD3/anti-CD28 paramagnetic beads, cells are debeaded<br>Cells are harvested as an expanded cohort; cells are washed in formulation buffer and cryopreserved |

## A. T cell Engineering Processes

T cell compositions produced using different non-expanded processes that differed in various features, including the starting source of cells (cryopreserved apheresis or fresh apheresis), concentration of oligomeric stimulatory reagent, and number of cells used for stimulation, were compared. T cell compositions also were produced in which cells were further cultivated for expansion. The processes were carried out on healthy donors or on patient donors.

## 1. Non-Expanded Processes

Leukapheresis samples were collected from human donors and washed. CD4+ and CD8+ cells were selected directly by immunoaffinity-based selection from the leukapheresis samples which had not been cryopreserved. After selection, the separate CD4+ and CD8+ T cell compositions were cryopreserved and then were thawed, and then the selected CD4+ and CD8+ T cells were mixed at a ratio of 1:1 of viable CD4+ T cells to viable CD8+ T cells to produce an input composition. About $600 \times 10^6$ cells from the mixed input cell composition (about $300 \times 10^6$ CD4+ and $300 \times 10^6$ CD8+) were stimulated by incubation with 0.8 $\mu$g per $1 \times 10^6$ cells anti-CD3/anti-CD28 Fab conjugated oligomeric streptavidin mutein reagents generated as described above in this Example. The stimulation was carried out for between 18-30 hours (24 $\pm$ 6 hours) in serum-free complete media containing basal media (*e.g.*, CTS™ OpTmizer basal media, Thermo Fisher), a T cell supplement (*e.g.*, 2.6% OpTmizer® T-cell Expansion Supplement, Thermo Fisher), an immune cell serum replacement (*e.g.*, 2.5% CTS™ Immune Cell Serum Replacement), 2 mM L-glutamine, a dipeptide form of L-glutamine (*e.g.*, 1.0% Glutamax™, Thermo Fisher), recombinant 100 IU/mL IL-2, recombinant 600 IU/mL IL-7, and recombinant 100 IU/mL IL-15. After stimulation, up to $300 \times 10^6$ cells were transduced by spinoculation with a lentiviral vector encoding either the exemplary anti-BCMA CAR or the exemplary anti-CD19 CAR.

After spinoculation, the cells were washed and resuspended at a density of up to about $0.75 \times 10^6$ cells/mL in basal media (*e.g.*, CTS™ OpTmizer basal media, Thermo Fisher) with 2 mM glutamine but without the addition of recombinant cytokines, and incubated at about 37.0 °C in an incubator. After about 48 hours $\pm$ 6 hours after initiation of the stimulation (about 24 hours after beginning the incubation), 1.0 mM D-biotin was added and mixed with the cells to dissociate anti-CD3 and anti-CD28 Fab reagents from the oligomeric streptavidin reagent. The cells were further incubated for an additional about 48 hours (about 96 hours $\pm$ 6 hours after initiation of stimulation or until day 5 of the process), and then were formulated with a cryoprotectant.

In another process, leukapheresis samples were collected from human donors, washed and cryopreserved. The cryopreserved leukapheresis samples were thawed, and separate compositions of CD4+ and CD8+ cells were selected from each sample by immunoaffinity-based selection, and then the CD4+ and CD8+ T cells were mixed with the goal to produce an input composition of up to about $900 \times 10^6$ viable CD4+ and CD8+ T cells, in which the ratio of viable CD4+ T cells to viable CD8+ T cells varied. The mixed input cell composition were stimulated by incubation with 1.2 $\mu$g per $1 \times 10^6$ cells anti-CD3/anti-CD28 Fab conjugated oligomeric streptavidin mutein reagents generated as described above in this Example (where 1.2 $\mu$g of the oligomeric stimulatory reagent includes 0.9 $\mu$g of oligomeric particles and 0.15 $\mu$g of anti-CD3 Fabs and 0.15 $\mu$g of anti-CD28 Fabs). The stimulation was carried out for between 16-24 hours (20 $\pm$ 4 hours) in the same serum-fee complete media described above. After stimulation, up to about $600 \times 10^6$ cells were transduced by spinoculation with a lentiviral vector encoding a CAR, in this case the same exemplary anti-BCMA CAR or exemplary anti-CD19 CAR described above. In this study, the cells that were incubated with the higher concentration of the oligomeric streptavidin mutein reagents exhibited an improved transduction efficiency (data not shown). After the spinoculation in this process, the cells were washed and resuspended at a density of $0.75 \times 10^6$ cells/mL in basal media ((*e.g.*, CTS™ OpTmizer basal media, Thermo Fisher) with 2 mM glutamine and 2.6% T cell supplement (e.g. 2.6% OpTmizer® supplement, Thermofisher) without the addition of recombinant cytokines, and incubated at about 37.0 °C in an incubator. After about 48 hours $\pm$ 6 hours after initiation of the stimulation (about 24 hours after beginning the incubation), 1.0 mM D-biotin was added and mixed with the cells to dissociate anti-CD3 and anti-CD28 Fab reagents from oligomeric streptavidin reagent. The cells were further incubated for an additional about 48 hours (about 96 hours ± 6 hours after initiation of stimulation or until day 5 of the process), and then were formulated with a cryoprotectant.

2. Expanded Process

In one process, anti-CD19 CAR T cells were engineered by the non-expanded process described above.

In another process, separate compositions of CD4+ and CD8+ cells were selected from human leukapheresis samples and were cryofrozen. The selected cell compositions were subsequently thawed and mixed at a ratio of 1:1 of viable CD4+ T cells to viable CD8+ T cells. Approximately $300 \times 10^6$ T cells ($150 \times 10^6$ CD4 and $150 \times 10^6$ CD8+ T cells) of the mixed composition were stimulated in the presence of paramagnetic polystyrene-coated beads with attached anti-CD3 and anti-CD28 antibodies at a 1:1 bead to cell ratio in serum free media containing recombinant IL-2, IL-7 and IL-15 for between 18 to 30 hours. Following the incubation, approximately $100 \times 106$ viable cells from the stimulated cell composition were concentrated in the serum free media containing recombinant IL-2, IL-7 and IL-15 The cells were transduced, by spinoculation at approximately 1600 g for 60 minutes, with a lentiviral vector encoding an exemplary CAR, in this case the exemplary anti-BCMA CAR described above. After spinoculation, the cells were resuspended in the serum free media containing recombinant IL-2, IL-7 and IL-15, and incubated for about 18 to 30 hours at about 37 °C. The cells were then cultivated for expansion by transfer to a bioreactor (e.g. a rocking motion bioreactor) in about 500 mL of the exemplary serum free media containing twice the concentration of IL-2, IL-7 and IL-15 as used during the incubation and transduction steps. When a set viable cell density was achieved, perfusion was initiated, where media was replaced by semi-continuous perfusion with continual mixing. The cells were cultivated the next day in the bioreactor until a threshold cell density of about $3 \times 10^6$ cells/mL was achieved, which typically occurred in a process involving 6-7 days of expansion. The anti-CD3 and anti-CD28 antibody conjugated paramagnetic beads were removed from the cell composition by exposure to a magnetic field. The cells where then collected, formulated and cryopreserved.

**B. Process Metrics**

Process metrics such as total live cells (FIG. 13A) and viability (FIG. 13B) were monitored during the manufacturing runs. As shown in FIG. 13A, minimal expansion of cells was observed at the day 5 time point, with robust expansion beginning after Day 5. FIG. 13B shows an initial decrease in viability of cells in the manufacturing runs which begins to increase as the cells start to expand after Day 5 of the processes.

Results of comparison of memory/differentiation phenotype at different time points in the manufacturing runs are shown in FIG. 13C (by CD5RA and CCR7 expression) and FIG. 13D (by CD27 and CCR7 expression). As shown, assessment of cells early during the manufacturing runs show that cells at about day 5 are substantially more enriched for less differentiated cell types, such as CCR7+CD45RA- or CCR7+CD27+ cells, compared to cells both at earlier times in the process (e.g. Day 1 or Day 2) or later times in the process (e.g. Day 9). The number of CD57+ cells was analyzed over the duration of the manufacturing process. As shown in FIG. 14, the number of CD8+CD57+ cells decreased over the course of the manufacturing process, while the number of CD4+CD57+ cells remained low throughout.

Sequences

| # | SEQUENCE | ANNOTATION |
|---|----------|------------|
| 1 | ESKYGPPCPPCP | spacer (IgG4hinge) (aa) |
| 2 | GAATCTAAGTACGGACCGCCCTGCCCCCCTTGCCCT | spacer (IgG4hinge) (nt) |
| 3 | ESKYGPPCPPCPGQPREPQVYTLPPSQEEMTKNQVSLTCLVKGFYPSDIAV<br>EWESNGQPENNYKTTPPVLDSDGSFFLYSRLTVDKSRWQEGNVFSCSVMHE<br>ALHNHYTQKSLSLSLSLGK | Hinge-CH3 spacer |
| 4 | ESKYGPPCPPCPAPEFLGGPSVFLFPPKPKDTLMISRTPEVTCVVVDVSQE<br>DPEVQFNWYVDGVEVHNAKTKPREEQFNSTYRVVSVLTVLHQDWLNGKEYK<br>CKVSNKGLPSSIEKTISKAKGQPREPQVYTLPPSQEEMTKNQVSLTCLVKG<br>FYPSDIAVEWESNGQPENNYKTTPPVLDSDGSFFLYSRLTVDKSRWQEGNV<br>FSCSVMHEALHNHYTQKSLSLSLGK | Hinge-CH2-CH3 spacer |

127

(continued)

| # | SEQUENCE | ANNOTATION |
|---|---|---|
| 5 | RWPESPKAQASSVPTAQPQAEGSLAKATTAPATTRNTGRGGEEKKKEKEKE EQEERETKTPECPSHTQPLGVYLLTPAVQDLWLRDKATFTCFVVGSDLKDA HLTWEVAGKVPTGGVEEGLLERHSNGSQSQHSRLTLPRSLWNAGTSVTCTL NHPSLPPQRLMALREPAAQAPVKLSLNLLASSDPPEAASWLLCEVSGFSPP NILLMWLEDQREVNTSGFAPARPPPQPGSTTFWAWSVLRVPAPPSPQPATY TCVVSHEDSRTLLNASRSLEVSYVTDH | IgD-hinge-Fc |
| 6 | LEGGGEGRGSLLTCGDVEENPGPR | T2A |
| 7 | MLLLVTSLLLCELPHPAFLLIPRKVCNGIGIGEFKDSLSINATNIKHFKNC TSISGDLHILPVAFRGDSFTHTPPLDPQELDILKTVKEITGFLLIQAWPEN RTDLHAFENLEIIRGRTKQHGQFSLAVVSLNITSLGLRSLKEISDGDVIIS GNKNLCYANTINWKKLFGTSGQKTKIISNRGENSCKATGQVCHALCSPEGC WGPEPRDCVSCRNVSRGRECVDKCNLLEGEPREFVENSECIQCHPECLPQA MNITCTGRGPDNCIQCAHYIDGPHCVKTCPAGVMGENNTLVWKYADAGHVC HLCHPNCTYGCTGPGLEGCPTNGPKIPSIATGMVGALLLLLVVALGIGLFM | tEGFR |
| 8 | FWVLVWGGVLACYS LLVTVAFII FWV | CD28 (amino acids 153-179 of Accession No. P 10747) |
| 9 | IEVMYPPPYLDNEKSNGTIIHVKGKHLCPSPLFPGPSKP FWVLVVVGGVLACYSLLVTVAFIIFWV | CD28 (amino acids 114-179 of Accession No. P 10747) |
| 10 | RSKRSRLLHSDYMNMTPRRPGPTRKHYQPYAPPRDFAAYRS | CD28 (amino acids 180-220 of P10747) |
| 11 | RSKRSRGGHSDYMNMTPRRPGPTRKHYQPYAPPRDFAAYRS | CD28 (LL to GG) |
| 12 | KRGRKKLLYIFKQPFMRPVQTTQEEDGCSCRFPEEEEGGCEL | 4-1BB (amino acids 214-255 of Q07011.1) |
| 13 | RVKFSRSADAPAYQQGQNQLYNELNLGRREEYDVLDKRRGRDPEMGGKPRR KNPQEGLYNELQKDKMAEAYSEIGMKGERRRGKGHDGLYQGLSTATKDTYD ALHMQALPPR | CD3 zeta |
| 14 | RVKFSRSAEPPAYQQGQNQLYNELNLGRREEYDVLDKRRGRDPEMGGKPRR KNPQEGLYNELQKDKMAEAYSEIGMKGERRRGKGHDGLYQGLSTATKDTYD ALHMQALPPR | CD3 zeta |
| 15 | RVKFSRSADAPAYKQGQNQLYNELNLGRREEYDVLDKRRGRDPEMGGKPRR KNPQEGLYNELQKDKMAEAYSEIGMKGERRRGKGHDGLYQGLSTATKDTYD ALHMQALPPR | CD3 zeta |
| 16 | RKVCNGIGIGEFKDSLSINATNIKHFKNCTSISGDLHILPVAFRGDSFTHT PPLDPQELDILKTVKEITGFLLIQAWPENRTDLHAFENLEIIRGRTKQHGQ FSLAVVSLNITSLGLRSLKEISDGDVIISGNKNLCYANTINWKKLFGTSGQ KTKIISNRGENSCKATGQVCHALCSPEGCWGPEPRDCVSCRNVSRGRECVD KCNLLEGEPREFVENSECIQCHPECLPQAMNITCTGRGPDNCIQCAHYIDG PHCVKTCPAGVMGENNTLVWKYADAGHVCHLCHPNCTYGCTGPGLEGCPTN GPKIPSIATGMVGALLLLLVVALGIGLFM | tEGFR |
| 17 | EGRGSLLTCGDVEENPGP | T2A |
| 18 | GSGATNFSLLKQAGDVEENPGP | P2A |
| 19 | ATNFSLLKQAGDVEENPGP | P2A |
| 20 | QCTNYALLKLAGDVESNPGP | E2A |
| 21 | VKQTLNFDLLKLAGDVESNPGP | F2A |
| 22 | -PGGG-(SGGGG)5-P- wherein P is proline, G is glycine and S is serine | Linker |

(continued)

| # | SEQUENCE | ANNOTATION |
|---|----------|------------|
| 23 | GSADDAKKDAAKKDGKS | Linker |
| 24 | atgcttctcctggtgacaagccttctgctctgtgagttaccacacccagca ttcctcctgatccca | GMCSFR alpha chain signal sequence |
| 25 | MLLLVTSLLLCELPHPAFLLIP | GMCSFR alpha chain signal sequence |
| 26 | MALPVTALLLPLALLLHA | CD8 alpha signal peptide |
| 27 | EPKSCDKTHTCPPCP | Hinge |
| 28 | ERKCCVECPPCP | Hinge |
| 29 | ELKTPLGDTHTCPRCPEPKSCDTPPPCPRCPEPKSCDTPPPCPRCPEPKSC DTPPPCPRCP | Hinge |
| 30 | ESKYGPPCPSCP | Hinge |
| 31 | X$_1$PPX$_2$P X1 is glycine, cysteine or arginine X2 is cysteine or threonine | Hinge |
| 32 | YGPPCPPCP | Hinge |
| 33 | KYGPPCPPCP | Hinge |
| 34 | EWVKYGPPCPPCP | Hinge |
| 35 | RASQDISKYLN | CDR L1 |
| 36 | SRLHSGV | CDR L2 |
| 37 | GNTLPYTFG | CDR L3 |
| 38 | DYGVS | CDR H1 |
| 39 | VIWGSETTYYNSALKS | CDR H2 |
| 40 | YAMDYWG | CDR H3 |
| 41 | EVKLQESGPGLVAPSQSLSVTCTVSGVSLPDYGVSWIRQPPRKGLEWLGVI WGSETTYYNSALKSRLTIIKDNSKSQVFLKMNSLQTDDTAIYYCAKHYYYG GSYAMDYWGQGTSVTVSS | VH |
| 42 | DIQMTQTTSSLSASLGDRVTISCRASQDISKYLNWYQQKPDGTVKLLIYHT SRLHSGVPSRFSGSGSGTDYSLTISNLEQEDIATYFCQQGNTLPYTFGGGT KLEIT | VL |
| 43 | DIQMTQTTSSLSASLGDRVTISCRASQDISKYLNWYQQKPDGTVKLLIYHT SRLHSGVPSRFSGSGSGTDYSLTISNLEQEDIATYFCQQGNTLPYTFGGGT KLEITGSTSGSGKPGSGEGSTKGEVKLQESGPGLVAPSQSLSVTCTVSGVS LPDYGVSWIRQPPRKGLEWLGVIWGSETTYYNSALKSRLTIIKDNSKSQVF LKMNSLQTDDTAIYYCAKHYYYGGSYAMDYWGQGTSVTVSS | scFv |
| 44 | KASQNVGTNVA | CDR L1 |
| 45 | SATYRNS | CDR L2 |
| 46 | QQYNRYPYT | CDR L3 |
| 47 | SYWMN | CDR H1 |
| 48 | QIYPGDGDTNYNGKFKG | CDR H2 |
| 49 | KTISSVVDFYFDY | CDR H3 |
| 50 | EVKLQQSGAELVRPGSSVKISCKASGYAFSSYWMNWVKQRPGQGLEWIGQI YPGDGDTNYNGKFKGQATLTADKSSSTAYMQLSGLTSEDSAVYFCARKTIS SVVDFYFDYWGQGTTVTVSS | VH |

| # | SEQUENCE | ANNOTATION |
|---|----------|------------|
| 51 | DIELTQSPKFMSTSVGDRVSVTCKASQNVGTNVAWYQQKPGQSPKPLIYSA TYRNSGVPDRFTGSGSGTDFTLTITNVQSKDLADYFCQQYNRYPYTSGGGT KLEIKR | VL |
| 52 | GGGGSGGGGSGGGGS | Linker |
| 53 | EVKLQQSGAELVRPGSSVKISCKASGYAFSSYWMNWVKQRPGQGLEWIGQI YPGDGDTNYNGKFKGQATLTADKSSSTAYMQLSGLTSEDSAVYFCARKTIS SVVDFYFDYWGQGTTVTVSSGGGGSGGGGSGGGGSDIELTQSPKFMSTSVG DRVSVTCKASQNVGTNVAWYQQKPGQSPKPLIYSATYRNSGVPDRFTGSGS GTDFTLTITNVQSKDLADYFCQQYNRYPYTSGGGTKLEIKR | scFv |
| 54 | HYYYGGSYAMDY | HC-CDR3 |
| 55 | HTSRLHS | LC-CDR2 |
| 56 | QQGNTLPYT | LC-CDR3 |
| 57 | gacatccagatgacccagaccacctccagcctgagcgccagcctgggcgac cgggtgaccatcagctgccgggccagccaggacatcagcaagtacctgaac tggtatcagcagaagcccgacggcaccgtcaagctgctgatctaccacacc agccggctgcacagcggcgtgcccagccggtttagcggcagcggctccggc accgactacagcctgaccatctccaacctggaacaggaagatatcgccacc tactttgccagcagggcaacacactgccctacacctttggcggcggaaca aagctggaaatcaccggcagcacctccggcagcggcaagcctggcagcggc gagggcagcaccaagggcgaggtgaagctgcaggaaagcggccctggcctg gtggcccccagccagagcctgagcgtgacctgcaccgtgagcggcgtgagc ctgcccgactacggcgtgagctggatccggcagccccccaggaagggcctg aatggctgggcgtgatctggggcagcgagaccacctactacaacagcgcc ctgaagagccggctgaccatcatcaaggacaacagcaagagccaggtgttc ctgaagatgaacagcctgcagaccgacgacaccgccatctactactgcgcc aagcactactactacggcggcagctacgccatggactactggggccagggc accagcgtgaccgtgagcagc | Sequence encoding scFv |
| 58 | GSTSGSGKPGSGEGSTKG | Linker |
| 59 | ASTKGPSVFPLAPCSRSTSESTAALGCLVKDYFPEPVTVSWNSGALTSGVH TFPAVLQSSGLYSLSSVVTVPSSNFGTQTYTCNVDHKPSNTKVDKTVERKC CVECPPCPAPPVAGPSVFLFPPKPKDTLMISRTPEVTCVVVDVSHEDPEVQ FNWYVDGVEVHNAKTKPREEQFNSTFRVVSVLTVVHQDWLNGKEYKCKVSN KGLPAPIEKTISKTKGQPREPQVYTLPPSREEMTKNQVSLTCLVKGFYPSD ISVEWESNGQPENNYKTTPPMLDSDGSFFLYSKLTVDKSRWQQGNVFSCSV MHEALHNHYTQKSLSLSPGK | Human IgG2 Fc (Uniprot P01859) |
| 60 | ASTKGPSVFPLAPCSRSTSESTAALGCLVKDYFPEPVTVSWNSGALTSGVH TFPAVLQSSGLYSLSSVVTVPSSSLGTKTYTCNVDHKPSNTKVDKRVESKY GPPCPSCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKCKVS NKGLPSSIEKTISKAKGQPREPQVYTLPPSQEEMTKNQVSLTCLVKGFYPS DIAVEWESNGQPENNYKTTPPVLDSDGSFFLYSRLTVDKSRWQEGNVFSCS VMHEALHNHYTQKSLSLSLGK | Human IgG4 Fc (Uniprot P01861) |
| 61 | GVQVETISPGDGRTFPKRGQTCVVHYTGMLEDGKKMDSSRDRNKPFKFMLG KQEVIRGWEEGVAQMSVGQRAKLTISPDYAYGATGHPGIIPPHATLVFDVE LLKLE | FKBP |
| 62 | GVQVETISPGDGRTFPKRGQTCVVHYTGMLEDGKKVDSSRDRNKPFKFMLG KQEVIRGWEEGVAQMSVGQRAKLTISPDYAYGATGHPGIIPPHATLVFDVE LLKLE | FKBP12v36 |
| 63 | MGSNKSKPKDASQRRR | acylation motif |

(continued)

| # | SEQUENCE | ANNOTATION |
|---|----------|------------|
| 64 | Met-Gly-Cys-Xaa-Cys | dual acylation region |
| 65 | Cys-Ala-Ala-Xaa | CAAX box |
| 66 | DPSKDSKAQVSAAEAGITGTWYNQLGSTFIVTAGADGALTGTYESAVGNAE SRYVLTGRYDSAPATDGSGTALGWTVAWKNNYRNAHSATTWSGQYVGGAEA RINTQWLLTSGTTEANAWKSTLVGHDTFTKVKPSAASIDAAKKAGVNNGNP LDAVQQ | Streptavidin Species: Streptomyces avidinii UniProt No. P22629 |
| 67 | EAGITGTWYNQLGSTFIVTAGADGALTGTYESAVGNAESRYVLTGRYDSAP ATDGSGTALGWTVAWKNNYRNAHSATTWSGQYVGGAEARINTQWLLTSGTT EANAWKSTLVGHDTFTKVKPSAAS | Minimal streptavidin Species: Streptomyces avidinii |
| 68 | EAGITGTWYNQLGSTFIVTAGADGALTGTY**IGAR**GNAESRYVLTGRYDSAP ATDGSGTALGWTVAWKNNYRNAHSATTWSGQYVGGAEARINTQWLLTSGTT EANAWKSTLVGHDTFTKVKPSAAS | Mutein Streptavidin Ile44-Gly45-Ala-46-Arg47 Species: Streptomyces avidinii |
| 69 | WSHPQFEK | Strep-tag® II |
| 70 | WSHPQFEKGGGSGGGSGGGSWSHPQFEK | Twin-Strep-tag |
| 71 | WSHPQFEKGGGSGGGSWSHPQFEK | Twin-Strep-tag |
| 72 | WSHPQFEKGGGSGGGSGGSAWSHPQFEK | Twin-Strep-tag |
| 73 | MEAGITGTWYNQLGSTFIVTAGADGALTGTY**IGAR**GNAESRYVLTGRYDSA PATDGSGTALGWTVAWKNNYRNAHSATTWSGQYVGGAEARINTQWLLTSGT TEANAWKSTLVGHDTFTKVKPSAAS | Mutein Streptavidin Ile44-Gly45-Ala-46-Arg47 Species: Streptomyces avidinii |
| 74 | -Trp-Xaa-His-Pro-Gln-Phe-Yaa-Zaa- | Streptavidin-binding peptide Xaa is any amino acid; Yaa is Gly or Glu Zaa is Gly, Lys or Arg |
| 75 | Trp-Arg-His-Pro-Gln-Phe-Gly-Gly | Streptavidin binding peptide, Strep-tag® |
| 76 | Trp-Ser-His-Pro-Gln-Phe-Glu-Lys-(Xaa)n-Trp-Ser-His-Pro-Gln-Phe-Glu-Lys- | Sequential modules of streptavidin-binding peptide Xaa is any amino acid; n is either 8 or 12 |
| 77 | Trp-Ser-His-Pro-Gln-Phe-Glu-Lys-(GlyGlyGlySer)n-Trp-Ser-His-Pro-Gln-Phe-Glu-Lys | Sequential modules of streptavidin-binding peptide; n is 2 or 3 |
| 78 | SAWSHPQFEKGGGSGGGSGGGSWSHPQFEK | Twin-Strep-tag |
| 79 | SAWSHPQFEKGGGSGGGSGGSAWSHPQFEK | Twin-Strep-tag |
| 80 | DPSKDSKAQVSAAEAGITGTWYNQLGSTFIVTAGADGALTGTY**VTAR**GNAE SRYVLTGRYDSAPATDGSGTALGWTVAWKNNYRNAHSATTWSGQYVGGAEA RINTQWLLTSGTTEANAWKSTLVGHDTFTKVKPSAASIDAAKKAGVNNGNP LDAVQQ | Mutein Streptavidin Val44-Thr45-Ala46-Arg47 Streptomyces avidinii |

| # | SEQUENCE | ANNOTATION |
|---|----------|------------|
| 81 | EAGITGTWYNQLGSTFIVTAGADGALTGTY**VTAR**GNAESRYVLTGRYDSAP ATDGSGTALGWTVAWKNNYRNAHSATTWSGQYVGGAEARINTQWLLTSGTT EANAWKSTLVGHDTFTKVKPSAAS | Mutein Streptavidin Val44-Thr45-Ala46-Arg47 Streptomyces avidinii |
| 82 | MEAGITGTWYNQLGSTFIVTAGADGALTGTY**VTAR**GNAESRYVLTGRYDSA PATDGSGTALGWTVAWKNNYRNAHSATTWSGQYVGGAEARINTQWLLTSGT TEANAWKSTLVGHDTFTKVKPSAAS | Mutein Streptavidin Val44-Thr45-Ala46-Arg47 Streptomyces avidinii |
| 83 | His-Pro-Gln-Phe | Streptavidin-binding peptide |
| 84 | Oaa-Xaa-His-Pro-Gln-Phe-Yaa-Zaa | Streptavidin-binding peptide Oaa is Trp, Lys or Arg; Xaa is any amino acid; Yaa is Gly or Glu Zaa is Gly, Lys or Arg |
| 85 | DPSKDSKAQVSAAEAGITGTWYNQLGSTFIVTAGADGALTGTY**IGAR**GNAE SRYVLTGRYDSAPATDGSGTALGWTVAWKNNYRNAHSATTWSGQYVGGAEA RINTQWLLTSGTTEANAWKSTLVGHDTFTKVKPSAASIDAAKKAGVNNGNP LDAVQQ | Mutein Streptavidin Ile44-Gly45-Ala-46-Arg47 Species: Streptomyces avidinii |
| 86 | EAGITGTWYNQLGSTFIVTAGADGALTGTYVTARGNAESRYVLTGRYDSAP ATDGSGTALGWTVAWKNNYRNAHSATTWSGQYVGGAEARINTQWLLTSGTT EENAGYSTLVGHDTFTKVKPSAAS | Mutein Streptavidin Ile44-Gly45-Ala-46-Arg47 and Glu117, Gly120, Try121 (mutein m1-9) Species: Streptomyces avidinii |
| 87 | DPSKDSKAQVSAAEAGITGTWYNQLGSTFIVTAGADGALTGTYVTARGNAE SRYVLTGRYDSAPATDGSGTALGWTVAWKNNYRNAHSATTWSGQYVGGAEA RINTQWLLTSGTTEENAGYSTLVGHDTFTKVKPSAAS | Mutein Streptavidin Ile44-Gly45-Ala-46-Arg47 and Glu117, Gly120, Try121 (mutein m1-9) |
| | | Species: Streptomyces avidinii |
| 88 | MEAGITGTWYNQLGSTFIVTAGADGALTGTYESAVGNAESRYVLTGRYDSA PATDGSGTALGWTVAWKNNYRNAHSATTWSGQYVGGAEARINTQWLLTSGT TEANAWKSTLVGHDTFTKVKPSAAS | Minimal streptavidin Species: Streptomyces avidinii |
| 89 | Trp-Ser-His-Pro-Gln-Phe-Glu-Lys-(GlyGlyGlySer)$_2$Gly-Gly-Ser-Ala-Trp-Ser-His-Pro-Gln-Phe-Glu-Lys | Mutein Streptavidin |
| 90 | Ala-Trp-Arg-His-Pro-Gln-Phe-Gly-Gly | Strep-tag® |
| 91 | LQQSGAELVKPGASVRLSCKASGYTFTEYIIHWIKLRSGQGLEWIGW FYPGSNDIQYNAKFKGKATLTADKSSSTVYMELTGLTSEDSAVYFC ARRDDFSGYDALPYWGQGTMVTV | VH anti-CD28 antibody CD28.3 |
| 92 | DIQMTQSPASLSVSVGETVTITCRTNENIYSNLAWYQQKQGKSPQLL IYAATHLVEGVPSRFSGSGSGTQYSLKITSLQSEDFGNYYCQHFWGT PCTFGGGTKLEIKR | VL anti-CD28 antibody CD28.3 |

(continued)

| # | SEQUENCE | ANNOTATION |
|---|----------|------------|
| 93 | QVQLQQSGAELARPGASVKMSCKASGYTFTRYTMHWVKQRPGQG LEWIGYINPSRGYTNYNQKFKDKATLTTDKSSSTAYMQLSSLTSEDS AVYYCARYYDDHYCLDYWGQGTTLTVSS | VH anti-CD3 antibody OKT3 |
| 94 | QIVLTQSPAIMSASPGEKVTMTCSASSSVSYMNWYQQKSGTSPKRWI YDTSKLASGVPAHFRGSGSGTSYSLTISGMEAEDAATYYCQQWSSN PFTFGSGTKLEIN | VL anti-CD3 antibody OKT3 |
| 95 | AMQVQLKQSGPGLVQPSQSLSITCTVSGFSLTTFGVHWVRQSPGKG LEWLGVIWASGITDYNVPFMSRLSITKDNSKSQVFFKLNSLQPDDTA IYYCAKNDPGTGFAYWQGTLVTVSSTKGPSVFPAPSSKSTSGGTAAL GCLVKDYFPEPVTVSWNSGALTSGVHTFPAVLQSSLYSLSSVVTVPS SSLGTQTYICNVNHKPSNTKVDKKVEPKSCGSAWSHPQFEKGGGSG GGSGGSAWSHPQFEK | Variable Heavy chain of Fab fragment m13B8.2 |
| 96 | AMDIQMTQSPASLSASVGETVTFTCRASEMIYSYLAWYQQKQGKSP QLLVHDAKTLAEGVPSRFSGGGSGTQFSLKINTLQPEDFGTYYCQAH YGNPPTFGGGTKLEIKRGIAAPSVFIFPPSDEQLKSGTASVVCLLNNF YPREAKVQWKVDNALQSGNSQESVTEQDSKDSTYSLSSTLTLSKAD YEKHKVYACEVTHQGLSSPVTKSFNRGECGS | Variable Light chain of Fab Fragment m13B8.2 |
| 97 | QVQLVQSGAEVKKPGATVKISCKVSGFNIKDTYIHWVQQAPGKGLE WMGRIDPANDNTLYASKFQGRVTITADTSTDTAYMELSSLRSEDTA VYYCARGYGYYVFDHWGQGTLVTVSS | Variable Heavy chain of huOKT8 |
| 98 | DVQITQSPSSLSASVGDRVTITCRTSRSISQYLAWYQQKPGKVPKLLI YSGSTLQSGVPSRFSGSGSGTDFTLTISSLQPEDVATYYCQQHNENPL TFGGGTKVEIK | Variable Light chain of huOKT8 |

**Claims**

1. A method for enriching T cells and generating an engineered population of T cells, the method comprising:

(a) performing a first selection, the first selection comprising enriching for either of CD57- or CD3+ T cells from a biological sample comprising primary human T cells, thereby generating an enriched T cell population; and
(b) performing a second selection on the cells from the enriched T cell population, wherein:

the first selection comprises enriching for CD57- T cells and the second selection comprises enriching for CD3+ T cells from the enriched population; or
the first selection comprises enriching for CD3+ T cells and the second selection comprises removing CD57+ T cells from the enriched T cell population,
wherein the method generates a depleted population that comprises fewer CD57+ T cells than the biological sample and is enriched for CD3+ T cells, and

(c) introducing a heterologous polynucleotide encoding a recombinant receptor into a population of T cells obtained from the depleted population, thereby generating an engineered population of T cells.

2. The method of claim 1, wherein the depleted population comprises at least one of the following:

(i) less than at or about 5% CD57+ T cells;
(ii) a frequency of CD57+ T cells that is less than at or about 35% of the frequency of CD57+ T cells present the biological sample;
(iii) CD4+ T cells, wherein at least at or about 95% of the CD4+ T cells are CD57-; and
(iv) CD8+ T cells, wherein at least at or about 95% of the CD8+ T cells are CD57-.

3. The method of claim 1 or claim 2, wherein the biological sample comprises an apheresis product or a leukapheresis product.

4. The method of any of claims 1-3, wherein:

   (i) at least at or about 95% of the CD4+ T cells of the depleted population comprises CD57-CD4+ T cells; and/or
   (ii) at least at or about 95% of the CD8+ T cells of the depleted population comprises CD57-CD8+ T cells; and/or
   (iii) at least at or about 95% of the CD3+ T cells of the depleted population comprises CD57-CD3+ T cells.

5. The method of any of claims 1-4, wherein:

   (i) the frequency of the CD57+ T cells in the depleted population is less than about or about 35%, 30%, 20%, 10%, 5%, 1% or 0.1% of the frequency of CD57+ T cells in the biological sample; and/or
   (ii) the depleted population comprises less than about or about 3%, less than about or about 2%, less than about or about 1%, less than about or about 0.1% or less than about or about 0.01% CD57+ T cells; and/or
   (iii) the depleted population is free or is essentially free of CD57+ T cells.

6. The method of any of claims 1-5, wherein:

   (i) the frequency of the naive-like T cells in the depleted population is at least about or about 10%, 20%, 30%, 40% or 50% greater than the frequency of naive-like T cells in the biological sample, optionally wherein the naive-like T cells are surface positive for one or more of markers selected from CD45RA, CD27, CD28, and CCR7; and/or
   (ii) the frequency of CD27+ T cells in the depleted population is at least about or about 10%, 20%, 30%, 40% or 50% greater than the frequency of the respective cells in the biological sample; and/or
   (iii) the frequency of CD28+ T cells in the depleted population is at least about or about 10%, 20%, 30%, 40% or 50% greater than the frequency of the respective cells in the biological sample; and/or
   (iv) the frequency of CD27+/CD28+ T cells in the depleted population is at least about or about 10%, 20%, 30%, 40% or 50% greater than the frequency of the respective cells in the biological sample.

7. The method of any of claims 1-6, wherein the method produces a T cell composition comprising at least at or about 90%, at least at or about 95%, at least at or about 97%, at least at or about 99% or a at least at or about 99.9% CD57-CD3+ T cells, optionally wherein the ratio of CD4+ and CD8+ T cells in the composition is between 3:1 and 1:3, between 2:1 and 1:2, between 1.5:1 and 1:1.5 or between 1.2:1 and 1:1.2, each inclusive, further optionally wherein the ratio of CD4+ and CD8+ T cells in the composition is at or about 1:1.

8. The method of any of claims 1-7, wherein the primary T cells are from a subject with a disease or condition, optionally wherein the disease or condition is a cancer.

9. The method of any of claims 1-8, wherein:

   (i) the removing of the CD57+ T cells comprises immunoaffinity-based selection, optionally wherein the immunoaffinity-based selection comprises contacting cells with an antibody capable of specifically binding to CD57 and recovering cells not bound to the antibody, thereby effecting negative selection, wherein the recovered cells are depleted for the CD57+ cells; and/or
   (ii) the enriching cells comprises immunoaffinity-based selection, optionally wherein the selection enriches for CD3 T cells and the immunoaffinity-based selection is effected by contacting cells with an antibody capable of specifically binding to CD3, and recovering cells bound to the antibody, thereby effecting positive selection, wherein the recovered cells are enriched for the CD3+ cells.

10. The method of claim 9, wherein:

    (i) the antibody is immobilized on a solid surface, optionally wherein the solid surface is a magnetic particle; and/or
    (ii) the antibody is immobilized on or attached to an affinity chromatography matrix.

11. The method of any of claims 1-10, wherein the biological sample comprising the primary T cells is a sample formulated with a cryoprotectant.

**12.** The method of any of claims 1-11, wherein, prior to the introducing, the depleted population is incubated under stimulatory conditions.

**13.** The method of any of claims 1-12, wherein the introducing comprises transduction with a viral vector comprising the heterologous polynucleotide, further optionally wherein the viral vector is a gammaretroviral vector or a lentiviral vector.

**14.** The method of any of claims 1-13, further comprising cultivating cells of the engineered population under conditions to promote proliferation or expansion of the engineered cells, thereby generating an expanded population of cells, optionally, wherein the cultivating is carried out in the presence of one or more recombinant cytokines, optionally comprising one or more of IL-2, IL-7 and IL-15.

**15.** The method of any of claims 1-14, wherein the recombinant receptor is a chimeric antigen receptor (CAR), optionally wherein the recombinant receptor comprises:

(i) an extracellular domain comprising an antigen-binding domain, optionally an scFv;
(ii) a spacer and/or a hinge region;
(iii) a transmembrane domain; and
(iv) an intracellular signaling domain comprising a costimulatory signaling region, optionally wherein:

(a) the intracellular signaling domain is or comprises an intracellular signaling domain of a CD3 chain, further optionally a CD3-zeta (CD3$\zeta$) chain or a signaling portion thereof; and/or
(b) the costimulatory signaling region comprises an intracellular signaling domain of a CD28, a 4-1BB or an ICOS or a signaling portion thereof.

**16.** A composition comprising cells produced by the method of any of claims 1-15.

**17.** A dose of T cells from an engineered population of T cells produced by the method of any of claims 1-15, for use in a method of treating a subject having or suspected of having a disease, disorder or condition, wherein the method comprises administering the dose of T cells to the subject.

**18.** A dose of T cells from an engineered population of T cells produced by the method of any of claims 1-15, for use in a method of treating a subject having or suspected of having a disease or condition, wherein the disease or condition is a cancer, and wherein the method comprises administering the dose of T cells to the subject.

**19.** The dose of T cells for use of claim 17 or claim 18, wherein:

(i) the recombinant receptor, optionally the CAR, specifically recognizes or specifically bind to an antigen associated with, or expressed or present on cells of, the disease or condition; and/or
(ii) the dose of T cells comprises between at or about $5 \times 10^6$ and at or about $1.5 \times 10^8$ recombinant receptor-expressing T cells, between at or about $5 \times 10^6$ and at or about $1 \times 10^8$ recombinant receptor-expressing T cells, between at or about $5 \times 10^6$ and at or about $50 \times 10^6$ recombinant receptor-expressing T cells, between at or about $5 \times 10^6$ and at or about $25 \times 10^6$ recombinant receptor-expressing T cells, between at or about $5 \times 10^6$ and at or about $10 \times 10^6$ recombinant receptor-expressing T cells, between at or about $10 \times 10^6$ and at or about $1.5 \times 10^8$ recombinant receptor-expressing T cells, between at or about $10 \times 10^6$ and at or about $1 \times 10^8$ recombinant receptor-expressing T cells, between at or about $10 \times 10^6$ and at or about $50 \times 10^6$ recombinant receptor-expressing T cells, between at or about $10 \times 10^6$ and at or about $25 \times 10^6$ recombinant receptor-expressing T cells, between at or about $25 \times 10^6$ and at or about $1.5 \times 10^8$ recombinant receptor-expressing T cells, between at or about $25 \times 10^6$ and at or about $1 \times 10^8$ recombinant receptor-expressing T cells, between at or about $25 \times 10^6$ and at or about $50 \times 10^6$ recombinant receptor-expressing T cells, between at or about $50 \times 10^6$ and at or about $1.5 \times 10^8$ recombinant receptor-expressing T cells, between at or about $50 \times 10^6$ and at or about $1 \times 10^8$ recombinant receptor-expressing T cells, between at or about $1 \times 10^8$ and at or about $1.5 \times 10^8$ recombinant receptor-expressing T cells, each inclusive; and/or
(iii) the dose of T cells are autologous to the subject being treated.

**Patentansprüche**

1. Verfahren zur Anreicherung von T-Zellen und Erzeugung einer konstruierten Population von T-Zellen, wobei das Verfahren Folgendes umfasst:

(a) Durchführen einer ersten Selektion, wobei die erste Selektion Anreichern auf entweder CD57- oder CD3+ T-Zellen aus einer primäre menschliche T-Zellen umfassenden biologischen Probe umfasst, wodurch eine angereicherte T-Zellpopulation erzeugt wird; und
(b) Durchführen einer zweiten Selektion an den Zellen aus der angereicherten T-Zellpopulation, wobei:

die erste Selektion Anreichern auf CD57- T-Zellen und die zweite Selektion Anreichern auf CD3+ T-Zellen aus der angereicherten Population umfasst; oder
die erste Selektion Anreichern auf CD3+ T-Zellen und die zweite Selektion Entfernen von CD57+ T-Zellen aus der angereicherten Population umfasst,
wobei mit dem Verfahren eine dezimierte Population erzeugt wird, die weniger CD57+ T-Zellen als die biologische Probe umfasst und auf CD3+ T-Zellen angereichert ist, und

(c) Einführen eines heterologen Polynukleotids, das einen rekombinanten Rezeptor codiert, in eine aus der dezimierten Population erhaltene Population von T-Zellen, wodurch eine konstruierte Population von T-Zellen erzeugt wird.

2. Verfahren nach Anspruch 1, wobei die dezimierte Population wenigstens eines der Folgenden umfasst:

(i) weniger als oder als etwa 5 % CD57+ T-Zellen;
(ii) eine Häufigkeit von CD57+ T-Zellen, die weniger als oder als etwa 35 % der vorliegenden CD57+ T-Zellen der biologischen Probe beträgt;
(iii) CD4+ T-Zellen, wobei wenigstens oder wenigstens etwa 95 % der CD4+ T-Zellen CD57- sind; und
(iv) CD8+ T-Zellen, wobei wenigstens oder wenigstens etwa 95 % der CD8+ T-Zellen CD57- sind.

3. Verfahren nach Anspruch 1 oder Anspruch 2, wobei die biologische Probe ein Aphereseprodukt oder ein Leukaphereseprodukt umfasst.

4. Verfahren nach einem der Ansprüche 1-3, wobei:

(i) wenigstens oder wenigstens etwa 95 % der CD4+ T-Zellen der dezimierten Population CD57-CD4+ T-Zellen umfassen; und/oder
(ii) wenigstens oder wenigstens etwa 95 % der CD8+ T-Zellen der dezimierten Population CD57-CD8+ T-Zellen umfassen; und/oder
(iii) wenigstens oder wenigstens etwa 95 % der CD3+ T-Zellen der dezimierten Population CD57-CD3+ T-Zellen umfassen.

5. Verfahren nach einem der Ansprüche 1-4, wobei:

(i) die Häufigkeit der CD57+ T-Zellen in der dezimierten Population weniger als etwa oder etwa 35 %, 30 %, 20 %, 10 %, 5 %, 1 % oder 0,1 % der Häufigkeit von CD57+ T-Zellen in der biologischen Probe beträgt; und/oder
(ii) die dezimierte Population weniger als etwa oder etwa 3 %, weniger als etwa oder etwa 2 %, weniger als etwa oder etwa 1 %, weniger als etwa oder etwa 0,1 % oder weniger als etwa oder etwa 0,01% CD57+ T-Zellen umfasst; und/oder
(iii) die dezimierte Population frei oder im Wesentlichen frei von CD57+ T-Zellen ist.

6. Verfahren nach einem der Ansprüche 1-5, wobei:

(i) die Häufigkeit der naivähnlichen T-Zellen in der dezimierten Population wenigstens etwa oder etwa 10 %, 20 %, 30 %, 40 % oder 50 % größer als die Häufigkeit von naivähnlichen T-Zellen in der biologischen Probe ist, gegebenenfalls wobei die naivähnlichen T-Zellen oberflächenpositiv für einen oder mehrere Marker ausgewählt aus CD45RA, CD27, CD28 und CCR7 sind; und/oder
(ii) die Häufigkeit von CD27+ T-Zellen in der dezimierten Population wenigstens etwa oder etwa 10 %, 20 %, 30 %, 40 % oder 50 % größer als die Häufigkeit der betreffenden Zellen in der biologischen Probe ist; und/oder

(iii) die Häufigkeit von CD28+ T-Zellen in der dezimierten Population wenigstens etwa oder etwa 10 %, 20 %, 30 %, 40 % oder 50 % größer als die Häufigkeit der betreffenden Zellen in der biologischen Probe ist; und/oder
(iv) die Häufigkeit von CD27+/CD28+ T-Zellen in der dezimierten Population wenigstens etwa oder etwa 10 %, 20 %, 30 %, 40 % oder 50 % größer als die Häufigkeit der betreffenden Zellen in der biologischen Probe ist.

7. Verfahren nach einem der Ansprüche 1-6, wobei mit dem Verfahren eine T-Zell-Zusammensetzung erstellt wird, die wenigstens oder wenigstens etwa 90 %, wenigstens oder wenigstens etwa 95 %, wenigstens oder wenigstens etwa 97 %, wenigstens oder wenigstens etwa 99 % oder wenigstens oder wenigstens etwa 99,9 % CD57-CD3+ T-Zellen umfasst, gegebenenfalls wobei das Verhältnis von CD4+ und CD8+ T-Zellen in der Zusammensetzung, jeweils einschließlich, zwischen 3:1 und 1:3, zwischen 2:1 und 1:2, zwischen 1,5:1 und 1:1,5 oder zwischen 1,2:1 und 1:1,2 liegt, ferner gegebenenfalls wobei das Verhältnis von CD4+ und CD8+ T-Zellen in der Zusammensetzung bei oder bei etwa 1:1 liegt.

8. Verfahren nach einem der Ansprüche 1-7, wobei die primären T-Zellen von einem Individuum mit einer Krankheit bzw. einem Leiden stammen, gegebenenfalls wobei es sich bei der Krankheit bzw. dem Leiden um eine Krebserkrankung handelt.

9. Verfahren nach einem der Ansprüche 1-8, wobei:

(i) das Entfernen der CD57+ T-Zellen auf Immunaffinität beruhende Selektion umfasst, gegebenenfalls wobei die auf Immunaffinität beruhende Selektion In-Kontakt-Bringen von Zellen mit einem Antikörper mit der Fähigkeit zur spezifischen Bindung an CD57 und Gewinnen von nicht an den Antikörper gebundenen Zellen umfasst, wodurch eine negative Selektion bewirkt wird, wobei die gewonnenen Zellen hinsichtlich der CD57+ T-Zellen dezimiert sind; und/oder
(ii) das Zellen Anreichern auf Immunaffinität beruhende Selektion umfasst, gegebenenfalls wobei die Selektion auf CD3 T-Zellen anreichert und die auf Immunaffinität beruhende Selektion bewirkt wird, indem Zellen mit einem Antikörper mit der Fähigkeit zur spezifischen Bindung an CD3 in Kontakt gebracht und an den Antikörper gebundene Zellen gewonnen werden, wodurch eine positive Selektion bewirkt wird, wobei die gewonnenen Zellen hinsichtlich der CD3+ T-Zellen angereichert sind.

10. Verfahren nach Anspruch 9, wobei:

(i) der Antikörper an einer festen Oberfläche immobilisiert ist, gegebenenfalls wobei es sich bei der festen Oberfläche um ein Magnetpartikel handelt; und/oder
(ii) der Antikörper an einer Affinitätschromatographiematrix immobilisiert oder gebunden ist.

11. Verfahren nach einem der Ansprüche 1-10, wobei es sich bei der die primären T-Zellen umfassenden biologischen Probe um eine mit einem Kryoprotektivum formulierte Probe handelt.

12. Verfahren nach einem der Ansprüche 1-11, wobei, vor dem Einführen, die dezimierte Population unter stimulierenden Bedingungen inkubiert wird.

13. Verfahren nach einem der Ansprüche 1-12, wobei das Einführen Transduktion mit einem das heterologe Polynukleotid umfassenden Virusvektor umfasst, ferner gegebenenfalls wobei es sich bei dem Virusvektor um einen Gammaretrovirusvektor oder einen Lentivirusvektor handelt.

14. Verfahren nach einem der Ansprüche 1-13, ferner umfassend Kultivieren von Zellen der konstruierten Population unter Bedingungen zur Förderung von Proliferation oder Expansion der konstruierten Zellen, wodurch eine expandierte Population von Zellen erzeugt wird, gegebenenfalls, wobei das Kultivieren in Gegenwart eines oder mehrerer rekombinanter Cytokine erfolgt, die gegebenenfalls eines oder mehrere von IL-2, IL-7 und IL-15 umfassen.

15. Verfahren nach einem der Ansprüche 1-14, wobei es sich bei dem rekombinanten Rezeptor um einen chimären Antigenrezeptor (CAR) handelt, gegebenenfalls wobei der rekombinante Rezeptor Folgendes umfasst:

(i) eine extrazelluläre Domäne, die eine antigenbindende Domäne, gegebenenfalls ein scFv umfasst;
(ii) eine Spacer- und/oder eine Hinge-Region;
(iii) eine Transmembrandomäne; und
(iv) eine intrazelluläre Signalgebungsdomäne, die eine Costimulator-Signalgebungsregion umfasst, gegebenenfalls wobei:

(a) die intrazelluläre Signalgebungsdomäne eine intrazelluläre Signalgebungsdomäne einer CD3-Kette, ferner gegebenenfalls eine CD3-zeta(CD3ζ)-Kette oder ein/einen Signalgebungsteil davon, ist oder umfasst; und/oder
(b) die Costimulator-Signalgebungsregion eine intrazelluläre Signalgebungsdomäne eines CD28, eines 4-1BB oder eines ICOS oder einen Signalgebungsteil davon umfasst.

16. Zusammensetzung, umfassend mit dem Verfahren nach einem der Ansprüche 1-15 erstellte Zellen.

17. Dosis von T-Zellen aus einer konstruierten Population von mit dem Verfahren nach einem der Ansprüche 1-15 erstellten T-Zellen, zur Verwendung bei einem Verfahren zur Behandlung eines Individuums, bei dem eine Krankheit, eine Störung oder ein Leiden vorliegt oder vermutet wird, wobei das Verfahren Verabreichen der Dosis von T-Zellen an das Individuum umfasst.

18. Dosis von T-Zellen aus einer konstruierten Population von mit dem Verfahren nach einem der Ansprüche 1-15 erstellten T-Zellen, zur Verwendung bei einem Verfahren zur Behandlung eines Individuums, bei dem eine Krankheit oder ein Leiden vorliegt oder vermutet wird, wobei es sich bei der Krankheit bzw. dem Leiden um eine Krebserkrankung handelt und wobei das Verfahren Verabreichen der Dosis von T-Zellen an das Individuum umfasst.

19. Dosis von T-Zellen zur Verwendung nach Anspruch 17 oder Anspruch 18, wobei:

(i) der rekombinante Rezeptor, gegebenenfalls der CAR, ein Antigen, das mit der Krankheit oder dem Leiden in Zusammenhang steht oder auf Zellen der Krankheit bzw. des Leidens exprimiert wird oder vorliegt, spezifisch erkennt oder daran spezifisch bindet; und/oder
(ii) die Dosis von T-Zellen, jeweils einschließlich, zwischen oder zwischen etwa $5 \times 10^6$ und oder und etwa $1,5 \times 10^8$ rekombinanten Rezeptor exprimierende T-Zellen, zwischen oder zwischen etwa $5 \times 10^6$ und oder und etwa $1 \times 10^8$ rekombinanten Rezeptor exprimierende T-Zellen, zwischen oder zwischen etwa $5 \times 10^6$ und oder und etwa $50 \times 10^6$ rekombinanten Rezeptor exprimierende T-Zellen, zwischen oder zwischen etwa $5 \times 10^6$ und oder und etwa $25 \times 10^6$ rekombinanten Rezeptor exprimierende T-Zellen, zwischen oder zwischen etwa $5 \times 10^6$ und oder und etwa $10 \times 10^6$ rekombinanten Rezeptor exprimierende T-Zellen, zwischen oder zwischen etwa $10 \times 10^6$ und oder und etwa $1,5 \times 10^8$ rekombinanten Rezeptor exprimierende T-Zellen, zwischen oder zwischen etwa $10 \times 10^6$ und oder und etwa $1 \times 10^8$ rekombinanten Rezeptor exprimierende T-Zellen, zwischen oder zwischen etwa $10 \times 10^6$ und oder und etwa $50 \times 10^6$ rekombinanten Rezeptor exprimierende T-Zellen, zwischen oder zwischen etwa $10 \times 10^6$ und oder und etwa $25 \times 10^8$ rekombinanten Rezeptor exprimierende T-Zellen, zwischen oder zwischen etwa $25 \times 10^6$ und oder und etwa $1,5 \times 10^8$ rekombinanten Rezeptor exprimierende T-Zellen, zwischen oder zwischen etwa $25 \times 10^6$ und oder und etwa $1 \times 10^8$ rekombinanten Rezeptor exprimierende T-Zellen, zwischen oder zwischen etwa $25 \times 10^6$ und oder und etwa $50 \times 10^6$ rekombinanten Rezeptor exprimierende T-Zellen, zwischen oder zwischen etwa $50 \times 10^6$ und oder und etwa $1,5 \times 10^8$ rekombinanten Rezeptor exprimierende T-Zellen, zwischen oder zwischen etwa $50 \times 10^6$ und oder und etwa $1 \times 10^8$ rekombinanten Rezeptor exprimierende T-Zellen, zwischen oder zwischen etwa $1 \times 10^8$ und oder und etwa $1,5 \times 10^8$ rekombinanten Rezeptor exprimierende T-Zellen umfasst; und/oder
(iii) die Dosis von T-Zellen für das behandelte Individuum autolog ist.

## Revendications

1. Procédé d'enrichissement de lymphocytes T et de génération d'une population modifiée de lymphocytes T, le procédé comprenant :

(a) la mise en oeuvre d'une première sélection, la première sélection comprenant l'enrichissement de lymphocytes T CD57- ou CD3+ à partir d'un échantillon biologique comprenant des lymphocytes T humains primaires, pour générer ainsi une population de lymphocytes T enrichie ; et
(b) la mise en oeuvre d'une seconde sélection sur les cellules provenant de la population de lymphocytes T enrichie, dans lequel

la première sélection comprend un enrichissement en lymphocytes T CD57- et la seconde sélection comprenant un enrichissement en lymphocytes T CD3+ à partir de la population enrichie ; ou
la première sélection comprend un enrichissement en lymphocytes T CD3+ et la seconde sélection comprend l'extraction des lymphocytes T CD57+ de la population de lymphocytes T enrichie,
le procédé générant une population appauvrie qui comprend moins de lymphocytes T CD57+ que l'échantillon biologique et est enrichie en lymphocytes T CD3+, et

(c) l'introduction d'un polynucléotide hétérologue codant pour un récepteur recombinant dans une population de lymphocytes T obtenue à partir de la population appauvrie, de façon à générer une population modifiée de lymphocytes T.

2. Procédé selon la revendication 1, dans lequel la population appauvrie comprend au moins l'un parmi ce qui suit :

(i) une quantité inférieure ou approximativement égale à 5 % de lymphocytes T CD57+ ;
(ii) une fréquence de lymphocytes T CD57+ inférieure ou environ égale à 35 % de la fréquence des lymphocytes T CD57+ présents dans l'échantillon biologique ;
(iii) des lymphocytes T CD4+, au moins ou environ 95 % des lymphocytes T CD4+ étant CD57- ; et
(iv) des lymphocytes T CD8+, au moins ou environ 95 % des lymphocytes T CD8+ étant CD57-.

3. Procédé selon la revendication 1 ou la revendication 2, dans lequel l'échantillon biologique comprend un produit d'aphérèse ou un produit de leucaphérèse.

4. Procédé selon l'une quelconque des revendications 1 à 3, dans lequel :

(i) au moins ou environ 95 % des lymphocytes T CD4+ de la population appauvrie comprennent des lymphocytes T CD57-CD4+ ; et/ou
(ii) au moins ou environ 95 % des lymphocytes T CD8+ de la population appauvrie comprennent des lymphocytes T CD57-CD8+ ; et/ou
(iii) au moins ou environ 95 % des lymphocytes T CD3+ de la population appauvrie comprennent des lymphocytes T CD57-CD3+.

5. Procédé selon l'une quelconque des revendications 1 à 4, dans lequel :

(i) la fréquence des lymphocytes T CD57+ dans la population appauvrie est inférieure à environ ou d'environ 35 %, 30 %, 20 %, 10 %, 5 %, 1 % ou 0,1 % de la fréquence des lymphocytes T CD57+ dans l'échantillon biologique ; et/ou
(ii) la population appauvrie comprend moins d'environ ou environ 3 %, moins d'environ ou environ 2 %, moins d'environ ou environ 1 %, moins d'environ ou environ 0,1 % ou moins d'environ ou environ 0,01 % de lymphocytes T CD57+ ; et/ou
(iii) la population appauvrie est exempte ou sensiblement exempte de lymphocytes T CD57+.

6. Procédé selon l'une quelconque des revendications 1 à 5, dans lequel :

(i) la fréquence des lymphocytes T de type naïf dans la population appauvrie est d'au moins environ ou d'environ 10 %, 20 %, 30 %, 40 % ou 50 % supérieure à la fréquence des lymphocytes T de type naïf dans l'échantillon biologique, éventuellement dans lequel les lymphocytes T de type naïf sont surface-positifs pour un ou plusieurs des marqueurs choisis parmi CD45RA, CD27, CD28 et CCR7 ; et/ou
(ii) la fréquence des lymphocytes T CD27+ dans la population appauvrie est d'au moins environ ou d'environ 10 %, 20 %, 30 %, 40 % ou 50 % supérieure à la fréquence des cellules respectives dans l'échantillon biologique ; et/ou
(iii) la fréquence des lymphocytes T CD28+ dans la population appauvrie est d'au moins environ ou d'environ 10 %, 20 %, 30 %, 40 % ou 50 % supérieure à la fréquence des cellules respectives dans l'échantillon biologique ; et/ou
(iv) la fréquence des lymphocytes T CD27+/CD28+ dans la population appauvrie est d'au moins environ ou d'environ 10 %, 20 %, 30 %, 40 % ou 50 % supérieure à la fréquence des cellules respectives dans l'échantillon biologique.

7. Procédé selon l'une quelconque des revendications 1 à 6, le procédé produisant une composition de lymphocytes T comprenant au moins ou environ 90 %, au moins ou environ 95 %, au moins ou environ 97 %, au moins ou environ 99 % ou au moins ou environ 99,9 % de lymphocytes T CD57-CD3+, éventuellement dans lequel le rapport des lymphocytes T CD4+ aux lymphocytes T CD8+ dans la composition est compris entre 3:1 et 1:3, entre 2:1 et 1:2, entre 1,5:1 et 1:1,5 ou entre 1,2:1 et 1:1,2, chacun limites comprises, éventuellement en outre dans lequel le rapport des lymphocytes T CD4+ aux lymphocytes T CD8+ dans la composition est de ou d'environ 1:1.

8. Procédé selon l'une quelconque des revendications 1 à 7, dans lequel les lymphocytes T primaires proviennent d'un sujet ayant une maladie ou un état pathologique, éventuellement dans lequel la maladie ou l'état pathologique est un cancer.

9. Procédé selon l'une quelconque des revendications 1 à 8, dans lequel :

(i) l'extraction des lymphocytes T CD57+ comprend une sélection à base d'une immunoaffinité, éventuellement dans lequel la sélection à base d'une immunoaffinité comprend la mise en contact des cellules avec un anticorps capable de se lier spécifiquement au CD57 et la récupération des cellules non liées à l'anticorps, en effectuant ainsi une sélection négative dans laquelle les cellules récupérées sont appauvries en lymphocytes CD57+ ; et/ou
(ii) l'enrichissement des cellules comprend une sélection à base d'une immunoaffinité, éventuellement dans lequel la sélection enrichit en lymphocytes T CD3 et la sélection à base d'une immunoaffinité est effectuée par mise en contact des cellules avec un anticorps capable de se lier spécifiquement à CD3 et récupération des cellules liées à l'anticorps, en effectuant ainsi une sélection positive, dans laquelle les cellules récupérées sont enrichies en les cellules CD3+.

10. Procédé selon la revendication 9, dans lequel :

(i) l'anticorps est immobilisé sur une surface solide, éventuellement dans lequel la surface solide est une particule magnétique ; et/ou
(ii) l'anticorps est immobilisé sur une matrice de chromatographie d'affinité ou y est fixé.

11. Procédé selon l'une quelconque des revendications 1 à 10, dans lequel l'échantillon biologique comprenant les lymphocytes T primaires est un échantillon formulé avec un cryoprotecteur.

12. Procédé selon l'une quelconque des revendications 1 à 11, dans lequel, avant l'introduction, la population appauvrie est incubée dans des conditions stimulatrices.

13. Procédé selon l'une quelconque des revendications 1 à 12, dans lequel l'introduction comprend une transduction avec un vecteur viral comprenant le polynucléotide hétérologue, éventuellement en outre dans lequel le vecteur viral est un vecteur gammarétroviral ou un vecteur lentiviral.

14. Procédé selon l'une quelconque des revendications 1 à 13, comprenant en outre la culture de cellules de la population modifiée dans des conditions visant à favoriser une prolifération ou une expansion des cellules modifiées, en générant ainsi une population expansée de cellules, éventuellement dans lequel la culture est mise en œuvre en présence d'une ou plusieurs cytokines recombinantes, comprenant éventuellement un ou plusieurs parmi l'IL-2, l'IL-7 et l'IL-15.

15. Procédé selon l'une quelconque des revendications 1 à 14, dans lequel le récepteur recombinant est un récepteur d'antigène chimère (CAR), éventuellement dans lequel le récepteur recombinant comprend :

(i) un domaine extracellulaire comprenant un domaine de liaison à l'antigène, de préférence un scFv ;
(ii) un espaceur et/ou une région charnière ;
(iii) un domaine transmembranaire ; et
(iv) un domaine de signalisation intracellulaire comprenant une région de signalisation costimulatrice, éventuellement dans lequel :

(a) le domaine de signalisation intracellulaire est ou comprend un domaine de signalisation intracellulaire d'une chaîne CD3, éventuellement en outre une chaîne CD3-zêta (CD3ζ) ou une partie de signalisation de cette dernière ; et/ou
(b) la région de signalisation costimulatrice comprend un domaine de signalisation intracellulaire d'un CD28, d'un 4-1BB ou d'un ICOS ou une partie de signalisation de celui-ci.

**16.** Composition comprenant des cellules produites par le procédé selon l'une quelconque des revendications 1 à 15.

**17.** Dose de lymphocytes T provenant d'une population modifiée de lymphocytes T produite par le procédé selon l'une quelconque des revendications 1 à 15, pour une utilisation dans une méthode de traitement d'un sujet ayant ou suspecté d'avoir une maladie, un trouble ou un état pathologique, la méthode comprenant l'administration de la dose de lymphocytes T au sujet.

**18.** Dose de lymphocytes T provenant d'une population modifiée de lymphocytes T produite par le procédé selon l'une quelconque des revendications 1 à 15, pour une utilisation dans une méthode de traitement d'un sujet ayant ou suspecté d'avoir une maladie ou un état pathologique, la maladie ou l'état pathologique étant un cancer, et la méthode comprenant l'administration de la dose de lymphocytes T au sujet.

**19.** Dose de lymphocytes T pour une utilisation selon la revendication 17 ou la revendication 18, dans laquelle :

(i) le récepteur recombinant, éventuellement le CAR, reconnaît spécifiquement ou se lie spécifiquement à un antigène associé à la maladie ou à l'état pathologique ou exprimé ou présent sur des cellules de la maladie ou de l'état pathologique ; et/ou

(ii) la dose de lymphocytes T est comprise entre une valeur de ou d'environ $5 \times 10^6$ et une valeur de ou d'environ $1,5 \times 10^8$ lymphocytes T exprimant un récepteur recombinant, entre une valeur de ou d'environ $5 \times 10^6$ et une valeur de ou d'environ $1 \times 10^8$ lymphocytes T exprimant un récepteur recombinant, entre une valeur de ou d'environ $5 \times 10^6$ et une valeur de ou d'environ $50 \times 10^6$ lymphocytes T exprimant un récepteur recombinant, entre une valeur de ou d'environ $5 \times 10^6$ et une valeur de ou d'environ $25 \times 10^6$ lymphocytes T exprimant un récepteur recombinant, entre une valeur de ou d'environ $5 \times 10^6$ et une valeur de ou d'environ $10 \times 10^6$ lymphocytes T exprimant un récepteur recombinant, entre une valeur de ou d'environ $10 \times 10^6$ et une valeur de ou d'environ $1,5 \times 10^8$ lymphocytes T exprimant un récepteur recombinant, entre une valeur de ou d'environ $10 \times 10^6$ et une valeur de ou d'environ $1 \times 10^8$ lymphocytes T exprimant un récepteur recombinant, entre une valeur de ou d'environ $10 \times 10^6$ et une valeur de ou d'environ $50 \times 10^6$ lymphocytes T exprimant un récepteur recombinant, entre une valeur de ou d'environ $10 \times 10^6$ et une valeur de ou d'environ $25 \times 10^6$ lymphocytes T exprimant un récepteur recombinant, entre une valeur de ou d'environ $25 \times 10^6$ et une valeur de ou d'environ $1,5 \times 10^8$ lymphocytes T exprimant un récepteur recombinant, entre une valeur de ou d'environ $25 \times 10^6$ et une valeur de ou d'environ $1 \times 10^8$ lymphocytes T exprimant un récepteur recombinant, entre une valeur de ou d'environ $25 \times 10^6$ et une valeur de ou d'environ $50 \times 10^6$ lymphocytes T exprimant un récepteur recombinant, entre une valeur de ou d'environ $50 \times 10^6$ et une valeur de ou d'environ $1,5 \times 10^8$ lymphocytes T exprimant un récepteur recombinant, entre une valeur de ou d'environ $50 \times 10^6$ et une valeur de ou d'environ $1 \times 10^8$ lymphocytes T exprimant un récepteur recombinant, entre une valeur de ou d'environ $1 \times 10^8$ et une valeur de ou d'environ $1,5 \times 10^8$ lymphocytes T exprimant un récepteur recombinant, chacun limites comprises ; et/ou

(iii) la dose de lymphocytes T est autologue au sujet soumis au traitement.

FIG. 1A

FIG. 1B

FIG. 1C

FIG. 1D

142

FIG. 2A

FIG. 2B

FIG. 2C

**FIG. 2D**

CD4+ CD57 Expression

FIG. 2E

EP 3 877 054 B1

FIG. 2F

## FIG. 3A

**FIG. 3B**

**FIG. 3C**

Naïve-like/$T_{CM}$
Markers

$T_{EM}/T_{EMRA}$
Markers

*Scaled*

Low %

High %

## FIG. 4A

**FIG. 4B**

100% CD57-  25% CD57+  75% CD57+  100% CD57+

**FIG. 4C**

**FIG. 5A**

**FIG. 5B**

**FIG. 5C**

UNDEPLETED

DEPLETED

## FIG. 5D

Ki-67 Expression at 72hrs

## FIG. 5E

## FIG. 5F

## FIG. 5G

FIG. 6A

FIG. 6B

**FIG. 6C**

FIG. 6D

% Ki-67+ of CD57+ SubTypes

● CD27- , CD45RA+

■ CD27+ , CD45RA+

▲ CD27+ , CD45RA-

▼ CD27- , CD45RA-

EP 3 877 054 B1

## FIG. 6E

### CD8+CD57+ Subgroups

### CD4+CD57+ Subgroups

**FIG. 7**

**FIG. 8A**

**FIG. 8B**

**FIG. 8C**

FIG. 9A

FIG. 9B

**CD4+ CAR+**

**CD8+ CAR+**

>CCR7+CD27+

<CCR7+CD27+

*P=0.1226

Survival Probability

Progression free survival (days)

>CCR7+CD27+

<CCR7+CD27+

*P=0.0435

Survival Probability

Progression free survival (days)

EP 3 877 054 B1

**FIG. 9D**

CD8+ CAR+

>CCR7+CD27+

<CCR7+CD27+

*P=0.2641

Duration of Response (days)

Probability of Remaining in Response (CR/PR)

**FIG. 9C**

CD4+ CAR+

>CCR7+CD27+

<CCR7+CD27+

*P=0.1101

Duration of Response (days)

Probability of Remaining in Response (CR/PR)

FIG. 10

## FIG. 11

Selected T Cell %CD27+CD28+ Composition in NHL Patients

**FIG. 12**

Selected T cells

FIG. 13A

FIG. 13B

EP 3 877 054 B1

FIG. 13C

FIG. 13D

EP 3 877 054 B1

**FIG. 14**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016187216 A
- US 7510687 B
- US 5538848 A
- US 5925517 A
- US 6174670 B
- US 6329144 B
- US 6326145 B
- US 6635427 B
- WO 2018170188 A
- US 2018064627 W
- WO 2013124474 A
- WO 2015164675 A
- WO 2009072003 A
- US 20110003380 A1
- WO 2016073602 A
- US 20170037369 A
- US 4452773 A, Molday
- EP 452342 B
- US 4795698 A
- US 5200084 A, Liberti
- US 6103493 A
- US 5506121 A
- WO 02077018 A
- US 7981632 B
- WO 2013011011 A
- US 7776562 B
- WO 02054065 A
- US 7482000 B
- US 20140295458 A
- US 6040177 A, Riddell
- US 5168049 A
- US 8298782 B
- US 8735540 B
- US 9023604 B
- WO 2014076277 A
- US 4361549 A
- US 6569997 B
- US 6303121 B
- WO 1999061065 A
- US 20010026932 A
- US 7547438 B
- WO 2001056603 A
- US 20080279851 A
- WO 2008051424 A

- WO 2013038191 A
- WO 2006054961 A
- WO 2007001459 A
- WO 2018197949 A
- WO 8602077 A
- DE 19641876 A1
- US 6022951 A
- WO 9840396 A
- WO 9624606 A
- US 6156493 A
- US 6165750 A
- US 6368813 B
- US 6123655 A
- US 6733433 B
- US 20080171951 A
- WO 0038762 A
- US 5219740 A
- US 6207453 B
- WO 200014257 A
- WO 2013126726 A
- WO 2012129514 A
- WO 2014031687 A
- WO 2013166321 A
- WO 2013071154 A
- WO 2013123061 A
- US 2002131960 A
- US 2013287748 A
- US 20130149337 A
- US 6451995 B
- US 7446190 B
- US 8252592 B
- US 8339645 B
- US 8398282 B
- US 7446179 B
- US 6410319 B
- US 7070995 B
- US 7265209 B
- US 7354762 B
- US 7446191 B
- US 8324353 B
- US 8479118 B
- EP 2537416 A
- WO 2014055668 A1
- US 8389282 B
- US 20160152723 A
- US 8822647 B
- US 20140271635 A
- WO 2014055668 A
- US 8911993 B

- US 8802374 B
- US 20070116690 A
- WO 03020763 A
- WO 04033685 A
- WO 2011044186 A
- WO 9613593 A
- WO 9618105 A
- WO 9960120 A
- WO 9918129 A
- WO 2006000830 A
- US 20170051035 A

- WO 1992008796 A
- WO 1994028143 A
- US 6060273 A
- US 20110003380 A
- WO 2010033140 A
- US 20030170238, Gruenberg
- US 4690915 A, Rosenberg
- US 5087616 A
- US 5323907 A
- US 5052558 A
- US 5033252 A

**Non-patent literature cited in the description**

- **WÖLF et al.** *Nature Protocols,* 2014, vol. 9 (4), 950-966
- **CASATI et al.** *Cancer Immunology,* 2016, vol. 62 (10), 1563-1573
- **HINRICHS et al.** *Journal of the American Society of Hematology,* 2011, vol. 117 (3), 808-814
- **OHKAWA et al.** *Immunology Oxford,* 2001, 281-290
- **FRAIETTA et al.** *Nat Med.,* 2018, vol. 24 (5), 563-571
- **LARSON et al.** *Cancer Res.,* 2018, vol. 78 (13
- **SINGH et al.** *Sci Trans Med.,* 2016, vol. 8 (320), 320-3
- **STRIOGA ; PASUKONIENE ; CHARACIEJUS.** *Immunol,* 2011
- **SHENDURE et al.** *Nat. Rev. Genet.,* 2004, vol. 5, 335-44
- **NOWROUSIAN.** *Euk. Cell,* 2010, vol. 9 (9), 1300-1310
- **SMITH et al.** *Clin Transl Immunology.,* January 2015, vol. 4 (1), e31
- **TERAKURA et al.** *Blood,* 2012, vol. 1, 72-82
- **WANG et al.** *J Immunother.,* 2012, vol. 35 (9), 689-701
- **HÜTTEN, A. et al.** *J. Biotech.,* 2004, vol. 112, 47-63
- **ILIADES, P. et al.** *FEBS Lett,* 1997, vol. 409, 437-441
- **STONE, E. et al.** *Journal of Immunological Methods,* 2007, vol. 318, 88-94
- **HOLT, L.J. et al.** *Trends Biotechnol.,* 2003, vol. 21 (11), 484-490
- **ILL. et al.** *Protein Eng,* 1997, vol. 10, 949-57
- **MARTIN et al.** *EMBO J,* 1994, vol. 13, 5303-5309
- **HOLLIGER et al.** *PNAS USA,* 1993, vol. 90, 6444-6448
- **TRAUNECKER et al.** *EMBO J,* 1991, vol. 10, 3655-3659
- **TRAUNECKER et al.** *Int J Cancer,* 1992, (7), 51-52

- **BES, C et al.** *J Biol Chem,* 2003, vol. 278, 14265-14273
- **STEMBERGER et al.** *pLoS One.,* 2012, vol. 7 (4), e35798
- **SMITH et al.** *Clin Transl Immunology,* January 2015, vol. 4 (1), e31
- **KLEBANOFF et al.** *J Immunother.,* 2012, vol. 35 (9), 651-660
- **TERAKURA et al.** *Blood.,* 2012, vol. 1, 72-82
- **WADHWA et al.** *Journal of Immunological Methods,* 2013, vol. 379 (1-2), 1-7
- **GEARING ; THORPE.** *Journal of Immunological Methods,* 1988, vol. 114 (1-2), 3-9
- **SOMAN et al.** *Journal of Immunological Methods,* 2009, vol. 348 (1-2), 83-94
- **ARAKAWA et al.** *J. Biochem.,* 1996, vol. 120, 657-662
- **VANHOVE et al.** *BLOOD,* 15 July 2003, vol. 102 (2), 564-570
- **PAULSEN et al.** *Cell Death & Differentiation,* 2011, vol. 18 (4), 619-631
- **TARABAN et al.** *Eur J Immunol.,* December 2002, vol. 32 (12), 3617-27
- **MITTLER et al.** *Immunol Res.,* 2004, vol. 29 (1-3), 197-208
- **BLAIR et al.** *JEM,* vol. 191 (4), 651-660
- **DENG et al.** *Hybrid Hybridomics.,* June 2004, vol. 23 (3), 176-82
- **MELERO et al.** *Clin Cancer Res.,* 01 March 2013, vol. 19 (5), 1044-53
- **PARK et al.** *Cancer Immunol Immunother.,* February 2012, vol. 61 (2), 203-14
- **ARGARANA et al.** *Nucleic Acids Res.,* 1986, vol. 14, 1871-1882
- **HOWARTH et al.** *Nat. Methods,* 2006, vol. 3, 267-73
- **ZHANG et al.** *Biochem. Biophys. Res. Commun.,* 2015, vol. 463, 1059-63
- **FAIRHEAD et al.** *J. Mol. Biol.,* 2013, vol. 426, 199-214
- **WU et al.** *J. Biol. Chem.,* 2005, vol. 280, 23225-31

- **LIM et al.** *Biochemistry,* 2010, vol. 50, 8682-91
- **WANG et al.** *J. Immunother.,* 2012, vol. 35 (9), 689-701
- **COOPER et al.** *Blood.,* 2003, vol. 101, 1637-1644
- **VERHOEYEN et al.** *Methods Mol Biol.,* 2009, vol. 506, 97-114
- **CAVALIERI et al.** *Blood.,* 2003, vol. 102 (2), 497-505
- **KOSTE et al.** *Gene Therapy,* 03 April 2014
- **CARLENS et al.** *Exp Hematol,* 2000, vol. 28 (10), 1137-46
- **ALONSO-CAMINO et al.** *Mol Ther Nucl Acids,* 2013, vol. 2, e93
- **PARK et al.** *Trends Biotechnol.,* November 2011, vol. 29 (11), 550-557
- **MILLER ; ROSMAN.** *BioTechniques,* 1989, vol. 7, 980-990
- **MILLER, A. D.** *Human Gene Therapy,* 1990, vol. 1, 5-14
- **SCARPA et al.** *Virology,* 1991, vol. 180, 849-852
- **BURNS et al.** *Proc. Natl. Acad. Sci. USA,* 1993, vol. 90, 8033-8037
- **BORIS-LAWRIE ; TEMIN.** *Cur. Opin. Genet. Develop.,* 1993, vol. 3, 102-109
- Remington's Pharmaceutical Sciences. 1980
- Remington: The Science and Practice of Pharmacy. Lippincott Williams & Wilkins, 01 May 2005
- **SADELAIN et al.** *Cancer Discov.,* April 2013, vol. 3 (4), 388-398
- **DAVILA et al.** *PLoS ONE,* 2013, vol. 8 (4), e61338
- **TURTLE et al.** *Curr. Opin. Immunol.,* October 2012, vol. 24 (5), 633-39
- **WU et al.** *Cancer,* March 2012, vol. 18 (2), 160-75
- **KOCHENDERFER et al.** *Nature Reviews Clinical Oncology,* 2013, vol. 10, 267-276
- **BRENTJENS et al.** *Sci Transl Med.,* 2013, vol. 5 (177
- **LING, N. R. et al.** *Leucocyte typing III,* 1987, 302
- **LING, N. R. et al.** *Leucocyte typing III.,* 1987, 302
- **HUDECEK et al.** *Clin. Cancer Res.,* 2013, vol. 19, 3153
- **FEDOROV et al.** *Sci. Transl. Medicine,* December 2013, vol. 5 (215
- **DE FELIPE.** *Genetic Vaccines and Ther.,* 2004, vol. 2, 13
- **DEFELIPE et al.** *Traffic,* 2004, vol. 5, 616-626
- **JORES et al.** *Proc. Nat'l Acad. Sci. U.S.A.,* 1990, vol. 87, 9138
- **CHOTHIA et al.** *EMBO J.,* 1988, vol. 7, 3745
- **LEFRANC et al.** *Dev. Comp. Immunol.,* 2003, vol. 27, 55
- **KOTB.** *Clinical Microbiology Reviews,* 1995, vol. 8, 411-426
- **JANEWAY et al.** Immunobiology: The Immune System in Health and Disease. Current Biology Publications, 1997, 4-33
- **KABAT et al.** Sequences of Proteins of Immunological Interest. US Dept. Health and Human Services, Public Health Service National Institutes of Health, 1991
- **HOLLER et al.** *Nat Immunol,* 2003, vol. 4, 55-62
- **HOLLER et al.** *Proc Natl Acad Sci USA,* 2000, vol. 97, 5387-92
- **LI et al.** *Nat Biotechnol,* 2005, vol. 23, 349-54
- **CHERVIN et al.** *J Immunol Methods,* 2008, vol. 339, 175-84
- **SINGH ; RAGHAVA.** *Bioinformatics,* 2001, vol. 17 (12), 1236-1237
- **SCHULER et al.** *Immunoinformatics Methods in Molecular Biology,* 2007, vol. 409 (1), 75-93
- **SINGH ; RAGHAVA.** ProPred: prediction of HLA-DR binding sites. *BIOINFORMATICS,* 2001, vol. 17 (12), 1236-1237
- **SCHULER et al.** SYFPEITHI, Database for Searching and T-Cell Epitope Prediction. *Immunoinformatics Methods in Molecular Biology,* 2007, vol. 409 (1), 75-93
- **SOO HOO, W. F. et al.** *PNAS (USA),* 1992, vol. 89, 4759
- **WÜLFING, C. ; PLÜCKTHUN, A.** *J. Mol. Biol.,* 1994, vol. 242, 655
- **KURUCZ, I. et al.** *PNAS (USA),* 1993, vol. 90, 3830
- **SCHLUETER, C. J. et al.** *J. Mol. Biol.,* 1996, vol. 256, 859
- **FEDOROV et al.** *Sci. Transl. Medicine,* 2013, vol. 5 (215
- **CHALLITA et al.** *J. Virol.,* 1995, vol. 69 (2), 748-755
- **LUPTON S. D. et al.** *Mol. and Cell Biol.,* 1991, vol. 11, 6
- **RIDDELL et al.** *Human Gene Therapy,* 1992, vol. 3, 319-338
- **LIU et al.** *Nature Biotech.,* April 2016, vol. 34 (4), 430-434
- **DE FELIPE et al.** *Traffic,* 2004, vol. 5, 616-626
- **BAUM et al.** *Molecular Therapy: The Journal of the American Society of Gene Therapy.,* 2006, vol. 13, 1050-1063
- **FRECHA et al.** *Molecular Therapy,* 2010, vol. 18, 1748-1757
- **HACKETT et al.** *Molecular Therapy,* 2010, vol. 18, 674-683
- **CHICAYBAM et al.** *PLoS ONE,* 2013, vol. 8 (3), e60298

- **VAN TEDELOO et al.** *Gene Therapy,* 2000, vol. 7 (16), 1431-1437
- **MANURI et al.** *Hum Gene Ther,* 2010, vol. 21 (4), 427-437
- **SHARMA et al.** *Molec Ther Nucl Acids,* 2013, vol. 2, e74
- **HUANG et al.** *Methods Mol Biol,* 2009, vol. 506, 115-126
- Current Protocols in Molecular Biology. John Wiley & Sons
- **JOHNSTON.** *Nature,* 1990, vol. 346, 776-777
- **BRASH et al.** *Mol. Cell Biol.,* 1987, vol. 7, 2031-2034
- **CHEADLE et al.** Chimeric antigen receptors for T-cell based therapy. *Methods Mol Biol.,* 2012, vol. 907, 645-66
- **BARRETT et al.** *Chimeric Antigen Receptor Therapy for Cancer Annual Review of Medicine,* 2014, vol. 65, 333-347
- Metastasis Research Protocols. Methods in Molecular Medicine. Humana Press Inc, vol. 2,58, 17-25
- **CHO et al.** *Lab Chip,* 2010, vol. 10, 1567-1573
- **GODIN et al.** *J Biophoton.,* 2008, vol. 1 (5), 355-376
- **ROSENBERG.** *Nat Rev Clin Oncol.,* 2011, vol. 8 (10), 577-85
- **THEMELI et al.** *Nat Biotechnol.,* 2013, vol. 31 (10), 928-933
- **TSUKAHARA et al.** *Biochem Biophys Res Commun,* 2013, vol. 438 (1), 84-9
- **KOCHENDERFER et al.** *J. Immunotherapy,* 2009, vol. 32 (7), 689-702
- **HERMAN et al.** *J. Immunological Methods,* 2004, vol. 285 (1), 25-40
- **WADWA et al.** *J. Drug Targeting,* 1995, vol. 3, 1-1 1
- Computational Molecular Biology. Oxford University Press, 1988
- Biocomputing: Informatics and Genome Projects. Academic Press, 1993
- Computer Analysis of Sequence Data. Humana Press, 1994
- **VON HEINJE, G.** Sequence Analysis in Molecular Biology. Academic Press, 1987
- Sequence Analysis Primer. M Stockton Press, 1991
- **CARRILLO et al.** *SIAM J Applied Math,* 1988, vol. 48, 1073
- **VOSS ; SKERRA.** *Protein Eng.,* 1997, vol. 1, 975-982
- **ARGARANA et al.** *Nucleic Acids Research,* 1986, 1871-1882